# EUROPEAN PATENT SPECIFICATION

(11) **EP 2 121 971 B1**
(45) Date of publication and mention of the grant of the patent: **30.03.2011**
(21) Application number: 07849642.9
(22) Date of filing: 27.12.2007
(51) Int. Cl.: C12Q 1/68

(54) **METHODS AND KITS FOR DIAGNOSIS OF MULTIPLE SCLEROSIS IN PROBABLE MULTIPLE SCLEROSIS SUBJECTS**
VERFAHREN UND KITS ZUR DIAGNOSE VON MULTIPLER SKLEROSE BEI WAHRSCHEINLICHEN MULTIPLE-SKLEROSE-PATIENTEN
MÉTHODES ET TROUSSES POUR LE DIAGNOSTIC DE LA SCLÉROSE EN PLAQUES CHEZ DES PATIENTS PRÉSENTANT UNE SCLÉROSE EN PLAQUES PROBABLE

(30) Priority: 29.12.2006 US 877682 P
(43) Date of publication of application: 25.11.2009
(73) Proprietor: Tel HaShomer Medical Research Infrastructure and Services Ltd., 52 621 Ramat Gan (IL)
(72) Inventor: ACHIRON, Anat, 69016 Tel-Aviv (IL); GUREVICH, Michael, 76236 Rechovot (IL)
(74) Representative: Fichter, Robert Arno
(86) International application number: PCT/IL2007/001616
(87) International publication number: WO 2008/081434

(56) References cited:
- WO-A-01/59063
- WO-A-01/87903
- WO-A-03/081201
- WO-A-03/102227
- CLANET M AND HOHLFELD R: "Int MS J. 2006 Jan ;13 (1):13-5 16420780 (P,S,E,B) MS treatment optimization: report from the 21st ETRIMS/10th ACTRIMS Congress, 28 September-1 October 2005, Thessaloniki, Greece." THE INTERNATIONAL MS JOURNAL, vol. 13, January 2006 (2006-01), pages 13-15, XP002482406
- COMI G ET AL: "Effect of early interferon treatment on conversion to definite multiple sclerosis: a randomised study" LANCET THE, LANCET LIMITED. LONDON, GB, vol. 357, no. 9268, 19 May 2001 (2001-05-19), pages 1576-1582, XP004249358 ISSN: 0140-6736
- KAPPOS L ET AL: "Treatment with interferon beta-1b delays conversion to clinically definite and McDonald MS in patients with clinically isolated syndromes" NEUROLOGY, vol. 67, no. 7, October 2006 (2006-10), pages 1242-1249, XP002482407 ISSN: 0028-3878
- POSER C M ET AL: "New diagnostic criteria for multiple sclerosis: guidelines for research protocols." ANNALS OF NEUROLOGY MAR 1983, vol. 13, no. 3, March 1983 (1983-03), pages 227-231, XP002482408 ISSN: 0364-5134
- POSER C M ET AL: "Diagnostic criteria for multiple sclerosis" CLINICAL NEUROLOGY AND NEUROSURGERY 2001 NL, vol. 103, no. 1, 2001, pages 1-11, XP002482409 ISSN: 0303-8467
- LINDBERG RAIJA L P ET AL: "Transcriptional profiling of multiple sclerosis: towards improved diagnosis and treatment" EXPERT REVIEW OF MOLECULAR DIAGNOSTICS, FUTURE DRUGS, LONDON, GB, vol. 6, no. 6, 1 November 2006 (2006-11-01), pages 843-855, XP009100096 ISSN: 1473-7159
- BOMPREZZI R ET AL: "Gene expression profile in multiple sclerosis patients and healthy controls: identifying pathways relevant to disease" HUMAN MOLECULAR GENETICS, OXFORD UNIVERSITY PRESS, SURREY, vol. 12, no. 17, 1 January 2003 (2003-01-01), pages 2191-2199, XP002349005 ISSN: 0964-6906

### SEQUENCE LISTING

<110> ACHIRON, Anat
   GUREVICH, Michael
<120> METHODS AND KITS FOR DIAGNOSIS OF MULTIPLE SCLEROSIS IN PROBABLE MULTIPLE SCLEROSIS SUBECTS
<130> 42664
<160> 58
<170> Patent In version 3.3
<210> 1
   <211> 1578
   <212> DNA
   <213> Homo sapiens
<400> 1
   gcccgctgcg gtaaatgggg cagaggccgg gaggggtggg ggttccccgc gccgcagcca 60
   tggagcagct tcgcgccgcc gcccgtctgc agattgttct gggccacctc ggccgcccct 120
   cggccggggc tgtcgtagct catcccactt cagggactat ttcctctgcc agtttccatc 180
   ctcaacaatt ccagtatact ctggataata atgttctaac cctggaacag agaaaatttt 240
   atgaagaaaa tgggtttcta gtaatcaaaa atcttgtacc tgatgccgat attcaacgct 300
   ttcggaatga gtttgaaaaa atctgcagaa aggaggtgaa accattagga ttaacagtaa 360
   tgagagatgt gaccatttcg aaatccgaat atgctccaag tgagaagatg atcacgaagg 420
   tccaggattt ccaggaagat aaggagctct tcagatactg cactctcccc gagattctga 480
   aatatgtgga gtgcttcact ggacctaata ttatggccat gcacacaatg ttgataaaca     540
   aacctccaga ttctggcaag aagacgtccc gtcaccccct gcaccaggac ctgcactatt     600
   tccccttcag gcccagcgat ctcatcgttt gcgcctggac ggcgatggag cacatcagcc 660
   ggaacaacgg ctgtctggtt gtgctcccag gcacacacaa gggctccctg aagccccacg 720
   attaccccaa gtgggagggg ggagttaaca aaatgttcca cgggatccag gactacgagg 780
   aaaacaaggc ccgggtgcac ctggtgatgg agaagggcga cactgttttc ttccatcctt 840
   tgctcatcca cggatctggt cagaataaaa cccagggatt ccggaaggca atttcctgcc 900
   atttcgccag tgccgattgc cactacattg acgtgaaggg caccagtcaa gaaaacatcg 960
   agaaggaagt tgtaggaata gcacataaat tctttggagc tgaaaatagc gtgaacttga 1020
   aggatatttg gatgtttcga gctcgacttg tgaaaggaga aagaaccaat ctttgaaata 1080
   gccatctgct ataactcttt caacagaaaa ccaaaaccaa acgaaatgtc taaggaaaat 1140
   gttttcttaa tgagatgatg taaccttttc tatcacttgt taaaagcaga aaacatgtat 1200
   caggtactta attgcataga gttagttttg cagcacaatg gtgttgcttt aatggaaaaa 1260
   aaaaacagta aaagtgaaat attactgttt taaggaaaac taatttaggg tggcagccaa 1320
   taaaggtggt tggtgtctaa tttaagtgtt aaatcaattt ctttcattca gttagctctt 1380
   tacccaagaa gaagtgaatg atttggagct tagggtatgt tttgtatccc ctttctgata 1440
   aacccattcc ctaccaattt tatgtcataa gagatttttt tcccccaaat ctagaacaat 1500
   gtataataca tttcacatct agtcaagggc ataggaacgg tgtcatggag tccaaataaa 1560
   gtggatattc ctgctcgg 1578
<210> 2
   <211> 1995
   <212> DNA
   <213> Homo sapiens
<400> 2
   aactcctggg ctcaagtgat cctcctgcct tagcctccta aagggctgag attacaggtg 60
   tgagccacca cgcctggcca agaagagagt tttaaatgtt aataaattgg cctgtgataa 120
   cactgaatta tattgggttt caagaaatca caaaaaaagc aacacctccc tgctgtcagc 180
   agctcataga atggtgggct agacataaac gtatacaagg aaattgccac attgtaagaa 240
   aatgtcctat tagtggcaca tactcatggc catggaagag caaagaagag gagatagaaa    300
   gagatagggt gaagggggga ggacaggaaa cccagtcaac ttttagttca tgtcttttaa    360
   aataaggaag agactctaat cagaagttga caagtgtcac cttccaatat tgtgactttc 420
   tcaatatcac agaggccagg gttttaagaa aaatcactct gcgaagatga cactgacttt 480
   ctcttaatct cccctacctc atcttcccaa aaagattaac tgaactaata gacttcaaac 540
   ttctggccta acttccagtt tacaggaaat catgaatgag agaaactgaa cagcctcaac 600
   aggaaacaat cagacacatc ctccatgtgg gatgtcctcc aaggcaagtg gcctggtctc 660
   tttctaaagt caatgtcaaa aaaaaaaaaa aaaaatccca ggcacagtgg ccttacactt 720
   gtaatcccag cactttgtga agccaaggcc attggatcac ctgaggtcag gagttcgagg 780
   ccagcctaac caatgtggta aaaccctgtc tctactaaaa atacaaaaat tagctgggcg 840
   tgttggtggg cacaagtaat cccacctact cggaaggctg agacaggaga atcacttgaa 900
   cccaggaggc ggaggttaca gtgagccaat atagcaccat tgccctccag cctgggtgac 960
   aagagcaaaa cgccatctca aaaaaaaaaa aaaaaaaaaa aagtgagaga actgttctag 1020
   atgctagatt aaacataaaa gagtttagga ctacaatatg aattgcaatg catgagcacc 1080
   tgtgtatctg aatttaggaa gaaaaaatcg ccataaaaga caatttggag acaactagat 1140
   agatttaaat atggactgga taccagatat aatgagaaaa ttatcgttca tttttcatat 1200
   tttgaaatgt tagtgtcttt atgtacgaga atgcccttac tattaggaaa cagatgctca 1260
   agaacttagg ccaggcacag tggctcatgc ctataatctg aatacttggg aggctgaggc 1320
   aggaggatca cttgagtctg agaccagcct gggcaacatg gtaagatttc tctctataga 1380
   aataaaatta aaattagcca ggtgtggtgg catgtgccta tagttccagc tacttgggag 1440
   gctgaggtgg gaggattagt tgagcccaga agttcgaggc tgcagtgagc tatgattgca 1500
   ccactgccct ccagcctggg tgacatagtg agaccctgcc ttaaaatata tatagagaga 1560
   gagggtaagc atcatgacat ctccttaccc tgattaatct caaactgttc agcaaaaaac 1620
   atatatatat atatacacac atccgcatac cttcgcatac acatatacac acacaaatag 1680
   agaaagcaaa tgcaggctgg gcacggttgc tcacacctat aatcccaaca ctttgggagg 1740
   ccaaggcagg cagatcacct gagatcagga gttcgagacc agcctggcca acatggcaaa 1800
   accccgtctc tactaaatat acaaaaaagt tagctggatg tagtggcggg cgcctgtaat 1860
   cccagctact cggggggcta aggcaggaga atcacttgaa cccgagaggc gggggttgca 1920
   gtgaacggag atggcgccac tgcattccag cctgggtgac agagcgagat gattgtctca 1980
   aaaaaaaaaa aaaaa 1995
<210> 3
   <211> 1403
   <212> DNA
   <213> Homo sapiens
<400> 3
   tttttttttt ttttcttttt ttttcctttt ttcagtgttg tatgtgtagc aggcacttga     60
   gtttatatga agatgttcgc ttcagtcaag gatggaagga gttgtgaggt ggaagggaga    120
   tgtgaggcag ggagagagga ggcaacagag tatgaattca tgaccaccag ccaccactag    180
   ccaagaatgt ccaggtagat tggggtggcc ttccccaaag catggaggat tttgtagatc    240
   tccttgatgt tcaaccgctg ctgtggttcc ctctgccagc accccagcat gacatcgtac    300
   acctctttgg ggcagactcg gggccgctcc aaaacacgac cttgggtaat gcactcaatg    360
   acctccgtgt ttgagagttg gaaccatggc tgctttccat aggtgaagat ctcccagagg    420
   atcaccccga agctccatac atcactctct gtagtgaact tccggtacat gatgctttca    480
   ggaggcatcc agcgaatggg gagcatggtg tgtcctccca ccctgtaata atccgtgctg    540
   tagacatctc tggacatgcc gaagtcccca atcttcacta gcagattcgc tccaaccagg    600
   cagttcctgg tggccaggtc tcggtgcaca aagtgctggg aggccaggta caccataccc    660
   gaggcgatct gactggcaat gtggagcatt tgggagagcc ccagctcacc cttggcctgg    720
   cgtggctgtc catccacaag gatcattgca tctggcccat gggccctgag gaacttattc    780
   aggtctccat gcttcatgta ttcaaagacc atgatgaggg ggtccccatc gccgcacact    840
   ccatagaact tgacaatgtg ctcatgctgc aggttggtga gcagctcggc ctccctctgg    900
   aaatccttcc gggcagccag ggtgggatcc ttcagggcct tcacagccac aagcatcttg    960
   tccttggtcg ggctgaggtt gtagcactcg gccaggaaga cctttccaaa ggctccctca   1020
   cccagttctc gcttcagcac gatgtctctc ctcttaatgt gctgcacatc tgctattctc   1080
   ccaatgggca tggcgtgctc ttcaggcggg gagacagaga ccatgatgtg gttcatgtaa   1140
   gccaggtctt cccgatgaga gaggttgatg ggcttccctt ccctatgcag cccgtcctcg   1200
   gagagcctgg actgtttgaa atccacggag tgccgggggt tcaggatcag agggtgcatg   1260
   atggggctgg gcatcagctg gatcacgcgt gtgctctcct gccgggggct ggatggcttc   1320
   gggtgggact cggaggacgc tgggcagtga ttattctcca tgggagacac tgacagaggg   1380
   taggactcct ggtggttgtt ctc                                            1403
<210> 4
   <211> 423
   <212> DNA
   <213> Homo sapiens
<220>
   <221> misc_feature
   <222> (97)..(97)
   <223> n is a, c, g, or t
<220>
   <221> misc_feature
   <222> (111)..(111) g, or t
   <223> n is a, c, g, or t
<400> 4
   ttttttttaa gataaattca tgcactttta ctatgcccat tgcacttctc atccatggat 60
   ttgccttgcc ttaagaatta accatggcct tgtggcntgg gtgacccagg ntgcttttat 120
   cttgcacagc taaagagggt ctgatgggtg gctcaacacc ccacccactc ctataccatg 180
   tcatcagtgt tcacattttc caaatgagtt gaagtgcctt gatctgtcca ctgccaccac 240
   caccagtgct gagttttccc atgtggtttt gcttttgtgg tgttactgcc ttgctgctag 300
   agcagcagga ctgtctgcgt agcgcctcca gcctgggacc tcactcagtt cccagggtat 360
   tcaaagacgg cagcggctcc cattccagtc ccgatgcaca tggacaccac tccgtatgcc 420
   ctc 423
<210> 5
   <211> 1207
   <212> DNA
   <213> Homo sapiens
<400> 5
   ccagaaatgg attcgaaata tcagtgtgtg aagctgaatg atggtcactt catgcctgtc 60
   ctgggatttg gcacctatgc gcctgcagag gttcctaaaa gtaaagcttt agaggccacc 120
   aaattggcaa ttgaagctgg cttccgccat attgattctg ctcatttata caataatgag 180
   gagcaggttg gactggccat ccgaagcaag attgcagatg gcagtgtgaa gagagaagac 240
   atattctaca cttcaaagct ttggtgcaat tcccatcgac cagagttggt ccgaccagcc 300
   ttggaaaggt cactgaaaaa tcttcaattg gattatgttg acctctacct tattcatttt 360
   ccagtgtctg taaagccagg tgaggaagtg atcccaaaag atgaaaatgg aaaaatacta 420
   tttgacacag tggatctctg tgccacgtgg gaggccgtgg agaagtgtaa agatgcagga 480
   ttggccaagt ccatcggggt gtccaacttc aaccgcaggc agctggagat gatcctcaac 540
   aagccagggc tcaagtacaa gcctgtctgc aaccaggtgg aatgtcatcc ttacttcaac 600
   cagagaaaac tgctggattt ctgcaagtca aaagacattg ttctggttgc ctatagtgct 660
   ctgggatccc accgagaaga accatgggtg gacccgaact ccccggtgct cttggaggac 720
   ccagtccttt gtgccttggc aaaaaagcac aagcgaaccc cagccctgat tgccctgcgc 780
   taccagctac agcgtggggt tgtggtcctg gccaagagct acaatgagca gcgcatcaga 840
   cagaacgtgc aggtgtttga attccagttg acttcagagg agatgaaagc catagatggc 900
   ctaaacagaa atgtgcgata tttgaccctt gatatttttg ctggcccccc taattatcca 960
   ttttctgatg aatattaaca tggagggcat tgcatgaggt ctgccagaag gccctgcgtg 1020
   tggatggtga cacagaggat ggctctatgc tggtgactgg acacatcgcc tctggttaaa 1080
   tctctcctgc ttggtgattt cagcaagcta cagcaaagcc cattggccag aaaggaaaga 1140
   caataatttt gttttttcat tttgaaaaaa ttaaatgctc tctcctaaag attcttcacc 1200
   taaaaaa 1207
<210> 6
   <211> 1883
   <212> DNA
   <213> Homo sapiens
<400> 6
   gtgagccacc gcccccagcc tggcctggca tttctttgag ttcaggaagt gtgacaagga     60
   tttggacacc cagaaataag cgtgtcgaga agagcacaag cagaggatcc agcgctcggc    120
   atggcggagc cagatctgga gtgcgagcag atccgtctga agtgtattcg taaggagggc    180
   ttcttcacgg tgcctccgga acacaggctg ggacgatgcc ggagtgtgaa ggagtttgag    240
   aagctgaacc gcattggaga gggtacctac ggcattgtgt atcgggcccg ggacacccag    300
   acagatgaga ttgtcgcact gaagaaggtg cggatggaca aggagaagga tggcatcccc    360
   atcagcagct tgcgggagat cacgctgctg ctccgcctgc gtcatccgaa catcgtggag    420
   ctgaaggagg tggttgtggg gaaccacctg gagagcatct tcctggtgat gggttactgt    480
   gagcaggacc tggccagcct cctggagaat atgccaacac ccttctcgga ggctcaggtc    540
   aagtgcatcg tgctgcaggt gctccggggc ctccagtatc tgcacaggaa cttcattatc    600
   cacagggacc tgaaggtttc caacttgctc atgaccgaca agggttgtgt gaagacagcg    660
   gatttcggcc tggcccgggc ctatggtgtc ccagtaaagc caatgacccc caaggtggtc    720
   actctctggt accgagcccc tgaactgctg ttgggaacca ccacgcagac caccagcatc    780
   gacatgtggg ctgtgggctg catactggcc gagctgctgg cgcacaggcc tcttctcccc    840
   ggcacttccg agatccacca gatcgacttg atcgtgcagc tgctgggcac gcccagtgag    900
   aacatctggc cgggcttttc caagctgcca ctggtcggcc agtacagcct ccggaagcag    960
   ccctacaaca acctgaagca caagttccca tggctgtcgg aggccgggct gcgcctgctg   1020
   cacttcctgt tcatgtacga ccctaagaaa agggcgacgg ccggggactg cctggagagc   1080
   tcctatttca aggagaagcc cctaccctgt gagccggagc tcatgccgac ctttccccac   1140
   caccgcaaca agcgggccgc cccagccacc tccgagggcc agagcaagcg ctgtaaaccc   1200
   tgacggtggg cctggcacac gcctgtattc ccacaccagg tcttccgatc agtggtgtct   1260
   gtgaagggtg ccgcgagcca ggctgaccag gcgcccggga tccagctcat ccccttggct   1320
   gggaacatcc tccactgact tcctcccact gtctgccctg aacccactgc tgcccccaga   1380
   aaaaggccgg gtgacaccgg gggctcccag cccgtgcacc ctggaagggc aggtctggcg   1440
   gctccatccg tggctgcagg ggtctcatgt ggtcctcctc gctatgttgg aaatgtgcaa   1500
   ccactgcttc ttgggaggag tggtgggtgc agtccccccg ctgtctttga gttgtggtgg   1560
   accgctggcc tgggatgaga gggcccagaa gaccttcgta tcccctctca gtcgcccggg   1620
   gctgtcccgt gcatgggttg gctgtgggga ccccaggtgg gcctggcagg actccagatg   1680
   aggacaagag ggacaaggta tggggtggga gccacaattg aggatacccc gagctaccag   1740
   gagagccctg ggctggaggc tgagctggat ccctgctccc cacacggagg acccaacagg   1800
   aggccgtggc tctgatgctg agcgaagcta taggctcttg ttggataaaa gcttttttaa   1860
   cagaaaaaaa aaaaaaaaaa aaa                                          1883
<210> 7
   <211> 1774
   <212> DNA
   <213> Homo sapiens
<400> 7
   tgttcctgcc tctccgccac ctccaccgcg gcttatcccc tggctggcgg cgttggcggg     60
   gcgggggaca gtagttgtag acgcccgccc ctgcctcaga gaagatacca cattgaatga    120
   acaaagactg aactttgttt acagatacct tttataaaag atgtctgtgg agtagtgtta    180
   cagttcaaat aacaaaggat tttgcattat gagatgagtg agagtggaga attggacaaa    240
   aattgatttt tgatctctga attgtggttt ttagctcacc agttattgtg tagtctcact    300
   atttgaaaat tcatgctgaa aaactgagtg aatatgatca gatttctcca ccaaattctc    360
   agcctgataa ggaaaatccc gttttgtcaa cctaatgaag cattcaaaga agacatatga    420
   ctcttttcaa gatgaacttg aagattatat taaagtacag aaagccagag gcttagagcc    480
   aaagacttgt ttcagaaaga tgaaagggga ctatttggaa acctgtgggt acaaaggaga    540
   ggttaattcc agacccacgt atagaatgtt tgaccagaga ctcccatctg aaaccatcca    600
   gacctaccca agatcatgca atattccaca aacagtggaa aatcggttgc ctcagtggtt    660
   accagcccat gacagcagat tgagactaga ctctctgagc tactgtcagt tcacgaggga     720
   ctgtttctca gaaaaaccag tacccctgaa ctttaatcaa caagaatata tttgtggctc     780
   acatggtgta gaacatagag tttacaagca cttctcctca gataacagta ccagtactca    840
   tcaagccagt cacaaacaga tacatcagaa gaggaaaagg cacccagagg aaggcagaga    900
   aaaatcagag gaggagcggt ctaagcataa gagaaaaaaa agctgcgagg aaattgactt    960
   agacaaacac aagagcatcc aaagaaagaa aacagaggtg gaaatagaaa ccgtacatgt   1020
   cagtacagaa aagcttaaga atcgaaagga gaaaaaaagc cgagatgtag tctctaagaa   1080
   agaggaacgt aagcgtacaa aaaagaaaaa ggaacaaggc caagaaagga cagaggagga   1140
   aatgctttgg gaccagtcta ttcttggatt ttgaagcttt caaagttggt tctcccaaag   1200
   ttaaattgaa aaaataggtg agagcttggt tttatgatat ccgtgttcat accacttttc   1260
   ttatgtgaat aggttcttta acttctaaca aaggcctagt aaacaaagtg tttagcatgc   1320
   ttgctctcca acacagaaat tgcttttcct cattttctaa aagcattatt acattttttg  1380
   aacatatagt gtaatttcct ttaatgaaag tgactctgct tttattcatc aaattgcttt   1440
   gatggtggaa atattttctg ttgggaggtt atttatttta aattggagga ttaatgacct   1500
   ttgcacaatc tgtttcttga ttgggtttgt tatagttttg agttgggtat tttatgttca   1560
   ttggtttttc tctgtgaagc aatttttttc tcctttatta gatctaactt gcagtgtatt   1620
   ttctaggctg gaaagtggaa aatgaaatat attatgatct taggttacat aaagtttcta   1680
   aagtttcaaa gagtcttgat acaaaatcag tttatattct gaaaatattt ataataaagt   1740
   attctaattt ctaaaaaaaa aaaaaaaaaa aaaa                                1774
<210> 8
   <211> 1597
   <212> DNA
   <213> Homo sapiens
<400> 8
   ggctgagcac tcaggcaatc acactctcag aaactgcggc ggctctggac tgcagcctcc      60
   aaggctccat gccagacaaa gctatgcgta gtcacacttg ctacaatagc ctggatggtt     120
   tcttttgtct ccaattattc acacacagca aatattttgc cagatatcga aaatgaagat     180
   ttcatcaaag actgcgttcg aatccataac aagttccgat cagaggtgaa accaacagcc     240
   agtgatatgc tatacatgac ttgggaccca gcactagccc aaattgcaaa agcatgggcc     300
   agcaattgcc agttttcaca taatacacgg ctgaagccac cccacaagct gcacccaaac     360
   ttcacttcac tgggagagaa catctggact gggtctgtgc ccattttttc tgtgtcttcc      420
   gccatcacaa actggtatga cgaaatccag gactataact tcaagactcg gatatgcaaa     480
   aaagtctgtg gccactacac tcaggttgtt tgggcagata gttacaaagt tggctgcgca     540
   gttcaatttt gccctaaagt ttctggcttt gacgctcttt ccaatggagc acattttata     600
   tgcaactacg gaccaggagg gaattaccca acttggccat ataagagagg agccacctgc     660
   agtgcctgcc ccaataatga caagtgtttg gacaatctct gtgttaacga cagcgagacc     720
   aagtcaaacg ttactactat gttgtatatc aggctggccc atatatccac gtaacagata     780
   cacttctctc tttctcattg ttaattcagt aattctaata ctgtctgtta taattaccat     840
   tttggtacag cacaagtacc ctaatttagt tcttttggac taatacaatt caggaaagaa     900
   aaaacccaaa aaccaacctc attcacatat ggcttttttt ttaacccaat aacaattagg     960
   tgtaccttcc tatttaaaca tttcataaaa aaatatatgt tatagcaata ctcttactca    1020
   aaagaagaaa tttcctaact ctatcagata aactcatctt tagtataaat aagcattatt    1080
   tgcaggttgc ctacaggtgg acttttagta agtaacctaa cccatgtttc agcttctaaa    1140
   tctcgaaaat gagcaaggta caggtacagt agcacatttt taggtgattc ttagtaactc    1200
   cagtagcctt cattagttaa aaacattatt attttttgca tgctgcttcg actctaaata    1260
   tcttgttttc cctgtctttt tggtttacta cttccccaga ttcagaacag aggagtaact    1320
   aggggatctg attttagagg cctaattttc tgttcatgga ctgttaaaag taaaaccaaa    1380
   ctttcaaaag ggataaacct aaatatttac ttgttatcat tagagaggga acatcaaatg    1440
   ctggcactat atacatacga tcagcctgat tatgatatca caatggtcgt aatgtataca    1500
   aagacttata taccactttc tcgtataaat ttttcaaaaa atacaataat aatataattt    1560
   ataaagaaca ctcttctatg aacaaccacc accacca                             1597
<210> 9
   <211> 734
   <212> DNA
   <213> Homo sapiens
<400> 9
   ccaaccaggg ctgcattgga ggttgaaatc acaaagatta gacacctttt tagataggtg      60
   ttcttcagca ccactgacaa cacggttctg acagtatttc atgacaatgg acggcgacag     120
   ctctacaaca gatgcttctc aactaggaat ctctgcagac tatattggag gaagtcatta     180
   tgttatacag cctcatgatg atactgagga cagcatgaat gatcatgaag acacaaatgg     240
   ttcaaaagaa agtttcagag aacaagatat atatcttcca atagcaaacg tggctaggat     300
   aatgaaaaat gccatacctc aaacgggaaa gattgcaaaa gatgccaaag aatgtgttca     360
   agaatgtgta agtgagttca tcagttttat aacatctgaa gcaagtgaaa ggtgccatca     420
   agagaaacgg aaaacaatca atggagaaga tattctcttt gctatgtcta ctttaggctt     480
   tgacagttat gtggaacctc tgaaattata ccttcagaaa ttcagagagg ctatgaaagg     540
   agaaaaggga attggtggag cagtcacagc tacagatgga ctaagtgaag agcttacaga     600
   ggaggcattt actaaccagt taccagctgg cttaataacc acagacggtc aacaacaaaa     660
   tgttatggtt tacacaacat catatcaaca gatttctggt gtccagcaaa ttcagttttc     720
   atgatctgaa gaaa                                                       734
<210> 10
   <211> 4423
   <212> DNA
   <213> Homo sapiens
<220>
   <221> misc_feature
   <222> (316)..(316)
   <223> n is a, c, g, or t
<220>
   <221> misc_feature
   <222> (1528)..(1528)
   <223> n is a, c, g, or t
<220>
   <221> misc_feature
   <222> (1537)..(1538)
   <223> n is a, c, g, or t
<220>
   <221> misc_feature
   <222> (1618)..(1618)
   <223> n is a, c, g, or t
<220>
   <221> mist_features
   <222> (1625)..(1625)
   <223> n is a, c, g, or t
<220>
   <221> misc_feature
   <222> (1720)..(1720)
   <223> n is a, c, g, or t
<220>
   <221> misc_feature
   <222> (1725)..(1727)
   <223> n is a, c, g, or t
<220>
   <221> misc_feature
   <222> (1747)..(1747)
   <223> n is a, c, g, or t
<220>
   <221> misc_feature
   <222> (2822)..(2825)
   <223> n is a, c, g, or t
<220>
   <221> misc_feature
   <222> (2829)..(2831)
   <223> n is a, c, g, or t
<220>
   <221> misc_feature
   <222> (2834)..(2839)
   <223> n is a, c, g, or t
<220>
   <221> misc_feature
   <222> (3066)..(3066)
   <223> n is a, c, g, or t
<220>
   <221> misc_feature
   <222> (3092)..(3092)
   <223> n is a, c, g, or t
<220>
   <221> misc_feature
   <222> (3099)..(3100)
   <223> n is a, c, g, or t
<220>
   <221> misc_feature
   <222> (3456)..(3456)
   <223> n is a, c, g, or t
<220>
   <221> misc_feature
   <222> (4110)..(4110)
   <223> n is a, c, g, or t
<220>
   <221> misc_feature
   <222> (4408)..(4416)
   <223> n is a, c, g, or t
<220>
   <221> misc_feature
   <222> (4418)..(4420)
   <223> n is a, c, g, or t
<400> 10
   gggggtttcc gcttccggca gcagcggctg cagcctcgct ctggtccctg cggctggcgg 60
   ccgagccgtg tgtctcctcc tccatcgccg ccatattgtc tgtgtgagca gaggggagag 120
   cggccgccgc cgctgccgct tccaccacag ctctatcaag gcttgtcaag cagtgtgctc 180
   atcacatggt aaatcatgca gcgtggaacc tcataaaatc tccaagaaac atcattcacc 240
   catactgact agtttcacat ctctttgttt gaagaaaaca ggtctgaaac aaggtcttac 300
   ccccagctgc ttctgnaaca cagtgactgc cagatctcca aacatcaagt ccagctttgt 360
   ccgccaacct gtctgacatg tcgggacccg tgccaagcag ggccagagtt tacacagatg 420
   ttaatacaca cagacctcga gaatactggg attacgagtc acatgtggtg gaatggggaa 480
   atcaagatga ctaccagctg gttcgaaaat taggccgagg taaatacagt gaagtatttg 540
   aagccatcaa catcacaaat aatgaaaaag ttgttgttaa aattctcaag ccagtaaaaa 600
   agaagaaaat taagcgtgaa ataaagattt tggagaattt gagaggaggt cccaacatca 660
   tcacactggc agacattgta aaagaccctg tgtcacgaac cccgccttgg gtttttgaac 720
   acgtaaacaa cacagacttc aagcaattgt accagacgtt aacagactat gatattcgat 780
   tttacatgta tgagattctg aaggccctgg attattgtca cagcatggga attatgcaca 840
   gagatgtcaa gccccataat gtcatgattg atcatgagca cagaaagcta cgactaatag 900
   actggggttt ggctgagttt tatcatcctg gccaagaata taatgtccga gttgcttccc 960
   gatacttcaa aggtcctgag ctacttgtag actatcagat gtacgattat agtttggata 1020
   tgtggagttt gggttgtatg ctggcaagta tgatctttcg gaaggagcca tttttccatg 1080
   gacatgacaa ttatgatcag ttggtgagga tagccaaggt tctggggaca gaagatttat 1140
   atgactatat tgacaaatac aacattgaat tagatccacg tttcaatgat atcttgggca 1200
   gacactctcg aaagcgatgg gaacgctttg tccacagtga aaatcagcac cttgtcagcc 1260
   ctgaggcctt ggatttcctg gacaaactgc tgcgatatga ccaccagtca cggcttactg 1320
   caagagaggc aatggagcac ccctatttct acactgttgt gaaggaccag gctcgaatgg 1380
   gttcatctag catgccaggg ggcagtacgc ccgtcagcag cgccaatatg atgtcaggga 1440
   tttcttcagt gccaacccct tcaccccttg gacctctggc aggctcacca gtgattgctg 1500
   ctgccaaccc ccttgggatg cctgttcnca gctgccnntg gcgctcagca gtaacggccc 1560
   tatctgtctc ctgatgcctg agcagaggtg ggggagtcca ccctctcctt gatgcagntt 1620
   gcgcntggcg gggaggggtg aaacacttca gaagcaccgt gtctgaaccg ttgcttgtgg 1680
   atttatagta gttcagtcat aaaaaaaaaa ttataatagn ctgannntct tttttctttt 1740
   ttttttnaac tcgaactttt cataactcag gggattccct gaaaaattac ctgcaggtgg 1800
   aatatttcat ggacaaattt ttttttctcc cctcccaaat ttagttcctc atcacaaaag 1860
   aacaaagata aaccagcctc aatcccggct gctgcattta ggtggagact tcttcccatt 1920
   cccaccattg ttcctccacc gtcccacact ttagggggtt ggtatctcgt gctcttctcc 1980
   agagattaca aaaatgtagc ttctcagggg aggcaggaag aaaggaagga aggaaagaag 2040
   gaagggagga cccaatctat aggagcagtg gactgcttgc tggtcgctta catcacttta 2100
   ctccataagc gcttcagtgg ggttatccta gtggctcttg tggaagtgtg tcttagttac 2160
   atcaagatgt tgaaaatcta cccaaaatgc agacagatac taaaaacttc tgttcagtaa 2220
   gaatcatgtc ttactgatct aaccctaaat ccaactcatt tatactttta tttttagttc 2280
   agtttaaaat gttgatacct tccctcccag gctccttacc ttggtctttt ccctgttcat 2340
   ctcccaacat gctgtgctcc atagctggta ggagagggaa ggcaaaatct ttcttagttt 2400
   tctttgtctt ggccattttg aattcattta gttactgggc ataacttact gctttttaca 2460
   aaagaaacaa acattgtctg tacaggtttc atgctagagc taatgggaga tgtggccaca 2520
   ctgacttcca ttttaagctt tctaccttct tttcctccga ccgtcccctt ccctcacatg 2580
   ccatccagtg agaagacctg ctcctcagtc ttgtaaatgt atcttgagag gtaggagcag 2640
   agccactatc tccattgaag ctgaaatggt agacctgtaa ttgtgggaaa actataaact 2700
   ctcttgttac agccccgcca ccccttgctg tgtgtatata tataatactt tgtccttcat 2760
   atgtgaaaga tccagtgttg gaattctttg gtgtaaataa acgtttggtt ttatttatca 2820
   annnnaaann naannnnnnt gtgtaaaggt cttgctgggt gggtgtctca tgttcacatc 2880
   tgaggggcct gcagccctgt accgtggagg cttcccaagg cccccatttt atacacccct 2940
   cgttcgaccc atggtaccgg gcagagcaga gaggccttaa aaaaaaagca ccacaagcca 3000
   aagcgtctct ggggattaag gatcctttgc cataaaactc atttgtgtct cagcagtgca 3060
   gggatngggg atggaaaaag tcgccagttt tngtgtgtnn gtgtgtgtgt gtaaagtgga 3120
   ctttccactg taatccaacc acctaagttt atcaggtgct tcactgagga agcctagttt 3180
   tttaagcaca atagcaaaac catcagctct gtattttctc ctgttatttc attacagtag 3240
   ctgcttgtgg gaactaggaa aaattcttcc aacatatttt aaggcctaaa atcttagttc 3300
   cccattctcc taccttatag attcacaggc ctttctcgcc taggcatcat agataaacgt 3360
   aattgtttgg ggagttgaat ttaatgaact tatctaactt tgtaacccat cttggcttta 3420
   gtaactttat caaggtggtg gctttagtga atatantggt aaactttaga ggacgctaaa 3480
   gcctcctttt atagcgcttc tcaacggtag ggagagctga agggaaaaca ttctgactgt 3540
   gtggcagggt gattttcttc cttatgacca cttacagtgg atatttattg tacttgaccc 3600
   ttttatgccc tagaatgctg tgagggttac catgttgaat ttgtgcagaa gctaaaagca 3660
   ccagatgtgc cagagatgca atttgtgatt atgtttgcac tggattgtga tttgaacagg 3720
   acacttataa ctaatgagtt ctttcttttg aggtggggag agggttgtaa atcaagactt 3780
   cataccctat ccttgtagct cggaaattga ggtgtagctt aggctgatgc ggagagctgc 3840
   agacagctgg acctccttac acccatcaga ccttggaata aaccgaaggt gaatgcagag 3900
   gactctgagg tgccacccaa gagcctttta aaataaatag aagtttttaa aaatgctact 3960
   taagagtcac tgcagaagca tggagagaga gaaaaaaaaa aaagacccaa ttgggtgggg 4020
   ggtggggggg aggcaagggt ttacagagta acaacttacg tgttgttgta aattacctca 4080
   tctgtatacc ttgtattggg acactatttn tcagcactac ctgtgtctgc attgcttaga 4140
   tggcaggaaa tggaatggcc aacatgaaag gttatgttgg caattactct gaaagatttt 4200
   ttaaagtatt tttaaacttt gtttgaagtg gtccctcccc tcttcctctg ctttttttta 4260
   agtttgaagc cagaaggccc tccccacctt gctttattgg ttgtaattgt attgtaattg 4320
   gtatgactaa accataactg tgctgggaag aagttgtgcc atgaaggtct ttaatttttt 4380
   ttaataaaaa catttaaata aatgaaannn nnnnnnannn aaa 4423
<210> 11
   <211> 2755
   <212> DNA
   <213> Homo sapiens
<400> 11
   caaataaacc cagcaaagga actagcagag ctggtgatgt ttatggcaca gattagtcac      60
   tgctacccag agtacctaag taattttcct caagaggtga aagatcttct ctcctgcaat     120
   cataccgtat tggatccaga tctgcgaatg acattttgca aagctttgat cttgctgaga     180
   aataagaatc tcatcaatcc atcaagcctg ctagaactct tctttgaact ttttcgttgc     240
   catgataaac ttctgcgaaa gactttatac acacatattg tgactgatat caagaatata     300
   aatgcaaaac acaagaacaa taaagtgaat gtagtattgc aaaatttcat gtacaccatg     360
   ttaagagata gcaatgcaac cgcagccaag atgtctttag atgtaatgat tgaactctac     420
   agaaggaaca tctggaatga tgcaaaaact gtcaatgtta tcacaactgc atgtttctct     480
   aaggtcacca agatattagt tgccgctttg acattctttc ttgggaaaga tgaagatgaa     540
   aaacaggaca gtgactccga atctgaggat gatggaccaa cagcaagaga cctgctagta     600
   caatatgcta cagggaagaa aagttccaaa aacaagaaaa agttggaaaa ggcaatgaaa     660
   gtgctcaaga aacaaaaaaa gaagaaaaaa ccagaggtgt ttaacttttc agccattctc     720
   ttgattcatg atccccaaga ttttgcggaa aaactactaa agcagcttga gtgctgtaag     780
   gagaggtttg aagtgaagat gatgctcatg aaccttatct ccagattggt gggaattcat     840
   gagcttttcc tcttcaattt ctatcccttt ttgcaaaggt ttctgcagcc ccaccaaaga     900
   gaagtaacca agatccttct gtttgctgca caagcatctc atcacctagt acccccagag     960
   attattcaat cattgcttat gactgtggca aacaattttg ttaccgacaa gaactctgga    1020
   gaagtcatga cagtaggaat caatgctata aaggagataa cagctcgatg tcctctggcc    1080
   atgactgaag aacttctcca agacctggct cagtataaaa cacacaagga taagaatgta    1140
   atgatgtctg ctagaacttt gattcacctc ttccgaacac tgaatcctca gatgctgcag    1200
   aagaaattcc ggggtaagcc tacagaggcc tccatagaag caagagtaca agaatatgga    1260
   gaattagatg ctaaagatta cattccagga gcagaagttc tggaagttga gaaagaagag 1320
   aatgctgaaa atgatgaaga tggatgggaa agtaccagtc tcagtgagga ggaggatgct 1380
   gatggtgaat ggattgatgt gcaacactct tccgatgaag aacagcaaga aatctccaag 1440
   aagctgaaca gcatgcccat ggaggagcgg aaggccaaag ctgcagccat cagcactagc 1500
   cgagttttaa ctcaggaaga cttccagaaa atccgcatgg cccaaatgag aaaagaactt 1560
   gatgctgccc ccgggaaatg ccagaagagg aaatacattg aaatagacag tgatgaagag 1620
   cccaggggtg aattactttc tcttcgggac attgaacgcc ttcataaaaa gccaaagtct 1680
   gacaaagaga caagactagc aactgcaatg gctggaaaga cagaccgaaa agaatttgtg 1740
   aggaagaaaa ccaaaacaaa tccattttcc agttcgacaa ataaagagaa gaaaaaacag 1800
   aagaacttta tgatgatgcg gtatagccag aatgtccggt caaaaaataa gcgttccttc 1860
   cgagaaaaac agttggcact acgagatgca cttttgaaaa agagaaaaag aatgaagtaa 1920
   cttcctggca agttttccat tcctagaaga atgctaagtt tgtgtccttg ctctgaaaat 1980
   tggtaaatca agcatgtttg tttacattaa aaagtccaga cacactgtat tgtgaaaact 2040
   gctgaacatg tggcagcaat tttgtgtttt tattttggag acggctaatg gtaggaatgt 2100
   taatgtaaat agtggtggta atgtaaaatc atttcattta tcattcatgc aaaaaaaagt 2160
   atgtattgag tgcctattca ttgtcactgt agatgcaaaa cgaatgagct gtaacccctt 2220
   cactcaaggc attgacagct tagctgtgag ggtggacaca catatgtgta tttacagtgc 2280
   agtgtaaata gttctgtagt agaggttaac tccatatttc tgtgaggtca ctgaggcctt 2340
   atggactaac tctgtgagga taggagttat atattcttat aagacaaaac aaaacaggac 2400
   aatgttacaa gagtaagagg ttcttacttg tacataggct ttcctgctga aaacaggccc 2460
   ctgctgtaca gattttgggt acataattta gctcttttag tcagtccaag agatttaagt 2520
   gacccccccc ccccccgtgt tttttttgtt tttgtttttg ttttgaatgc catgtaaagg 2580
   ctttttggtt aagacctcac ttttaaaact gccttaagta taaatagtac ctttggaata 2640
   tatttagttc atcatttgag ctgccttcat actggtttcc tcagccttcc ttcagcctgt 2700
   aatattttca gcccactgtt taccttgtct caataaaagg tttctaatgc caaat      2755
<210> 12
   <211> 3029
   <212> DNA
   <213> Homo sapiens
<400> 12
   gcggccgcgt cgacgaaatg tctggatttg gactaaagaa aaaaggaaag gctagcagtc     60
   atccaacaga atcatgagac agactttgcc ttgtatctac ttttgggggg gccttttgcc    120
   ctttgggatg ctgtgtgcat cctccaccac caagtgcact gttagccatg aagttgctga    180
   ctgcagccac ctgaagttga ctcaggtacc cgatgatcta cccacaaaca taacagtgtt    240
   gaaccttacc cataatcaac tcagaagatt accagccgcc aacttcacaa ggtatagcca    300
   gctaactagc ttggatgtag gatttaacac catctcaaaa ctggagccag aattgtgcca    360
   gaaacttccc atgttaaaag ttttgaacct ccagcacaat gagctatctc aactttctga    420
   taaaaccttt gccttctgca cgaatttgac tgaactccat ctcatgtcca actcaatcca    480
   gaaaattaaa aataatccct ttgtcaagca gaagaattta atcacattag atctgtctca    540
   taatggcttg tcatctacaa aattaggaac tcaggttcag ctggaaaatc tccaagagct    600
   tctattatca aacaataaaa ttcaagcgct aaaaagtgaa gaactggata tctttgccaa    660
   ttcatcttta aaaaaattag agttgtcatc gaatcaaatt aaagagtttt ctccagggtg    720
   ttttcacgca attggaagat tatttggcct ctttctgaac aatgtccagc tgggtcccag    780
   ccttacagag aagctatgtt tggaattagc aaacacaagc attcggaatc tgtctctgag    840
   taacagccag ctgtccacca ccagcaatac aactttcttg ggactaaagt ggacaaatct    900
   cactatgctc gatctttcct acaacaactt aaatgtggtt ggtaacgatt cctttgcttg    960
   gcttccacaa ctagaatatt tcttcctaga gtataataat atacagcatt tgttttctca   1020
   ctctttgcac gggcttttca atgtgaggta cctgaatttg aaacggtctt ttactaaaca   1080
   aagtatttcc cttgcctcac tccccaagat tgatgatttt tcttttcagt ggctaaaatg   1140
   tttggagcac cttaacatgg aagataatga tattccaggc ataaaaagca atatgttcac   1200
   aggattgata aacctgaaat acttaagtct atccaactcc tttacaagtt tgcgaacttt   1260
   gacaaatgaa acatttgtat cacttgctca ttctccctta cacatactca acctaaccaa   1320
   gaataaaatc tcaaaaatag agagtgatgc tttctcttgg ttgggccacc tagaagtact   1380
   tgacctgggc cttaatgaaa ttgggcaaga actcacaggc caggaatgga gaggtctaga   1440
   aaatattttc gaaatctatc tttcctacaa caagtacctg cagctgacta ggaactcctt   1500
   tgccttggtc ccaagccttc aacgactgat gctccgaagg gtggccctta aaaatgtgga   1560
   tagctctcct tcaccattcc agcctcttcg taacttgacc attctggatc taagcaacaa   1620
   caacatagcc aacataaatg atgacatgtt ggagggtctt gagaaactag aaattctcga   1680
   tttgcagcat aacaacttag cacggctctg gaaacacgca aaccctggtg gtcccattta   1740
   tttcctaaag ggtctgtctc acctccacat ccttaacttg gagtccaacg gctttgacga   1800
   gatcccagtt gaggtcttca aggatttatt tgaactaaag atcatcgatt taggattgaa   1860
   taatttaaac acacttccag catctgtctt taataatcag gtgtctctaa agtcattgaa   1920
   ccttcagaag aatctcataa catccgttga gaagaaggtt ttcgggccag ctttcaggaa   1980
   cctgactgag ttagatatgc gctttaatcc ctttgattgc acgtgtgaaa gtattgcctg   2040
   gtttgttaat tggattaacg agacccatac caacatccct gagctgtcaa gccactacct   2100
   ttgcaacact ccacctcact atcatgggtt cccagtgaga ctttttgata catcatcttg   2160
   caaagacagt gccccctttg aactcttttt catgatcaat accagtatcc tgttgatttt   2220
   tatctttatt gtacttctca tccactttga gggctggagg atatcttttt attggaatgt   2280
   ttcagtacat cgagttcttg gtttcaaaga aatagacaga cagacagaac agtttgaata   2340
   tgcagcatat ataattcatg cctataaaga taaggattgg gtctgggaac atttctcttc   2400
   aatggaaaag gaagaccaat ctctcaaatt ttgtctggaa gaaagggact ttgaggcggg   2460
   tgtttttgaa ctagaagcaa ttgttaacag catcaaaaga agcagaaaaa ttatttttgt   2520
   tataacacac catctattaa aagacccatt atgcaaaaga ttcaaggtac atcatgcagt   2580
   tcaacaagct attgaacaaa atctggattc cattatattg gttttccttg aggagattcc   2640
   agattataaa ctgaaccatg cactctgttt gcgaagagga atgtttaaat ctcactgcat   2700
   cttgaactgg ccagttcaga aagaacggat aggtgccttt cgtcataaat tgcaagtagc   2760
   acttggatcc aaaaactctg tacattaaat ttatttaaat attcaattag caaaggagaa   2820
   actttctcaa tttaaaaagt tctatggcaa atttaagttt tccataaagg tgttataatt   2880
   tgtttattca tatttgtaaa tgattatatt ctatcacaat tacatctctt ctaggaaaat   2940
   gtgtctcctt atttcaggcc tatttttgac aattgactta attttaccca aaataaaaca   3000
   tataagcacg caaaaaaaaa aaaaaaaaa                                     3029
<210> 13
   <211> 2727
   <212> DNA
   <213> Homo sapiens
<400> 13
   aatattttat accaacgttg agcaagatgc agaaaccaac agacaatgcc aatttacgtg     60
   tcattatgga actacttatg agattatgac tgtacccaat gacccttaca cttttctctc    120
   ttgtggtgaa gatggaactg ttaggtggtt tgatacacgc atcaaaacta gctgcacaaa    180
   agaagattgt aaagatgata ttttaattaa ctgtcgacgt gctgccacgt ctgttgctat    240
   ttgcccacca ataccatatt accttgctgt tgggttgttc tgacagctca gtacgaatat    300
   atgatcggcg aatgctgggc acaagagcta cagggaatta tgcaggtcga gggactactg    360
   gaatggttgc ccgttttatt ccttcccatc ttaataataa gtcctgcaga gtgacatctc    420
   tgtgttacag tgaagatggt caagagattc tcgttagtta ctcttcagat tacatatatc    480
   tttttgaccc gaaagatgat acagcacgag aacttaaaac tccttctgcg ggaaggagag    540
   aaggagaaga gttgcgacaa ccaccagtta agcgtttgag acttcgtggt gattggtcag    600
   atactggacc cagagcaagg ccggagagtg aacgagaacg agatggagag ccagagtccc    660
   caatgtgtcc attgatgcca gagaatgtcc tgatatgtta tcaagatggt ttgaagaagc    720
   aagtgaggtt gcacaaagca atagaggacg aggaagatct cgacccagag gtggaacaag    780
   tcaatcagat atttcaactc ttcctacggt cccatcaagt cctgatttgg aagtgagtga    840
   aactgcaatg gaagtagata ctccagctga acaatttctt cagccttcta catcctctac    900
   aatgtcagct caggctcatt cgacatcatc tcccacagaa agccctcatt ctactccttt    960
   gctatcttct ccagacagtg aacaaaggca gtctgttgag gcatctggac accacacaca   1020
   tcatcagtct gattctcctt cttctgtggt taacaaacag ctcggatcca tgtcacttga   1080
   cgagcaacag gataacaata atgaaaagct gagccccaaa ccagggacag gtgaaccagt   1140
   tttaagtttg cactacagca cagaaggaac aactacaagc acaataaaac tgaactttac   1200
   agatgaatgg agcagtatag catcaagttc tagaggaatt gggagccatt gcaaatctga   1260
   gggtcaggag gaatctttcg tcccacagag ctcagtgcaa ccaccagaag gagacagtga   1320
   aacaaaagct cctgaagaat catcagagga tgtgacaaaa tatcaggaag gagtatctgc   1380
   agaaaaccca gttgagaacc atatcaatat aacacaatca gataagttca cagccaagcc   1440
   attggattcc aactcaggag aaagaaatga cctcaatctt gatcgctctt gtggggttcc   1500
   agaagaatct gcttcatctg aaaaagccaa ggaaccagaa acttcagatc agactagcac   1560
   tgagagtgct accaatgaaa ataacaccaa tcctgagcct cagttccaaa cagaagccac   1620
   tgggccttca gctcatgaag aaacatccac cagggactct gctcttcagg acacagatga   1680
   cagtgatgat gacccagtcc tgatcccagg tgcaaggtat cgagcaggac ctggtgatag 1740
   acgctctgct gttgcccgta ttcaggagtt cttcagacgg agaaaagaaa ggaaagaaat 1800
   ggaagaattg gatactttga acattagaag gccgctagta aaaatggttt ataaaggcca 1860
   tcgcaactcc aggacaatga taaaagaagc caatttctgg ggtgctaact ttgtaatgag 1920
   tggttctgac tgtggccaca ttttcatctg ggatcggcac actgctgagc atttgatgct 1980
   tctggaagct gataatcatg tggtaaactg cctgcagcca catccgtttg acccaatttt 2040
   agcctcatct ggcatagatt atgacataaa gatctggtca ccattagaag agtcaaggat 2100
   ttttaaccga aaacttgctg atgaagttat aactcgaaac gaactcatgc tggaagaaac 2160
   tagaaacacc attacagttc cagcctcttt catgttgagg atgttggctt cacttaatca 2220
   tatccgagct gaccggttgg agggtgacag atcagaaggc tctggtcaag agaatgaaaa 2280
   tgaggatgag gaataataaa ctctttttgg caagcactta aatgttctga aatttgtata 2340
   agacatttat tatatttttt tctttacaga gctttagtgc aattttaagg ttatggtttt 2400
   tggagttttt cccttttttt gggataacct aacattggtt tggaatgatt gtgtgcatga 2460
   atttgggaga ttgtataaaa caaaactagc agaatgtttt taaaactttt tgccgtgtat 2520
   gaggagtgct agaaaatgca aagtgcaata ttttccctaa ccttcaaatg tgggagcttg 2580
   gatcaatgtt gaagaataat tttcatcata gtgaaaatgt tggttcaaat aaatttctac 2640
   acttgccatt tgcatgtttg ttgctttcta attaaagaaa ctggttgttt taagatcaaa 2700
   aaaaaaaaaa aaaaaaaaaa aaaaaaa 2727
<210> 14
   <211> 3555
   <212> DNA
   <213> Homo sapiens
<220>
   <221> misc_feature
   <222> (37)..(37)
   <223> n is a, c, g, or t
<220>
   <221> misc_feature
   <222> (91)..(91)
   <223> n is a, c, g, or t
<220>
   <221> misc_feature
   <222> (98).. (98)
   <223> n is a, c, g, or t
<220>
   <221> misc_feature
   <222> (104)..(104)
   <223> n is a, c, g, or t
<220>
   <221> misc_feature
   <222> (197)..(197)
   <223> n is a, c, g, or t
<220>
   <221> misc_feature
   <222> (251)..(251)
   <223> n is a, c, g, or t
<220>
   <221> misc_feature
   <222> (340)..(367)
   <223> n is a, c, g, or t
<220>
   <221> misc_feature
   <222> (395)..(411)
   <223> n is a, c, g, or t
<220>
   <221> misc feature
   <222> (454)..(511)
   <223> n is a, c, g, or t
<220>
   <221> misc_feature
   <222> (532)..(561)
   <223> n is a, c, g, or t
<220>
   <221> misc_feature
   <222> (649)..(703)
   <223> n is a, c, g, or t
<220>
   <221> misc_feature
   <222> (746)..(769)
   <223> n is a, c, g, or t
<220>
   <221> misc_feature
   <222> (853)..(867)
   <223> n is a, c, g, or t
<220>
   <221> misc feature
   <222> (922)..(944)
   <223> n is a, c, g, or t
<220>
   <221> misc_feature
   <222> (1040)..(1062)
   <223> n is a, c, g, or t
<220>
   <221> misc_feature
   <222> (1472)..(1986)
   <223> n is a, c, g, or t
<220>
   <221> misc_feature
   <222> (1910)..(1924)
   <223> n is a, c, g, or t
<220>
   <221> misc_feature
   <222> (1951)..(1966)
   <223> n is a, c, g, or t
<220>
   <221> misc_feature
   <222> (1985)..(1985)
   <223> n is a, c, g, or t
<220>
   <221> misc_feature
   <222> (2062)..(2062)
   <223> n is a, c, g, or t
<220>
   <221> misc_feature
   <222> (2378)..(2463)
   <223> n is a, c, g, or t
<220>
   <221> misc_feature
   <222> (2959)..(2954)
   <223> n is a, c, g, or t
<220>
   <221> misc_feature
   <222> (2960)..(2960)
   <223> n is a, c, g, or t
<220>
   <221> misc_feature
   <222> (2966)..(2967)
   <223> n is a, c, g, or t
<220>
   <221> misc_feature
   <222> (2970)..(2971)
   <223> n is a, c, g, or t
<220>
   <221> misc_feature
   <222> (2973)..(2973)
   <223> n is a, c, g, or t
<220>
   <221> misc_feature
   <222> (2975)..(2975)
   <223> n is a, c, g, or t
<220>
   <221> misc_feature
   <222> (2986)..(2986)
   <223> n is a, c, g, or t
<220>
   <221> misc_feature
   <222> (2998)..(2998)
   <223> n is a, c, g, or t
<220>
   <221> misc_feature
   <222> (3072)..(3072)
   <223> n is a, c, g, or t
<220>
   <221> misc_feature
   <222> (3086)..(3092)
   <223> n is a, c, g, or t
<220>
   <221> misc_feature
   <222> (3095)..(3096)
   <223> n is a, c, g, or t
<220>
   <221> misc_feature
   <222> (3099)..(3102)
   <223> n is a, c, g, or t
<220>
   <221> misc_feature
   <222> (3105)..(3107)
   <223> n is a, c, g, or t
<220>
   <221> misc_feature
   <222> (3119)..(3120)
   <223> n is a, c, g, or t
<220>
   <221> misc_feature
   <222> (3123)..(3125)
   <223> n is a, c, g, or t
<220>
   <221> misc feature
   <222> (3127)..(3127)
   <223> n is a, c, g, or t
<220>
   <221> misc_feature
   <222> (3180)..(3216)
   <223> n is a, c, g, or t
<400> 14
   gcacagcgcg actaagcaga agaaagatca aaaaccngga aaagaggaga agagcaaaca      60
   ggcactttga ggaacaatcc cctttaactc naagccgnac agcngtctag gaattcaagt     120
   tcagtgccta ccgaagacaa aggcgccccg agggagtggc ggtgcgaccc cagggcgtgg     180
   gcccggccgc ggagccnaca ctgcccggct gacccggtgg tctcggacca tgtctcccgc     240
   cccaagaccc ncccgttgtc tcctgctccc cctgctcacg ctcggcaccg cgctcgcctc     300
   cctcggctcg gcccaaagca gcagcttcag ccccgaagcn nnnnnnnnnn nnnnnnnnnn     360
   nnnnnnnccc ggggacctac gtacccacac acagnnnnnn nnnnnnnnnn nctcagcggc     420
   catcgctgcc atgcagaagt tttacggctt gcannnnnnn nnnnnnnnnn nnnnnnnnnn     480
   nnnnnnnnnn nnnnnnnnnn nnnnnnnnnn ngttccagac aagtttgggg cnnnnnnnnn     540
   nnnnnnnnnn nnnnnnnnnn nctacgccat ccagggtctc aaatggcaac ataatgaaat     600
   cactttctgc atccagaatt acacccccaa ggtgggcgag tatgccacnn nnnnnnnnnn     660
   nnnnnnnnnn nnnnnnnnnn nnnnnnnnnn nnnnnnnnnn nnnttccgcg aggtgcccta     720
   tgcctacatc cgtgagggcc atgagnnnnn nnnnnnnnnn nnnnnnnnnt ttgccgaggg     780
   cttccatggc gacagcacgc ccttcgatgg tgagggcggc ttcctggccc atgcctactt     840
   cccaggcccc aannnnnnnn nnnnnnncca ctttgactct gccgagcctt ggactgtcag     900
   gaatgaggat ctgaatggaa annnnnnnnn nnnnnnnnnn nnnnacgagc tgggccatgc     960
   cctggggctc gagcattcca gtgacccctc ggccatcatg gcaccctttt accagtggat    1020
   ggacacggag aattttgtgn nnnnnnnnnn nnnnnnnnnn nncatccagc aactttatgg    1080
   gggtgagtca gggttcccca ccaagatgcc ccctcaaccc aggactacct cccggccttc    1140
   tgttcctgat aaacccaaaa accccaccta tgggcccaac atctgtgacg ggaactttga    1200
   caccgtggcc atgctccgag gggagatgtt tgtcttcaag gagcgctggt tctggcgggt    1260
   gaggaataac caagtgatgg atggataccc aatgcccatt ggccagttct ggcggggcct    1320
   gcctgcgtcc atcaacactg cctacgagag gaaggatggc aaattcgtct tcttcaaagg    1380
   agacaagcat tgggtgtttg atgaggcgtc cctggaacct ggctacccca agcacattaa    1440
   ggagctgggc cgagggctgc ctaccgacaa gnnnnnnnnn nnnnnnttct ggatgcccaa    1500
   tggaaagacc tacttcttcc gtggaaacaa gtactaccgt ttcaacgaag agctcagggc    1560
   agtggatagc gagtacccca agaacatcaa agtctgggaa gggatccctg agtctcccag    1620
   agggtcattc atgggcagcg atgaagtctt cacttacttc tacaagggga acaaatactg    1680
   gaaattcaac aaccagaagc tgaaggtaga accgggctac cccaagtcag ccctgaggga    1740
   ctggatgggc tgcccatcgg gaggccggcc ggatgagggg actgaggagg agacggaggt    1800
   gatcatcatt gaggtggacg aggagggcgg cggggcggtg agcgcggctg ccgtggtgct    1860
   gcccgtgctg ctgctgctcc tggtgctggc ggtgggcctt gcagtcttcn nnnnnnnnnn 1920
   nnnngggacc cccaggcgac tgctctactg nnnnnnnnnn nnnnnngaca aggtctgacg 1980
   cccanccgcc ggcccgccca ctcctaccac aaggactttg cctctgaagg ccagtggcag 2040
   caggtggtgg tgggtgggct gntcccatcg tcccgagccc cctccccgca gcctccttgc 2100
   ttctctctgt cccctggctg gcctccttca ccctgaccgc ctccctccct cctgccccgg 2160
   cattgcatct tccctagata ggtcccctga gggctgagtg ggagggcggc cctttccagc 2220
   ctctgcccct caggggaacc ctgtagcttt gtgtctgtcc agccccatct gaatgtgttg 2280
   ggggctctgc acttgaaggc aggaccctca gacctcgctg gtaaaggtca aatggggtca 2340
   tctgctcctt ttccatcccc tgacatacct taacctcnnn nnnnnnnnnn nnnnnnnnnn 2400
   nnnnnnnnnn nnnnnnnnnn nnnnnnnnnn nnnnnnnnnn nnnnnnnnnn nnnnnnnnnn 2460
   nnnggctaaa aggaatctaa tcttgttgag ggtagagacc ctgagacagt gtgagggggt 2520
   ggggactgcc aagccaccct aagaccttgg gaggaaaact cagagagggt cttcgttgct 2580
   cagtcagtca agttcctcgg agatctgcct ctgcctcacc taccccaggg aacttccaag 2640
   aaggagcctg agccactggg gactaagtgg gcagaagaaa cccttggcag ccctgtgcct 2700
   ctcgaatgtt agccttggat ggggctttca cagttagaag agctgaaacc aggggtgcag 2760
   ctgtcaggta gggtggggcc ggtgggagag gcccgggtca gagccctggg ggtgagcctg 2820
   aaggccacag agaaagaacc ttgcccaaac tcaggcagct ggggctgagg cccaaaggca 2880
   gaacagccag agggggcagg aggggaccaa aaaggaaaat gaggacgtgc agcagcattg 2940
   gaaggctggg gccnggcagn ccaggnnaan ncnancaggg ggccancagg gtgggctngt 3000
   ggagctctca ggaagggccc tgaggaaggc acacttgctc ctgttggtcc ctgtccttgc 3060
   tgcccaggca gngtggaggg gaaggnnnnn nnagnnagnn nnagnnncag agaaggcann 3120
   cannngngga atgaggggct tcacgagagg ccacagggcc tggctggcca cgctgtcccn 3180
   nnnnnnnnnn nnnnnnnnnn nnnnnnnnnn nnnnnngctc gcttaggctg ggtccacgct 3240
   tccctggtgc cagcacccct caagcctgtc tcaccagtgg cctgccctct cgctccccca 3300
   cccagcccac ccattgaagt ctccttgggc caccaaaggt ggtggccatg gtaccgggga 3360
   cttgggagag tgagacccag tggagggagc aagaggagag ggatgtcggg ggggtggggc 3420
   acggggtagg ggaaatgggg tgaacggtgc tggcagttcg gctagatttc tgtcttgttt 3480
   gtttttttgt tttgtttaat gtatattttt attataatta ttatatatga attccaaaaa 3540
   aaaaaaaaaa aaaaa 3555
<210> 15
   <211> 213
   <212> DNA
   <213> Homo sapiens
<400> 15
   tgggaggaag gcttgggagt cccctaagac acatcttcag tcacttctct ctgcctgtga     60
   ctcaggaaga ccagctcctc ctactgtctt ctgtgctagg gatcacttcc ttgttgagtg    120
   gggccggagt tttaagagga tcttctgctc ctcctcatct ggtccctttc cttccaaggc    180
   ccccgagagg aaggcatgcg gtgggcccta ctg                                213
<210> 16
   <211> 1670
   <212> DNA
   <213> Homo sapiens
<400> 16
   gggcaaaccc tttgaaaaat attctaaatg aaaatgacat agtattcata gtggaaaaag     60
   tgcctttaga aaaggaagaa acaagtcata ttgaagaact tcaatctgaa gaaactgcca    120
   tatctgattt ctctactggc gaaaatgttg gaccacttgc tttaccagtt gggaaggcaa    180
   ggcagttaat tggactttac accatggctc acaatcctaa tatgacccat ttgaagatta    240
   atctgcctgt tactgccctt cccccccttt gggtaagatg tgacagttca gatcctgaag    300
   gtacttgttg gctaggagct gagcttatca caacaaacaa cagcattaca ggaattgtct    360
   tatatgtggt cagttgtaaa gctgataaaa attattctgt aaatcttgaa aacctaaaaa    420
   atttacacaa gaaaagacat cacttgtcta ctgtaacatc caaaggcttt gcccagtatg    480
   agctctttaa gtcctctgcc ttggatgata caatcacagc atcacaaact gcgatcgctt    540
   tggatatttc ctggagtcct gtggatgaga ttcttcaaat ccctccactc tcttcaactg    600
   caatctgaat attaaagtgg aatcaggaga gcccagaggt cctttgaatc atctctacag    660
   agaactgaaa tttcttcttg ttttggctga tggtttgagg actggtgtca ctgaatggct    720
   cgagcccctg gaagcaaaat ctgctgttga acttgttcag gaatttctga atgacttaaa    780
   taagctggat ggatttggtg attctacaaa aaaagacact gaggttgaga ccttgaagca    840
   tgacactgct gcagtcgatc gttccgtcaa gcgtcttttc aaagttcgga gtgatcttga    900
   ttttgctgag caactgtggt gcaaaatgag cagtagtgtg atttcatacc aagactaggt    960
   gaagtgtttc acattgatca tccagagtct acaacgtggt gatatacagc catggctcca   1020
   tagtggaagt aacagtttac taagtaagct cattcatcag tcttatcatg gaaccatgga   1080
   cacagtttct ctcagtggga ctattccagt tcaaatgctt ttggaaattg gtttggacaa   1140
   actaaagaaa gattatatca gttttttcat aggtcaggaa cttgcatctt tgaatcattt   1200
   ggaatacttc attgctccat cagtagatat acaagaacag gtttatcgtg tccaaaaact   1260
   ccaccatatt ctagaaatat tagtcagttg catgcctttc attaaatctc aacatgaact   1320
   cctcttttct ttaacacaga tctgcataaa gtattacaaa caaaatcctc ttgatgagca   1380
   acacattttt cagctgccag tcagaccaac tgctgtaaag aacttatatc aaagtgagaa   1440
   gccacagaaa tggagagtgg aaatatatag tggtcaaaag aagattaaga cagtttggca   1500
   actgagtgac agctcaccca taggccatct gaattttcac aaacctgatt tttcggaatt   1560
   aacactaagc ggtagcctgg aagaaaggat attctttact aacatggtta cctgcagcca   1620
   ggtgcatttc aagtgaagtg tgctgatgaa gtcctctata agcacaagcc               1670
<210> 17
   <211> 1914
   <212> DNA
   <213> Homo sapiens
<400> 17
   cggacgcgtg ggcggacgcg tggggccttc ctgggattgg agtctcgagc tttcttcgtt     60
   cgttcgccgg cgggttcgcg cccttctcgc gcctcggggc tgcgaggctg gggaaggggt    120
   tggagggggc tgttgatcgc cgcgtttaag ttgcgctcgg ggcggccatg tcggccggcg    180
   aggtcgagcg cctagtgtcg gagctgagcg gcgggaccgg aggggatgag gaggaagagt    240
   ggctctatgg cgatgaaaat gaagttgaaa ggccagaaga agaaaatgcc agtgctaatc    300
   ctccatctgg aattgaagat gaaactgctg aaaatggtgt accaaaaccg aaagtgactg    360
   agaccgaaga tgatagtgat agtgacagcg atgatgatga agatgatgtt catgtcacta    420
   taggagacat taaaacggga gcaccacagt atgggagtta tggtacagca cctgtaaatc    480
   ttaacatcaa gacaggggga agagtttatg gaactacagg gacaaaagtc aaaggagtag    540
   accttgatgc acctggaagc attaatggag ttccactctt agaggtagat ttggattctt    600
   ttgaagataa accatggcgt aaacctggtg ctgatctttc tgattatttt aattatgggt    660
   ttaatgaaga tacctggaaa gcttactgtg aaaaacaaaa gaggatacga atgggacttg    720
   aagttatacc agtaacctct actacaaata aaattacggt acagcaggga agaactggaa    780
   actcagagaa agaaactgcc cttccatcta caaaagctga gtttacttct cctccttctt    840
   tgttcaagac tgggcttcca ccgagcagga gattacctgg ggcaattgat gttatcggtc    900
   agactataac tatcagccga gtagaaggca ggcgacgggc aaatgagaac agcaacatac    960
   aggtcctttc tgaaagatct gctactgaag tagacaacaa ttttagcaaa ccacctccgt   1020
   ttttccctcc aggagctcct cccactcacc ttccacctcc tccatttctt ccacctcctc   1080
   cgactgtcag cactgctcca cctctgattc caccaccggg ttttcctcct ccaccaggcg   1140
   ctccacctcc atctcttata ccaacaatag aaagtggaca ttcctctggt tatgatagtc   1200
   gttctgcacg tgcatttcca tatggcaatg ttgcctttcc ccatcttcct ggttctgctc   1260
   cttcgtggcc tagtcttgtg gacaccagca agcagtggga ctattatgcc agaagagaga   1320
   aagaccgaga tagagagaga gacagagaca gagagcgaga ccgtgatcgg gacagagaaa   1380
   gagaacgcac cagagagaga gagagggagc gtgatcacag tcctacacca agtgttttca   1440
   acagcgatga agaacgatac agatacaggg aatatgcaga aagaggttat gagcgtcaca   1500
   gagcaagtcg agaaaaagaa gaacgacata gagaaagacg acacagggag aaagaggaaa   1560
   ccagacataa gtcttctcga agtaatagta gacgtcgcca tgaaagtgaa gaaggagata   1620
   gtcacaggag acacaaacac aaaaaatcta aaagaagcaa agaaggaaaa gaagcgggca   1680
   gtgagcctgc ccctgaacag gagagcaccg aagctacacc tgcagaatag gcatggtttt   1740
   ggccttttgt gtatattagt accagaagta gatactataa atcttgttat ttttctggat   1800
   aatgtttaag aaatttacct taaatcttgt tctgtttgtt agtatgaaaa gttaactttt   1860
   tttccaaaat aaaagagtga atttttcatg ttaagttaaa aaaaaaaaaa aaaa         1914
<210> 18
   <211> 652
   <212> DNA
   <213> Homo sapiens
<400> 18
   ggacggaagg agcggcggcg acggaggagg aggatggagg cggtggtgtt cgtcttctct 60
   ctcctcgatt gttgcgcgct catcttcctc tcggtctact tcataattac attgtctgat 120
   ttagaatgtg attacattaa tgctagatca tgttgctcaa aattaaacaa gtgggtaatt 180
   ccagaattga ttggccatac cattgtcact gtattactgc tcatgtcatt gcactggttc 240
   atcttccttc tcaacttacc tgttgccact tggaatatat atcgatacat tatggtgccg 300
   agtggtaaca tgggagtgtt tgatccaaca gaaatacaca atcgagggca gctgaagtca 360
   cacatgaaag aagccatgat caagcttggt ttccacttgc tctgcttctt catgtatctt 420
   tatagtatga tcttagcttt gataaatgac tgaagctgga gaagccgtgg ttgaagtcag 480
   cctacactac agtgcacagt tgaggagcca gagacttctt aaatcatcct tagaaccgtg 540
   accatagcag tatatatttt cctcttggaa caaaaaacta tttttgctgt atttttacca 600
   tataaagtat ttaaaaaaca ggaaaaaaaa aaaaaaaaaa aaaaaaaaaa aa 652
<210> 19
   <211> 3346
   <212> DNA
   <213> Homo sapiens
<400> 19
   acgtactcca tgcgctacct gctgcccagc gtcgtgctcc tgggcacggc gcccacctac      60
   gtgttggcct ggggggtctg gcggctgctc tccgccttcc tgcccgcccg cttctaccaa      120
   gcgctggacg accggctcta ctgcgtctac cagagcatgg tgctcttctt cttcgagaat     180
   tacaccgggg tccagatatt gctatatgga gatttgccaa aaaataaaga aaatataata     240
   tatttagcaa atcatcaaag cacagttgac tggattgttg ctgacatctt ggccatcagg     300
   cagaatgcgc taggacatgt gcgctacgtg ctgaaagaag ggttaaaatg gctgccattg     360
   tatgggtgtt actttgctca gcatggagga atctatgtaa agcgcagtgc caaatttaac     420
   gagaaagaga tgcgaaacaa ggtgcagagc tacgtggacg caggaactcc aatgtatctt     480
   gtgatttttc cagaaggtac aaggtataat ccagagcaaa caaaagtcct ttcagctagt     540
   caggcatttg ctgcccaacg tggccttgca gtattaaaac atgtgctaac accacgaata     600
   aaggcaactc acgttgcttt tgattgcatg aagaattatt tagatgcaat ttatgatgtt     660
   acggtggttt atgaagggaa agacgatgga gggcagcgaa gagagtcacc gaccatgacg     720
   gaatttctct gcaaagaatg tccaaaaatt catattcaca ttgatcgtat cgacaaaaaa     780
   gatgtcccag aagaacaaga acatatgaga agatggctgc atgaacgttt cgaaatcaaa     840
   gataagatgc ttatagaatt ttatgagtca ccagatccag aaagaagaaa aagatttcct     900
   gggaaaagtg ttaattccaa attaagtatc aagaagactt taccatcaat gttgatctta     960
   agtggtttga ctgcaggcat gcttatgacc gatgctggaa ggaagctgta tgtgaacacc    1020
   tggatatatg gaaccctact tggctgcctg tgggttacta ttaaagcata gacaagtagc    1080
   tgtctccaga cagtgggatg tgctacattg tctatttttg gcggctgcac atgacatcaa    1140
   attgtttcct gaatttatta aggagtgtaa ataaagcctt gttgattgaa gattggataa    1200
   tagaatttgt gacgaaagct gatatgcaat ggtcttgggc aaacatacct ggttgtacaa    1260
   ctttagcatc ggggctgctg gaagggtaaa agctaaatgg agtttctcct gctctgtcca    1320
   tttcctatga actaatgaca acttgagaag gctgggagga ttgtgtattt tgcaagtcag    1380
   atggctgcat ttttgagcat taatttgcag cgtatttcac tttttctgtt attttcaatt    1440
   tattacaact tgacagctcc aagctcttat tactaaagta tttagtatct tgcagctagt    1500
   taatatttca tcttttgctt atttctacaa gtcagtgaaa taaattgtat ttaggaagtg    1560
   tcaggatgtt caaaggaaag ggtaaaaagt gttcatgggg aaaaagctct gtttagcaca    1620
   tgattttatt gtattgcgtt attagctgat tttactcatt ttatatttgc aaaataaatt 1680
   tctaatattt attgaaattg cttaatttgc acaccctgta cacacagaaa atggtataaa 1740
   atatgagaac gaagtttaaa attgtgactc tgattcatta tagcagaact ttaaatttcc 1800
   cagctttttg aagatttaag ctacgctatt agtacttccc tttgtctgtg ccataagtgc 1860
   ttgaaaacgt taaggttttc tgttttgttt tgttttttta atatcaaaag agtcggtgtg 1920
   aaccttggtt ggaccccaag ttcacaagat ttttaaggtg atgagagcct gcagacattc 1980
   tgcctagatt tactagcgtg tgccttttgc ctgcttctct ttgatttcac agaatattca 2040
   ttcagaagtc gcgtttctgt agtgtggtgg attcccactg ggctctggtc cttcccttgg 2100
   atcccgtcag tggtgctgct cagcggcttg cacgcagact tgctaggaag aaatgcagag 2160
   ccagcctgtg ctgcccactt tcagagttga actctttaag cccttgtgag tgggcttcac 2220
   cagctactgc agaggcattt tgcatttgtc tgtgtcaaga agttcacctt ctcaagccag 2280
   tgaaatacag acttaatttg tcatgactga acgaatttgt ttatttccca ttaggtttag 2340
   tggagctaca cattaatatg tatcgcctta gagcaagagc tgtgttccag gaaccagatc 2400
   acgattttta gccatggaac aatatatccc atgggagaag acctttcagt gtgaactgtt 2460
   ctatttttgt gttataattt aaacttcgat ttcctcatag tcctttaagt tgacatttct 2520
   gcttactgct actggatttt tgctgcagaa atgtatcagt ggcccacatt aaacatacca 2580
   gttggatcat gataagcaaa atgaaagaaa taatgattaa gggaaaatta agtgactgtg 2640
   ttacactgct tctcccatgc cagagaataa actctttcaa gcatcatctt tgaagagtcg 2700
   tgtggtgtga attggtttgt gtacattaga atgtatgcac acatccatgg acactcagga 2760
   tatagttggc ctaataatcg gggcatgggt aaaacttatg aaaatttcct catgctgaat 2820
   tgtaattttc tcttacctgt aaagtaaaat ttagatcaat tccatgtctt tgttaagtac 2880
   agggatttaa tatattttga atataatggg tatgttctaa atttgaactt tgagaggcaa 2940
   tactgttgga attatgtgga ttctaactca ttttaacaag gtagcctgac ctgcataaga 3000
   tcacttgaat gttaggtttc atagaactat actaatcttc tcacaaaagg tctataaaat 3060
   acagtcgttg aaaaaaattt tgtatcaaaa tgtttggaaa attagaagct tctccttaac 3120
   ctgtattgat actgacttga gttattttct aaaattaaga gccgtatacc tacctgtaag 3180
   tcttttcaca tatcatttaa acttttgttt gtattattac tgatttacag cttagttatt 3240
   aatttttctt tataagaatg ccgtcgatgt gcatgctttt atgtttttta gaaaagggtg 3300
   tgtttggatg aaagtaaaaa aaaaaataaa atctttcact gtctct 3346
<210> 20
   <211> 459
   <212> DNA
   <213> Homo sapiens
<220>

   <221> misc_feature
   <222> (12)..(13)
   <223> n is a, c, g, or t
<220>
   <221> misc_feature
   <222> (445)..(446)
   <223> n is a, c, g, or t
<220>
   <221> misc_feature
   <222> (448)..(453)
   <223> n is a, c, g, or t
<400> 20
   tttttttttt tnnttttttt ttttttgtat gttggaacgt gccccagaat gaggcagttg     60
   gcaaacttct caggacaatg aatccttccc gtttttcttt ttatgccaca cagtgcattg    120
   ttttttctac ctgcttgtct tatttttaga ataatttaga aaaacaaaac aaaggctgtt    180
   tttcctaatt ttggcatgaa cccccccttg ttccaaatga agacggcatc acgaagcagc    240
   tccaaaagga aaagcttggg cggtgcccag cgtgcccgct gcccatcgac gtctgtcctg    300
   gggacgtgga gggtggcagc gtccccgcct gcaccagtgc cgtcctgctg atgtggtagg    360
   ctagcaatat tttggttaaa atcatgtttg tgactgtaac catttgtatg aattatttta    420
   aagaaataaa aatcctggaa agagnnannn nnnccagca                           459
<210> 21
   <211> 2574
   <212> DNA
   <213> Homo sapiens
<220>
   <221> misc_feature
   <222> (200)..(200)
   <223> n is a, c, g, or t
<220>
   <221> misc_feature
   <222> (444)..(444)
   <223> n is a, c, g, or t
<220>
   <221> misc feature
   <222> (551)..(551)
   <223> n is a, c, g, or t
<220>
   <221> misc_feature
   <222> (614)..(614)
   <223> n is a, c, g, or t
<220>
   <221> misc_feature
   <222> (631)..(631)
   <223> n is a, c, g, or t
<220>
   <221> misc_feature
   <222> (853)..(853)
   <223> n is a, c, g, or t
<220>
   <221> misc_feature
   <222> (855)..(855)
   <223> n is a, c, g, or t
<220>
   <221> misc feature
   <222> (964)..(964)
   <223> n is a, c, g, or t
<220>
   <221> misc_feature
   <222> (978)..(978)
   <223> n is a, c, g, or t
<220>
   <221> misc_feature
   <222> (1024)..(1024)
   <223> n is a, c, g, or t
<220>
   <221> misc_feature
   <222> (1060)..(1060)
   <223> n is a, c, g, or t
<220>
   <221> misc_feature
   <222> (1067)..(1067)
   <223> n is a, c, g, or t
<220>
   <221> misc_feature
   <222> (1075)..(1075)
   <223> n is a, c, g, or t
<220>
   <221> misc_feature
   <222> (1086)..(1086)
   <223> n is a, c, g, or t
<220>
   <221> misc_feature
   <222> (1091)..(1091)
   <223> n is a, c, g, or t
<220>
   <221> misc_feature
   <222> (1100)..(1100)
   <223> n is a, c, g, or t
<220>
   <221> misc_feature
   <222> (1110)..(1110)
   <223> n is a, c, g, or t
<220>
   <221> misc_feature
   <222> (1114).. (1114)
   <223> n is a, c, g, or t
<220>
   <221> misc_feature
   <222> (1116)..(1116)
   <223> n is a, c, g, or t
<220>
   <221> misc_feature
   <222> (1120)..(1120)
   <223> n is a, c, g, or t
<220>
   <221> misc_feature
   <222> (1130)..(1130)
   <223> n is a, c, g, or t
<220>
   <221> misc_feature
   <222> (1140)..(1140)
   <223> n is a, c, g, or t
<220>
   <221> misc_feature
   <222> (1143)..(1143)
   <223> n is a, c, g, or t
<220>
   <221> misc_feature
   <222> (1150)..(1150)
   <223> n is a, c, g, or t
<220>
   <221> misc_feature
   <222> (1158)..(1159)
   <223> n is a, c, g, or t
<220>
   <221> misc_feature
   <222> (1164)..(1164)
   <223> n is a, c, g, or t
<220>
   <221> misc_feature
   <222> (1169)..(1169)
   <223> n is a, c, g, or t
<220>
   <221> misc_feature
   <222> (1171)..(1171)
   <223> n is a, c, g, or t
<220>
   <221> misc_feature
   <222> (1181)..(1181)
   <223> n is a, c, g, or t
<220>
   <221> misc_feature
   <222> (1187)..(1187)
   <223> n is a, c, g, or t
<220>
   <221> misc_feature
   <222> (1348)..(1348)
   <223> n is a, c, g, or t
<220>
   <221> misc_feature
   <222> (1602)..(1602)
   <223> n is a, c, g, or t
<220>
   <221> misc_feature
   <222> (1659)..(1659)
   <223> n is a, c, g, or t
<220>
   <221> misc_feature
   <222> (1737)..(1737)
   <223> n is a, c, g, or t
<220>
   <221> misc_feature
   <222> (1741)..(1741)
   <223> n is a, c, g, or t
<400> 21
   cggcacgagg gtttgatcta atcttaatca cagtgagctc ttcattagca caatatgtgg     60
   tttgccccaa gtgtgcacta tttaatactt tgtaatatgc caccaagagt actgacattt    120
   agagttgttt aaaggccgag aactggaaac agcctttccc tcattttctg tgtattggtg    180
   atgggagtaa taacattttn ggggagcttt ttaaatttca cagaagagga aagttgcctg    240
   ctctggcagg tatgtgcaag atagagtgtg tttcatttgt tctgttgcca agaattagtg    300
   ctgtactatt gtagttcctt taggatttgt atgtgctctg ggctcatgaa gatattgcat    360
   catgagcttc agcagttgta ctcttttttg atgacctaaa aagggcttat ttctgaggaa    420
   tgaaaggttc ccatcattga ctanggatgt ggaaaacctt tcctagctta gagcatttgt    480
   atctatattt taaagtcaga gttcatgtta cctgttttaa tcacatgact gcatgtccca 540
   gtacacaaaa nggcactggt tggcattctt cttaatgtat ttagtaaaga tcagaagaaa 600
   tcctttaaga gttnaaatgt ccctggaaca ngcatacagg ctctagtcaa gaatgaatta 660
   gagtgaagga aagctgtgtg acacctggca ttcctctgtt catggagctt ctttgaggct 720
   tgaagattga ttttaccatc tagaccactc tgcctattct tcaaccacct tggttacttt 780
   gacataggaa ttgacttctt ttccttgaat ggaaaacact ttgaaataat aataaacatt 840
   gttataaact aananatgtg agagtgctta gttgaaacaa aaaggagttt tagtagacag 900
   tattatacta tctttgaaaa tcaaggagaa gtttatgcaa cttaaaatgt gtacaaactg 960
   cagngcaatc tactgttngg tgaatgtcag tgtattatca ggaaacatgt ctatacaatc 1020
   acangagtta tatttcctca caaacttctt tgtgaagagn gaaatgngtt tctgntacct 1080
   ctgggnttca ncttacgggn catattttgn gcangnattn atgtgattgn tgcctatgcn 1140
   tgnatgaatn gaatgaannt ttcnagttng nacattgcct naaatcnata acttgatgat 1200
   gcttgggaaa gactcaacag ttaaaacttc atgaagttct aatgtctgtg ttccaaaaca 1260
   catcacatta ttaggatgta gggagatatg tatgtgtgct ccctggggtg gggatttcta 1320
   gttactagac catctccatt tttagcantt ggcatcctca tgatactttt ataaatacga 1380
   cattaacagg agagcagcag tacgattttg ccgatggaat aacagatttg ccggcaatca 1440
   ctgaaagagt gcaaatatcg ggtccttgtg acttcaacgg actcttccaa attgtatgaa 1500
   tgtatcaatg tattagataa acccagtttc agaatgataa agaaaaaatg ttagaccaaa 1560
   taatgcggct agttaacagt ggtacgattt ctagcccgtg gntttaaaat gcacttaaag 1620
   tcctgtcctt gccttttatt ttctgaactt gatgttttng cattctttga gttcagttta 1680
   aagacaacta cgagcatctg taaccaatct gacaataatg tgttcatcag gtgcctntgg 1740
   nattaaatca catactggca tatttaagct gaatgtcaat ctggaaaata aattgactgt 1800
   attaacggaa ataccactct ttgtgtagat atttgtcgta tattgaagaa aaagctaaaa 1860
   agaatggaaa tcgcatgact ataacttaag tctttcttca aagtgcatgc agtcttttgc 1920
   gatacctcat tcagccaagt attggtattc ttcctcattc ggtataaggc agctttcaat 1980
   ttgcttagag ggcaacattg gaaggttaga gttcatcaga aacagaattc taaaatgtga 2040
   gttcaattca ataaatttga atttctgtag gaagaatcaa atcaccgatt taaagattgc 2100
   aatatataat aatcattttt aaagtattgg attaaatctg ataggttttc cagaaatgaa 2160
   caaaaatcag ctctaaaacc aaagctgatt tttagaaaat ttgaaaatgt aaatcagccc 2220
   tatccatact atagtttctc taaaacttta tctgaaagag tcattttaaa ataactatta 2280
   aacaatgtaa ctgctatctt aatgttctga aataagttaa aacattttaa aatatgaata 2340
   ctgtaaagga aataaacggt gggaaggaaa agtagagaaa gaaatgccaa ttccagtcca 2400
   aagctttatt tgccaagttt tcttagaatg aattttacca atttatgaat tcttgtaagc 2460
   ggaatgtaaa acggaaatac tgaaagactt ttgcctaaag tggcattatt gactgctggt 2520
   gtgatgctac tgtaatgtaa taaattatta agttgttgca aaaaaaaaaa aaaa 2574
<210> 22
   <211> 1904
   <212> DNA
   <213> Homo sapiens
<400> 22
   ggaagggagg gagcgaaggt aggaggcagg gcttgcctca ctggccaccc tcccaacccc     60
   aagagcccag ccccatggtc cccgccgccg gcgcgctgct gtgggtcctg ctgctgaatc    120
   tgggtccccg ggcggcgggg gcccaaggcc tgacccagac tccgaccgaa atgcagcggg    180
   tcagtttacg ctttgggggc cccatgaccc gcagctaccg gagcaccgcc cggactggtc    240
   ttccccggaa gacaaggata atcctagagg acgagaatga tgccatggcc gacgccgacc    300
   gcctggctgg accagcggct gccgagctct tggccgccac ggtgtccacc ggctttagcc    360
   ggtcgtccgc cattaacgag gaggatgggt cttcagaaga gggggttgtg attaatgccg    420
   gaaaggatag caccagcaga gagcttccca gtgcgactcc caatacagcg gggagttcca    480
   gcacgaggtt tatagccaat agtcaggagc ctgaaatcag gctgacttca agcctgccgc    540
   gctcccccgg gaggtctact gaggacctgc catgccatct cctgaggatc tgcggctggt    600
   gctgatgccc tttccgggcg ccttcgagtt ggggcgctga gccagctccg cacggagcac    660
   aagccttgca cctatcaaca atgtccctgc aaccgacttc gggaagagtg ccccctggac    720
   acaagtctct gtactgacac caactgtgcc tctcagagca ccaccagtac caggaccacc    780
   actaccccct tccccaccat ccacctcaga agcagtccca gcctgccacc cgccagcccc    840
   tgcccagccc tggctttttg gaaacgggtc aggattggcc tggaggatat ttggaatagc    900
   ctctcttcag tgttcacaga gatgcaacca atagacagaa accagaggta atggccactt    960
   catccacatg aggagatgtc agtatctcaa cctctcttgc cctttcaatc ctagcgccca   1020
   ctagatattt ttagtacaga aaaacaaaac tggaaaacac attgtttggt cttgtgtttc   1080
   tttacagagg tacctgaggg aggagagaca taaatccctt catccctaag actgaactat   1140
   gtaactagca gcctctggct tgttttctac tccctgtccc tcaggataaa atgttgatat   1200
   tgctcatttt cctcatttcc aacattgttt taaaacaagt acttctttta caggcttgaa   1260
   aaatctcaaa taaacgctaa gaaaagggag taggaagaac aaggagttga gcccttgaaa   1320
   gatgacagtg gtcttcttgc cttcatgctt ggccctctct cctcaaaagg gcaatgttgg   1380
   tacaaaattc catctcagcc actttcgagg agttatcttc attagctata tccatccttt   1440
   aatccaacac acacctgcaa tgattactgt gcaactattt tgcttaattt tttatttgaa   1500
   aaaatgtatt taaaagtcca acaacttttt aatataaatt acgactctca aacccattcc   1560
   catcacttta ttagtgatgg tagcatacat attagagaag gtagctaaag gcaagagagc   1620
   accaaaggaa aaagactgtc caaagaacag gtattagaat gaggccgaag atcacggtga   1680
   ccagagattt ctaggagtct ctaacctttc caccctatcc tgttaaccct ttagatctct   1740
   agtataacac tcaggctact gaggtatttt agagcaacaa gctgggttac tttcagagca   1800
   accagcttga ctggaactga gagtaaattg ggaatgtatg accaatctta gaccctgaaa   1860
   aatggcagaa aatacatgga aatttgaaaa aaaaaaaaaa aaaa                    1904
<210> 23
   <211> 12609
   <212> DNA
   <213> Homo sapiens
<220>
   <221> misc_feature
   <222> (35)..(37)
   <223> n is a, c, g, or t
<220>
   <221> misc_feature
   <222> (239)..(239)
   <223> n is a, c, g, or t
<220>
   <221> misc_feature
   <222> (259)..(259)
   <223> n is a, c, g, or t
<220>
   <221> misc_feature
   <222> (711)..(711)
   <223> n is a, c, g, or t
<220>
   <221> misc_feature
   <222> (877)..(880)
   <223> n is a, c, g, or t
<220>
   <221> misc_feature
   <222> (882)..(882)
   <223> n is a, c, g, or t
<220>
   <221> misc_feature
   <222> (884)..(884)
   <223> n is a, c, g, or t
<220>
   <221> misc_feature
   <222> (896)..(896)
   <223> n is a, c, g, or t
<220>
   <221> misc_feature
   <222> (1172)..(1172)
   <223> n is a, c, g, or t
<220>
   <221> misc_feature
   <222> (1183)..(1183)
   <223> n is a, c, g, or t
<220>
   <221> misc_feature
   <222> (1191)..(1192)
   <223> n is a, c, g, or t
<220>
   <221> misc_feature
   <222> (1194)..(1194)
   <223> n is a, c, g, or t
<220>
   <221> misc_feature
   <222> (1196)..(1197)
   <223> n is a, c, g, or t
<220>
   <221> misc_feature
   <222> (1199)..(1201)
   <223> n is a, c, g, or t
<220>
   <221> misc_feature
   <222> (1298)..(1298)
   <223> n is a, c, g, or t
<220>
   <221> misc_feature
   <222> (1446)..(14446)
   <223> n is a, c, g, or t
<220>
   <221> misc_feature
   <222> (1846)..(1846)
   <223> n is a, c, g, or t
<220>
   <221> misc_feature
   <222> (1911)..(1911)
   <223> n is a, c, g, or t
<220>
   <221> misc_feature
   <222> (2909)..(2909)
   <223> n is a, c, g, or t
<220>
   <221> misc_feature
   <222> (3161)..(3162)
   <223> n is a, c, g, or t
<220>
   <221> misc_feature
   <222> (3231)..(3231)
   <223> n is a, c, g, or t
<220>
   <221> misc_feature
   <222> (3594)..(3594)
   <223> n is a, c, g, or t
<220>
   <221> misc_feature
   <222> (4382)..(4382)
   <223> n is a, c, g, or t
<220>
   <221> misc_feature
   <222> (4462).. (4462)
   <223> n is a, c, g, or t
<220>
   <221> misc_feature
   <222> (4656)..(4656)
   <223> n is a, c, g, or t
<220>
   <221> misc_feature
   <222> (4708)..(4708)
   <223> n is a, c, g, or t
<220>
   <221> misc_feature
   <222> (5214)..(5214)
   <223> n is a, c, g, or t
<220>
   <221> misc_feature
   <222> (8307)..(8308)
   <223> n is a, c, g, or t
<220>
   <221> misc_feature
   <222> (8310)..(8310)
   <223> n is a, c, g, or t
<220>
   <221> misc_feature
   <222> (8318)..(8318)
   <223> n is a, c, g, or t
<220>
   <221> misc_feature
   <222> (8336)..(8336)
   <223> n is a, c, g, or t
<220>
   <221> misc_feature
   <222> (8351)..(8351)
   <223> n is a, c, g, or t
<220>
   <221> misc_feature
   <222> (8658)..(8658)
   <223> n is a, c, g, or t
<220>
   <221> misc_feature
   <222> (8883)..(8886)
   <223> n is a, c, g, or t
<220>
   <221> misc feature
   <222> (8886)..(8889)
   <223> n is a, c, g, or t
<220>
   <221> misc_feature
   <222> (8891)..(8891)
   <223> n is a, c, g, or t
<220>
   <221> misc_feature
   <222> (8984)..(8988)
   <223> n is a, c, g, or t
<220>
   <221> misc_feature
   <222> (9192)..(9192)
   <223> n is a, c, g, or t
<220>
   <221> misc_feature
   <222> (9203)..(9203)
   <223> n is a, c, g, or t
<220>
   <221> misc_feature
   <222> (9273)..(9274)
   <223> n is a, c, g, or t
<220>
   <221> misc_feature
   <222> (9276)..(9276)
   <223> n is a, c, g, or t
<220>
   <221> misc_feature
   <222> (9278)..(9279)
   <223> n is a, c.g, or t
<220>

   <221> misc_feature
   <222> (9281)..(92B5)
   <223> n is a, c, g, or t
<220>
   <221> misc feature
   <222> (9287)..(9287)
   <223> n is a, c, g, or t
<220>
   <221> misc_feature
   <222> (9309)..(9309)
   <223> n is a, c, g, or t
<220>
   <221> misc_feature
   <222> (9698)..(9699)
   <223> n is a, c, g, or t
<220>
   <221> misc_feature
   <222> (10403)..(10403)
   <223> n is a, c, g, or t
<220>
   <221> misc_feature
   <222> (11142)..(11142)
   <223> n is a, c, g, or t
<220>
   <221> misc_feature
   <222> (11381)..(11381)
   <223> n is a, c, g, or t
<220>
   <221> misc_feature
   <222> (11383)..(11383)
   <223> n is a, c, g, or t
<220>
   <221> misc_feature
   <222> (11385)..(11387)
   <223> n is a, c, g, or t
<400> 23
   tttttttttt tttttttttt tttttttttt ttttnnntta tacacatcca attttattaa 60
   cattcactta taaattatct gcccttgtca tatattggaa tcgtgttttt taggcaaata 120
   taacaatcct ctactgcagg agagaaagtc ttcttggtga tatgagaaat actggtgtga 180
   taatttcata aattattcaa gtctgtggaa acactccagt tcagagaagg gtacctggna 240
   tttgttttca gctttctcna ttttcacatg cacgttaggc tatctggttt tgatagtgcg 300
   tgttttcttc tattaagtga gatagctttc tcaataatta tatcagaaac agcagcagtg 360
   agtgactaga gggcctctac tggcctcagg cattttctaa aaaccggaat tagctgtcaa 420
   attttgagaa tacagagctt cccacggcct cccccatccc tgcatttggt gtgcacctca 480
   caatttcatg caatttagtg acttgccaag ctcctgcagg catctgtgtt tgtgctgaag 540
   atgtctgtct cctctgcggt gcaccgtgtc gtgtatcaca gcatattgga atgggtctgt 600
   aggtaggaat agctgatttg ttattcagga tggctttaac agccgctgga aggaacacac 660
   catcttatgg tagttgctct tccacacagc tttgttctct gcatgggtga ngggtatctc 720
   tggggtccat caactattac atcatgatgc ctgtgcaata tttacttgct tacgtccaat 780
   ctactgggtc cctgaagaat aagctttttt cttcttctta gctaatattt acccctcctt 840
   ttctctaatg atagagtact gggaagcaga aatcccnnnn tncnccttct ctcacnaact 900
   ccccagcctc cccactcatt ctcagtcctg gggactgggg gctagtggct gagtgtgggc 960
   tatactgcct tgctgtttgc aatgaaacaa ttgtaaaata aagagagcag ccaaaacagg 1020
   gagttatgag ctgagaatat gggaataagc acacaattag ttctgacgaa agaaatatct 1080
   tcctgatgtt attaacaatc tactagaaat tgtgtcctct ttaccaacag aaaaatattg 1140
   atgtgcaaga aaataagctt atttctaact tnatttttta agnggtagaa nntntnntnn 1200
   nttttgcata catacaattt atttatttat attatagccc tgtatgatac aagcattttc 1260
   tcatctaaaa gtgttatttt aactcagaaa acatactngc attaagctct tgagcctcag 1320
   aatggagcac agagggaaca aaaccctttt gaaattaaaa atattattca gcagaaactg 1380
   atacttttga accaatatga atttgaaata actaaatatt ttttcaaaat ctcccttaac 1440
   atcaangttc aagatgattg catttatttt gatgtcacta tataaataca aggatgttaa 1500
   ctgatactca cctaagtcaa atattccagt tgcattaaca aacatacaca taacaaatat 1560
   aaatttatat caaatgtcaa atttccccat tatcttctcc tcttcttcat taggaatttg 1620
   atgttctgaa aagtgccttt tttgtctgct tcgatttttt gttgctgttt tttttttttt 1680
   tttgctttat aatttctaag catttaggca gaaaagaaag aggcattaaa aatacaactc 1740
   agaaatattt aaattttgat gaaataagag tcttgttagt tgcagtctct tttgaatact 1800
   ttccttggga gaaaatatgc tgtgaaacag tgatctgcca ctacanagtg gttggggaca 1860
   ttaggtcatt tctagaaagc aattatatac acctaatggc ttattcccta naaaatgaac 1920
   tattatggtg tcagaaaagg ctagatactt tatcatctga aagacaggtt gctcagatgt 1980
   atacaaatga atttattctc ttgacctcta gttcagatgt cagaaatatt ttgtactaaa 2040
   acagagatag gttcctatat tggaatatgt tcaatattta ctacctttct cttctattct 2100
   ctatccataa aactctgcct gattaaaata ttttaaagtg tactttttac ctgatactgt 2160
   taatagcatt gatgatcctg gattttaaag atggctgctt tagtctactt tgattttggg 2220
   aaagccattt taaacaaaca aatatgtggt atcatttaaa gttttataaa gaattaagag 2280
   taactgaatg acagctgaat ttctcatgta tttattgact gcctcctagg ttcaaagttc 2340
   tgtgctgcat gcagacatat caaaatttct tatatctttg gatcatgaat gtcagggtct 2400
   ttcagctcct ggtcagaaaa aatactgtca tgtcagagtg aggaattctt agtatgaggt 2460
   ggtgctgggg agtaccagta gcttcagttc aaaaattcat tgctctgggt cccaacagct 2520
   ttgttccaag tctacatctt aacagtatgt ggaaggattg gttcaggctt cctttatatt 2580
   ctacatagat cttgatttgg gatttttagg aaccaatagc ttaagctatg ttagaacaat 2640
   ccaaaagctt taaaaattgt cagagcaaac cctatagcaa accctatcta gaccttaaac 2700
   cttggcgttg ataactgaga atttaaagac tctggaggca gtgttttgtt cctgtggggg 2760
   agaggccagg tcaacttgca gagtatctcc cagagtctgg tatcaagtgt cttcactccc 2820
   tgtcacatca cttttttccc aacactttca ttatccatat tttgctctgc tattccaaat 2880
   gtgtagtctc taagaataac ctgaacacnc caggttattc tctccctcga caaattctga 2940
   gtgcatgatt taatacaaaa acaatttgaa taagagattt ttatcttgct acattttgaa 3000
   aagcccttac agatacagtt ttaaatatgt aattgaaatg tggcattttc ttgagtattc 3060
   aatacatttc ttggattttt ttttcctaac tgcaggaaca gcatacataa aaacgctcgg 3120
   gtcctgaagc aattctttaa gcacagcaaa cgttgagtca nntggctttc actccatgtt 3180
   tataaaaggc aatgccacca tcataagttt ctcaataatt cctatcactt ntaagcattt 3240
   tcatgtatta atctcaatat attttaatag catacatatg gtctcaaaaa accctttcct 3300
   ggggcttact gttggctttt tattccccca gatcttctta aaatcttagc ctatacatga 3360
   cttagaacat gtttttaaca agttatatga aaatctcctt ttgtgctcca cattgacaga 3420
   gcctgtctga atcccagaga aggcaatcac atcctgttat gttgtcagtg tgagaggcaa 3480
   aagctccaaa gtactaagtt cctagagctg taaatccatt gtgcttcatg ttataaataa 3540
   agaaattgcc ctgtgacagc tacagtttgg aagcattatc atttctcttt tcanctctca 3600
   cttttaatct tttcagttat tttccttagt aaaacttagc caccaaagta tgacttcttg 3660
   tgtcacaaac ctatatgcca caattaaaag aaagaaattc cagccaacca tgtcagtggc 3720
   tgtagtcagc atttaagaca aaactgactc ctatcgattt agcagaagag agttctccct 3780
   gccccgtgtc tccttccatc ctgccgctcc ctgccctctc cagccttttg ggcaagcctt 3840
   ttcttcctga tgcattgcga atgtgtgatg tacgctttct agcagatgca tttgaggtta 3900
   ggtcttcgca accatgaaaa cacagttata aattgttctc ggagctaacc cactcaacat 3960
   cttggcactt ggttgttttt aacttttcaa caataggcac tgttcactcc cctccatctc 4020
   agttctttat tcagtgatca ggcaggacac tgatgaaagg ctagtcacgg cttcccccca 4080
   tggtatcagt gtggtggcat gtgatgattc acatggggtg tgtttgatcc gaggaagccc 4140
   agtctctgct tattcttcct ttgttctgtc atctgctcct tgagctccgc cagctgaccc 4200
   gacagctgtt tgaccagact catggtcgat tccaacttct cctgaaggct ccgaatttca 4260
   ttttgctcac tgtcgccttc attgctaacg agggacatgg ctcgcatccg aggaaaccaa 4320
   tccaaattct tctccacaat catttgagcc acataacttt caggtccagt gtattctgtt 4380
   gngtctttaa ctttcaccag gactatgaag tacaaataat gccacatatt gtgttctgac 4440
   ttaatgtgct cctcaaatga anccgtttta ttatcaaact tgtctctctc aagtccacag 4500
   atgaaacaag ttgtctttag aatttcttct tttttctgtt tttcgcttct gagatcagca 4560
   aaagtatcga tgataacacc aaaaatcaag ttcagaacaa taatgataac aatgaaataa 4620
   aaaagaaggt cataaaccac tcgggcagca aacaanggct catctttcga tggccttctt 4680
   agcacatccc ccacaccacc gccattcntg aggccctggt tcagcacggt gacaatgcac 4740
   ataaggagag tgtcacacgt cctttcaatt ccatcttcat actcttcatc agctgtattt 4800
   gaagctggaa ttgtgggtga acagttctcc ttggcacatg cttccatcat ggtagttaaa 4860
   gtcatagtag gcacttgatg actgcctgta acaggagttc ggtttttcag cctatcaact 4920
   tccatagtga agtcatcctt caaaaaaagg aacccaataa tggaaaacag gtagacgagg 4980
   atgagagcca ggactgcagt tagaataata gagcggccat ttcgtgtgac actttttatg 5040
   acattcagca aagtctcttc cctgtacacc aaatcaaaaa gcaggaagct atagaagaat 5100
   tcatggacaa aaaggcccag catgcaaacc aggacatacg ccacgtgata gagaaaggcc 5160
   atatccagga tgactgctcg gtacccacgg gtgaacgtgc cacgatttcc aacnaaactc 5220
   accagaaaaa caattttatt acaaagatta gctgcaccaa gaagtattaa tgtaggccca 5280
   agacctattg tatatattga tctgagcatt attgatacaa gaaacggccg aatacccaca 5340
   ggcttggaga agaaaaacag catagatgtg cagatcgcaa ctgctatcca aagaagaacc 5400
   gagaacaatg gagaaagtgt accttcatct ccatcatccc caaatgggta gaagagagca 5460
   acagctaaat tgatgaacac agccaggttg aaggaaatgc tcccccagag agagatgtgc 5520
   ctcgagaacc agaacagtgc agggttattc ctgattttct tctgccactt catttcattg 5580
   tagagatctt ctgtttgctg gaaaaagtca ttcactttac ttccttgttc atccctttca 5640
   gttgtattga acacacggca cttggattct cgagtgaggt attcacatat attggggaca 5700
   ggaaaaacta tttgttccat ggtcctatca tgccggacaa tctcaatctg tgcagtgtgg 5760
   ttggcataat actttaaggc ttcatctcct tcatctggat ccgatcctgg tttgagcatc 5820
   tgctgcaaca gtttattgtg gcgggccaac tgatgggcca gaatatagat attgtgtcca 5880
   acatcttttg gagaaacacc atcatctcca ccctcatcat ccccatggtc acattccaat 5940
   ccttggttat aggcattctt catcacatcc accagttctc tgggtctcat gttaaaaaga 6000
   attctttctg cattctcact gtcatgtctg ctttccataa tggccagcaa aagtttagat 6060
   gcattgttct ttagctggag caccaggtcc attcggtatt taccaagagg gtttatgtca 6120
   ttcagaatca aagcaatgat gatatcaatc ccattagact catgtgtagc gatacaggtc 6180
   tgattttcat ggcaagggcc ctggcaatac tcagtcaagc tctccaggtt ctggttgacc 6240
   agcgctacat tcttctcatt gatgtagaga cccaacaggc ccaggccacc ggttgtactt 6300
   ccacaaatgc agtccagaaa ctgaagggtc tcacagacta ggttgtaatt tgttttgttg 6360
   ttttgattcc tcaagaagtt ctgcaattcc cggttgtgat tctcacacag taactgaaga 6420
   aatctcagta ttggctgcat gatggcaatt gcgggactca ttgttacttc ctctgcggat 6480
   ttttcctcag tgtttcccgc ttctggtcct gtgcacataa tgtctatttc tggatccatt 6540
   tctcttctgt atacacaata tgctttggat gttgctgaag aagcttctgt taattgccct 6600
   ttcattccct cttttaaatg tagtgttgaa tctcttactc tcattcgtgg accagatgtc 6660
   atcaattcat tgtcatcgtc cctttttttg ttacctaaat ctatggtatt aactgtcact 6720
   gttgatctta tttctttctg agcagccttc attcgatcat agagaacttt aaagaatttt 6780
   tctgactttt tttgttcatg caactgctgg tagaaagaat actgtgtttg tgtatttcct 6840
   ccttcaagca aggcaatgcc gaggaaaatg ccttctgaaa aaattctgtc atttttggtg 6900
   ttcactataa catcgatgac aagttctgat gcaccttctt tatccagcag acactgaatg 6960
   tctgacattg atatccccat cttatctgaa tcttgtccag aaaagcttcc tcccacctgt 7020
   gcagttttgg agtaggctcc tgataggtgt ccattcacac caatactata atcaccttta 7080
   aagtatcgat tcagaagtat ctttcttaat gtgttaccct cttccacaaa gctgtctttc 7140
   ttctctaaca tttctcgtaa tgtctgaaga attttaatgc acagtttttc ttctttctcc 7200
   attagtttct ttgtatgatt aatcaacttc gacatgaaag cgccacatct tattcttgca 7260
   tcgcttccct cagggaacag cagttctgga ctgtacaata catcaaccaa cactgagaat 7320
   tcagcctgca tcattgggct gaactggtgc tccaaggagg ccactacatc ctgtaacttt 7380
   tcaataatat ttctgtaatc ccaagcaggc cctccaagag cttccttaaa gcgtggccca 7440
   gagcgagctg atagtctcca acccattgct gctctctgca ccatatttga atggctcttc 7500
   atgaaaagag tattaacttg gctgtccaaa tccactggaa tggcaattcc acgatttttt 7560
   gccacttcag ccaaagttct gatacaggat tccactgagg ctttctgcgc tgggtttggc 7620
   caggtgcaat tgtaaattct gaaggcagat tgcagtagct gaataaaaac tggctgatgt 7680
   gtctggaggc tggtactatt gtctgaaaag ggagaattaa agaagccgct cacaatattc 7740
   attattgact cagtaacaca cttttccaaa aagatgtctg catgtttcct gtctgtagtt 7800
   gtgttgcaaa cccttgccat atccaccaag aagttctcaa ataatttcca aatgtggtta 7860
   cttgtataga tttctttcat ttccacttca gtgtcaacat aacagtgatt aacaaagttc 7920
   acataagcaa ttttaacctc agggatgcag tcgtcatggg tcaccaccct cactatgtcg 7980
   tccagcggga gaagggaatt acacttgatt tcagtgtaga catttttccc ctctgtgcat 8040
   gctgccagca actccaccag ggtgatgtgg taggctaagg ggccactctc atcccctcgg 8100
   tctctctctg aacacatcat atggagaagg attggaaatg atgctctatc attgtaaaat 8160
   atcagcacgt cttcaccccc atttatcaac tctgtcatta ccatatcctg gcatttcttc 8220
   acatatttac catctgcttt tacaattgtt tgcaaaaacc tcaggtactc cacgtggcgg 8280
   ccatgtgtct caatgcagtg cacaaanngn tgtacaantc tctcgctaat ttcgtngcac 8340
   agatggtaat ngttcatgaa gatgtgccgc atggtttctg cttcaaggag acctggagtt 8400
   aaaaacaaat tcagatgttt atgaagaaga acttgattct gtggatttcc tcgacagaaa 8460
   ttctgcagaa atgtatgggc tagattcatt acttcattca tcttttcatc attcttttca 8520
   tagggtatct gcagaagatc caacaccacc gaatgcgccc ccatattttt cagtaatcgt 8580
   tgatgttgat tccgacactt tttattctgc acacagagtt tacttagcct gatcaaaatc 8640
   tcctttacaa tccggtantt attgctcttg ttgctgtcaa tctgaggttt ctttgttcca 8700
   tcctgcactg gacttaaaat gtttgattcc tcaattggct cttcaccacc tttcacttga 8760
   ctttccccta tttctccatt ctcatagttg ctgctcttct ccacccatag ctcagacttt 8820
   tctactgtca gtcgaagctg gtctagatct gccttgattt gcttgtagtt atctacgtct 8880
   tgnnnngnna ncagtaattg cacctgctta aatgcctgta aaacctctgc cctctggctg 8940
   aagtgcttaa acaacagctg cagggctcca gacagcaaag gcgngtagtc gtgcatgatc 9000
   agatgaatga ggacccgtaa aaacgtcctg cctccttcat cgtcaagttg aactggattt 9060
   ttttcttttc ttcccgcaaa catagtttct gcctgagctg caatttcatc tatatcagga 9120
   acaatagctg atggtagtaa agtgtctgga gatccactgg cagatgtctc cgcattgtca 9180
   ttgtcctctc cnaactcctt ctnatatatt gacagcatat atgagatcct ataatccagt 9240
   ctgacactca ggataaactg caaaatctca atnntntnna nnnnngngtc catcacagtc 9300
   acatcctcnt gctcggtggg gctcccttgc ttgctcgggt ggatgctggg tggcacatcc 9360
   ggcacgctca tggggaagat ggagcctcta ctgagtacca tctgggtcat catctctccc 9420
   accccatgaa tggttctcat cacattgttt cctccatctt gaaatttgct taatctttca 9480
   aagtatgatg acatgggggc ctgtacaatg tctaaaatag ccagaagtgt tcttgttagc 9540
   cttaataact cactgaaact ataaaatcca aagtatataa gattccgagc caagtggacc 9600
   acctcaaatg tcagtttatt tttttcttta tccccaaaag gaaagggctg gtttacaact 9660
   tctttcaaat attcttcaac aaattccatt gtcagggnna atttcctctt catatcattt 9720
   ctggaagagt ctgttataga atcatattca tgaattgtga tctttgtggg gatttctgtc 9780
   cagagcctgg catagcgaac aggcaccacg gactcctggg gatcccggtc aacgtgcatg 9840
   tggagcatga ggcgacagaa ggacgctcgg aggtcgaacg gcaggctctc atccgacaca 9900
   caccgcagga tcaggtctac agacagctgt gtagaaatct ggtttatggc cagatactgg 9960
   cgatccaagc acatccttgc aaagaggttt agctggtacc tgtaataggt aagaacttct 10020
   aagtcagctt tggtgccttc ttttgcctct tgagcaaggt gcctgatagc tttgccatga 10080
   ggttccttgt tgctgtcaat ccaatagagc caaacttctt catcatcaat gtcatctgaa 10140
   aggatggagc tctccatggg gttgtctgct tgcattgaga ccaccttagt ttgaatgaga 10200
   atgtctgcat tgcctggact caacataaat ttacagatga gttcttgagt tacagggata 10260
   gcagtggtat tagacacaca cagatctgac aaataatcca aaaaccttgg ctcccgattt 10320
   ctcctgagta aactgacaaa tgtttctatt tcttttgctg tgatatgttt ctctagtagt 10380
   tttctgttgt tgtgcaacaa agntgtgata gtatcttctg ccaaaatatc atagccaatc 10440
   tgggactgca tgacacagaa attcttagca atatattcct gatttttccg gtaatcctgc 10500
   tgcgagtgtc tcaggacgcg gtaacagagc cgcagcatgt acttgtaggg tgcatatctt 10560
   tgatccccca gatcttcaag tctcagcatc gagccttctc ctgctttctc tttaaagggt 10620
   gctttaagaa ttccaaatac ctgtgccagt atgttttgtt ccctcatcaa tttttgacgc 10680
   tctcggtttg gcttagtgat aaccacatcc agaacttctt gtccattatt aggcacatca 10740
   gcaacaaaga atatgagatc ttccaataat ttggttacaa acctcctttc attctgagtt 10800
   attgtgccgt tttctagctt tttaactgtg gtcgctagta ctttattggc atcattggca 10860
   aagtctaagt ctcgaacttc agacagtgga acagacacga ttgcgaacgc ttctttatct 10920
   tctttggttt ggcaggttcc aatctttaac ataacaggcc tctcttcatc tgtgtctatg 10980
   gggatactag tactggttac ccatgtgttg gtgcataaat gccttaaccg aacatatgag 11040
   ttccttggaa ccaggcagtc agctctctga agagttgtgg catctagttc aaaaagggat 11100
   gcaatgtcat tgccatgcgg gactgaaacc aaagtataca tnatcttctc ccctgcctgg 11160
   cgttttttct ttgaagttgg agggactcca tctctcacat tttttccttc attttgggca 11220
   tctcgataat caggattaag ctctgcagct aaatagtttc cagttgcaag atgcttaaat 11280
   ctgaacaagc tgttccactg tcctgcaccc ccacggcatg ggtcatgatg aaccacctct 11340
   atttcccaga gtgctttaga actagtagca gaagtagctg ntngnnncaa ggtcgtacga 11400
   aggaaaatgt gctgtttttt ctcatattca tcacaagtca aaaacttctc ttgttccgca 11460
   tgaaataatc taacaacgtc ccctcctttt aatacatcct ctcgatagga actatatttc 11520
   atgaataaag tgattttcca gctggtgttg caattgacag cattcacctc tttacaccct 11580
   gggttatcaa gaagctctat gttgctggca tgtagtggct gccctgcatt cacaggcatc 11640
   aaaacaactt tatctcctac aacaatattg tcaccctcgc ttctcagttt ccagaatgga 11700
   tgaatataaa accaagaccc ttcatttcct gcagcatcca aggacacacg catggcattc 11760
   ttctccagta aagcaggtaa tctcttgttg acagtaagat atttgttgct ttttatatgc 11820
   agtagttgta taacattact gtattttaca atttctccca acagtttctt attctccgat 11880
   tcattttgtt tttgttccag ttctgcagcg tgctgtagtt tcttcagcaa ggctgcctcg 11940
   gtgtggttcc cttgtttggc ttgctttgct ttccaatatt gcttctgggc agaatatctg 12000
   ttcatagggc acaccttgaa aaggcagtct ctgaacttct tgggagggtt ggcaaggtcc 12060
   ccggcctctg ggtgcaccac acatctgtca tccactaacc ccaaggtgct gatgaagccg 12120
   ttgaccgagc cctccgcgta cagggacacg atgtccccta tgtagaggaa gctggacatt 12180
   ttctcagtca tgctgcttca tgctgcttca tgttccacag tggacgtccc tcttcttccc 12240
   tgcgccctcg ccgccctctc tccagggagc cgccgcggca gaagcggatc ggatcgcggg 12300
   actacagcgg ccaagagcca cggcggaggc acggcccgag ccactgagcg tcgcgctcag  12360
   ccgtggtgcg cgccggggaa gccagaccgg ggccaagcgc cagctcggga caccccgcga  12420
   agaggcagcc caggcgccca gagaagccgc agccgccgcc cgctccccgg caggtttcct  12480
   gttcctttct gaagttttct ctccaacacc tttgctcctc ctcctcctcc ttccctctcc  12540
   gctcccacct cgtgtccact ccctgctctg cgaccgctcg gccgtcacag cgccggcggg  12600
   agctggaag                                                         12609
<210> 24
   <211> 3976
   <212> DNA
   <213> Homo sapiens
<400> 24
   gaattccggg cccccggccg tccctcttaa ggctcttcct ccgctccgcc agtgtccggc      60
   cgcgggccgg ccttagtgac tggggcggcg ggccccgggg ccgcggcgtg gggcgggcag     120
   gcggacgccg gccgcgggct gctttcgtcg gctcccaagc tctcccggag cgagcagccg     180
   cccgcgagcc gccgcggagc ctcctcgccc gctcccgccg gcgagcaaga tgatgtgcga     240
   ggtgatgccg accatcagcg aagcagaagg cccccctgga ggaggtggag gccatggttc     300
   cggctcccct tcacagccag atgcagattc acattttgaa cagttgatgg tctccatgct     360
   agaagaaagg gaccgccttc ttgatacact gagagagact caagaaacgc tggccttaac     420
   ccaggggaag ttacacgagg ttggtcatga aagagattcc ttgcagagac agctcaacac     480
   ggcacttcca caggagttcg cagcacttac taaagaactc aatgtatgca gggaacagct     540
   ccttgaaagg gaagaagaaa ttgctgaact gaaagcagaa aggaataaca ccaggctgct     600
   gttagagcat ttggaatgcc ttgtctccag gcatgagcgg tctcttagga tgaccgtggt     660
   gaagagacaa gcgcagtctc cagcaggcgt gtccagcgaa gtggaagtgc tgaaagcact     720
   gaagtcctta tttgaacacc acaaagctct ggatgaaaag gtgagagagc gattacgagt     780
   agcacttgaa agatgtagtt tgttagaaga ggaattaggt gccacacaca aagagctaat     840
   gattcttaaa gaacagaata atcagaaaaa aactctaaca gatggagtgc tggacataaa     900
   ccatgaacaa gaaaatacac caagcacgag tggaaagaga tcttctgatg gttctttaag     960
   ccacgaggaa gaccttgcta aagtaattga gctccaagaa atcataagta agcagtcaag    1020
   ggaacagagc caaatgaaag aacgcctggc ttccctttcc agtcatgtga cagaactgga    1080
   agaggatctg gacacggcta gaaaagatct catcaaatct gaagaaatga acacaaaatt    1140
   gcaacgagat gtccgtgaag ccatggccca aaaggaagat atggaagaga gaatcactac    1200
   tcttgaaaaa cgctacctcg ctgcacagcg tgaagccaca tctgtgcatg acctcaatga    1260
   taaacttgaa aatgaaattg caaataaaga ttctatgcat cgacagactg aagataaaaa    1320
   ccgccagtta caggagcgct tggaattggc agagcaaaag ctgcaacaga cactgaggaa    1380
   ggcagagacg ctcccggagg tggaggcgga gctggcccag agggtggcag cgctttccaa    1440
   ggctgaagag agacacggca acattgaaga aaggttacga cagatggaag cacagttgga    1500
   ggagaagaat caagaactgc agcgggcaag gcaaagagaa aaaatgaacg aagaacataa    1560
   taaacgttta tcagacactg ttgacaagct gctttcagaa tctaatgaga ggcttcaact    1620
   tcatcttaaa gagagaatgg ctgctttgga agataagaac tctcttttaa gagaagttga 1680
   aagtgcaaaa aagcagttag aagaaacaca acacgataag gatcagcttg tcctaaacat 1740
   tgaagcactg agggctgaac tagaccacat gagactaaga ggtgcttcac ttcatcatgg 1800
   ccgaccccac ttgggcagtg tcccagattt caggttcccc atggcagacg gccacacaga 1860
   ctcctacagc accagtgcag tgctgcggcg cccacagaaa ggccggctgg cagccctgcg 1920
   agatgagcct tccaaggtac aaactcttaa tgagcaggat tgggaacgtg cccagcaagc 1980
   tagtgtcttg gcaaatgtag cacaagcatt cgagagtgat gctgacgtgt ctgatggtga 2040
   agatgacagg gacactctcc tcagctcagt tgacctgcta tcgcccagcg ggcaggccga 2100
   cgcgcacaca ctagccatga tgcttcagga gcagctggac gccatcaaca aagagatcag 2160
   gttgattcag gaagaaaaag aaaatacaga gcagcgggca gaggagattg aaagtcgagt 2220
   tggcagtgga agtctagaca atcttggtcg ttttagatca atgagctcca ttccccccta 2280
   ccctgcttcc tcgcttgcta gctcctcccc tccgggcagt gggcgctcca ccccacgaag 2340
   gatccctcac agcccagctc gggaagtgga cagactgggc gtcatgaccc ttttgccacc 2400
   ttccagagaa gaggtacgag atgacaagac aaccataaag tgtgaaacct ccccgccttc 2460
   ctccccgaga gcccttcggt tagaccggct gcacaaaggg gcgctgcaca ccgtcagcca 2520
   cgaggacatc agggacataa ggaactccac aggctcccag gatggtcccg tgagcaaccc 2580
   cagcagtagc aacagtagcc aggactcgct ccacaaagcc ccaaagaaga aaggcattaa 2640
   gtcctccatt ggccgcttgt ttggcaagaa agaaaagggc cgacctggac aaactggcaa 2700
   agaagcatta ggacaagctg gtgtttccga gacggataac tcatctcagg atgccttggg 2760
   acttagcaaa ttggggggac aggctgaaaa aaatcgtaaa cttcaaaaaa agcatgaatt 2820
   gctggaggaa gcccggagac aaggtttacc ttttgcccaa tgggacgggc caacggttgt 2880
   ggtctggcta gagctctggg ttgggatgcc agcctggtat gtggctgcct gccgagcaaa 2940
   cgtgaaaagc ggggccatca tgtcggccct gtccgacaca gagatccagc gtgagattgg 3000
   catcagcaac cccctgcaca ggctgaagct gaggctggcc atccaggaga tcatgtcgct 3060
   gaccagcccg tctgccccgc ccacatctag aacgacactc gcctatgggg acatgaacca 3120
   cgagtggatc ggcaacgagt ggctccccag cctgggcctc ccccagtacc gcagctactt 3180
   catggagtgc cttgtagacg ccaggatgct ggaccacttg accaagaaag accttcgagg 3240
   gcagctgaaa atggtcgaca gttttcacag aaacagtttc cagtgtggaa ttatgtgcct 3300
   gagaaggtta aattatgacc ggaaagaact ggaaagaaaa agagaagaaa gtcagagtga 3360
   aataaaagac gtgcttgttt ggagcaatga tcgagtgatt cgctggatcc tgtcaattgg 3420
   ccttaaagaa tatgcaaaca atcttataga gagtggtgtt cacggagcac ttctggcctt 3480
   agatgaaacc ttcgacttca gtgcactggc actgctgtta cagatcccga cgcagaacac 3540
   acaggctcgt gctgtcttgg aaagagaatt taacaacctt ttggtcatgg ggactgatag 3600
   aaggtttgat gaagatgatg ataaaagctt taggagagca ccttcatgga gaaaaaagtt 3660
   tagaccaaag gacattcgtg gcttagctgc tgggtcagca gagactctcc ctgcaaactt 3720
   ccgggtgact tcttctatgt cttccccctc tatgcagcca aagaagatgc agatggacgg 3780
   tatgtgatgg gtcacactaa cctgtcactt gttgggagca tgagcagctt tctgtctgga 3840
   acattaataa tgatctaaaa cggcctattt aatatgttac aaggcacttg agtatggttg 3900
   catgtccaaa tataaatgtt tttaaattaa ctctaacatt tgtttataaa agtttaacca 3960
   taataataga attttt 3976
<210> 25
   <211> 1579
   <212> DNA
   <213> Homo sapiens
<400> 25
   cagctgaggc gttctccgct tagtgcctac ggcatgggtc agctctttct gccacctccg 60
   ccttcgcggt cctcacctct ttgcgggtga agttaccttt tgggtttcca gaccttgggg 120
   gcctgaggca aactcgggga cgaaccccta acccacctct cttttctgtc attctccaac 180
   tcttactttg taccacggaa tcacttggcc ttaaagctag ccagctcggc cgtgccttga 240
   ggccaggttc gagagataaa ggtaaccgtg gggaaagcgg ccccccagtg gacaaaggcg 300
   gaggcaagaa tagagcaggc ctgagggcca taggcgatga gaataggcag ttgaggggtg 360
   aaaaagaaaa caaacaaaca acaacaacaa accctgaaga gtactgaaga tttagaaggg 420
   actggaaagg acttgttgcg caatggaaga atctgactct gagaaaacga cggagaaaga 480
   aaatctgggg ccgagaatgg atccaccact aggggaaccg gaaggatcgc ttgggtgggt 540
   gctaccaaat acagccatga agaaaaaggt gctgttgatg ggtaaaagtg ggtctggtaa 600
   gaccagcatg aggtctatta tctttgcaaa ttatattgcc agagacacac gtcgccttgg 660
   cgcaacaata ctagaccgta tacatagtct tcaaattaat agcagtttga gcacctactc 720
   tctcgtagac tctgttggaa atacaaagac atttgatgta gaacattctc atgttcgatt 780
   tctgggaaac ctggtattga acctgtggga ttgtggtggg caagacacct tcatggaaaa 840
   ttatttcact agccaacggg acaacatctt ccgaaatgtg gaggttctga tttatgtctt 900
   tgatgtggag agccgcgaac tggaaaagga catgcactat taccaatcat gcctggaggc 960
   cattctgcag aattctccag atgccaaaat attttgcttg gtacacaaaa tggatctggt 1020
   acaggaggat caacgggacc tgatttttaa agagcgagaa gaagatttga ggcgtttgtc 1080
   tcgcccattg gaatgttctt gtttccgaac atctatctgg gatgaaaccc tctataaggc 1140
   ttggtccagc atagtttatc agctgattcc caatgttcag cagctggaaa tgaacctaag 1200
   gaattttgct gaaattatcg aagctgatga agtacttctt tttgagagag ctacttttct 1260
   ggtaatttct cactatcagt gtaaagagca gcgtgatgcc catagatttg agaaaataag 1320
   caacattatt aagcagttca agctgagctg cagcaagctg gctgcctctt tccagagtat 1380
   ggaagtcagg aactctaact tcgctgcttt cattgacatc tttacatcca acacttatgt 1440
   gatggttgta gtgtctgatc cgtccattcc ttctgcagct actctgatca acatccgcaa 1500
   tgccaggaaa cactttgaaa agctggaaag agtggatgga ccaaagcagt gtcttctcat 1560
   gcgctaaaca ttagtagta 1579
<210> 26
   <211> 7017
   <212> DNA
   <213> Homo sapiens
<400> 26
   cctcgctccc tctccggtaa tgagggggct gagctgtccc tccgaggagg gggcctggtg 60
   tggataaaag agacgaaaaa gccgggggag gtttccaaaa ataaaaccgt ccgggtcccc 120
   ttcagacggc tgcaggcaca gggaggaggc gcgaaggtgc agcagccgtg cgagcccagc 180
   tggagtagga gcgcggactc gaggctcggg gcgcgcagcc ctcgttccgc cgagagccgg 240
   gcccccagtc ggccgcttca gggcccccta gactcagaga agctggccgc cgggcggggc 300
   cgggagaaca gcccgcgggc gtccagcgtg ccgaccacaa agctcttcgc ggtgcccgcg 360
   cgcaccactc tccagccgcc ccgcgccatg aggccgcgga ccaaagcccg cagcccgggg 420
   cgcgccctgc ggaacccctg gagaggcttc ctgccgctca ccctggctct cttcgtgggc 480
   gcgggtcatg cccaaaggga ccccgtaggg agatacgagc cggctggtgg agacgcgaat 540
   cgactgcggc gccctggggg cagctacccg gcagcggctg cagccaaggt gtacagtctg 600
   ttccgggagc aggacgcgcc tgtcgcgggc ttgcagcccg tggagcgggc ccagccgggc 660
   tgggggagcc ccaggaggcc caccgaggcg gaggccagga ggccgtcccg cgcgcagcag 720
   tcgcggcgtg tccagccacc tgcgcagacc cggagaagca ctcccctggg ccagcagcaa 780
   ccagcacccc ggacccgggc cgcgccggct ctcccacgcc tggggacccc acagcggtct 840
   ggggctgcgc ccccaacccc gccgcgaggg cggctcacgg ggaggaacgt ctgcggggga 900
   cagtgctgcc caggatggac aacagcaaac agcaccaacc actgtatcaa acccgtttgc 960
   gagccgccgt gccagaaccg gggctcctgc agccgcccgc agctctgtgt ctgccgctct 1020
   ggtttccgtg gagcccgctg cgaggaggtc attcccgatg aggaatttga cccccagaac 1080
   tccaggctgg cacctcgacg ctgggccgag cgttcaccca acctgcgcag gagcagtgcg 1140
   gctggagagg gcaccttggc cagagcacag ccgccagcac cacagtcgcc gcccgcacca 1200
   cagtcgccac cagctgggac cctgagtggc ctcagccaga cccacccttc ccagcagcac 1260
   gtggggttgt cccgcactgt ccgacttcac ccgactgcca cggccagtag ccagctctct 1320
   tccaacgccc tgcccccggg accaggcctt gagcagagag atggcaccca acaggcggta 1380
   cctctggagc acccctcatc cccctggggg ctgaacctca cggagaaaat caagaagatc 1440
   aagatcgtct tcactcccac catctgcaag cagacctgtg cccgtggaca ctgtgccaac 1500
   agctgtgaga ggggcgacac caccaccctg tacagccagg gcggccatgg gcacgatccc 1560
   aagtctggct tccgcatcta tttctgccag atcccctgcc tgaacggagg ccgctgcatc 1620
   ggcagggacg aatgctggtg ccccgccaac tccaccggga agttctgcca cctgcctatc 1680
   ccgcagccgg acagggagcc tccagggagg gggtcccgcc ccagggcctt gctggaagcc 1740
   ccactgaagc agtccacttt cacactgccg ctctccaacc agctggcctc cgtgaacccc 1800
   tccctggtga aggtgcacat tcaccaccca cccgaggcct cagtgcagat ccaccaggtg 1860
   gcccaggtgc ggggcggggt ggaggaggcc ctagtggaga acagcgtgga gaccagaccc 1920
   ccgccctggc tgcctgccag ccctggccac agcctctggg acagcaacaa catccctgct 1980
   cggtctggag agccccctcg gccactgccc ccagcagcac ccaggcctcg aggactgctg 2040
   ggccggtgtt acctgaacac tgtgaacgga cagtgtgcca accctctgct ggagctgact 2100
   acccaggagg actgctgtgg cagtgtggga gccttctggg ggtgactttt gtgtgcccca 2160
   tgcccaccca gaccagcctc cccggtgatt gagaatggcc agctggagtg tcctcagggg 2220
   tacaagagac tgaacctcac tcactgccaa gatatcaacg agtgcttgac cctgggcctg 2280
   tgcaaggacg cggagtgtgt gaataccagg ggcagctacc tgtgcacatg cagacctggc 2340
   ctcatgctgg atccatcgcg gagccgctgt gtgtcggaca aggcaatctc catgctgcag 2400
   ggactgtgct accggtcgct ggggcccggc acctgcaccc tgcctttggc ccagcggatc 2460
   accaagcaga tatgctgctg cagccgcgtg ggcaaagcat ggggcagcga gtgtgagaaa 2520
   tgccctctgc ctggcacaga ggccttcaga gagatctgcc ctgccggcca cggctacacc 2580
   tacgcgagct ccgacatccg cctgtccatg aggaaagccg aggaggagga actggcaagg 2640
   cccccaaggg agcaagggca gaggagcagc ggggcactgc ccgggccagc agagaggcag 2700
   cccctccggg tcgtcacgga cacctggctt gaggccggga ccatccctga caagggtgac 2760
   tctcaggctg gccaggtcac gaccagtgtc actcatgcac ctgcctgggt cacagggaat 2820
   gccacaaccc caccaatgcc tgaacagggg attgcagaga tacaggaaga acaagtgacc 2880
   ccctccaccg atgtgctggt gaccctgagc accccaggca ttgacagatg cgctgctgga 2940
   gccaccaacg tctgtggccc tggaacctgc gtgaacctcc ccgatggata cagatgtgtc 3000
   tgcagccctg gctaccagct gcaccccagc caggcctact gcacagatga caacgagtgt 3060
   ctgagggacc cctgcaaggg aaaagggcgc tgcatcaacc gcgtggggtc ctactcctgc 3120
   ttctgctacc ctggctacac tctggccacc tcaggggcga cacaggagtg tcaagatatc 3180
   aatgagtgtg agcagccagg ggtgtgcagc ggggggcagt gcaccaacac cgagggctcg 3240
   taccactgcg agtgtgatca gggctacatc atggtcagga aaggacactg ccaagatatc 3300
   aacgaatgcc gtcaccccgg tacctgccct gatgggagat gcgtcaattc ccctggctcc 3360
   tacacttgtc tggcctgtga ggagggctac cggggccaga gtgggagctg tgtagatgtg 3420
   aatgagtgtc tgactcccgg ggtctgtgcc catggaaagt gcaccaacct agaaggctcc 3480
   ttcagatgct cttgtgagca gggctatgag gtcacctcag atgagaaggg ctgccaagat 3540
   gtggatgagt gtgccagccg ggcctcatgc cccacaggcc tctgcctcaa cacggagggc 3600
   tccttcgcct gctctgcctg tgagaacggg tactgggtga atgaagacgg cactgcctgt 3660
   gaagacctag atgagtgtgc cttcccggga gtctgcccct ccggagtctg caccaacacg 3720
   gctggctcct tctcctgcaa ggactgcgat gggggctacc ggcccagccc cctgggtgac 3780
   tcctgtgaag atgtggatga atgtgaagac ccccagagca gctgcctggg aggcgagtgc 3840
   aagaacactg tgggctccta ccagtgcctc tgtccccagg gcttccagct ggccaatggc 3900
   accgtgtgtg aggatgtgaa tgagtgcatg ggggaggagc actgcgcacc ccacggcgag 3960
   tgcctcaaca gccacgggtc tttcttctgt ctgtgcgcgc ctggcttcgt cagcgcagag 4020
   gggggcacca gctgccagga tgtggacgag tgtgccacca cagacccgtg tgtgggaggg 4080
   cactgtgtca acaccgaggg ctccttcaac tgtctatgtg agactggctt ccagccctcc 4140
   ccagagagtg gagagtgtgt ggatattgac gagtgtgagg actatggaga cccggtgtgt 4200
   ggcacctgga agtgtgaaaa cagccctggc tcctaccgct gtgttctggg ctgccagcct 4260
   ggcttccaca tggccccgaa cggagactgc attgacatag acgagtgcgc caacgacacc 4320
   atgtgtggca gccacggctt ctgtgacaac actgatggct ccttccgctg cctctgtgac 4380
   cagggcttcg agatctctcc ctcaggctgg gactgtgtgg atgtgaacga gtgtgagctt 4440
   atgctggcgg tatgtggggc cgcgctctgt gagaacgtgg agggctcctt cctgtgcctc 4500
   tgtgccagtg acctggagga gtacgatgcc caggaggggc actgccgccc acggggggct 4560
   ggaggtcaga gtatgtctga ggccccaacg ggggaccatg ccccggcccc cacccgcatg 4620
   gactgctact ccgggcagaa gggccatgcg ccctgctcca gtgtcctggg ccggaacacc 4680
   acacaggctg aatgctgctg cacccagggc gctagctggg gagatgcctg tgacctctgc 4740
   ccgtctgagg actcagctga attcagcgag atctgcccta gtggaaaagg ctacattcct 4800
   gtggaaggag cctggacgtt tggacagacc atgtacacag atgcggatga gtgtgtgata 4860
   ttcgggcctg gtctctgccc gaacggccgg tgcctcaaca ccgtgcctgg ttatgtctgc 4920
   ctgtgcaatc ccggcttcca ctacgatgct tcccacaaga agtgtgagga tcacgatgag 4980
   tgccaggacc tggcctgtga gaatggcgag tgcgtcaaca cggagggctc cttccactgc 5040
   ttctgcagcc ccccgctcac cctggacctc agccagcagc gctgcatgaa cagcaccagc 5100
   agcacggagg acctccctga ccacgacatc cacatggaca tctgctggaa aaaagtcacc 5160
   aatgatgtgt gcagcgaacc cctgcgtggg caccgcacca cctacacgga atgctgctgc 5220
   caggacggcg aggcctggag ccagcagtgt gctctgtgtc cccgaggagg ctctgaggtc 5280
   tatgctcagc tgtgcaacgt ggctcgcatt gaggcagagc gggaggccgg ggtccacttc 5340
   cggccaggct atgagtatgg ccccgggccc gatgacctgc actacagcat ctatggccca 5400
   gatggggccc ccttctacaa ctacctgggc cccgaggaca ccgtccctga gcctgccttc 5460
   cccaacacag ccggtcactc agcggaccgc acacccatcc ttgagtctcc tttgcagccc 5520
   tcagaactcc agccccacta cgtggccagc catccagagc cccagccggg cttcgaaggg 5580
   cttcaggcgg aggagtgcgg catcctgaac ggctgtgaga atggccgctg tgtgcgcgtg 5640
   cgggagggct acacctgtga ctgttttgag ggcttccagc tggatgcggc ccacatggcc 5700
   tgcgtagatg tgaatgagtg tgatgacttg aacgggcctg ctgtgctctg tgtccatggt 5760
   tactgcgaga acacagaggg ctcctaccgc tgccactgct ccccgggata tgtggctgag 5820
   gcagggcccc cccactgcac tgccaaggag tagcagtcag gggtcagtgt ggcaactacc 5880
   tggaaatggc ctccagtcac aggcaggggc cttgaggatg atttcctagc tgggaagaca 5940
   ccgtgacatc aggccagagg tttccaatca gccttgcctg ctttcatctc tcccagctta 6000
   gcctctggct gtaagcttcg gtcattgcct ccatgccctt gcttggctca agcaccacca 6060
   atcgctttaa tgcttcagcc accgcatgag gccctgtcca ccacctttcc tggccttgct 6120
   atgggatgct taccaaagga tggccctcat ccaccctccc aagctgtgcg agcatgcaag 6180
   gccccatggc ctcacactgc agacacccct ttccagccac aatccaccat catcctgacg 6240
   atcccacaac tgggacagag gctacatctg ccctagggag gtccttcaga atctgtggag 6300
   caagaaagga tttggggaag cttggggact gactccagag ccccctccta agaaccatca 6360
   ccaccactca gccaatctgt tctgggccct gattttgcca cacctccatc ctgtagccca 6420
   ttctctgacc ccaaggagtg gcagaagatc ccttcactca gagaagcaag gctgatatta 6480
   gcttgttgaa tgtaagagac acaaatgaag aagaacaaag agcctgagaa agcagcaaga 6540
   ggacatgatg aaaaatacgt ggagttgatg agaaagggga gccaaggctt tatacgtcta 6600
   aagaaaatat tcagtagctg aatccgccca gtgatagcct gtgggcacca gcagcaaggg 6660
   ctgccatggg atacagcacc catctacaaa gacctctatt acataaacac tgcttcttac 6720
   aggaaacaaa cctcttctgg gatctccttt tgtgaaaacc agtttgatgt gctaaaagta 6780
   aaaagtctat tttccagtgt ggtcttgttc agaagcagcc agatttccaa tgttgttttt 6840
   cccctccact cagaaacccc tgccctttcc cttcagaaaa cgatggcagg cattcctctg 6900
   agtttacaag cagagactca ctccaaccca aactagctgg gagttcagaa ccatggtgga 6960
   ataaagaaat gtgcatctgg tccaaaaaaa aaaaaaaaaa aaaaaaaaaa aaaaaaa 7017
<210> 27
   <211> 1484
   <212> DNA
   <213> Homo sapiens
<400> 27
   tggctgcgag aagacgacag aagacattag actcaggcac ctctgaaatt gtgaaatctc 60
   ccagaatcga gtgttctaag acaagaagag aaatgcaatc agtggttcaa ctcataatga 120
   caagagacag tgatggttat gaaaactcaa cagatggtga aatgtgtgac aaagatgctc 180
   tggaggaaga ttcagaaagc gttagtgaaa taggaagtga tgaggaatct gaaaatgaaa 240
   ttacaagtgt tggtagagct tcaggtgatg acgatggaag tgaagatgat gaagaggagg 300
   atgaagatga agaggaggat gaagatgagg atagtgagga tgatgataaa agtgacggtg 360
   gccctgatct tgcaaggggt aaaggaaata tagaaactag ttctgaagat gaagatgata 420
   cggcagattt gtttccagaa gaatctggtt ttgagcatgc ttggagagaa ttagataaag 480
   atgctcctcg tgctgatgag attacacgtc gattagtagt ttgtaacatg gactgggata 540
   gattaaaggc aaaagatttg ctggctctgt tcaattcatt taaacccaaa ggaggtgtaa 600
   tattttccgt caagatatat ccttcagaat ttagaaagga gaggatgaag gaagagcaag 660
   ttcaaggacc agtagagcta ttaagtattc ctgaagatgc cccagaaaaa gactggacgt 720
   ctagagaaaa attgagagat tatcaattca aacgactgaa gtactattat gcagtagtag 780
   actgtgattc tccggaaaca gctagtaaaa tttatgagga ttgtgatggc ctggaatttg 840
   aaagtagttg ttctttcata gatctaaggt atataccaga tgatattact tttgatgatg 900
   agcctaagga tgcagcctca gaagtgaatt taacagcata taaaccaaaa tatttcactt 960
   ctgctgcaat gggaacatca acggtggaaa tcacttggga tgagactgat catgaaagaa 1020
   ttacaatgct caacaggaag tttaaaaagg aagagctttt ggacatggat tttcaagcct 1080
   acttagcttc ctctagtgaa gacgaagagg agatagaaga ggagctacaa ggtgatgatg 1140
   gagtcaatgt agaagaagat gggaaaacaa agaaaagtca gaaggatgat gaagagcaaa 1200
   ttgctaaata caggcagctc ttgcaggtta ttcaagaaaa agaaaagaaa ggcaaagaaa 1260
   atgatatgga aatggaaatt aaatgggttc caggtcttaa agaaagtgca gaagagatgg 1320
   tcaaaaacaa attggaagga aaggataaac tgaccccttg ggaacaattt ttagaaaaga 1380
   agaaagagaa aaaaagactg aaaaggaaac agaaggctct tgctgaagag gccagtgaag 1440
   aggaacttcc ctcttggatg ttgatttgaa tgacccatac tttg 1484
<210> 28
   <211> 3237
   <212> DNA
   <213> Homo sapiens
<220>
   <221> misc_feature
   <222> (17)..(17)
   <223> n is a, c, g, or t
<220>
   <221> misc_featuee
   <222> (38)..(38)
   <223> n is a, c, g, or t
<220>
   <221> misc_feature
   <222> (42)..(42)
   <223> n is a, c, g, or t
<220>
   <221> misc_feature
   <222> (44)..(45)
   <223> n is a, c, g, or t
<220>
   <221> misc_feature
   <222> (49)..(49)
   <223> n is a, c, g, or t
<220>
   <221> misc_feature
   <222> (51)..(51)
   <223> n is a, c, g, or t
<220>
   <221> misc_feature
   <222> (55)..(55)
   <223> n is a, c, g, or t
<220>
   <221> misc_feature
   <222> (63)..(63)
   <223> n is a, c, g, or t
<220>
   <221> misc_feature
   <222> (65)..(67)
   <223> n is a, c, g, or t
<220>
   <221> misc_feature
   <222> (69)..(71)
   <223> n is a, c, g, or t
<220>
   <221> misc_feature
   <222> (98)..(100)
   <223> n is a, c, g, or t
<220>
   <221> misc_feature
   <222> (102)..(103)
   <223> n is a, c, g, or t
<220>
   <221> misc_feature
   <222> (105)..(109)
   <223> n is a, c, g, or t
<220>
   <221> misc_feature
   <222> (111)..(113)
   <223> n is a, c, g, or t
<220>
   <221> misc_feature
   <222> (251)..(251)
   <223> n is a, c, g, or t
<220>
   <221> misc_feature
   <222> (261)..(261)
   <223> n is a, c, g, or t
<220>
   <221> misc_feature
   <222> (269)..(264)
   <223> n is a, c, g, or t
<220>
   <221> misc_feature
   <222> (944)..(944)
   <223> n is a, c, g, or t
<220>
   <221> misc_feature
   <222> (952)..(953)
   <223> n is a, c, g, or t
<220>
   <221> misc_feature
   <222> (955)..(955)
   <223> n is a, c, g, or t
<220>
   <221> misc_feature
   <222> (957)..(960)
   <223> n is a, c, g, or t
<220>
   <221> misc_feature
   <222> (962)..(962)
   <223> n is a, c, g, or t
<220>
   <221> misc_feature
   <222> (964)..(966)
   <223> n is a, c, g, or t
<220>
   <221> misc feature
   <222> (1005)..(1005)
   <223> n is a, c, g, or t
<220>
   <221> misc_feature
   <222> (1761)..(1761)
   <223> n is a, c, g, or t
<220>
   <221> misc_feature
   <222> (2023)..(2023)
   <223> n is a, c, g, or t
<220>
   <221> misc_feature
   <222> (2618)..(2618)
   <223> n is a, c, g, or t
<220>
   <221> misc_feature
   <222> (3222)..(3222)
   <223> n is a, c, g, or t
<400> 28
   aaatgtagtg ccacagnaac cctgcacgtg acgcttgngt cntnntggna natanaggcg     60
   cgngnnngnn ngtctaggga atcgaggtgc cggctgcnnn tnncnnnnna nnngcccaga    120
   caggcggtgg gtgggaatgc ctcacttcag tttgaagagg gtccggatcc aaaggggtta    180
   aaacgagcga cccccgatcc ccgaccacac ttcccgcctc cctaaaacgc acaccccgct    240
   agccatgggc ngccgcgacc nccngttcaa agtgctggtg gtgggggacg ccgcagtggg    300
   caagacgtcg ctggtgcagc gatattccca ggacagcttc agcaaacact acaagtccac    360
   ggtgggagtg gattttgctc tgaaggttct ccagtggtct gactacgaga tagtgcggct    420
   tcagctgtgg gatattgcag ggcaggagcg cttcacctct atgacacgat tgtattatcg    480
   ggatgcctct gcctgtgtta ttatgtttga cgttaccaat gccactacct tcagcaacag    540
   ccagaggtgg aaacaggacc tagacagcaa gctcacacta cccaatggag agccggtgcc    600
   ctgcctgctc ttggccaaca agtgtgatct gtccccttgg gcagtgagcc gggaccagat    660
   tgaccggttc agtaaagaga acggtttcac aggttggaca gaaacatcag tcaaggagaa    720
   caaaaatatt aatgaggcta tgagagtcct cattgaaaag atgatgagaa attccacaga    780
   agatatcatg tctttgtcca cccaagggga ctacatcaat ctacaaacca agtcctccag    840
   ctggtcctgc tgctagtagt gtttggctta ttttccatcc cagttctggg aggtctttta    900
   agtctcttcc ctttggttgc ccacctgacc attttattaa gtanatttga anngncnnnn    960
   gncnnntgtc cagtaaggag gcccattgtc acttagaaaa gacanctgga acccatgtgc   1020
   atttctgcat ctcctggatt agcctttcac atgttgctga ctcacattag tgccagttag   1080
   tgccttcggt gtaagatctt ctcatcagcc ctcaatttgt gatccggaat tttgtgagaa   1140
   ggattagaaa tcagcacctg cgttttagag atcataattc tcacctactt ctgagcttat   1200
   ttttccattt gatattcatt gatatcatga cttccaattg agaggaaaat gagatcaaat   1260
   gtcatttccc aaatttcttg taggccgttg tttcagattc tttctgtctt ggaatgtaaa   1320
   catctgattc tggaatgcag aaggaggggt ctgggcatct gtggattttt ggctactaga   1380
   agtgtcccag aagtcactgt atttttgaaa cttctaacgt cataattaag tttctcttgt   1440
   cttggcatca agaatagtca agttttttgg ccgggcatgg tggctcatgc ctgtaatccc   1500
   agcacttggg gaggccaagg caggcggatc acatgaggcc aggaattcga gaccaacctg   1560
   gtcagcatgg caaaaccccg tctctactaa aagtacaaaa attagccagg cgtgatggca   1620
   cgtgtctgta atcccagcta ctctggagac tgaggtggga gaatcgcttg agactgggag   1680
   gcagaggttg cagtgaaccg agatcatgcc accgcacttc agcctgggtg acagagaagg   1740
   actccgtctc aaaaaaaaaa naaaaaagaa tagtcatttt taaactacct atctcatgca   1800
   atgaaagcat tttcttccac aaagagctta atcctcatga taggattgcc tagtgtctcc   1860
   catttgcagg tttctgggtt gatgtcttaa tgcataatac tgcaagtgac atcagctggc   1920
   tgtgatgctt cgaaataggt ctgctcctca cagctttggg aatctgaatg gaagaagaaa   1980
   agagagaagt taacaacctc cactggggca actttgtgaa cangtaggca cttagtcata   2040
   ggaaacatat tatgtgcagg tcctagcctg gggtaggaaa gtagatagac agaaaatcat   2100
   taggtaattt aagtactaaa ttgggcaggg ctttttagta tcaaatcact actagaccgt   2160
   ttaatttgtt aaattatctc taggatggtg atttataacc tacccaaagt tatcgatatt   2220
   cttactaaac tctgaggcct gaagttctgt gatagacctt aaataagtgt cctaagtcag   2280
   tggttcccaa atctggctgg tcgggaatac ctgggaagtt tgttaaaatt ttttaaaaat   2340
   gttttaagat ttttgggtcc tgagccaggc gtggtggctc acacctgtaa tcccagcact   2400
   ttgggaggct gaggcaggtg gatcgcctga ggtcaggagt tcaagatcaa cctggccaac   2460
   atactgaaac cccgtctcta ctaaaaataa gaaaaattag ctgggcgtgg tggcgggcac   2520
   ctgtaatccc agctacttgg gaggctgagg caggagaatc acttgaacct gggagttaga   2580
   ggttgcagtg agctgagatc acaccattgc gcttcagncc tgggcaacaa gagtgaaact   2640
   ccatctccaa aaaaaaaaaa caaaaagaaa aagattttgg ggtcccgacc tcaaacctac   2700
   tgaatcagaa tttctaggga tgaagcctag gaatgtgttg ttgttttcag agcttccctg   2760
   gtgattgtga taagcctggt ttggaaacca ttgctggaga actttgtaaa gatacagaga   2820
   cccagacctt ttgtatttac atttaaatac aaatacaaat cctgggtttc tatatgttct   2880
   gttagctttt caggtgattc tgctacacag acgttgaaaa ccactgccct aagaaagaga   2940
   tcagaggcca catatcagag agaaaaggga ccaaaccttc ggtggtttgt tgtgtgtcgt   3000
   tttaatgcca attattttaa cttgcacagt cttctgaaac cttgtattaa tagttctctt   3060
   ttgtattacc attttcaggt agggttttga tcactatgat tctgaagata atagtgaaat   3120
   agtgaatttc attgatatga agagataatt gattttcatt cattggtttg aacacctgca   3180
   aaatcacaaa taaatgagaa ctaagtcttg taaaaaaaaa anaaaaaaaa aaaaaaa      3237
<210> 29
   <211> 1285
   <212> DNA
   <213> Homo sapiens
<220>
   <221> misc_feature
   <222> (25)..(25)
   <223> n is a, c, g, or t
<400> 29
   taacgctccc taaactgcca cttgntcagc tccgcgccta aggtgtctat tagtgcgcct     60
   gcgctgtgac ctagaatggg cgcatgcgcc gagcggaact ggctggtttg aaaaccatgg    120
   cgtgggtacc agcggagtcc gcagtggaag agttgatgcc tcggctattg ccggtagagc    180
   cttgcgactt gacggaaggt ttcgatccct cggtaccccc gaggacgcct caggaatacc    240
   tgaggcgggt ccagatcgaa gcagctcaat gtccagatgt tgtggtagct caaattgacc    300
   caaagaagtt gaaaaggaag caaagtgtga atatttctct ttcaggatgc caacccgccc    360
   ctgaaggtta ttccccaaca cttcaatggc aacagcaaca agtggcacag ttttcaactg    420
   ttcgacagaa tgtgaacaaa catagaagtc actggaaatc acaacagttg gatagtaatg    480
   tgacaatgcc aaaatctgaa gatgaagaag gctggaagaa attttgtctg ggtgaaaagt    540
   tatgtgctga cggggctgtt ggaccagcca caaatgaaag tcctggaata gattatgtac    600
   aaattggttt tcctcccttg cttagtattg ttagcagaat gaatcaggca acagtaacta    660
   gtgtcttgga atatctgagt aattggtttg gagaaagaga ctttactcca gaattgggaa    720
   gatggcttta tgctttattg gcttgtcttg aaaagccttt gttacctgag gctcattcac    780
   tgattcggca gcttgcaaga aggtgctctg aagtgaggct cttagtggat agcaaagatg    840
   atgagagggt tcctgctttg aatttattaa tctgcttggt tagcaggtat tttgaccaac    900
   gtgatttagc tgatgagcca tcttgatgta gctgatctct cagggataga agatatttct    960
   catgaaggca gcctaactct gaggaaaaca atgccaattc aagtacagat ttcaacacat   1020
   cttcaacact atgtgaaggg ttcacatctt aacctgtgca attcagattg atactcagaa   1080
   tatgggttga tttgaatatc tgaaatatca atggaaaatc ccactcagtt tttgatgaac   1140
   agtttgaaca gttttctgta atcaagcagc ttgcatagaa attgtatgat gaaattttac   1200
   ataggttctt ggtgctgttt tgttcttttt ttgttttttg ttgttttgtt atttacttat   1260
   atacatataa aattttattg aaaat                                         1285
<210> 30
   <211> 738
   <212> DNA
   <213> Homo sapiens
<220>
   <221> misc_feature
   <222> (38)..(38)
   <223> n is a, c, g, or t
<220>
   <221> misc_feature
   <222> (614)..(614)
   <223> n is a, c, g, or t
<400> 30
   tttttttttt tttttttttt tttttttttt ttttggtngg tggtggttgt tcatagaaga    60
   gtgttcttta taaattatat tattattgta ttttttgaaa aatttatact agaaagtggt    120
   atatatgtct ttgtatacat tacgaccatt gtgattatcc attaaatcca gtgtctgatg    180
   ctattggtta gtaatgatgt cccagcattt gatgttccct ctctaatgat aacaagtaaa    240
   tatttaggtt tatccctttt gaaagtttgg ttttactttt aacagtccat gaacagaaaa    300
   ttaaggcctc taaaatcaga tcccctagtt actcctctgt tctgaatctg gggaagtagt    360
   aaaccaaaaa gacagggaaa accagatatt tagagtcgaa gcagcatgca aaaaataata    420
   atgtttttaa ctaatgaagg ctactggagt tactaagaat cacctaaaaa tgtgctactg    480
   taccttgctc attttgcaga tttagaagct gaaacatggg ttaggttact tactaaaagt    540
   ccacctgtgg caacctgcaa ataatgctta tttatactaa agatgagttt atctgataga    600
   gttaggaaat ttcntctttt gagtaagagt attgctataa catatatttt tttctgaaat    660
   gttttaaaat agaagtacac ctaattgtta ttggttaaaa aaaaagccat atgtgaatga    720
   ggttggtttt tgggtttt                                                738
<210> 31
   <211> 2352
   <212> DNA
   <213> Homo sapiens
<400> 31
   tttttttttt tttttttttt tgaaaacaga ataagttact ttattacaag aacaaaacag    60
   acttgctaat tttatgtctc ctttcaccca tatcacaaat gcaaagtata tattatcata    120
   agatggaaat taccaaactc caagatactc tgcataaatg aaataaactc aacttgcaaa    180
   attagcaagt agaacattca agtatctaca gtcattataa atagtgtaaa gtacatgcca    240
   tatctacagt gtgggaagag tataaacatg ggaaaatgag ttcttaagga acaattagaa    300
   tgtttgtttg gacaatttaa aatattaaca agggtactca aatacagaat caaccaaaac    360
   atttattttt tctctttttt caagaaaaac tgtcgagctt cttcccaggc ctgctgagga    420
   aatctgttag ccagctcaag atagtcttgg ggaccaaggc atttcccttt gggttggtaa    480
   ggctggtttt ccgcttgacc tggagtctgt gccaaatcct gaatgaaagt ggccagtaaa    540
   acacagctca tctcttgctc cagaatgatc ttgttctgat ggttgttgta acaagcagcg    600
   ataagtgaag ggaacagtac tttgatcagc cgtgggtcac tgaaatactg gaagggcaac    660
   tggcagagct tctgcagcac tgtggggtgg cggccggact gcacgatcac ctgaaaagca    720
   ggcagatgaa gagctgcaaa gctgttgaag aaacgtaaac tctgaatggc cacttggatg    780
   gtattttgag tgtaattctc cttgggactg gcagtgctgg ggtccaagat ggtgccatgg    840
   aagaggacac agtagagcat atgaagaact ccagccaggt cggttgcctg aagagcagct    900
   gtcagccctg tgggatcctg gcgattattg tcaaatatgc tgtatgacct tccagtgaca    960
   gcaaagcaca gtgtacacat tgcatgtaag agtcctgcgg catgctgcag aaatatggcc   1020
   atcttgggat tctcatccac tgggccttgc accgagagga agcaggcaca cagtttgtca   1080
   atcagaccca tgttcaccac gtagctgata aggtcctgaa ctcgattgtt aaaaggatca   1140
   ccttgtgagg ttttgttttt catttcctgt gttggtattt ttggggtagc tggctggcag   1200
   tttccatctg gtcgattggc aatcaggcag cccaaaacca cagcactgac tttgagaagt   1260
   ccagttgtca agccttcaaa aacttgttta tttgtatttc tccccaaaat agtattattt   1320
   tcatctggaa cataaaccgt caactggtgt atcaggaggt ccattaagaa ggtaatcttg   1380
   ttactaaaca gaacatcact gcagttttct gaacagttat tgcaggtgag gttgtaaaca   1440
   ttgattgcat tgcagagaga cttaggagga attcttaaaa ctgtgttcac atttgtggct   1500
   gggactactg cttgaaggat gtgttcaagg gctgttaatc caccagcagc ttgaaatgca   1560
   atctgatctg ccacattctc tttttccagt attctagtga tctctcctag ggtccgatcc   1620
   aaagcagaca ctttattgtt tgcccatgag ccactgtctt gaacttgtac ttgttttaga   1680
   agatctttgg ctaatcgctg aagctttgct ttataaggtg aatcagagcc agaatttttg   1740
   gtttccatta aactctcata ttccttagcc ctgaagttca tccgggcttt tatctttttg   1800
   gctttttttt tatttttttg ccgctcttct ccatctttca aagcttctgc tggagctgta   1860
   ctttcaacca caatgtcaat aatgtacttc ttcaaggaaa gatgctccac ttcttcatct   1920
   gacagttcac gcccctggat gctggtattc tctctcacgg cttgctggtg ttttctccct   1980
   ttaacatggc taaaaagata tacctctgaa gagatcagga cattgcagag agaacactgc   2040
   ttctttcttt cataaggggt cagtttgggg gcataatcag tatttgcatg tcgcccactg   2100
   cttagctcag cagctttttc ttttctttgt tcaatctgtt ccatgtgcct tcgaatactt   2160
   tcatcatgct tgagctgaat ttttttctgt aactcttcca tagcttcttg ttgagcagct   2220
   gtgagtgctg ccaatcgttc ttccctgtct ctagctcttt cccgggctgc atcctcacgg   2280
   gttttttcct tttcttgcct ctgttgttca attcgggctt cttgttcttt cctcttcatt   2340
   aacaattctt ct                                                       2352
<210> 32
   <211> 1180
   <212> DNA
   <213> Homo sapiens
<400> 32
   ccgggtcact gttaaggggg ccgccatggc ccggctgtgg ggcgcgctga gtctttggcc     60
   actgtgggcg gccgtgccgt ggggcggggc ggcagccgtc ggtgtccggg cttgcagctc    120
   cacggccgcc ccggacggcg tcgagggccc ggcgctgcgg cgctcctatt ggcgccacct    180
   gaggcgtctg gtgctgggtc ctcccgaacc gccgttctcg cacgtgtgcc aagtcgggga    240
   cccggtgctg cgcggcgtgg cggccccggt ggagcgggcg cagctaggcg ggcccgagct    300
   gcagcggctg acgcaacggc tggtccaggt gatgcggcgg cggcgctgcg tgggcctaag    360
   cgcgccgcag ctgggggtgc cgcggcaggt gctggcgctg gagctccccg aggcgctgtg    420
   tcgggagtgc ccgccccgcc agcgcgcgct ccgccaaatg gagcccttcc ccctgcgcgt    480
   gttcgtgaac cccagcctgc gagtgcttga cagccgcctg gtcacctttc ccgagggctg    540
   cgagagcgtc gccggcttcc tggcctgcgt gccccgcttc caggcggtgc agatctcagg    600
   gctggacccc aatggagaac aggtggtgtg gcaggcgagc gggtgggcag cccgcatcat    660
   ccagcacgag atggaccacc tgcagggctg cctgtttatt gacaaaatgg acagcaggac    720
   gttcacaaac gtctattgga tgaaggtgaa tgactaaagc tttgctactg gggctgagga   780
   ttccggatac caagacgcaa acactttcac tttgagctgg gcaaatctta cttggcatca    840
   acttggatgg ctcgcatatg acaggaaact ggattgccaa ggcatggcag actgagctgg    900
   aggaagatgt cagaaatgtt tgccctgaaa tcagttacgg acaaaatgtt ggctacaact    960
   tgaggagaaa aatcaccccc aaaggagtgg acatttccta acaattctgt atggaggaag   1020
   gtggggtaat tgcattcgtc tgcagtagac acgagttcct cggacctgta taatctccca   1080
   aagccaaggt ttggtaataa tgtagtcccc aaatacctga aagctgtctt taaaaatgca   1140
   ggtaagcatg tgactggcaa aaaaaaaaaa aaaaaaaaaa                         1180
<210> 33
   <211> 1899
   <212> DNA
   <213> Homo sapiens
<400> 33
   ccaagccagt ggacaccccc cccacagatc accgtccagc ctgcagcgtt cgcatttagc      60
   ccaggaatca tcagtgctgc ttccctcggg ggacagaccc agatcctggg gtccctcact     120
   acagctccag tcattaccag cgccattccc agcatgccag ggatcagcag tcagatcctc     180
   accaatgctc agggacaggt tattggaacc cttccatggg tagtgaactc agctagtgtg     240
   gcggccccag caccagccca aagcctgcag gtccaggccg tgacccccca gctgttgttg    300
   aacgcccagg gccaggtgat tgcgaccctg gctagcagcc ccctgcctcc acctgtggct     360
   gtccggaagc caagcacacc tgagtcccct gctaagagtg aggtgcagcc catccagccc     420
   acaccaaccg tgccccagcc tgctgtggtc attgccagcc cagctccagc cgccaagcca     480
   tctgcctctg ctcctatccc aattacctgc tcagagaccc ccaccgtcag ccagttggtg     540
   tccaagccac atactccaag tctggatgag gatgggatca acttagaaga gatccgggag     600
   tttgccaaga actttaagat ccggcggctc tcgctgggcc ttacacagac ccaggtgggt     660
   caggctctga ctgcaacgga aggtccagcc tacagccagt cagccatctg ccggttcgag     720
   aagctagaca tcacacccaa gagtgcccag aagctaaagc cggtgctgga aaagtggcta     780
   aacgaagctg aactgcggaa ccaggaaggc cagcagaacc tgatggagtt tgtgggaggc     840
   gagccctcca agaaacgcaa acgccgcacc tccttcaccc cccaggccat agaggctctc     900
   aatgcctatt ttgagaagaa cccactgccc acaggccagg agatcactga aattgctaag     960
   gagctcaact acgaccgtga ggtagtgcgg gtctggttct gcaatcggcg ccagacgctc    1020
   aagaacacca gcaagctgaa cgtctttcag atcccttagg gctcagccct ggccctgtgt    1080
   tctagcactt tgtccatttc ccgtggcatc cggctgcagc cactgccatg acagcacctg    1140
   tcattttgcc acgtgcagct gtgctcaccc caggtcatca gactccaccg tgtgcatgtg    1200
   catcaatgtc cctcttttct cccacacatc tcacatcatg gggaggccag agggggccac    1260
   acgagagctc caggctctgg gctggtcact ccgaagaaga ggatttgtga cgtcacttag    1320
   agaagcacct tgctagcatg gtttctgaag ggtgaattct ggtggggaac cagaaactcc    1380
   ctgtctttgg ggcagggcta aagcagctcc taaggaccac tggccattag ctcttgcttt    1440
   tgatggcatt ctctttccac cttgtcttct cctttgctcc tctgtgttag tgtggcaggt    1500
   atgacaactc atccagtgga aacacagcct cacactgccc ttccgccccc cacactttgc    1560
   ctgcaggtgc accgaaagac ctgggagata aaattcaaaa aagtgtgatg tgctgctcag    1620
   aaggtcagac tccatgtctg ccttgacctc aaggtcagaa ggttcccaaa cccctggggc    1680
   tggaacatgg gatctcctct tccacctctt cctggttcct ttgcggggaa aattgcacta    1740
   aaacagaacc ttttcttaat ccatgttgga aggaagcaac agtgaactct acctgttctg    1800
   gagttctcct gggtctccag aaggttggga atttagaaaa taaggctgtt ctttcatatt    1860
   ttaatttaat atatgtcaat ggccatccct cccacaaaa                           1899
<210> 34
   <211> 2352
   <212> DNA
   <213> Homo sapiens
<400> 34
<210> 35
   <211> 3059
   <212> DNA
   <213> Homo sapiens
<400> 35
   gccccccccc cacgcctcct cccctcctcc cgcccgcgcg tgctcccgcc cctcgccgcc 60
   gctcgctcgc tcgccggccg gcctccgctc ggccccctcc ctcagctccg gtgcgcggcg 120
   gccgacgacc cgcggcctgg gcctcggcgc gccaccggcg cccgcgcgga gcggcccggg 180
   ggccctcacg caggcccatg gcggcggcgc ggcgcgctct cgggccgggc accacgcggg 240
   cgcggggccg ggggcgcggg gccgggggcg ggtcccgccg ggcgctgggc cgtgggccgc 300
   ctggagggcc gcgagttcga gtatctgatg aagaagcgct cggtgaccat cggccgcaac 360
   tcgtcgcagg gctcggtgga cgtgagcatg ggccactcga gcttcatctc ccggcgccac 420
   ctcgagatct tcacgccccc gggcggcgcg gccatggcgg ggccgctccg gagctgccgc 480
   ccgcgcagcc caggcccgac gccggcggcg acttctacct gcgctgcttg ggcaagaacg 540
   gggtattcgt ggacggcgtg ttccagaggc gcggggcgcc gccgctgcag ctgccgcgcg 600
   tgtgcacatt caggttcccg agcacaaaca tcaagataac gttcactgcc ctgtccagcg 660
   agaagagaga gaagcaggag gcgtctgagt ctccagtgaa ggccgtacag ccacacatct 720
   cgcccctgac catcaacatt ccagacacca tggcccacct catcagccct ctgccctccc 780
   ccacgggaac catcagcgct gcaaactcct gcccctccag cccccgggga gcggggtctt 840
   cagggtacaa ggtgggccga gtgatgccat ctgacctcaa tttaatggct gacaactcac 900
   agcctgaaaa tgaaaaggaa gcttcaggtg gagacagccc gaaggatgat tcaaagccgc 960
   cttactccta cgcgcagctg atagttcagg cgattacgat ggctcccgac aaacagctca 1020
   ccctgaacgg gatttataca cacatcacta aaaattatcc ctactacagg actgcggaca 1080
   agggctggca gaattcaatt cgccacaatc tctctctgaa tcgttatttc atcaaagtgc 1140
   cgcgttccca ggaagaacca ggcaaaggct cgttctggag gatagaccca gcctctgaaa 1200
   gcaaattaat agaacaggct tttaggaaac gacggcctag gggcgtgccc tgctttagaa 1260
   cccctctggg accgctctct tctaggagtg ccccagcctc tcccaatcac gcgggagtgc 1320
   tgtctgctca ctctagtggc gcccagaccc ctgagagcct gtcgagggaa ggttcgccgg 1380
   cccccctgga gcctgagcct ggcgctgcac agcccaaact cgctgtcatc caggaagccc 1440
   ggtttgccca gagcgcccca gggtcacctc tgtccagtca gccagtctta atcaccgtcc 1500
   agcggcagct accacaggcc atcaagcctg tcacctacac tgtggccacc ccagtgacca 1560
   cctcgacctc ccagccaccc gtcgtgcaga cggttcacgt cgtccaccag atcccagcgg 1620
   tgtcggtcac cagtgtggcc ggactggccc cagcgaacac gtacactgtc tctggacaag 1680
   ctgtggtcac cccggcagcc gtgctggccc ctcctaaggc agaggcccag gagaatggag 1740
   accacaggga agtcaaagtg aaagtagagc ctattcccgc cattggccac gccacgctcg 1800
   gcactgccag ccggatcatt cagacggcac agaccacccc ggtccagacg gtgaccatag 1860
   tacaacaggc acctctaggt caacaccagc taccaataaa aactgtaaca caaaacggca 1920
   ctcacgtggc atcagtcccc actgcggtcc acggccaggt gaacaatgcc gcggcgagtc 1980
   ctttgcacat gttggcaaca cacgcatccg catcggcctc cctgcccaca aagcgccaca 2040
   acggtgacca gccggagcag ccggagctga agcggatcaa gacagaagac ggcgagggca 2100
   tcgtcattgc cctgagcgtg gacacgccac cggcagccgt aagggaaaag ggtgtccaga 2160
   actagcgacc gggagagctt ttctttaacg atatcaactc tgtggtgcca aaaggagacg 2220
   cggcctcccg ccagcactcg gggtgcaggg gccctgtggt tggacttcac ctctcagcac 2280
   tgaaaaccca aaacccagct ggccttaaca ctccttaaag acagaagtca cacttgaaca 2340
   aaacccacac acaacaaaac ctgatttggg agacggtgtc tccactgagc acctgctggg 2400
   ctgagcttct acctacgagt gaaactctgt cctcccgcga ggaccaggca tcgctgtgtg 2460
   aggacggcac ggccagcgcc tgctgtgagt gggtctccca agactaggcc tcaggacgcg 2520
   gggggagcca tccccgccgc cctcacagga cccaccaggc agcggagaca tgtggaatta 2580
   gagtattttg aggtgtcctt tctttacaaa ataatggggt cttgggcatt tcacatcact 2640
   ccatttctac tgagactttc agaatcacac aggccctttc cgtggatttc atttggggca 2700
   aagaaacaac atagttttgt ttttgttttc agcctatgga atgatttcct tttgtctgtc 2760
   ttgttcaagt tcagacgaag ctactctggc atctgcacat ttccgtgtta cagcagctgc 2820
   ctgatgaatt ttatccacct ccatttcagc atgtggctcg cgtggacagg tggacggacg 2880
   ctgtggccgc atggaacctt gagaacccag ggacgagcca gtgccgggaa ggaactgccg 2940
   ggactcaccg agctgcactt aactgttctc tttctggcta ttttttgttg tttgtttctt 3000
   tgtgttgact ttgtccctgg caaaattttc cactctgagt aaaacaagtc tcggaattc 3059
<210> 36
   <211> 1549
   <212> DNA
   <213> Homo sapiens
<400> 36
   aaaccctctg taaagtaaca gaagttagaa ggggaaatgt cgcctctctg aagattaccc      60
   aaagaaaaag tgatttgtca ttgctttata gactgtaaga agagaacatc tcagaagtgg     120
   agtcttaccc tgaaatcaaa ggatttaaag aaaaagtgga atttttcttc agcaagctgt     180
   gaaactaaat ccacaacctt tggagaccca ggaacaccct ccaatctctg tgtgttttgt     240
   aaacatcact ggagggtctt ctacgtgagc aattggattg tcatcagccc tgcctgtttt     300
   gcacctggga agtgccctgg tcttacttgg gtccaaattg ttggctttca cttttgaccc     360
   taagcatctg aagccatggg ccacacacgg aggcagggaa catcaccatc caagtgtcca     420
   tacctcaatt tctttcagct cttggtgctg gctggtcttt ctcacttctg ttcaggtgtt     480
   atccacgtga ccaaggaagt gaaagaagtg gcaacgctgt cctgtggtca caatgtttct     540
   gttgaagagc tggcacaaac tcgcatctac tggcaaaagg agaagaaaat ggtgctgact     600
   atgatgtctg gggacatgaa tatatggccc gagtacaaga accggaccat ctttgatatc     660
   actaataacc tctccattgt gatcctggct ctgcgcccat ctgacgaggg cacatacgag     720
   tgtgttgttc tgaagtatga aaaagacgct ttcaagcggg aacacctggc tgaagtgacg     780
   ttatcagtca aagctgactt ccctacacct agtatatctg actttgaaat tccaacttct     840
   aatattagaa ggataatttg ctcaacctct ggaggttttc cagagcctca cctctcctgg     900
   ttggaaaatg gagaagaatt aaatgccatc aacacaacag tttcccaaga tcctgaaact     960
   gagctctatg ctgttagcag caaactggat ttcaatatga caaccaacca cagcttcatg    1020
   tgtctcatca agtatggaca tttaagagtg aatcagacct tcaactggaa tacaaccaag    1080
   caagagcatt ttcctgataa cctgctccca tcctgggcca ttaccttaat ctcagtaaat    1140
   ggaatttttg tgatatgctg cctgacctac tgctttgccc caagatgcag agagagaagg    1200
   aggaatgaga gattgagaag ggaaagtgta cgccctgtat aacagtgtcc gcagaagcaa    1260
   ggggctgaaa agatctgaag gtagcctccg tcatctcttc tgggatacat ggatcgtggg    1320
   gatcatgagg cattcttccc ttaacaaatt taagctgttt tacccactac ctcaccttct    1380
   taaaaacctc tttcagatta agctgaacag ttacaagatg gctggcatcc ctctcctttc    1440
   tccccatatg caatttgctt aatgtaacct cttcttttgc catgtttcca ttctgccatc    1500
   ttgaattgtc ttgtcagcca attcattatc tattaaacac taatttgag                1549
<210> 37
   <211> 1584
   <212> DNA
   <213> Homo sapiens
<220>
   <221> misc_feature
   <222> (308)..(311)
   <223> n is a,c,g, or t
<220>
   <221> misc_feature
   <222> (572)..(572)
   <223> n is a, c, g, or t
<220>
   <221> misc_feature
   <222> (894)..(894)
   <223> n is a, c, g, or t
<220>
   <221> misc_feature
   <222> (925)..(925)
   <223> n is a, c, g, or t
<400> 37
   tttttttttt tttttttttt ttttttacga tagatcacag ttttttattc ttaatgtcac     60
   aagcaggaga aaaatctcac attcatacta aaaattccaa ctagactcaa caggaatgaa    120
   gtctctattt gtaatggaaa gtcccagcct cccgctgccg tccagtgtgt gtactgtaca    180
   catccacact cactctcact cagggttccc ggaccggctg tcctgcctgc ggaactgagg    240
   taaacaagct caggtactga cactaggagg gtctacctta cataaggtac aggtagaagc    300
   ttgattgnnn nctaggccca ggcccaccca gaccctccaa tcctaacagg tatttaggct    360
   tgaggttcac tccctcctca gctgcacacg cagccaggta taacactcgc cctcagtcac    420
   aacggggagg gggcaccggt tacatctaca tcacattatt tataaaataa gaattacatt    480
   tcatataaca tggccagaag gagctctagt cccccaggaa agctgccggg gacagcattt    540
   gagcctcttc tttgcacagg cataacttaa cnatacagct aattcctagt taatagcatt    600
   tatacttaac cacctcaatg aaccaagctt gaaggaattt aaaaggcaat ttagcttaaa    660
   tacaaaaata aatttttgtt aaaaaacgtt taaatatttt tttcttttaa tttagacaca    720
   cgcattcata cttctcccaa agaggttggg cgtgacagca aggcgcttgg gcctggggta    780
   tgtggtttca gaaggacgga aggaaaggat gggctgcaga gggccctgtt tgggaaaaat    840
   aggattttaa aaatatggtt cattaattta ggttttctaa catctacttt gggntgacgt    900
   agcctccagt gaggtcagtt aagtnggaca gaaaccgcag agggaagagg tctttgcttc    960
   ccctgggccc attctccctg gctgccagcc cttgaagtca gaacaccatg ggaaaattca   1020
   ggagtcggca cttgtagccg tcaagtggtg ctactttcca cattcaatta gcgaggcccc   1080
   agaaaacttg aaacaggaag tccggcacta ccaacatgcc gccactcata caactcaact   1140
   cttcctccaa actcgattca aagagcaata cacagagcgt cagacaaagg aacacaggac   1200
   aaggcttgaa acatacaatc ctggagaggc agacgagggc ccaatggggc caaagggtag   1260
   ctgggcgagg aggcagctga agcagcggtg ggcatcctgc ctgctccaag agcttctccc   1320
   ttcacccctg gctctgccag gctaacagtc ttgagccacc aagctctgag gggccaaact    1380
   ggcagagggt tggagatgaa acattgattc aggcttggca ggaagaaaac ggcaggagca   1440
   gcacccactg gggtctccaa ggcaaagcta gattcaaggg catttaaaaa ggagggaatc   1500
   ctgcaaggaa ccggtgaggg acacagcagc aagaaatggg gaaacagagt ccccagaagg   1560
   gaccagagga agagaaccac ttct                                          1584
<210> 38
   <211> 3969
   <212> DNA
   <213> Homo sapiens
<400> 38
   gaattccgaa agcaaggaga tggccaccaa ggagaagctg cagtgtctga aagatttcca     60
   caaggacatg gtgaagccct caccagggaa gagcccaggc acgcggcctg aggacgaggc    120
   tgagggaaaa cctccgcaga gggagaagtg gtctagcaag atcgactttg tgctctctgt    180
   ggctggcggc ttcgtgggct tgggcaacgt ctggcgcttc ccgtacctct gctacaagaa    240
   tggtggaggt gcgtttctca taccgtattt tattttcctg tttgggagcg gcctgcctgt    300
   gtttttcttg gagatcatca taggccagta cacctctgaa gggggcatca cctgctggga    360
   aaagatctgc cccttgttct ctggtatcgg ctatgcctcc gttgtaattg tgtccctcct    420
   gaatgtctac tacatcgtca tcctggcctg ggccacatac tacctgttcc agtccttcca    480
   gaaggagctg ccctgggcac actgcaacca cagctggaac acacctcact gcatggagga    540
   caccatgcgc aagaacaaga gtgtctggat caccatcagc tccaccaact tcacctcccc    600
   tgtcatcgag ttctgggagc gcaacgtgct gagcttgtcc cctggaatcg accacccagg    660
   ctctctgaaa tgggacctcg ctctctgcct tcttttagtc tggctagtgt gtttcttctg    720
   catctgcaag ggcgtcaggt ccactgggaa ggtcgtctac ttcacagcca cttttccatt    780
   cgccatgctc ctggtgctgc tggtccgagg gctgacgctg ccgggcgcgg gccgaggcat    840
   caagttctat ctgtatcctg acatcacccg ccttgaggac ccacaggtgt ggattgacgc    900
   tgggactcag atattcttct cttatgccat ctgcctgggg gctatgacct cgctggggag    960
   ctacaacaag tacaagtata actcgtacag ggactgtatg ctgctgggat gcctgaacag   1020
   tggtaccagt tttgtgtctg gcttcgcaat tttttccatc ctgggcttca tggcacaaga   1080
   gcaaggggtg gacattgctg atgtggctga gtcaggtcct ggcctggcct tcattgccta   1140
   cccaaaagct gtgacaatga tgccgctgcc cacattttgg tccattcttt tttttattat   1200
   gcttctcttg cttggactgg atagccagtt tgttgaagtt gaaggacaga tcacatcctt   1260
   ggttgatctt tacccatcct tcctaaggaa gggttatcgt cgggaaatct tcatcgcctt   1320
   cgtgtgtagc atcagctacc tgctggggct gacgatggtg acggagggtg gcatgtatgt   1380
   gtttcagctc tttgactact atgcagctag cggtgtatgc cttttgtggg ttgcattctt   1440
   tgaatgtttt gttattgcct ggatatatgg aggtgataac ctttatgatg gtattgagga   1500
   catgattggc tatcggcccg ggccctggat gaagtacagc tgggtgatca ctccagttct   1560
   ctgtgttgga tgtttcatct tctcgctcgt caagtacgta cccctgacct acaacaaaac   1620
   atacgtgtcc ccaacttggg ccattgggct gggctggagc ctggcccttt cctccatgct   1680
   ctgcgttccc ttggtcatcg tcatccgcct ctgccagact gaggggccgt tccttgtgag   1740
   agtcaagtac ctgctgaccc caagggaacc caaccgctgg gctgtggagc gcgagggagc   1800
   cacaccttac aactctcgca ccgtcatgaa cggcgctctc gtgaaaccga cccacatcat   1860
   tgtggagacc atgatgtgag ctctctcggg tcgacggggc cggcggcttt cctgctgttt 1920
   actaacatta gattcacata ggaccaggtt tacagagctt tatatttgca ctaggatttt 1980
   tttttttttg taattgtcac agaaaatgta attgtgggta tgtgtgcgtg cgtgtgtgtg 2040
   tgtgtgtgtg tgtatcgtgt gtgtgtgttt tgttttgatt tgggggatat tttgtacaaa 2100
   aagaaaaccc acgggaagat gtccgtggag aggcagagct ttcatactga attagatgta 2160
   ttttatggga atttggtaaa tttttctttg tatttttttt tttacatata agtatatata 2220
   cacttagaga ttgtcatata cttttaccac ttgaattgat cttcttgcca gcaatagatc 2280
   tcattttcaa aagcaattct tcggtgctgt gtagctggca gaaagttctg tccagtaaac 2340
   gcaggatgga attttcctgg gactctacac ccatcttaag gtggtatacc ttccaaatcc 2400
   tggttcagat ggaagaaata gcaggagaga ggacccatta gctggcagac ccaggggaag 2460
   aaaggagggc tgtgaggaga tacctcatta aacttggctt agtgaagaag agagatgcca 2520
   aaggaatgaa ccaacccttc acataaagga gactggctga agctgaatga ggaggcccta 2580
   tagcagaagt ctgattctaa gagcagtaga aacttgtacc agaagcaaaa tcccactttt 2640
   aattttgaga tggtgagtgg atagtcagta gaccgtcaga accactggcc agagagggag 2700
   ctgctagaga tccaagaagg ctggcaggaa tgaggctcac aactcagcct cgcaagaggt 2760
   ggcagaggca caggaggcca cagtccttcc tggggcattc caggcagaga aggagcagag 2820
   gctctcccgg caggagctgg ggtctcaggg ctcagatgag tctgttgcat ttgaatgggg 2880
   tcatagcagg ttctggtcat tccccaagca acatctcagc atctcttaaa gttgcctgca 2940
   ggaatgaagc atgacatacc tgttgaggga ctaggggagt ggtggggagg tgagtggacc 3000
   aaaggatata ggccccaggc atgcagatgg gcccggtgtc ggggaggggt gctttctttc 3060
   ctcatctccc cactccccac tctcagcctg ggagactcct gccaagccct cattaaagat 3120
   gccaccctgg gctgccctgg cacctagcaa ggcacaccaa gaacagcttt tgagtcgtat 3180
   cctccactgg ggaagtgctc ccagttcaga acaagggcag cccgtggtgc tgacctagga 3240
   tataacaaag ctcttcactt caaaacccct gcaatagctg ggtttacaga catttaccac 3300
   ctggggaccc aaaagagaag gcctaggaga gttttctaga aggttgggat tgtcagggtc 3360
   ctggcccccc agaactggct tgatcaaggg ccttatgtgg agcagaggtt gtctctgaac 3420
   caggagagaa ggtactatac ctttcaaatc cccagggcag acacaccccc acccagcccc 3480
   tatttggacc taaactgtgc catttgaaca gtcacttcca agctcagtct aaatgaaacc 3540
   gaaacgtgac cacgcacaaa ggcagtcact gctcgagggg tgcagaccgc agaattttca 3600
   cagcaggggc tcttggaact ctggaaaccc ccttcttaaa tttgggagga ggagtatgcc 3660
   tttggtgtcc ccctcccaag ggcaattctg aaccccatct ttggcaggca tacatatttc 3720
   actgtttcca aagctatcta ctctgccaaa caacacccag tcctattcca aactctcaac 3780
   gattctatct tgttcctgtt tttctatgta tttatggttg ccgtttgtgt ctgatttgat 3840
   tttactgttt tttccctgat tttatggagt agcattgtga cctgttttcc tttgtcttat 3900
   ataactttag taaactaacc actgtcaatg attgagggca ggtggcacgt ggggaagagg 3960
   gcggaattc 3969
<210> 39
   <211> 5257
   <212> DNA
   <213> Homo sapiens
<400> 39
   aaaaattttc tgttaccaaa ttttacaact tctaataaga ctactataac tttatgtaaa 60
   ctgatgaaga tgtgctgatt aacatattct gtgatatggt ttacaacttt taatcataat 120
   tgtccatgat tttggaatgc tgttatttat cagtaaatgt aaaatatttg aggcatttag 180
   ccatacacac actagaactt tttaaaactt tgtcctatag tgtaattata aactgatgac 240
   tattatcttc atacattgag tcttcatgca tcaatgaaat gaaaaatata ggagtagatg 300
   tccacgccca cagaccctgg tgcgatgccc cacccagggc cttcgccggg gcctgggcct 360
   tcccctgggc caattcttgg gcctagtcca ggaccaggac catccccagg ttccgtccac 420
   agcatgatgg ggccaagtcc tggacctcca agtgtctccc atcctatgcc gacgatgggg 480
   tccacagact tcccacagga aggcatgcat caaatgcata agcccatcga tggtatacat 540
   gacaagggga ttgtagaaga catccattgt ggatccatga agggcactgg tatgcgacca 600
   cctcacccag gcatgggccc tccccagagt ccaatggatc aacacagcca aggttatatg 660
   tcaccacacc catctccatt aggagcccca gagcacgtct ccagccctat gtctggagga 720
   ggcccaactc cacctcagat gccaccaagc cagccggggg ccctcatccc aggtgatccg 780
   caggccatga gccagcccaa cagaggtccc tcacctttca gtcctgtcca gctgcatcag 840
   cttcgagctc agattttagc ttataaaatg ctggcccgag gccagcccct ccccgaaacg 900
   ctgcagcttg cagtccaggg gaaaaggacg ttgcctggct tgcagcaaca acagcagcag 960
   caacagcagc agcagcagca gcagcagcag cagcagcagc agcaacagca gccgcagcag 1020
   cagccgccgc aaccacagac gcagcaacaa cagcagccgg cccttgttaa ctacaacaga 1080
   ccatctggcc cggggccgga gctgagcggc ccgagcaccc cgcagaagct gccggtgccc 1140
   gcgcccggcg gccggccctc gcccgcgccc cccgcagccg cgcagccgcc cgcggccgca 1200
   gtgcccgggc cctcagtgcc gcagccggcc ccggggcagc cctcgcccgt cctccagctg 1260
   cagcagaagc agagccgcat cagccccatc cagaaaccgc aaggcctgga ccccgtggaa 1320
   attctgcaag agcgggaata cagacttcag gcccgcatag ctcataggat acaagaactg 1380
   gaaaatctgc ctggctcttt gccaccagat ttaagaacca aagcaaccgt ggaactaaaa 1440
   gcacttcggt tactcaattt ccagcgtcag ctgagagagg aggtggtggc ctgcatgcgc 1500
   agggacacga ccctggagac ggctctcaac tccaaagcat acaaacggag caagcgccag 1560
   actctgagag aagctcgcat gaccgagaag ctggagaagc agcagaagat tgagcaggag 1620
   aggaaacgcc gtcagaaaca ccaggaatac ctgaacagta ttttgcaaca tgcaaaagat 1680
   tttaaggaat atcatcggtc tgtggccgga aagatccaga agctctccaa agcagtggca 1740
   acttggcatg ccaacactga aagagagcag aagaaggaga cagagcggat tgaaaaggag 1800
   agaatgcggc gactgatggc tgaagatgag gagagttata gaaaactgat tgatcaaaag 1860
   aaagacaggc gtttagctta ccttttgcag cagaccgatg agtatgtagc caatctgacc 1920
   aatctggttt gggagcacaa gcaagcccag gcagccaaag agaagaagaa gaggaggagg 1980
   aggaagaaga aggctgagga gaatgcagag ggtggggagt ctgccctggg accggatgga 2040
   gagcccatag atgagagcag ccagatgagt gacctccctg tcaaagtgac tcacacagaa 2100
   accggcaagg ttctgttcgg accagaagca cccaaagcaa gtcagctgga cgcctggctg 2160
   gaaatgaatc ctggttatga agttgcccct agatctgaca gtgaagagag tgattctgat 2220
   tatgaggaag aggatgagga agaagagtcc agtaggcagg aaaccgaaga gaaaatactc 2280
   ctggatccaa atagcgaaga agtttctgag aaggatgcta agcagatcat tgagacagct 2340
   aagcaagacg tggatgatga atacagcatg cagtacagtg ccaggggctc ccagtcctac 2400
   tacaccgtgg ctcatgccat ctcggagagg gtggagaaac agtctgccct cctaattaat 2460
   gggaccctaa agcattacca gctccagggc ctggaatgga tggtttccct gtataataac 2520
   aacttgaacg gaatcttagc cgatgaaatg gggcttggaa agaccataca gaccattgca 2580
   ctcatcactt atctgatgga gcacaaaaga ctcaatggcc cctatctcat cattgttccc 2640
   ctttcgactc tatctaactg gacatatgaa tttgacaaat gggctccttc tgtggtgaag 2700
   atttcttaca agggtactcc tgccatgcgt cgctcccttg tcccccagct acggagtggc 2760
   aaattcaatg tcctcttgac tacttatgag tatattataa aagacaagca cattcttgca 2820
   aagattcggt ggaaatacat gatagtggac gaaggccacc gaatgaagaa tcaccactgc 2880
   aagctgactc aggtcttgaa cactcactat gtggccccca gaaggatcct cttgactggg 2940
   accccgctgc agaataagct ccctgaactc tgggccctcc tcaacttcct cctcccaaca 3000
   atttttaaga gctgcagcac atttgaacaa tggttcaatg ctccatttgc catgactggt 3060
   gaaagggtgg acttaaatga agaagaaact atattgatca tcaggcgtct acataaggtg 3120
   ttaagaccat ttttactaag gagactgaag aaagaagttg aatcccagct tcccgaaaaa 3180
   gtggaatatg tgatcaagtg tgacatgtca gctctgcaga agattctgta tcgccatatg 3240
   caagccaagg ggatccttct cacagatggt tctgagaaag ataagaaggg gaaaggaggt 3300
   gctaagacac ttatgaacac tattatgcag ttgagaaaaa tctgcaacca cccatatatg 3360
   tttcagcaca ttgaggaatc ctttgctgaa cacctaggct attcaaatgg ggtcatcaat 3420
   ggggctgaac tgtatcgggc ctcagggaag tttgagctgc ttgatcgtat tctgccaaaa 3480
   ttgagagcga ctaatcaccg agtgctgctt ttctgccaga tgacatctct catgaccatc 3540
   atggaggatt attttgcttt tcggaacttc ctttacctac gccttgatgg caccaccaag 3600
   tctgaagatc gtgctgcttt gctgaagaaa ttcaatgaac ctggatccca gtatttcatt 3660
   ttcttgctga gcacaagagc tggtggcctg ggcttaaatc ttcaggcagc tcatacagtg 3720
   gtcatctttg acagcgactg gaatcctcat caggatctgc aggcccaaga ccgagctcac 3780
   cgcatcgggc agcagaacga ggtccgggta ctgaggctct gtaccgtgaa cagcgtggag 3840
   gaaaagatcc tcgcggccgc aaaatacaag ctgaacgtgg atcagaaagt gatccaggcg 3900
   ggcatgtttg accaaaagtc ttcaagccac gagcggaggg cattcctgca ggccatcttg 3960
   gagcatgaag aggaaaatga ggaagaagat gaagtaccgg acgatgagac tctgaaccaa 4020
   atgattgctc gacgagaaga agaatttgac ctttttatgc ggatggacat ggaccggcgg 4080
   agggaagatg cccggaaccc gaaacggaag ccccgtttaa tggaggagga tgagctgccc 4140
   tcctggatca ttaaggatga cgctgaagta gaaaggctca cctgtgaaga agaggaggag 4200
   aaaatatttg ggagggggtc ccgccagcgc cgtgacgtgg actacagtga cgccctcacg 4260
   gagaagcagt ggctaagggc catcgaagac ggcaatttgg aggaaatgga agaggaagta   4320
   cggcttaaga agcgaaaaag acgaagaaat gtggataaag atcctgcaaa agaagatgtg   4380
   gaaaaagcta agaagagaag aggccgccct cccgctgaga aactgtcacc aaatcccccc   4440
   aaactgacaa agcagatgaa cgctatcatc gatactgtga taaactacaa agatagttca   4500
   gggcgacagc tcagtgaagt cttcattcag ttaccttcaa ggaaagaatt accagaatac   4560
   tatgaattaa ttaggaagcc agtggatttc aaaaaaataa aggaaaggat tcgtaatcat   4620
   aagtaccgga gcctaggcga cctggagaag gatgtcatgc ttctctgtca caacgctcag   4680
   acgttcaacc tggagggatc ccagatctat gaagactcca tcgtcttaca gtcagtgttt   4740
   aagagtgccc ggcagaaaat tgccaaagag gaagagagtg aggatgaaag caatgaagag   4800
   gaggaagagg aagatgaaga agagtcagag tccgaggcaa aatcagtcaa ggtgaaaatt   4860
   aagctcaata aaaaagatga caaaggccgg gacaaaggga aaggcaagaa aaggccaaat   4920
   cgaggaaaag ccaaacctgt agtgagcgat tttgacagcg atgaggagca ggatgaacgt   4980
   gaacagtcag aaggaagtgg gacggatgat gagtgatcag tatggacctt tttccttggt   5040
   agaactgaat tccttcctcc cctgtctcat ttctacccag tgagttcatt tgtcatatag   5100
   gcactgggtt gtttctatat catcatcgtc tataaactag ctttaggata gtgccagaca   5160
   aacatatgat atcatggtgt aaaaaacaca cacatacaca aatatttgtg accaaatggg   5220
   cctcaaagat tcagattgaa acaaacaaaa agctttt                            5257
<210> 40
   <211> 1635
   <212> DNA
   <213> Homo sapiens
<400> 40
   atgggcgagg tggaggcccc gggccgcttg tggctcgaga gcccccctgg ggaggcgccc 60
   cccatcttcc tgccctcgga cgggcaagcc ctggtcctgg gcaggggacc cctgacccag 120
   gttacggacc ggaagtgctc cagaactcaa gtggagctgg tcgcagatcc tgagacccgg 180
   acagtggcag tgaaacagct gggagttaac ccctcaacta ccgggaccca ggagttgaag 240
   ccggggttgg agggctctct gggggtgggg gacacactgt atttggtcaa tggcctccac 300
   ccactgaccc tgcgctggga agagacccgc acaccagaat cccagccaga tactccgcct 360
   ggcacccctc tggtgtccca agatgagaag agagatgctg agctgccgaa gaagcgtatg 420
   cggaagtcaa accccggctg ggagaacttg gagaagttgc tagtgttcac cgcagctggg 480
   gtgaaacccc agggcaaggt ggctggcttt gatctggacg ggacgctcat caccacacgc 540
   tctgggaagg tctttcccac tggccccagt gactggagga tcttgtaccc agagattccc 600
   cgtaagctcc gagagctgga agccgagggc tacaagctgg tgatcttcac caaccagatg 660
   agcatcgggc gcgggaagct gccagccgag gagttcaagg ccaaggtgga ggctgtggtg 720
   gagaagctgg gggtcccctt ccaggtgctg gtggccacgc acgcaggctt gtaccggaag 780
   ccggtgacgg gcatgtggga ccatctgcag gagcaggcca acgacggcac gcccatatcc 840
   atcggggaca gcatctttgt gggagacgca gccggacgcc cggccaactg ggccccgggg 900
   cggaagaaga aagacttctc ctgcgccgat cgcctgtttg ccctcaacct tggcctgccc 960
   ttcgccacgc ctgaggagtt ctttctcaag tggccagcag ccggcttcga gctcccagcc 1020
   tttgatccga ggactgtctc ccgctcaggg cctctctgcc tccccgagtc cagggccctc 1080
   ctgagcgcca gcccggaggt ggttgtcgca gtgggattcc ctggggccgg gaagtccacc 1140
   tttctcaaga agcacctcgt gtcggccgga tatgtccacg tgaacaggga cacgctaggc 1200
   tcctggcagc gctgtgtgac cacgtgtgag acagccctga agcaagggaa acgggtcgcc 1260
   atcgacaaca caaacccaga cgccgcgagc cgcgccaggt acgtccagtg tgcccgagcc 1320
   gcgggcgtcc cctgccgctg cttcctcttc accgccactc tggagcaggc gcgccacaac 1380
   aaccggtttc gagagatgac ggactcctct catatccccg tgtcagacat ggtcatgtat 1440
   ggctacagga agcagttcga ggccccaacg ctggctgaag gcttctctgc catcctggag 1500
   atcccgttcc ggctatgggt ggagccgagg ctggggcggc tgtactgcca gttctccgag 1560
   ggctgagccc cgcccagctc ccctccacaa taaacgctgt ttctccttga aaaaaaaaaa 1620
   aaaaaaaaaa aaaaa 1635
<210> 41
   <211> 978
   <212> DNA
   <213> Homo sapiens
<400> 41
   ccgcgtcggt gcccgcgccc ctccccgggc cccgccatgg gcctcaccgt gtccgcgctc      60
   ttttcgcgga tcttcgggaa gaagcagatg cggattctca tggttggctt ggatgcggct     120
   ggcaagacca caatcctgta caaactgaag ttgggggaga ttgtcaccac catcccaacc     180
   ataggcttca atgtagaaac agtggaatat aagaacatct gtttcacagt ctgggacgtg     240
   ggaggccagg acaagattcg gcctctgtgg cggcactact tccagaacac tcagggcctc     300
   atctttgtgg tggacagtaa tgaccgggag cgggtccaag aatctgctga tgaactccag     360
   aagatgctgc aggaggacga gctgcgggat gcagtgctgc tggtatttgc caacaagcag     420
   gacatgccca acgccatgcc cgtgagcgag ctgactgaca agctggggct acagcactta     480
   cgcagccgca cgtggtatgt ccaggccacc tgtgccaccc aaggcacagg tctgtacgat     540
   ggtctggact ggctgtccca cgagctgtca aagcgctaac cagccagggg caggcccctg     600
   atgcccggaa gctcctgcgt gcatccccgg gatgaccaga ctcccggact cctcaggcag     660
   tgccctttcc tcccactttt cctcccccat agccacaggc ctctgctcct gctcctgcct     720
   gcatgttctc tctgttgttg gagcctggag ccttgctctc tgggcacaga ggggtccact     780
   ctcctgcctg ctgggaccta tggaaggggc ttcctggcca aggccccctc ttccagagga     840
   ggagcaggga tctgggtttc cttttttttt tctgttttgg gtgtactcta ggggccaggt     900
   tgggaggggg aaggtgaggg cttcgggtgg tgctataatg tggcactgga tcttgagtaa     960
   taaatttgct gtggtttg                                                  978
<210> 42
   <211> 2334
   <212> DNA
   <213> Homo sapiens
<400> 42
   agacacctct gccctcacca tgagcctctg gcagcccctg gtcctggtgc tcctggtgct      60
   gggctgctgc tttgctgccc ccagacagcg ccagtccacc cttgtgctct tccctggaga     120
   cctgagaacc aatctcaccg acaggcagct ggcagaggaa tacctgtacc gctatggtta    180
   cactcgggtg gcagagatgc gtggagagtc gaaatctctg gggcctgcgc tgctgcttct    240
   ccagaagcaa ctgtccctgc ccgagaccgg tgagctggat agcgccacgc tgaaggccat    300
   gcgaacccca cggtgcgggg tcccagacct gggcagattc caaacctttg agggcgacct    360
   caagtggcac caccacaaca tcacctattg gatccaaaac tactcggaag acttgccgcg    420
   ggcggtgatt gacgacgcct ttgcccgcgc cttcgcactg tggagcgcgg tgacgccgct    480
   caccttcact cgcgtgtaca gccgggacgc agacatcgtc atccagtttg gtgtcgcgga    540
   gcacggagac gggtatccct tcgacgggaa ggacgggctc ctggcacacg cctttcctcc    600
   tggccccggc attcagggag acgcccattt cgacgatgac gagttgtggt ccctgggcaa    660
   gggcgtcgtg gttccaactc ggtttggaaa cgcagatggc gcggcctgcc acttcccctt    720
   catcttcgag ggccgctcct actctgcctg caccaccgac ggtcgctccg acggcttgcc    780
   ctggtgcagt accacggcca actacgacac cgacgaccgg tttggcttct gccccagcga    840
   gagactctac acccgggacg gcaatgctga tgggaaaccc tgccagtttc cattcatctt    900
   ccaaggccaa tcctactccg cctgcaccac ggacggtcgc tccgacggct accgctggtg    960
   cgccaccacc gccaactacg accgggacaa gctcttcggc ttctgcccga cccgagctga   1020
   ctcgacggtg atggggggca actcggcggg ggagctgtgc gtcttcccct tcactttcct   1080
   gggtaaggag tactcgacct gtaccagcga gggccgcgga gatgggcgcc tctggtgcgc   1140
   taccacctcg aactttgaca gcgacaagaa gtggggcttc tgcccggacc aaggatacag   1200
   tttgttcctc gtggcggcgc atgagttcgg ccacgcgctg ggcttagatc attcctcagt   1260
   gccggaggcg ctcatgtacc ctatgtaccg cttcactgag gggcccccct tgcataagga   1320
   cgacgtgaat ggcatccggc acctctatgg tcctcgccct gaacctgagc cacggcctcc   1380
   aaccaccacc acaccgcagc ccacggctcc cccgacggtc tgccccaccg gaccccccac   1440
   tgtccacccc tcagagcgcc ccacagctgg ccccacaggt cccccctcag ctggccccac   1500
   aggtcccccc actgctggcc cttctacggc cactactgtg cctttgagtc cggtggacga   1560
   tgcctgcaac gtgaacatct tcgacgccat cgcggagatt gggaaccagc tgtatttgtt   1620
   caaggatggg aagtactggc gattctctga gggcaggggg agccggccgc agggcccctt   1680
   ccttatcgcc gacaagtggc ccgcgctgcc ccgcaagctg gactcggtct ttgaggagcc   1740
   gctctccaag aagcttttct tcttctctgg gcgccaggtg tgggtgtaca caggcgcgtc   1800
   ggtgctgggc ccgaggcgtc tggacaagct gggcctggga gccgacgtgg cccaggtgac   1860
   cggggccctc cggagtggca gggggaagat gctgctgttc agcgggcggc gcctctggag   1920
   gttcgacgtg aaggcgcaga tggtggatcc ccggagcgcc agcgaggtgg accggatgtt   1980
   ccccggggtg cctttggaca cgcacgacgt cttccagtac cgagagaaag cctatttctg   2040
   ccaggaccgc ttctactggc gcgtgagttc ccggagtgag ttgaaccagg tggaccaagt   2100
   gggctacgtg acctatgaca tcctgcagtg ccctgaggac tagggctccc gtcctgcttt   2160
   gcagtgccat gtaaatcccc actgggacca accctgggga aggagccagt ttgccggata   2220
   caaactggta ttctgttctg gaggaaaggg aggagtggag gtgggctggg ccctctcttc   2280
   tcacctttgt tttttgttgg agtgtttcta ataaacttgg attctctaac cttt         2334
<210> 43
   <211> 2427
   <212> DNA
   <213> Homo sapiens
<400> 43
   agcctgtgac tttcctccct ggacaaaggc atcatgagtt gtcagatctc ttgcaaatct      60
   cgaggaagag gaggaggtgg aggaggattc cggggcttca gcagcggctc agctgtggtg     120
   tctggtggaa gccggagatc aacttccagc ttctcctgct tgagccgcca tggtggtggt     180
   ggcgggggct tcggtggagg cggctttggc agtcggagtc ttgttggcct tggagggacc     240
   aagagcatct ccattagtgt ggctggagga ggtggtggct ttggcgccgc tggtggattt     300
   ggtggcagag gaggtggttt tggaggcggc agcggctttg gaggcggcag cggctttgga     360
   ggtggcagcg gcttcagtgg tggtggtttc ggtggaggcg gctttggtgg aggccgcttt     420
   ggaggttttg ggggccctgg tggtgttgga ggtttagggg gtcctggtgg ctttgggcct      480
   ggaggatacc ctggtggcat ccacgaagtc tctgtcaacc agagcctcct gcagcctctc     540
   aacgtgaaag ttgacccaga gatccagaat gtgaaggccc aagagcgtga gcagatcaaa     600
   actctcaaca acaaatttgc ctccttcatt gacaaggtgc ggttcttgga gcagcagaac     660
   caggtgttac agaccaaatg ggagctgcta caacaaatga atgttggcac ccgccccatc     720
   aacctggagc ccatcttcca ggggtatatc gacagcctca agagatatct ggatgggctc     780
   actgcagaaa gaacatcaca gaattcagag ctgaataaca tgcaggatct tgtggaggat     840
   tataagaaga agtatgagga tgaaatcaat aagcgcacag ctgctgagaa tgattttgtg     900
   acgcttaaaa aggacgtgga caatgcctac atgataaagg tggagttgca gtccaaggtg     960
   gacctgctga accaggaaat tgagtttctg aaagttctct atgatgcgga gatatcccag    1020
   atacatcaga gtgtcactga caccaacgtc atcctctcca tggacaacag ccgcaacctg    1080
   gacttggata gcatcatcgc cgaggtcaag gcccagtatg aggagatcgc ccagaggagc    1140
   aaggaagaag cggaggccct gtaccacagc aagtatgagg agctccaggt gactgtcggg    1200
   agacatggag acagcctgaa agagatcaag atagagatca gcgagctgaa ccgcgtgatc    1260
   cagaggctgc agggggagat cgcacatgtg aagaagcagt gtaagaatgt gcaagatgcc    1320
   atcgcagatg ccgagcagcg tggggagcat gccctcaagg atgccaggaa caagttgaat    1380
   gacctggagg aggccctgca gcaggccaag gaggacttgg cgcggctgct gcgtgactac    1440
   caggagctga tgaacgtgaa gctggcccta gatgtggaga tcgccaccta ccgcaaactg    1500
   ctggagggcg aggagtgcag gatgtctgga gacctcagca gcaatgtgac tgtgtctgtg    1560
   acaagcagca ccatttcatc aaatgtggca tccaaggctg cctttggagg ttctggaggt    1620
   agagggtcca gttccggagg aggatacagc tctggaagca gcagttatgg ctctggaggc    1680
   cgacagtctg gctccagagg cggtagtgga ggaggaggtt ctatctctgg aggaggatat    1740
   ggctctggcg gtggttctgg aggaagatac ggatctggtg gtggctctaa gggagggtcc    1800
   atctctggag gaggatatgg ctctggaggt ggaaaacaca gctctggagg tggctctaga    1860
   ggaggctcca gctctggagg aggatatggc tctggaggtg ggggttctag ctctgtaaag    1920
   ggtagctcag gtgaagcttt tggttccagc gtgaccttct cttttagata aagatgagcc    1980
   cccaccacca ccgactctcc caacccagac tctcccactc cagaatgtag aagcctgtct 2040
   ctgtacctct aactggcagc aagttaaatt tttgtcattt atctctgatg gcactttgag 2100
   ggaaaagaat gtccacatac agtttttgaa agatcttctc tccaaaccag ttagttagag 2160
   ccagtgacgc ctctgtgttc tggggcggaa tctgtgctgt ctaggtttgt gcttctagcc 2220
   atgcccattc ccgcccccac catgcctctt tgcattgccc attttccaga tgtgtattct 2280
   gttgaggacc caggcccatc cagggatttc atctctaagc ctggcagtgc tggggggaaa 2340
   tgtgtttctg tgtatatagc tcctcttgtc cactctgctt tcggaagtgc tgtggtctgg 2400
   gggtcttcat aataaacctc atttgca 2427
<210> 44
   <211> 852
   <212> DNA
   <213> Homo sapiens
<400> 44
   caccctgtaa gtggctgcgg cgggaagatg gcggagctgc gcgtgctcgt agctgtcaag 60
   agggtcatcg actacgccgt gaagatccga gtgaagcctg acaggaccgg tgtggtcacg    120
   gatggtgtga agcactccat gaaccccttc tgtgagatcg cggtggagga ggctgtgcgg    180
   ctcaaggaga agaagctggt gaaggaggtc atcgccgtca gctgtgggcc tgcacagtgc 240
   caggagacga ttcgtaccgc cctggccatg ggtgcagacc gaggtatcca cgtggaggtg 300
   cccccagcag aagcagaacg cttgggtccc ctgcaggtgg ctcgggtcct ggccaagctg 360
   gcagagaagg agaaggtgga cctggtgctg ctgggcaaac aggccatcga tgatgactgt 420
   aaccagacag ggcagatgac agctggattt cttgactggc cacagggcac attcgcctcc 480
   caggtgacgc tggaggggga caagttgaaa gtggagcggg agatcgatgg gggcctggag 540
   accctgcgcc tgaagctgcc agctgtggtg acagctgacc tgaggctcaa cgagccccgc 600
   tacgccacgc tgcccaacat catgaaagcc aagaagaaga agatcgaggt gatcaagcct 660
   ggggacctgg gtgtggacct gacctccaag ctctctgtga tcagtgtgga ggacccgccc 720
   cagcgcacgg ccggcgtcaa ggtggagacc actgaggacc tggtggccaa gctgaaggag 780
   attgggcgga tttgagcccc tcccagagat ggcaataaaa ctgactctca acatcaaaaa 840
   aaaaaaaaaa aa 852
<210> 45
   <211> 581
   <212> DNA
   <213> Homo sapiens
<400> 45
   acaactgtgt tcactagcaa cctcaaacag acaccatggt gcacctgact cctgaggaga     60
   agtctgccgt tactgccctg tggggcaagg tgaacgtgga tgaagttggt ggtgaggccc    120
   tgggcaggct gctggtggtc tacccttgga cccagaggtt ctttgagtcc tttggggatc    180
   tgtccactcc tgatgctgtt atgggcaacc ctaaggtgaa ggctcatggc aagaaagtgc    240
   tcggtgcctt tagtgatggc ctggctcacc tggacaacct caagggcacc tttgccacac    300
   tgagtgagct gcactgtgac aagctgcacg tggatcctga gaacttcagg ctcctgggca    360
   acgtgctggt ctgtgtgccg gcccatcact ttggcaaaga attcacccaa ccagtgcagg    420
   ctgcctatca gaaagtggtg gctggtgtgg ctaatgccct ggcccacaag tatcactaag    480
   ctcgctttct tgctgtccaa tttctattaa aggttccttt gttccctaag tccaactact    540
   aaactggggg atattatgaa gggccttgag catctggatt c                        581
<210> 46
   <211> 2446
   <212> DNA
   <213> Homo sapiens
<400> 46
   ctgggtctgg gtctgagcct aggcatgagt gaggccacca gtgaggcaga ggaggaggcg 60
   gccacggccg aggcggtggg acgcctggcc acgacgctgt ggctgcggct ccgcggctgg 120
   gaggcggtgc tggcggcggc gcagcggttg ctggtgtggg agaagccgct gcacagcctg 180
   gtcacggcgg ccgcgctcaa cggcctcttc tggttgctgt cttcctcgtc cctccggccc 240
   ttcttcctac tcagcgtctc acttttggcc tattttctgc tggatctctg gcagcctcgc 300
   tttctccctg acgtttcagc atcatcccca gaggagccac actctgacag tgagggtgcg 360
   gggtcaggcg cccggccgca cctgctgagt gtgcccgagt tgtgcagata cctggctgag 420
   agctggctca ccttccagat tcacctgcag gagctgctgc agtacaagag gcagaatcca 480
   gctcagttct gcgttcgagt ctgctctggc tgtgctgtgt tggctgtgtt gggacactat 540
   gttccaggaa ttatgatttc ctacattgtc ttgttgagta tcctgctgtg gcccctggtg 600
   gtttatcatg agctgatcca gaggatgtac actcgcctgg agcccctgct catgcagctg 660
   gactacagca tgaaggcaga agccaatgcc ctgcatcaca aacacgacaa gaggaagcgt 720
   caggggaaga atgcaccccc aggaggtgat gagccactgg cagagacaga gagtgaaagc 780
   gaggcagagc tggctggctt ctccccagtg gtggatgtga agaaaacagc attggccttg 840
   gccattacag actcagagct gtcagatgag gaggcttcta tcttggagag tggtggcttc 900
   tccgtatccc gggccacaac tccgcagctg actgatgtct ccgaggattt ggaccagcag     960
   agcctgccaa gtgaaccaga ggagacccta agccgggacc taggggaggg agaggaggga    1020
   gagctggccc ctcccgaaga cctactaggc cgtcctcaag ctctgtcaag gcaagccctg 1080
   gactcggagg aagaggaaga ggatgtggca gctaaggaaa ccttgttgcg gctctcatcc    1140
   cccctccact ttgtgaacac gcacttcaat ggggcagggt cccccccaga tggagtgaaa    1200
   tgctcccctg gaggaccagt ggagacactg agccccgaga cagtgagtgg tggcctcact    1260
   gctctgcccg gcaccctgtc acctccactt tgccttgttg gaagtgaccc agccccctcc    1320
   ccttccattc tcccacctgt tccccaggac tcaccccagc ccctgcctgc ccctgaggaa 1380
   gaagaggcac tcaccactga ggactttgag ttgctggatc agggggagct ggagcagctg 1440
   aatgcagagc tgggcttgga gccagagaca ccgccaaaac cccctgatgc tccacccctg 1500
   gggcccgaca tccattctct ggtacagtca gaccaagaag ctcaggccgt ggcagagcca 1560
   tgagccagcc gttgaggaag gagctgcagg cacagtaggg cttcctggct aggagtgttg 1620
   ctgtttcctc ctttgcctac cactctgggg tggggcagtg tgtggggaag ctggctgtcg 1680
   gatggtagct attccaccct ctgcctgcct gcctgcctgc tgtcctgggc atggtgcagt 1740
   acctgtgcct aggattggtt ttaaatttgt aaataatttt ccatttgggt tagtggatgt 1800
   gaacagggct agggaagtcc ttcccacagc ctgcgcttgc ctccctgcct catctctatt 1860
   ctcattccac tatgccccaa gccctggtgg tctggccctt tctttttcct cctatcctca 1920
   gggacctgtg ctgctctgcc ctcatgtccc acttggttgt ttagttgagg cactttataa 1980
   tttttctctt gtcttgtgtt cctttctgct ttatttccct gctgtgtcct gtccttagca 2040
   gctcaacccc atcctttgcc agctcctcct atcccgtggg cactggccaa gctttaggga 2100
   ggctcctggt ctgggaagta aagagtaaac ctggggcagt gggtcaggcc agtagttaca 2160
   ctcttaggtc actgtagtct gtgtaacctt cactgcatcc ttgccccatt cagcccggcc 2220
   tttcatgatg caggagagca gggatcccgc agtacatggc gccagcactg gagttggtga 2280
   gcatgtgctc tctcttgaga ttaggagctt ccttactgct cctctgggtg atccaagtgt 2340
   agtgggaccc cctactaggg tcaggaagtg gacactaaca tctgtgcagg tgttgacttg 2400
   aaaaataaag tgttgattgg ctagaaaaaa aaaaaaaaaa aaaaaa 2446
<210> 47
   <211> 468
   <212> DNA
   <213> Homo sapiens
<220>
   <221> misc_feature
   <222> (6).. (6)
   <223> n is a, c, g, or t
<220>
   <221> misc_feature
   <222> (9)..(10)
   <223> n is a, c, g, or t
<220>
   <221> misc_feature
   <222> (12)..(12)
   <223> n is a, c, g, or t
<220>
   <221> misc_feature
   <222> (15).. (15)
   <223> n is a, c, g, or t
<220>
   <221> misc_feature
   <222> (18)..(18)
   <223> n is a, c, g, or t
<220>
   <221> misc feature
   <222> (30)..(30)
   <223> n is a, c, g, or t
<220>
   <221> misc_feature
   <222> (33)..(33)
   <223> n is a, c, g, or t
<220>
   <221> misc_feature
   <222> (36)..(36)
   <223> n is a, c, g, or t
<220>
   <221> misc_feature
   <222> (39).. (39)
   <223> n is a, c, g, or t
<220>
   <221> misc_feature
   <222> (42)..(42)
   <223> n is a, c, g, or t
<220>
   <221> misc_feature
   <222> (45)..(45)
   <223> n is a, c, g, or t
<220>
   <221> misc_feature
   <222> (51).. (51)
   <223> n is a, c, g, or t
<220>
   <221> misc_feature
   <222> (57)..(57)
   <223> n is a, c, g, or t
<220>
   <221> misc_feature
   <222> (60)..(60)
   <223> n is a, c, g, or t
<220>
   <221> misc_feature
   <222> (71)..(72)
   <223> n is a, c, g, or t
<220>
   <221> misc feature
   <222> (75) .. (75)
   <223> n is a, c, g, or t
<220>
   <221> misc_feature
   <222> (78)..(78)
   <223> n is a, c, g, or t
<220>
   <221> misc_feature
   <222> (93)..(93)
   <223> n is a, c, g, or t
<220>
   <221> misc_feature
   <222> (99)..(99)
   <223> n is a, c, g, or t
<220>
   <221> misc feature
   <222> (129)..(129)
   <223> n is a, c, g, or t
<220>
   <221> misc_feature
   <222> (132)..(132)
   <223> n is a. c, g, or t
<220>
   <221> misc feature
   <222> (135)..(135)
   <223> n is a. c, g, or t
<220>
   <221> misc feature
   <222> (138)..(138)
   <223> n is a, c, g, or t
<220>
   <221> misc feature
   <222> (148)..(150)
   <223> n is a, c, g, or t
<220>
   <221> misc_feature
   <222> (153)..(153)
   <223> n is a, c, g, or t
<220>
   <221> misc_feature
   <222> (156)..(156)
   <223> n is a, c, g, or t
<220>
   <221> misc_feature
   <222> (159).. (159)
   <223> n is a, c, g, or t
<220>
   <221> misc feature
   <222> (291)..(291)
   <223> n is a, c, g, or t
<220>
   <221> misc_feature
   <222> (294)..(294)
   <223> n is a, c, g, or t
<220>
   <221> misc_feature
   <222> (318)..(318)
   <223> n is a, c, g, or t
<220>
   <221> misc_feature
   <222> (321)..(321)
   <223> n is a, c, g, or t
<220>
   <221> misc_feature
   <222> (331)..(331)
   <223> n is a, c, g, or t
<220>
   <221> misc_feature
   <222> (333)..(333)
   <223> n is a, c, g, or t
<220>
   <221> misc feature
   <222> (339)..(339)
   <223> n is a, c. g, or t
<220>
   <221> misc_feature
   <222> (384)..(384)
   <223> n is a, c, g, or t
<220>
   <221> misc_feature
   <222> (387)..(387)
   <223> n is a, c, g, or t
<220>
   <221> misc_feature
   <222> (405)..(405)
   <223> n is a, c, g, or t
<220>
   <221> misc_feature
   <222> (414)..(414)
   <223> n is a, c, g, or t
<220>
   <221> misc_feature
   <222> (448)..(448)
   <223> n is a, c, g, or t
<220>
   <221> misc_feature
   <222> (451)..(451)
   <223> n is a, c, g, or t
<220>
   <221> misc_feature
   <222> (454)..(454)
   <223> n is a, c, g, or t
<220>
   <221> misc_feature
   <222> (458)..(458)
   <223> n is a, c, g, or t
<400> 47
   atggtncann tnacnccngt ggagaagtcn gcngtnacng cnctntgggg naaggtnaan      60
   gtggatgaag nnggnggnga ggccctgggc agnctgctng tggtctaccc ttggacccag     120
   aggttcttng antcnttngg ggatctgnnn acnccngang cagttatggg caaccctaag     180
   gtgaaggctc atggcaagaa agtgctcggt gcctttagtg atggcctggc tcacctggac     240
   aacctcaagg gcacctttgc cacactgagt gagctgcact gtgacaagct ncangtggat     300
   cctgagaact tcaggctnct nggcaacgtg ntngtctgng tgctggccca tcactttggc     360
   aaagaattca ccccaccagt gcangcngcc tatcagaaag tggtngctgg tgtngctaat     420
   gccctggccc acaagtatca ctaagctngc nttnttgntg tccaattt                  468
<210> 48
   <211> 576
   <212> DNA
   <213> Homo sapiens
<400> 48
   actcttctgg tccccacaga ctcagagaga acccaccatg gtgctgtctc ctgccgacaa      60
   gaccaacgtc aaggccgcct ggggtaaggt cggcgcgcac gctggcgagt atggtgcgga     120
   ggccctggag aggatgttcc tgtccttccc caccaccaag acctacttcc cgcacttcga     180
   cctgagccac ggctctgccc aggttaaggg ccacggcaag aaggtggccg acgccctgac     240
   caacgccgtg gcgcacgtgg acgacatgcc caacgcgctg tccgccctga gcgacctgca     300
   cgcgcacaag cttcgggtgg acccggtcaa cttcaagctc ctaagccact gcctgctggt     360
   gaccctggcc gcccacctcc ccgccgagtt cacccctgcg gtgcacgcct ccctggacaa     420
   gttcctggct tctgtgagca ccgtgctgac ctccaaatac cgttaagctg gagcctcggt     480
   ggccatgctt cttgcccctt gggcctcccc ccagcccctc ctccccttcc tgcacccgta     540
   cccccgtggt ctttgaataa agtctgagtg ggcggc                               576
<210> 49
   <211> 756
   <212> DNA
   <213> Homo sapiens
<400> 49
   cctctcccac cggcccgatg agctgcagcg gctccggcgc ggaccccgag gcggcgccgg      60
   cctccgccgc ctcggccccg ggccccgcgc ccccggtctc ggctcccgcc gcgctgccct     120
   ccagcaccgc cgcggagaac aaggccagcc ccgcggggac agcgggggga cctggggctg     180
   gagcagctgc tgggggcacg ggacccttgg cggcgcgggc cggggagcca gctgagcggc     240
   gtggggcggc tccggtgtcg gcgggtggcg cggcgccccc ggagggggcc atatctaacg    300
   gggtttacgt actgccgagc gcggccaacg gagacgtgaa gcccgtggtg tccagcacgc    360
   ctttggtgga cttcttgatg cagctggaag attacacgcc tacgatccca gatgcagtga    420
   ctggttacta cctgaaccgt gctggctttg aggcctcaga cccacgcata attcggctca    480
   tctccttagc tgcccagaaa ttcatctcag atattgccaa tgatgcccta cagcactgca    540
   aaatgaaggg cacggcctcc ggcagctccc ggagcaagag caaggaccgc aagtacactc    600
   taaccatgga ggacttgacc cctgccctca gcgagtatgg catcaatgtg aagaagccgc    660
   actacttcac ctgagccacc caacctaaat gtacttatct gtccccatgt ccccacacca    720
   gcctgttttc ataataaact ttattgtgac aggcta                              756
<210> 50
   <211> 1211
   <212> DNA
   <213> Homo sapiens
<400> 50
   ggcacgaggg gagccgaagc agcagcgcag gttgtccccg tttcccctcc cccttccctt      60
   ctccggttgc cttcccgggc cccttacact ccacagtccc ggtcccgcca tgtcccagaa     120
   acaagaagaa gagaaccctg cggaggagac cggcgaggag aagcaggaca cgcaggagaa     180
   agaaggtatt ctgcctgaga gagctgaaga ggcaaagcta aaggccaaat acccaagcct     240
   aggacaaaag cctggaggct ccgacttcct catgaagaga ctccagaaag ggcaaaagta     300
   ctttgactca ggagactaca acatggccaa agccaagatg aagaataagc agctgccaag     360
   tgcaggacca gacaagaacc tggtgactgg tgatcacatc cccaccccac aggatctgcc     420
   ccagagaaag tcctcgctcg tcaccagcaa gcttgcgggt ggccaagttg aatgatgctg     480
   cccggggctc tgccagatcc tgagacgctt cccctccctg ccccacccgg gtcctgtgct     540
   ggctcctgcc ccttcctgct tttgcagcca ggggtcagga ggtggctcgg gtgtgggctg     600
   gagaggcaga agccctttcc tgttggtgtc ccagcacatg gagccccttg ggctgagcac     660
   caagaccttg aacctttttt gttttacctt ttttccaaat aacagttggg agaaatatca     720
   atgaaattct gggggtgggg gtggggttga aagggtgggg tgggagatat ggaggagtat     780
   gaattagggc ttggagttgg taaaaacatt cctgactatc cttcttaacc acgtggctga     840
   tgtggggtag gtatgagggg aaggaagtgg agtagcctaa tgaaaagggg ttctagttga     900
   gctctgtaga taaatgcctt gtttcagtgt ggttggagac ctggtgtcag ataaaagaaa     960
   ctccatccgc acagacagat gcaaacagct cctctagttc tgcagagcta gttgagaact    1020
   caacattaat cattttaaaa agtactgtcc ttgaaataga tttgctgtgg gaagaagggc    1080
   agtgagtgtg ggagaaagga gccgtgagcg tggggaaccc cacagagccc aaaggacttt    1140
   ttcagtattc gaaataaaca aaacaaaaac ccatgaaaaa acccaaccca gaaaaaaaaa    1200
   aaaaaaaaaa a                                                         1211
<210> 51
   <211> 1216
   <212> DNA
   <213> Homo sapiens
<400> 51
   ctgaagaccc agagagattt catgctcagt tttagcaccg acccccagga cttcatccag      60
   gaatggctcc gttcccagcg ccgagacctc aagatcatca ctgatgtgat tggaaatcct     120
   gaggaggaga gacgagctgc tttctaccac cagccctggg cccaggaagc agtaggcagg     180
   cacatctttg ccaaggtgca gcagcgaagg caggaactgg aacaggtgct gggaattcgc     240
   ctgacctaac tgctcaggga tctttcttcc cagccctgga gcctggaggg agaccaccct     300
   ctgggtcctt gctggggccg cagacacgta ggctggggtg aggagtgtct gctgtcaccc     360
   tctactctcc agctttagtt ttataaatgt agtgatagga ttccttgttg cttggtcccc     420
   aaagccttat actttttgca ttggctttaa ttgggttcag cagatgcctc ctctgccccc     480
   ctgcaggcag gcccaagtag gactgctgga ggctgtgctt tgacattgta agacatttcc     540
   gaaccaaagg ctgctgggtt tgcatgttta cagactcccc ctggggcgag ggtcagagct     600
   ggctctgggg agctgggcta ggaagaggag gtgcagccta gactcttcct agcctttcta     660
   aaccaaagtt ctttgccatt cctacaagcc cagccttgct gctggttttt tcctttcctt     720
   tgggtatttg cactattttg ggagcaagtt ttctatgtgg gagccacttt ttttgtacag     780
   gggtaagttg ggggttttca gggagcctgt taggtgcctc cttcttttct ttcctcaatc     840
   tatgcaagcg gctctggccg ccatcatctc ctgggatgcc agagggctgc ctctccagcg     900
   gcttgggccg gggaggggac actccagttc tctagcatgg cctgaggtat ggggtatgtg     960
   catgtggagg ccagggtaag gtgaatgggg aggctgggag gactggtgtt gccctttgga    1020
   gcttggtgag gagggtgggc ctagggcttg gcgagtgcca cttttggcag gtttggaaat    1080
   ttccaaataa atccttttgt ctaaaaaaaa aaaaaaaaaa aaaaaaaaaa aaaaaaaaaa    1140
   aaaaaaaaaa aaaaaaaaaa aaaaaaaaaa aaaaaaaaaa aaaaaaaaaa aaaaaaaaaa    1200
   aaaaaaaaaa aaaaaa                                                    1216
<210> 52
   <211> 366
   <212> DNA
   <213> Homo sapiens
<220>
   <221> misc feature
   <222> (19)..(19)
   <223> n is a, c, g, or t
<220>
   <221> misc_feature
   <222> (84)..(85)
   <223> n is a, c, g, or t
<220>
   <221> misc_feature
   <222> (89)..(89)
   <223> n is a, c, g, or t
<220>
   <221> misc_feature
   <222> (93)..(99)
   <223> n is a, c, g, or t
<220>
   <221> misc_feature
   <222> (96)..(101)
   <223> n is a, c, g, or t
<220>
   <221> misc_feature
   <222> (172)..(172)
   <223> n is a, c, g, or t
<220>
   <221> misc_feature
   <222> (191)..(191)
   <223> n is a, c, g, or t
<220>
   <221> misc_feature
   <222> (267)..(267)
   <223> n is a, c, g, or t
<220>
   <221> misc_feature
   <222> (278)..(278)
   <223> n is a, c, g, or t
<220>
   <221> misc_feature
   <222> (282)..(282)
   <223> n is a, c, g, or t
<220>
   <221> misc_feature
   <222> (285)..(285)
   <223> n is a, c, g, or t
<220>
   <221> misc_feature
   <222> (301)..(301)
   <223> n is a, c, g, or t
<220>
   <221> misc_feature
   <222> (328)..(328)
   <223> n is a, c, g, or t
<400> 52
   tttttttttt ttttgccgnc cactcagact ttattcaaag accaggaagg gccggtgcaa      60
   ggaggggagg agggcccgtt gganngccna gcnngnnnnn ngaacggcta ccgaggctcc     120
   agcttaacgg tatttggagg tcagcacggt gctcacagaa gccaggaact tntccaggga     180
   ggcgtgcacc ncaggggttg aactcggcgg ggaggtgggc ggccagggtc accagcaggc     240
   agtggcttag gagcttgaag ttgaccnggg tccacccnaa gnttntgcgc gtgcaggtcg     300
   ntcagggcgg acagcgcgtt tgggcatntc gtccacgtgc gccacggctt tgggtcaagc     360
   gcgttc                                                                366
<210> 53
   <211> 3775
   <212> DNA
   <213> Homo sapiens
<400> 53
   cattcataag actcagagct acggccacgg cagggacacg cggaaccaag acttggaaac      60
   ttgattgttg tggttcttct tgggggttat gaaatttcat taatcttttt tttttccggg     120
   gagaaagttt ttggaaagat tcttccagat atttcttcat tttcttttgg aggaccgact     180
   tacttttttt ggtcttcttt attactcccc tccccccgtg ggacccgccg gacgcgtgga     240
   ggagaccgta gctgaagctg attctgtaca gcgggacagc gctttctgcc cctgggggag     300
   caacccctcc ctcgcccctg ggtcctacgg agcctgcact ttcaagaggt acagcggcat     360
   cctgtggggg cctgggcacc gcaggaagac tgcacagaaa ctttgccatt gttggaacgg 420
   gacgttgctc cttccccgag cttccccgga cagcgtactt tgaggactcg ctcagctcac 480
   cggggactcc cacggctcac cccggacttg caccttactt ccccaacccg gccatagcct 540
   tggcttcccg gcgacctcag cgtggtcaca ggggcccccc tgtgcccagg gaaatgtttc 600
   aggctttccc cggagactac gactccggct cccggtgcag ctcctcaccc tctgccgagt 660
   ctcaatatct gtcttcggtg gactccttcg gcagtccacc caccgccgcg gcctcccagg 720
   agtgcgccgg tctcggggaa atgcccggtt ccttcgtgcc cacggtcacc gcgatcacaa 780
   ccagccagga cctccagtgg cttgtgcaac ccaccctcat ctcttccatg gcccagtccc 840
   aggggcagcc actggcctcc cagcccccgg tcgtcgaccc ctacgacatg ccgggaacca 900
   gctactccac accaggcatg agtggctaca gcagtggcgg agcgagtggc agtggtgggc 960
   cttccaccag cggaactacc agtgggcctg ggcctgcccg cccagcccga gcccggccta 1020
   ggagaccccg agaggagacg ctcaccccag aggaagagga gaagcgaagg gtgcgccggg 1080
   aacgaaataa actagcagca gctaaatgca ggaaccggcg gagggagctg accgaccgac 1140
   tccaggcgga gacagatcag ttggaggaag aaaaagcaga gctggagtcg gagatcgccg 1200
   agctccaaaa ggagaaggaa cgtctggagt ttgtgctggt ggcccacaaa ccgggctgca 1260
   agatccccta cgaagagggg cccgggccgg gcccgctggc ggaggtgaga gatttgccgg 1320
   gctcagcacc ggctaaggaa gatggcttca gctggctgct gccgcccccg ccaccaccgc 1380
   ccctgccctt ccagaccagc caagacgcac cccccaacct gacggcttct ctctttacac 1440
   acagtgaagt tcaagtcctc ggcgacccct tccccgttgt taacccttcg tacacttctt 1500
   cgtttgtcct cacctgcccg gaggtctccg cgttcgccgg cgcccaacgc accagcggca 1560
   gtgaccagcc ttccgatccc ctgaactcgc cctccctcct cgctcggtga actctttaga 1620
   cacacaaaac aaacaaacac atgggggaga gagacttgga agaggaggag gaggaggaga 1680
   aggaggagag agaggggaag agacaaagtg ggtgtgtggc ctccctggct cctccgtctg 1740
   accctctgcg gccactgcgc cactgccatc ggacaggagg attccttgtg ttttgtcctg 1800
   cctcttgttt ctgtgccccg gcgaggccgg agagctggtg actttgggga cagggggtgg 1860
   gaaggggatg gacaccccca gctgactgtt ggctctctga cgtcaaccca agctctgggg 1920
   atgggtgggg aggggggcgg gtgacgccca ccttcgggca gtcctgtgtg aggatgaagg 1980
   gacgggggtg ggaggtaggc tgtggggtgg gctggagtcc tctccagaga ggctcaacaa 2040
   ggaaaaatgc cactccctac ccaatgtctc ccacacccac cctttttttg gggtgcccag 2100
   gttggtttcc cctgcactcc cgaccttagc ttattgatcc cacatttcca tggtgtgaga 2160
   tcctctttac tctgggcaga agtgagcccc cccttaaagg gaattcgatg cccccctaga 2220
   ataatctcat ccccccaccc gacttctttt gaaatgtgaa cgtccttcct tgactgtcta 2280
   gccactccct cccagaaaaa ctggctctga ttggaatttc tggcctccta aggctcccca 2340
   ccccgaaatc agcccccagc cttgtttctg atgacagtgt tatcccaaga ccctgccccc 2400
   tgccagccga ccctcctggc cttcctcgtt gggccgctct gatttcaggc agcaggggct 2460
   gctgtgatgc cgtcctgctg gagtgattta tactgtgaaa tgagttggcc agattgtggg 2520
   gtgcagctgg gtggggcagc acacctctgg ggggataatg tccccactcc cgaaagcctt 2580
   tcctcggtct cccttccgtc catccccctt cttcctcccc tcaacagtga gttagactca 2640
   agggggtgac agaaccgaga agggggtgac agtcctccat ccacgtggcc tctctctctc 2700
   tcctcaggac cctcagccct ggcctttttc tttaaggtcc cccgaccaat ccccagccta 2760
   ggacgccaac ttctcccacc ccttggcccc tcacatcctc tccaggaagg cagtgagggg 2820
   ctgtgacatt tttccggaga agatttcaga gctgaggctt tggtaccccc aaacccccaa 2880
   tatttttgga ctggcagact caaggggctg gaatctcatg attccatgcc cgagtccgcc 2940
   catccctgac catggttttg gctctcccac cccgccgttc cctgcgcttc atctcatgag 3000
   gatttcttta tgaggcaaat ttatattttt taatatcggg gggtggacca cgccgccctc 3060
   catccgtgct gcatgaaaaa cattccacgt gccccttgtc gcgcgtctcc catcctgatc 3120
   ccagacccat tccttagcta tttatccctt tcctggtttc cgaaaggcaa ttatatctat 3180
   tatgtataag taaatatatt atatatggat gtgtgtgtgt gcgtgcgcgt gagtgtgtga 3240
   gcgcttctgc agcctcggcc taggtcacgt tggccctcaa agcgagccgt tgaattggaa 3300
   actgcttcta gaaactctgg ctcagcctgt ctcgggctga cccttttctg atcgtctcgg 3360
   cccctctgat tgttcccgat ggtctctctc cctctgtctt ttctcctccg cctgtgtcca 3420
   tctgaccgtt ttcacttgtc tcctttctga ctgtccctgc caatgctcca gctgtcgtct 3480
   gactctgggt tcgttgggga catgagattt tattttttgt gagtgagact gagggatcgt 3540
   agatttttac aatctgtatc tttgacaatt ctgggtgcga gtgtgagagt gtgagcaggg 3600
   cttgctcctg ccaaccacaa ttcaatgaat ccccgacccc cctaccccat gctgtacttg 3660
   tggttctctt tttgtatttt gcatctgacc ccggggggct gggacagatt ggcaatgggc 3720
   cgtcccctct ccccttggtt ctgcactgtt gccaataaaa agctcttaaa aacgc 3775
<210> 54
   <211> 1970
   <212> DNA
   <213> Homo sapiens
<220>
   <221> misc_feature
   <222> (853)..(853)
   <223> n is a, c, g, or t
<220>
   <221> misc feature
   <222> (979)..(979)
   <223> n is a, c, g, or t
<220>
   <221> misc_feature
   <222> (1049)..(1049)
   <223> n is a, c, g, or t
<220>
   <221> misc_feature
   <222> (1061)..(1061)
   <223> n is a, c, g, or t
<220>
   <221> misc_feature
   <222> (1579)..(1579)
   <223> n is a, c, g, or t
<220>
   <221> misc_feature
   <222> (1614)..(1614)
   <223> n is a, c, g, or t
<220>
   <221> misc_feature
   <222> (1632)..(1632)
   <223> n is a, c, g, or t
<220>
   <221> misc_feature
   <222> (1870)..(1870)
   <223> n is a, c, g, or t
<220>
   <221> misc_feature
   <222> (1877) ..(1877)
   <223> n is a, c, g, or t
<220>
   <221> misc feature
   <222> (1948)..(1948)
   <223> n is a, c, g, or t
<400> 54
   tttttttttt tgaaagggtc aaaaatttta ttagcaggac tttttgtgtt tttgaatata      60
   caggtttcct gctgtccagg gtaaagggga agtggtgtct tgtggcccga ggtttggggc     120
   gcttgccttg ggactccatc cccatctctt tcccgctagc gcagctgggg gaaggtgcct     180
   gcttgccggc cccacggatt cttcggctgt ggcataaggc actgtgtgtt ctgcaggaag     240
   gcgctcatgg ctggctggta gatgagaggc ggccttctca ggggctgcaa agctgaggct     300
   gttgaggggt tgagttgaac ttggggcccc tagtgaggag gccccaggcg atgggagcac     360
   ggcctggtga gtctggtgcc gctgcctccc cactggccct gcaccaggtc tcaggatggg     420
   atcatgcctt tgctcctctt gcggtcggcc atggttctcc ggttgtggtt ggctcttgtc     480
   gccttgttgg cttccttctt cctgcgttcc tgggttgtct cgcggctctg cccatggcct     540
   cgggggctgc cggccactgc tgttgagctg tcatgccggt accctttctt ggcgagaaag     600
   gccatgcgcc tggcttctgc cttctctctc agcactgcag ggtcctgaac aaaatggtcg     660
   ggcttgggag cctcctcgtc agcatcgtct tcctcatcgt cgtcatcctc ctcctgccct     720
   tctctaggca ctttggttct cagcacctga gggatggtga atggcctgcg gctgatgagc     780
   tcgtcatcag agtctgcatc attggcgccc acctggttgc catcgtatgt gtcatcgtac     840
   tcatcctcgt agntagacac tgtggtaggg caggctctcg cctggctgca gtggcacctg     900
   caatggcata agcatgggtc tggggggctc aggggctggc ctggtttccc tggcccagca     960
   gagacctggc cttggtggnc tacctcctcc accaccacgc tgtactgctc gtagcgctgc    1020
   cgctgtgccg ccactgcacg cttgtcgtnc agcaaactcc ncgtgttttc ctccttcctg    1080
   gtgctcttgc ccttgtgcac ccggctcagg tctactgagt ccctgctgaa cacatcaaac    1140
   tcgtcattct ggaagacgtt gtggcgagac gtcagcaggg gtgtagggtc tggtttcatt    1200
   tctctgtcta ggttgcggtc cagctggctg agggtggggg ccagccgctc ctccaggata    1260
   ttgttgatca cctgctctgg gtcgtagtgg tagtactcca ggcaggccag gatgaagccc    1320
   tcaccaaggt ctggcagcag gtccttcact tgggagatga gagagtccag ttccacccca    1380
   cacatggcag ggcccacagc cgcggctgct cccatgcact cctcttcctc cgagttctcc    1440
   ggatgtgatg atgcttgact gactgcctct gctgtcaccg tgaccccgtt aggctcccca    1500
   ttaggctcct caatcaccga tgggtcttta gcatctgtgg ctttccgtct gtccacccct    1560
   tcccatgcac tctcgactnc ctggaggatg taggcagtcc gcgtctcgtc caanactgat    1620
   gaggcctgct gnagcaagct gatgtcttcg gccacgggga agagtgcatt catagtcccc    1680
   ggaggaacct cttctcctgc agcaaggagc tgaagatctg aaggaactct tcgatgaagc    1740
   cctgaatgtt gtcacagctg ctttctagga tgggaaggag gcagatctgg ttcaggatga    1800
   tgtggaaaat ctccattagc ttcttcctgg aatgggagag cctctgccac aggtcaccaa    1860
   gaagcctgtn acaaaantcg tgcttctgga aggtctggca agccaaaggg aagatatcca    1920
   aaaaggccca aagtgtggtg caggtatnaa aaaggtagag aacaatgtcc                1970
<210> 55
   <211> 3869
   <212> DNA
   <213> Homo sapiens
<220>
   <221> misc feature
   <222> (889)..(890)
   <223> n is a, c, g, or t
<220>
   <221> misc_feature
   <222> (896)..(896)
   <223> n is a, c, g, or t
<220>
   <221> misc_feature
   <222> (970)..(973)
   <223> n is a, c, g, or t
<220>
   <221> misc_feature
   <222> (1415)..(1415)
   <223> n is a, c, g, or t
<220>
   <221> misc feature
   <222> (1417)..(1417)
   <223> n is a, c, g, or t
<220>
   <221> misc_feature
   <222> (2174)..(2174)
   <223> n is a, c, g, or t
<220>
   <221> misc_feature
   <222> (2270)..(2270)
   <223> n is a, c, g, or t
<220>
   <221> misc_feature
   <222> (2272)..(2272)
   <223> n is a, c, g, or t
<220>
   <221> misc_feature
   <222> (2322)..(2322)
   <223> n is a, c, g, or t
<220>
   <221> misc_feature
   <222> (2675)..(2676)
   <223> n is a, c, g, or t
<220>
   <221> misc_feature
   <222> (2702)..(2702)
   <223> n is a, c, g, or t
<220>
   <221> misc feature
   <222> (2780)..(2780)
   <223> n is a, c, g, or t
<220>
   <221> misc_feature
   <222> (3238)..(3238)
   <223> n is a, c, g, or t
<220>
   <221> misc_feature
   <222> (3278)..(3278)
   <223> n is a, c, g, or t
<220>
   <221> misc_feature
   <222> (3302)..(3302)
   <223> n is a, c, g, or t
<220>
   <221> misc feature
   <222> (3347)..(3347)
   <223> n is a, c, g, or t
<220>
   <221> misc_feature
   <222> (3366)..(3366)
   <223> n is a, c, g, or t
<220>
   <221> misc_feature
   <222> (3380)..(3380)
   <223> n is a, c, g, or t
<220>
   <221> misc_feature
   <222> (3409)..(3410)
   <223> n is a, c, g, or t
<220>
   <221> misc_feature
   <222> (3687)..(3687)
   <223> n is a, c, g, or t
<220>
   <221> misc feature
   <222> (3696)..(3701)
   <223> n is a, c, g, or t
<220>
   <221> misc feature
   <222> (3703)..(3703)
   <223> n is a, c, g, or t
<400> 55
   cctggtttct ctacatacta accccatgtc tctctggact cacacctggc tcaggccttc     60
   cagaccaaac tcatacttcc tcctcaccag aagtttccga atctcacact agagaagatt    120
   tgtcaccttt aaagtccact cacattattt tgactcaggg ttttgttttt tgtctatttc    180
   ccctcctgca actctccttt aaaagtgaaa agagagagaa tggtttaagt agtctgttgc    240
   tcataagcac gtgtctgcag tctgtcactt gcaaggcctt ggtgatcacc atgcagcact    300
   tgtgcgcaca cacaccccca caagtctttt ttcctaataa tccattttcc acattgccat    360
   gagggtaaag ggttccaatc aaaaccacca ttaaactgca tagtttctag tcttccctca    420
   gccctgatgc taattatgat agtttgcata gacataactt aaacttgtga ttctccctcc 480
   ttccagagct ttttgtagcc ctttttcccc ccaaaattag ttttgttcaa aatcaagcat 540
   tgggcccagc cattaagata agacctaata agtgagagat acagttccca agagtttaca 600
   gatccaggtg ttcacatggt ggtggccagt ctaccgttta tcttgtcaag gttgttactg 660
   ctaatttggt gtcactttgg tcttgggcag cggtggtggg cgacgtatct aatgagcgtg 720
   ccattgactt ctccatgttt gtctgtgtac gaggggtgca ggttgtctgt aatccactaa 780
   agtgtcagcc acacccagct ttagtaactc gctaatcgca caccctgggt atatcagcca 840
   ggggctgcta cagttaatgg tacacagttg atggtacaca ggctgaaann gataangaat 900
   caagacccta ctacatttcc aaagccgaaa agccatgagg gttacaattt aaatgagctt 960
   gggaaggaan nnncccgaag acgtctccag cctgaagggt cagagccgct ggaagcctgg 1020
   ctgtctctgg gctttctcgt ctcagtgcag caaggcaccg tgatcaacca acccgtgatg 1080
   gggaaggccc tggttctgtc tttgtttggg ggtaaatagc tgtgtggtgt tgagtgactg 1140
   tgattggctt ttccagctcc tttaacacaa actacaaccc actcacgata caacttcggc 1200
   agatgtggtc aaattgatgt ttgttgactt gcctcatttc cttccagctc ttaagttcca 1260
   aagtctaact gggatggtag atgtgaaatg catgattgtc gtcctaattt ctgcatgtgt 1320
   ttatgacaat gtgttgaggg gagggggagg agagccagag agcattttca ggaaccgagg 1380
   tctttccttg tcttactctg ggggaatgcc atgtngnctc tgatagagtt gtctatcggt 1440
   ctgtttgtga atgccctttc gcttaaactt ccagcccttc tggtggcatc cttggttgta 1500
   aggtggctgc tgtataacgt ccactctctt tcatatttaa tatgtgtgat tgttactgtt 1560
   gtctttttgt tttaaagtaa ccataagagg agatgatctg agttatattt attgatcaga 1620
   ggaatagagt gcagtaaaac tcatactggc atttcgcttt tctcatttcc ctgtaaatgg 1680
   gcgggtatta atccattccc accttctaat gtccttgatg tccagctggc tttgtgggtg 1740
   tgcaatggtt ttactgtact gcctcgatct gtgccaggca tttatacaca gccacatgtg 1800
   cgagtcaggt agagcgtatg tggtcacccc catggctcac ggagttagct gaatggcctc 1860
   tccgctgtcc ctagatggca gaaacggtgg acacaagcga aatggtcaac ggcgctacag 1920
   aacaaaggac gagctctaaa gagtccagcc ccatcccctc cccgacctct gatcgtaaag 1980
   ccaagactgc cctcccagcc cagagtgccg ccaccttacc agccagaacc caggagacac 2040
   cttcggccca gatggaaggc ttcctcaatc ggaaacacga gtgggaggcc cacaataaga 2100
   aagcctcaag caggtcctgg cacaatgttt attgtgtcat aaataaccaa gaaatgggtt 2160
   tctacaaaga tgcnaagact gctgcttctg gaattcccta ccacagcgag gtccctgtga 2220
   gtttgaaaga agctgtctgc gaagtggccc ttgattacaa aaagaagaan cncgtattca 2280
   agctaagact aaatgatggc aatgagtacc tcttccaagc cnaagacgat gaggaaatga 2340
   acacatggat ccaggctatc tcttccgcca tctcctctga taaacacgag gtgtctgcca 2400
   gcacccagag cacgccagca tccagccgcg cgcagaccct ccccaccagc gtcgtcacca 2460
   tcaccagcga gtccagtccc ggcaagcggg aaaaggacaa agagaaagac aaagagaagc 2520
   ggttcagcct ttttggcaaa aagaaatgaa ctcctttcct tcacctcctg cccttctctt 2580
   accttttcag tgaaattcca gcatgcaagc tcagaaccaa cacattactc tctgtgccta 2640
   atgttcctca atgtggttga tttttttttt ttttnnaatt tatagagcat ttcggggggg 2700
   gntgggggaa acacacctaa acactttatc tccaagttac aaaagtttga ggtgcagagg 2760
   gaaggccaga tttttttttn aatgaaatta tatagattag atctcagtat ttaaactgtt 2820
   cctcaatttt gtgaggctgt gttggaaata acccgcctct agtgctgttg gtatgcaagg 2880
   cagcggtgct taatcaatat ttcctgtgct caccagaggc aaaatgtacc aatatcctga 2940
   caccattctc tctccattta cttctggtgg ttaccctgac tcttgactct tagaagtgcc 3000
   cgagatgggg ctaaccttta ttaaacagat cgcatattat gatcttgctg cagccacagt 3060
   gcagctccac attaactcta cagaccaaac catttgtatc tggcatcact tactaacaca 3120
   cgacatgcgg cttttctgca tcaactgcta tgacggttaa gaatgtcagt atacaagaag 3180
   gaatagaaaa ctgatactgt tttaaataat ctgtaatttc aatttttttt tttttttngc 3240
   tgaaatacat tatattgtac gtttgagata attctagnta caaagtataa taaaactaga 3300
   tngtataata aaccctttaa atcattggta agtgtacaag tggtggnaac tgaagcattt 3360
   actggnacaa agtaatgttn actctaatgg ttacttgctc gtgcgttgnn ccacactgtg 3420
   ttataatttg cttcatttcc ttgctatttg atacatagtg tgcatttctc tgtcactgta 3480
   actattgtaa tgacaaattt tcatcttact gcacaatcaa aatgacattg ataggaatga 3540
   actccagagg ctgggcctga acagggaggt ggtcgctcag gcctggtgct cagtcgtacg 3600
   acctgtacct ctcaactttt gccctatctg ttaaatatat gctatgtcat taaatgcttt 3660
   taaatctaaa aaaaaaaaaa aaaaaanaaa aaaaannnnn ngngtgtgca tgcccagtag 3720
   ggaaactgca aagggagaaa tgacaaacaa gaaacatttt acaaccagtc tgggctcact 3780
   tttgcatttt ttatgcatgt ctggtgcaca agctttgaaa actacagcaa acagtaataa 3840
   atgtgactgt tttgtagtta taaaaaaaa 3869
<210> 56
   <211> 3964
   <212> DNA
   <213> Homo sapiens
<400> 56
<210> 57
   <211> 4914
   <212> DNA
   <213> Homo sapiens
<220>
   <221> misc_feature
   <222> (1323)..(1323)
   <223> n is a, c, g, or t
<220>
   <221> misc_feature
   <222> (1705)..(1705)
   <223> n is a, c, g, or t
<220>
   <221> misc_feature
   <222> (2315)..(2315)
   <223> n is a, c, g, or t
<220>
   <221> misc_feature
   <222> (2317)..(2317)
   <223> n is a, c, g, or t
<220>
   <221> misc_feature
   <222> (2319)..(2324)
   <223> n is a, c, g, or t
<220>
   <221> misc_feature
   <222> (2327)..(2328)
   <223> n is a, c, g, or t
<220>
   <221> misc_feature
   <222> (2330)..(2330)
   <223> n is a, c, g, or t
<220>
   <221> misc_feature
   <222> (2332)..(2336)
   <223> n is a, c, g, or t
<220>
   <221> misc_feature
   <222> (2338)..(2338)
   <223> n is a, c, g, or t
<220>
   <221> misc_feature
   <222> (2591)..(2591)
   <223> n is a, c, g, or t
<220>
   <221> misc_feature
   <222> (2593)..(2594)
   <223> n is a, c, g, or t
<220>
   <221> misc_feature
   <222> (2602)..(2603)
   <223> n is a, c, g, or t
<220>
   <221> misc_feature
   <222> (2626).. (2627)
   <223> n is a, c, g, or t
<220>
   <221> misc_feature
   <222> (2629)..(2630)
   <223> n is a, c, g, or t
<220>
   <221> misc_feature
   <222> (2664)..(2664)
   <223> n is a, c, g, or t
<220>
   <221> misc_feature
   <222> (2785)..(2785)
   <223> n is a, c, g, or t
<220>
   <221> misc_feature
   <222> (3284)..(3284)
   <223> n is a, c, g, or t
<220>
   <221> misc_feature
   <222> (3619)..(3619)
   <223> n is a, c, g, or t
<220>
   <221> misc_feature
   <222> (3622)..(3622)
   <223> n is a, c, g, or t
<220>
   <221> misc_feature
   <222> (3630)..(3630)
   <223> n is a, c, g, or t
<220>
   <221> misc_feature
   <222> (3632)..(3632)
   <223> n is a, c, g, or t
<220>
   <221> misc_feature
   <222> (3636)..(3636)
   <223> *n is a,* c, g, or t
<220>
   <221> misc_feature
   <222> (3646)..(3648)
   <223> n is a, c, g, or t
<220>
   <221> misc_feature
   <222> (3650)..(3652)
   <223> n is a, c, g, or t
<220>
   <221> misc_feature
   <222> (3659)..(3655)
   <223> n is a, c, g, or t
<220>
   <221> misc_feature
   <222> (3657)..(3659)
   <223> n is a, c, g, or t
<220>
   <221> misc_feature
   <222> (3678) .. (3679)
   <223> n is a, c, g, or t
<220>
   <221> misc_feature
   <222> (3886)..(3886)
   <223> n is a, c, g, or t
<220>
   <221> misc_feature
   <222> (4736)..(4737)
   <223> n is a, c, g, or t
<400> 57
   aactgggctg gccatagggg gaagcggcgc gttaaatccg ccccctctcc ataccctact      60
   ttcaaggggt gggcggtaga ggggaggttg gggttggagc agcggcgagg gggcccctcc     120
   cttgcacctc cccccaccgg acttggtcgc gcccgaagtg taacactttc tctttgtcgg     180
   aggagctcct ctgtttcctg tgcagtagct cccgttgcgg cggcacccgt ggcagccctg     240
   gcggacgcag gagcgatggc agcgaccgat atagctcgcc aggtgggtga aggttgccga     300
   actgtccccc tggctggaca tgtggggttt gacagcttgc ctgaccagct ggtgaataag     360
   tccgtcagcc agggcttctg cttcaacatc ctgtgcgtgg gagagacagg tttgggcaag     420
   tccaccctca tggacaccct gttcaacacc aaattcgaag gggagccagc cacccacaca     480
   cagccgggtg tccagctcca gtctaatacc tatgacctcc aagagagcaa cgtgaggcta     540
   aagctcacga tcgttagcac agttggcttt ggggaccaga tcaacaaaga ggacagctac     600
   aagcctatcg tggaattcat cgatgcacaa ttcgaggcct acctgcagga agagctaaag     660
   atccgaagag tgctacacac ctaccatgac tcccgaatcc atgtctgctt gtatttcatt     720
   gcccccacgg gtcattccct gaagtctctg gacctagtga ctatgaagaa gctggacagt     780
   aaggtgaaca tcatccccat cattgccaaa gcagatgcca tttcgaagag tgagctaaca     840
   aagttcaaaa tcaaaatcac cagcgagctt gtcagcaacg gagtccagat ctatcagttt     900
   cctacagatg atgagtcggt ggcagagatc aatggaacca tgaacgccca cctgccgttt     960
   gctgtcattg gcagcacaga agaactgaag ataggcaaca agatgatgag ggcgcggcag    1020
   tatccttggg gcactgtgca ggttgaaaac gaggcccact gcgactttgt gaagctgcgg 1080
   gagatgctga ttcgggtcaa catggaggat ctgcgggagc agacccacac ccggcactat 1140
   gagctgtatc gccgctgtaa gctggaggag atgggcttca aggacaccga ccctgacagc 1200
   aaacccttca gtttacagga gacatatgag gccaaaagga acgagttcct aggggaactc 1260
   cagaaaaaag aagaggagat gagacagatg ttcgtccagc gagtcaaaga gaaagaagcg 1320
   ganctcaaag aggcagagaa agagctgcac gagaagtttg accgtctgaa gaaactgcac 1380
   caggacgaga agaagaaact ggaggataag aagaaatccc tggatgatga agtgaatgct 1440
   ttcaagcaaa gaaagacggc ggctgagctg ctccagtccc agggctccca ggctggaggc 1500
   tcacagactc tgaagagaga caaagagaag aaaaattaac tctgctgttt gctgcatgct 1560
   gcatgagacc cagggtcctg tttgggcttc ctgtagacac ccttttcctg cgcaacagag 1620
   ctgggcctcc ctttctctaa tttccccctt aacatgcctg gggggcatac aatccaaccc 1680
   gcgccctctc ctctcttcct gccanggttt atagaaacct gagaatctga gggtgatgtc 1740
   tggccgctgg tcaagaagcc aacagtcatg tggctcgcag atgcatcctg catcccagtc 1800
   cccctcccag cacccccagc catcccccct gtcttccccc acatctttgc cagaggtgtg 1860
   acatggtcag ggggcccatc tgctactctt tcccaccagc tcccctgttc cagttctggt 1920
   tgctgttagt ttccctgagg tatttgcaac caccatggct gggtaaccac cgatcagcac 1980
   agctgtcccc ttggtctcct gtatcccagt cactagtcct ccctggtcca ccccaccctc 2040
   atcctcagga gccacagcca tttcttagag ggtttcaaaa ggacagcctt tggcgccttt 2100
   tccttctaac ctttgagtcc agccctttcc agttttcatt cactcgaagt aactgcactc 2160
   aagctgtgct caaaatcggc aacgcattta tttacaccaa gcccttccca taaaacacaa 2220
   ctgctgaaga aaatagcaga cgtttcccct ctctctaact ctgggtatcc cacagatgca 2280
   aaagggagaa taaacctgaa tattattacc agccnanann nnnnaannan annnnnancg 2340
   aattcttctt gtgaggtatt tcagcatctc ggggggtaat ttccggaagg gctccatact 2400
   gtcccaataa ggtgaggcca gtagcaggaa taataaatcc cactttgtag gctggaaaac 2460
   tgagctgtca aaagaatcaa gtgtttgggg gtttgctctg atgagtcttc tagttcattt 2520
   ggtgaatgtc atgatgattt ttaacatgca ttttgcatgc atcccccaat aagaagagat 2580
   gagactcggc ngnngagaag annaggccct taactttctt tccaanntnn ggagttgaga 2640
   gtttaaaaat attccagccc taangttttt atcatgggtc ccatctgata gtggctttgg 2700
   gaacctctgt gaagtagaga gccctccctt gtcagggtta tgaggcacag tggcctttgg 2760
   tgtttggcca gtgacagtgt gaganatgga gttgacctgg caatgatctg tggctaacat 2820
   gccgtctctc tgcccttcct ttgcagtaat ccatggctgt gtactgaata gtattccccg 2880
   ctacagctgg actggactcc atttagcctt ttaagccgag gttcctattt taactgacag 2940
   ctttcctttg gggtgccagg cagcgaggcc ccccacccct atcctgccat gtacttcaag 3000
   ctcacttctt ctttttgagt tccgcaactt gctcctgcct cccagcccca ctggcactga 3060
   ccatgaccac ctacttctat ttttttttta gagtttcttt ttttgatcac ttactttcaa 3120
   agcacacagt caaacaaggt tatgccaaat ttccaggcct ttttgaagta ttgagaaggg 3180
   gaaggggatt tctcacttca attatagatc ataataggaa gcaaaaagaa aaaaatgaaa 3240
   agcaaacata tgcacgcact tttcttgttg acaaagcaag aatntaggtt tgctgtgtag 3300
   gtttggtgct ctattgattg gtgagtgacc agagcaagta tgaaggtgat gctgccaaag 3360
   cacaagcctt tttgaagtat tgagaagggg aaggggattt ctcacttcaa ttatagatca 3420
   taataggaag caaaaagaaa aaaatgaaaa gcaaacatat gcacgcactt ttcttgttga 3480
   caaagcaaga atataggttt gctgtgtagg tttggtgctc tattgattgg tgagtgacca 3540
   gagcaagtat gaaggtgatg ctgccaaagc acaagccagt ttcttgggaa aattcaagtt 3600
   acagtggagt atttttttna anaccatatn cntggnggta gaaacnnncn nncnncnnna 3660
   aaaaaaaaaa aaaaaaannt ctgctcagat actcagccag tagctcagag agatgctgag 3720
   ttaggcctgt caggtctcct tgggaaaggc ttcatatttg caactttgat gattctatgt 3780
   ccagcttcag agctgctttc ccagaaattc acgcttaaac aaccaaccgg taaccaccac 3840
   ttccccacac cgccgcctgg taattatttg cattacaaac cggagncgcc ctcatttgca 3900
   tttgtgtaca gattaactag ttaaggcttg agaagctctg aataattcaa aagtattaga 3960
   cccacacagc cttggagaga ccttcagaaa ctaaggagga gttttatatt aagggagaca 4020
   ttttagtcag taagacgata taacctactt actccgtaag gggaaatgaa ggcccggaga 4080
   agggaaggga cttgaccgag gtcccacttc tgtttcgagg cagaagccag actaattttc 4140
   atgcctcctg actcccaatc agtttcacaa agggattcaa tctgtttata tacgttacat 4200
   tcctggatac gaggtctttt gatgttcaga gtaactgact agttagtatt agaagaccct 4260
   cgaggttttt ttccacagaa aaacatctga agatggattg ggtgagggct ggcaaaacga 4320
   aggcatgccg ggccagctcc ttaacccaat gacccagtga tgctgcaagg ctggaacggg 4380
   gtccaggaga ctgtgtgtaa caggtgccct aggtgaccct tataatcagg gaagtttggt 4440
   gaacaaaaat cgaacccatg agtgaacata aattaaaaag ttgatcaacc tattaaaatg 4500
   tgtatttcat tgggtagctt ttctcactgt agacagattt tttccttctt caatgaaaag 4560
   gcttttaaat tagtacaact gttactattt aaaaaaaaaa taccctaagt actctgttta 4620
   cttctggtga aacaaaacca gtcattagaa atggtctgtg cttttatttt cccagactgg 4680
   agtggctttt ctgaaacaca cacacacaca cacacacaca cacacacaca cacacnngta 4740
   cacacatccc tcacttctct taagccaaga agtttgcttt ccctagctgc agtgtagatg 4800
   gctcttgttt ttgttttttt gttttaatca tttggcattc acatgtggct gttaatatgt 4860
   gcttgttttt aattaaaaca agaagcttta aaaaaaaaaa aaaaaaaaaa aaaa 4914
<210> 58
   <211> 2809
   <212> DNA
   <213> Homo sapiens
<400> 58
   ccggctcgcg gagagcgtag cgcggccttg gtggcggaat ggcgttgagt gacggcccgg     60
   ccccgccatc tggttaaagg gactcgttca acacggaagt gtcccggggc tgcattgtgc    120
   tacagctaga atgaagcaca tcaacctatc atttgcagcg tgtggatttc tgggcattta    180
   ccacttgggg gcagcatctg cactttgcag acatggcaaa aaacttgtga aggatgtcaa    240
   agccttcgct ggggcgtctg cgggatcgtt ggttgcttct gttctgctaa cagcaccaga    300
   aaaaatagag gaatgtaacc aatttaccta caagtttgcc gaagaaatca gaaggcagtc 360
   tttcggggca gtaacgcccg gttatgactt catggcccga ctaagaagtg ggatggagtc 420
   gattcttcct cccagcgctc acgagctggc ccagaaccga ctgcacgtat ccatcaccaa 480
   cgccaaaacc agagaaaatc acttagtctc cactttttcc tccagggagg acctcattaa 540
   ggtcctccta gccagcagtt ttgtgcccat ttatgcagga ctgaagctag tggaatacaa 600
   agggcagaag tgggtggacg gaggcctcac caacgctctt cccatcctgc ccgtcggccg 660
   gacagtaacc atctccccct tcagtggacg actggacatc tccccgcagg acaaagggca 720
   gctagatctg tatgttaata tcgccaagca ggatatcatg ttgtccctgg caaacctggt 780
   gagactcaac caagcccttt ttcccccaag caagaggaaa atggaatctt tgtatcagtg 840
   tggttttgat gacactgtta agtttttact taaagaaaat tggtttgaat aaaatgcata 900
   aaagtttata atgcaaaaca cgttttagat agtttttgat ggaagtttct aatcaaatcc 960
   tttttagaaa atctatcatg actcaattgt attactcctt gtaatatttg tgtttatttt 1020
   taatatttga attattactt agcacatgga tataagaggc actttattag aatttgacaa 1080
   cgtcattaag aaggatacac ttaggtgata aggaatcatt tttggtgaac tgttgtgtcc 1140
   tttaaaaaat ggaggaggat aggttctttg ggtaaattga ggaacatgga aaatggaggg 1200
   ccaccactta ggttccatgg agaatatgtg gcatgatctt gacgtgaagc atgggtgttt 1260
   ccgtgactta gctgtgtccc tgtagctctg gaactgaagg aactaggtag gagaggaagg 1320
   atttgagaaa taggcaactc atggttaaag acctttgatc aggactggag gaacaaaggt 1380
   ttgtgtatta gtccattttc acactgctga gaaagacata cccaagactg ggcaatttac 1440
   aaaagaaagg tttaatgggc tcacagttcc acatgactga ggaggcctca caattgtggt 1500
   ggaaggtgaa aggcatgtct cacatggcag cagacaagag aagaaaactt gtgcagggaa 1560
   actccccttt ataaaaccat cggatctctt gagacgcgtt cactatcatg agaatagcat 1620
   gggaaagacc tgcctccatg attcagttac ctcccactgg gtccctccca caacgtgtgg 1680
   caattgtggg agctacatac acttcaagat gagatttggg tggggacaca gccaaaccat 1740
   aacagtttgc tttccacagg tgtgtgcagt tgctcccagt gcagttaggg gcagatatcc 1800
   agaagcctag gagattttga agaggcttat cccctctcat cttcctcccc taaaccccac 1860
   cccagtttag gagattatca agctggttgt cattcattct ctctctctct ctctctctct 1920
   ctctctctct ctctctctct ctctcgccat acctctctga gtatttagct attgttctat 1980
   atactggata catctattat gggaacttaa atagataaaa tactactaaa cacaaagaac 2040
   caagtagcct agtaaataag aattaaatcc agaataatca ttcacttctc atttttaaat 2100
   cacgttgtac tgatcccagg ttttttactt tctgagtagt tgtcgatcac tttgaggact 2160
   tgtgtatggg cttgtaccaa tttactgaca ctgtcttaaa tcctaaatct gtttttatga 2220
   ggttttgcca agcaagaact cttgattact aagaggcagt tagagacaaa ccaatttaat 2280
   tccatgtctt caattgtcac gcatttcttc ttttactctt tgaaactatt ctttgagtca 2340
   attttatggt tctcagaagc caaaatacac aacttttagc acataaacac caacgatggc 2400
   ctctttttga ggagttatgc atagacccac tctagagtaa tgatggtccc tgtggtatat 2460
   actttctcct actctagcaa acatgtagtt taatcttaat gtgttgtttc cataagtgac 2520
   atgaagtgga tagattctca attgttatgt ccacttattc actaggtaaa ttttcagttt 2580
   taatactttt ctccttaccc cttcctttga tcatttcatg tgaatattct atttgtatga 2640
   tacactgtat ttcaataaat tccttgttga tgtaccctta aattgaagaa atttaagctg 2700
   caaaaccaaa tctcattgta taagactttt ttgaagtatc ttgtatcgac tacatatgta 2760
   tttgaccctg tgggaggatg gtacttttct ttttaacttt tattttaag 2809

## Description

The present invention concerns a method of determining a probability of a subject diagnosed with probable multiple sclerosis to develop definite multiple sclerosis according to claim 1.

The present disclosure relates to genetic markers which are differentially expressed between subjects diagnosed with probable multiple sclerosis (MS) which further develop the definite diagnosis of MS and control subjects, more particularly, but not exclusively, to methods and kits using same for determining the probability of a subject diagnosed with probable multiple sclerosis to develop a definite diagnosis of multiple sclerosis and for treating subjects diagnosed with probable multiple sclerosis.

Multiple sclerosis (MS) is the most common central nervous system (CNS) disease affecting young adults (disease onset between 20 to 40 years of age), and the third leading cause for disability after trauma and rheumatic diseases. Disease prevalence in USA is 120/100,000, (250,000 to 350,000 cases), and in Israel about 30/100,000. MS is a multifactorial disease that develops in genetically predisposed subjects exposed to yet undefined environmental factors and which results in irreversible neurological disability.

The diagnosis of MS is defined primary by clinical terms and relies on a combination of history, neurological examination and ancillary laboratory and neuro-imaging studies. Typically, at onset of MS, an otherwise healthy person presents with the acute or sub-acute neurological symptomatology (attack). The symptoms usually remain for several days to few weeks, and then partially or completely resolve. The neurological symptoms are accompanied by demyelinating lesions on brain MRI, Thus, the laboratory diagnosis of probable MS is based on: 1) Cerebro-spinal fluid (CSF) evaluation of IgG synthesis, oligoclonal bands; and 2) MRI of the brain and spinal cord. After a period of remission, a second attack will occur. During this period between the first and second attacks, the patient is diagnosed as probable MS. Only when the second attack occurs, the diagnosis of clinically definite MS is established.

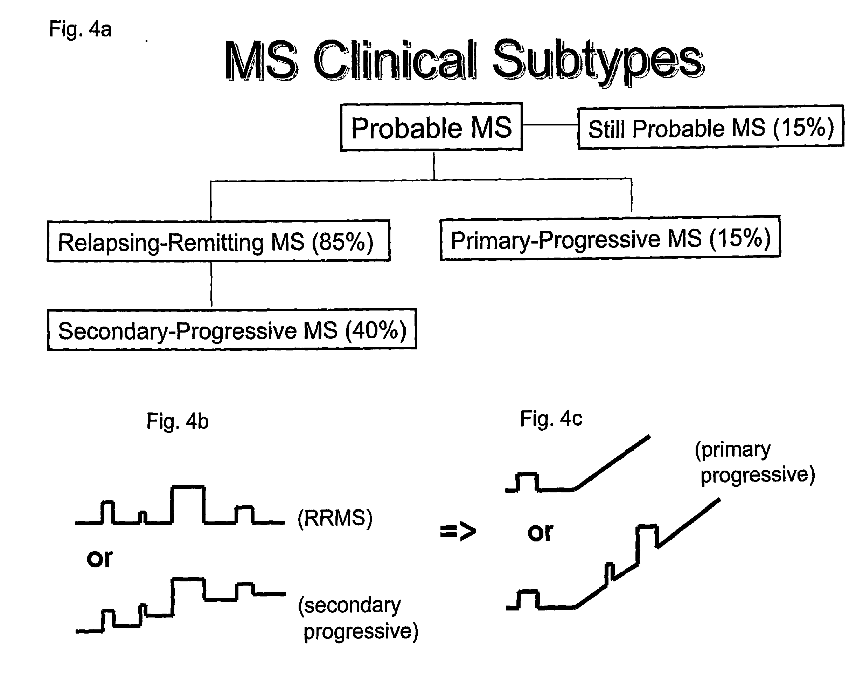

In about 85 % of the patients with definite diagnosis of MS, the disease course is relapsing-remitting definite MS (RRMS), which is characterized by attacks during which new neurological symptoms and signs appear, or existing neurological symptoms and signs worsen. Usually an attack develops within a period of several days, lasts for 6-8 weeks, and then gradually resolves. During an acute attack, scattered inflammatory and demyelinating CNS lesions produce varying combinations of motor, sensory, coordination, visual, and cognitive impairments, as well as symptoms of fatigue and urinary tract dysfunction. The outcome of an attack is unpredictable in terms of neurological squeal, but it is well established that with each attack, the probability of complete clinical remission decreases, and neurological disability and handicap are liable to develop. In about 15 % of patients the disease has a primary progressive course, characterized by gradual onset of neurological symptoms that progress over time. In a subset of patients (about 40 %), the disease has a secondary progressive course, *i.e*., it is first characterized by relapses and remission and then gradually progresses (See Figures 4a-c). The only course of MS in which treatment was effectively established is RRMS. Various immunomodulatory drugs have been shown to reduce the number and severity of acute attacks; and thereby to decrease the accumulation of neurological disability.

The main pathologic findings in MS are the presence of infiltrating mononuclear cells predominantly T lymphocytes and macrophages that surpass the blood brain barrier and induce an active inflammation within the brain and spinal cord, attacking the myelin and resulting in gliotic scars and axonal loss. These inflammatory (acute and chronic) processes can be visualized by brain and spinal cord magnetic resonance imaging (MRI) as hyperintense T2 or hypointense T1 lesions. Thus, MRI examination can serve for the diagnosis of the disease and as a surrogate marker to follow disease activity by measuring lesion load within the brain.

The etiology of MS is still unknown. The pathogenesis of MS involves autoimmune mechanisms associated with autoreactive T cells against myelin antigens. It is well established that not one dominant gene determines genetic susceptibility to develop MS, but rather many genes, each with different influence, are involved. The initial pathogenic process that triggers the disease might be caused by one group of genes, while other groups are probably involved in disease activity and progression (5, 6).

In a previous epidemiological study the present inventors have shown that 57.6 % of patients with probable MS experience a second attack within one year from onset, and thus convert to definite MS (7). In other studies, the progression to clinically definite MS in patients with an abnormal brain MRI was 49 % and 65 % in the first 5 years, 41 % and 68 % within 2 years, and 24 % and 45 % within 1 year (8, 9, respectively). Prediction of disease progression rate is especially important during the initial stage, when patients first present with neurological symptomatology and are defined to suffer from probable MS. At this early stage the immunological process of epitope spreading which is associated with exposure of the immune system to myelin antigens is still limited and significant disability has not yet developed.

The potential application of DNA microarray technology for understanding neurological disorders was discussed in a recent review (12). In MS, microarray analysis of brain lesions and brains of mice with experimental allergic encephalomyelitis (EAE) - the experimental animal model of MS - identified genes that contribute to lesion pathology (13). Similarly, different expression of transcribed genes encoding inflammatory cytokines was demonstrated in acute inflammatory brain lesions compared with 'silent' lesions without inflammation, using a large-scale gene microarray analysis (14).

In the peripheral blood of MS patients, simultaneous inhibitory and stimulatory effects of inflammatory T cells and macrophages reflect their potential role within the ongoing autoimmune response was reported. Analysis of the expression pattern in peripheral blood mononuclear cells (PBMC) obtained from MS patients during a stable clinical remission revealed 34 genes out of more than 4000 tested that were significantly different from controls (15). In a previous study by the present inventors (16) PBMC gene expression pattern of 26 RRMS patients and 18 healthy subjects demonstrated significantly different pattern of 1109 genes between patients and healthy subjects. This signature contains genes that implicate the underlying processes involved in MS pathogenesis including T-cell activation and expansion, inflammation and apoptosis. To determine disease stage related gene expression signatures MS patients were evaluated during an acute relapse and in remission (16; PCT Pub. No. WO03081201A2, EP1532268A2, AU3214604AH, US20060003327A1, to the present inventors). This analysis demonstrated 721 differentiating genes including genes that play a regulatory role in epitope spreading and in macrophage recruitment to the inflammatory injury. Apoptotic-related genes such as cyclin G1 (CCG1) - the mediator of p53-dependent apoptosis and the caspases 2, 8 and 10 were significantly down-expressed.

WO 0187903 relates to novel single nucleotide polymorphisms in the human acetyl-Coenzyme A acyltransferase 1 (peroxisomal 3-oxoacyl-Coenzyme A thiolase) (ACAA1) gene. In addition, various genotypes, haplotypes and haplotype pairs for the ACAA1 gene that exist in the population are described. Compositions and methods for haplotyping and/or genotyping the ACAA1 gene in an individual are also disclosed. Polynucleotides containing one or more of the ACAA1 polymorphisms disclosed herein are also described.

The method of determining a probability of a subject diagnosed with probable multiple sclerosis to develop definite multiple sclerosis is defined in claim 1.

According to some embodiments of the invention, the reference cell is of an unaffected subject.

According to some embodiments of the invention, the probability of the subject diagnosed with probable multiple sclerosis to develop definite multiple sclerosis is higher than about 75 %.

According to some embodiments of the invention, detecting the level of expression is effected using an RNA detection method.

According to some embodiments of the invention, the cell of the subject is a blood cell.

According to some embodiments of the invention, wherein said detecting said level of expression is effected using a protein detection method.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. In case of conflict, the patent specification, including definitions, will control. In addition, the materials, methods, and examples are illustrative only and not intended to be limiting.

### BRIEF DESCRIPTION OF THE DRAWINGS

The invention is herein described, by way of example only, with reference to the accompanying drawings. With specific reference now to the drawings in detail, it is stressed that the particulars shown are by way of example and for purposes of illustrative discussion of the preferred embodiments of the present invention only, and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice.

In the drawings:

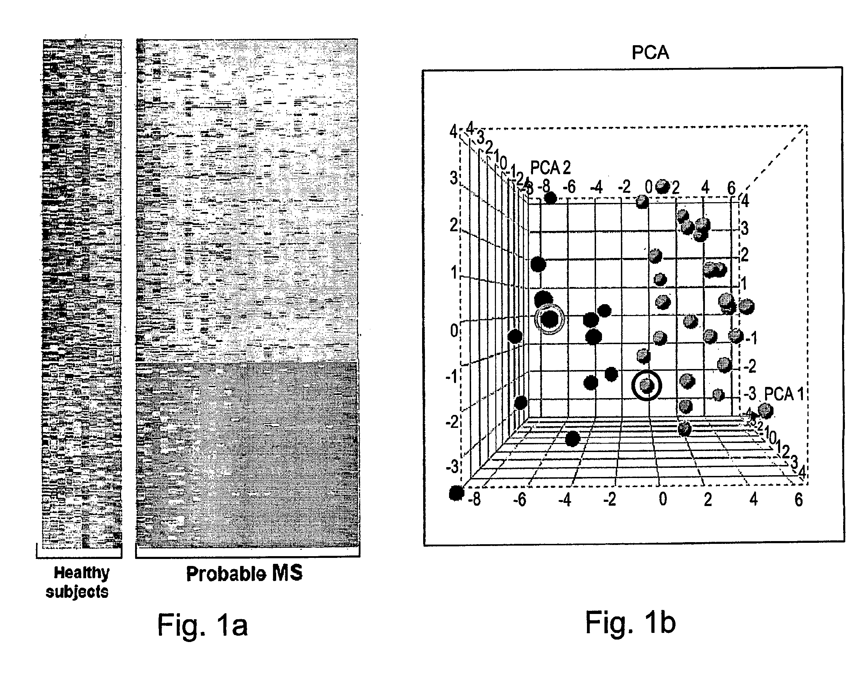

FIGs. 1a-b depicts the most informative genes differentially expressed between probable MS and healthy subjects. Figure 1a - Infogramm of 554 most informative genes (listed in Table 1) discriminated between probable MS patients (N = 28) and healthy subjects (N = 10); Each row represents a gene and each column represents a patient's sample. Figure 1b - PCA analysis demonstrating only two classification errors (marked by circles; 5 %) using the 554 most informative genes. Blue dots - healthy subjects, red dots - probable MS patients;

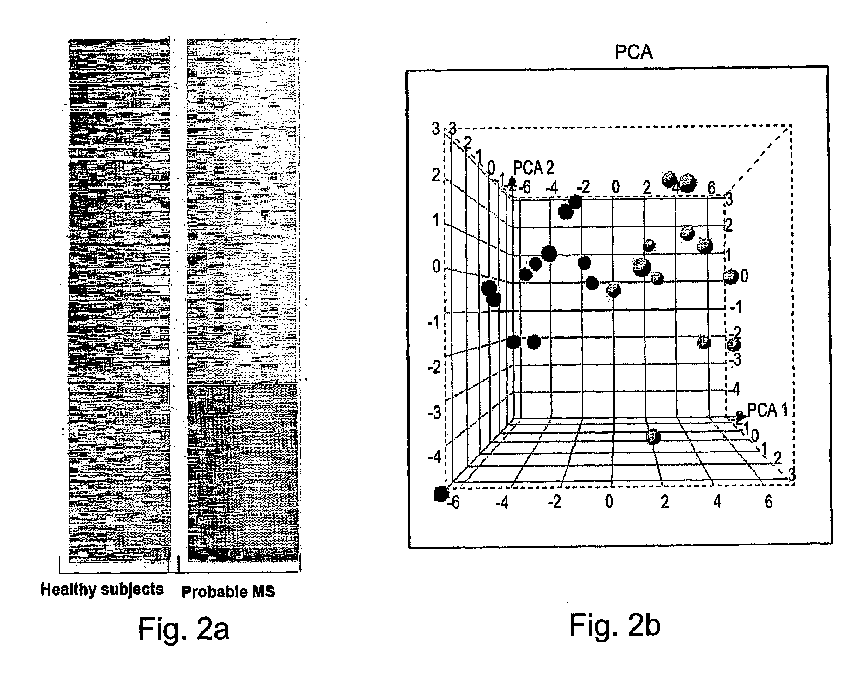

FIGs. 2a-b depicts the most informative genes differentially expressed between probable MS patients that converted to definite MS during a 2-year follow-up period and healthy subjects. Figure 2a - Infogramm of the 1517 most informative genes (listed in Table 2) that discriminated between probable MS patients (N = 12) that converted to definite MS during a 2-year follow-up period and healthy subjects (N = 11); Each row represents a gene and each column represents a patient's sample. All genes passed FDR criteria at p < 0.03 and 8 genes passed Bonfferoni correction at p < 3.2 x 10⁻⁵. Figure 2b - PCA applied to the 1517 most informative genes resulted in two clusters, healthy subjects (blue dots) and probable MS patients (red dots) with no classification errors (0 classification errors).

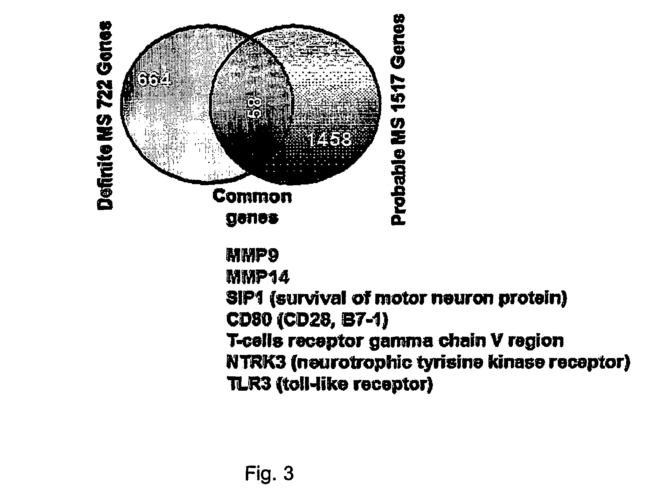

FIG. 3 is a Van-diagram demonstrating intersecting genes between probable which further developed a definite diagnosis of MS (the 1517 genes listed in Table 2 which differentiate between probable patients that developed to definite MS during 2 years follow up and healthy controls) and definite (the 722 genes listed in Table 4 which differentiate between MS patients with a definite diagnosis of MS, i.e., after at least the second neurological attack and healthy controls) PBMC gene expression signatures. Note that the 58 intersecting genes (listed in Table 5) share the same expression pattern, *i.e*., either upregulation or downregulation relative to control subjects in both subjects diagnosed with probable MS which further developed definite MS within a 2-year period (i.e., probable MS subjects who develop clinical symptoms/MRI pattern which fit the diagnosis of a definite MS) and the subjects diagnosed with definite MS (i.e., an expression pattern determined in subjects with a definite diagnosis of MS);
FIGs. 4a-c depict the various multiple sclerosis subtypes. Figure 4a - a flow chart of the MS clinical subtypes. A subject diagnosed with probable MS can develop a diagnosis of definite MS (in about 85 % of the cases) during 5 years follow up period or remain diagnosed as probable MS (in 15 % of the cases). Of the subjects diagnosed with definite MS, 85 % exhibit a disease course of relapsing-remitting MS (RRMS) and about 15 % exhibit a primary progressive course of disease. Of the patients developed RRMS, 40 % will develop a secondary progressive MS course. Figure 4b schematically illustrates the disease courses of RRMS or secondary progressive MS. Figure 4c schematically illustrates the disease course of primary progressive MS with or without attacks/remission periods.

### DESCRIPTION OF SPECIFIC EMBODIMENTS OF THE INVENTION

The present disclosure, in some embodiments thereof, relates to genetic markers which are differentially expressed between probable multiple sclerosis (MS) subjects that further converted to the definite diagnosis of MS and healthy controls. More particularly, but not exclusively, such differentially expressed markers can be used to determine the probability of a subject diagnosed with probable MS to develop a definite diagnosis of MS. In addition, the present disclosure, in some embodiments thereof, can be used to select a treatment regimen for subjects diagnosed with probable MS based on the expression pattern of such genetic markers.

Before explaining at least one embodiment of the invention in detail, it is to be understood that the invention is not limited in its application to the details set forth in the following description or exemplified by the Examples. The invention is capable of other embodiments or of being practiced or carried out in various ways. Also, it is to be understood that the phraseology and terminology employed herein is for the purpose of description and should not be regarded as limiting.

The present disclosure relates to genetic markers which are predictive to the definite diagnosis of MS in subjects diagnosed with probable MS, *i.e.,* following the first neurological attack.

As is shown in Figures 1a-b, Table 1 and is described in Example 1 of the Examples section which follows, the present inventors have uncovered 554 genes which are differentially expressed in PBMC between subjects with probable MS and healthy controls. Following a 2-years follow up, the subjects diagnosed with probable MS were divided to those who eventually developed a diagnosis of definite MS (convertors to definite MS) or sustained the diagnosis of probable MS (non-convertors to definite MS). Analysis of the gene expression pattern of probable MS subjects which further converted to definite MS revealed 1517 genes which are differentially expressed as compared to healthy controls (Table 2, Figures 2a-b and Example 2 of the Examples section which follows). In addition, analysis of the gene expression pattern of probable MS subjects which did not convert to definite MS within a period of 2 years revealed 503 genes which are differentially expressed as compared to healthy controls (Table 3, Example 2 of the Examples section which follows). Moreover, comparison of the gene expression pattern of subjects with a definite diagnosis of MS to that of healthy controls revealed 722 genes which are differentially expressed (Table 4, Example 2 of the Examples section which follows). Furthermore, comparison of the differentiating genes between probable MS subjects which further converted to definite MS to that of subjects with the definite diagnosis of MS revealed that the expression pattern of 58 genes is common between the two groups of samples (Table 5, Example 3 of the Examples section which follows). In addition, application of the SVM software based on RBF kernel on a randomly assigned training set of 80 % of the 40 probable MS patients revealed optimal sets of genes and their prediction power (average error in test set) of the probability of a subject diagnosed with probable MS to develop the definite diagnosis of MS (Table 6, Example 3 of the Examples section which follows). These results suggest that the expression pattern of each of the 58 genes and/or a combination of several or all of the 58 genes has a predictive value in determining the probability of a subject diagnosed with probable MS to develop the definite diagnosis of MS.

Thus, according to one aspect of the present invention there is provided a method of determining a probability of a subject diagnosed with probable multiple sclerosis to develop definite multiple sclerosis. The method is effected by determining in a cell of the subject a level of expression of at least the polynucleotide sequence SEQ ID NOs: 4 wherein an alteration above a predetermined threshold in the level of expression of the at least one polynucleotide sequence in the cell of the subject relative to a level of expression of the at least one polynucleotide sequence in a reference cell is indicative of the probability of the subject diagnosed with probable multiple sclerosis to develop definite multiple sclerosis.

As used herein, the phrase "a subject diagnosed with probable multiple sclerosis" refers to a mammal, preferably a human being, who is diagnosed with probable multiple sclerosis, e.g., a subject who experienced one neurological attack affecting the CNS and accompanied by demyelinating lesions on brain magnetic resonance imaging (MRI). The neurological attack can involve acute or sub-acute neurological symptomatology (attack) manifested by various clinical presentations like unilateral loss of vision, vertigo, ataxia, incoordination, gait difficulties, sensory impairment characterized by paresthesia, dysesthesia, sensory loss, urinary disturbances until incontinence, diplopia, dysarthria, various degrees of motor weakness until paralysis, cognitive decline either as a monosymptomatic or in combination. The symptoms usually remain for several days to few weeks, and then partially or completely resolve.

The diagnosis of probable MS can also include laboratory tests involving evaluation of IgG synthesis and oligoclonal bands (immunoglobulins found in 85-95 % of subjects diagnosed with definite MS) in the cerebrospinal fluid (CSF, obtained by e.g., lumbar puncture) which provide evidence of chronic inflammation of the central nervous system. Combined with MRI of the brain and spinal cord and clinical data, the presence of oligoclonal bands can help make a definite diagnosis of MS.

As used herein, the phrase "determining a probability" refers to the likelihood of a subject diagnosed with probable MS to develop the definite diagnosis of MS within a certain time period. According to an embodiment of the invention, such probability can be high, e.g., more than 51 %, at least 60 %, at least 70 %, at least 80 %, at least 85 %, at least 87 %, at least 90 %, at least 95 %, at least 99 %, e.g., 100 %, that a subject diagnosed with probable MS will develop the definite diagnosis of MS. It will be appreciated that the time period during which the subject diagnosed with probable MS will convert to the definite diagnosis of MS can be within 1 year since onset of probable MS, within 2-3 years, within 3-5 years, or more.

As used herein the phrase "develop definite multiple sclerosis" refers to a subject who is diagnosed with probable MS and which experiences at least a second neurological attack affecting the CNS and accompanied by demyelinating lesions on brain magnetic resonance imaging (MRI), wherein the neurological attacks are associated with the appearance of new neurological symptoms and signs or the worsening of existing neurological symptoms and signs. It will be appreciated that the disease course of patients diagnosed with definite MS can be a relapsing-remitting multiple sclerosis (RRMS) (occurring in 85 % of the patients), a primary progressive multiple sclerosis (occurring in 15 % of the patients) or a secondary progressive multiple sclerosis (occurring in 40 % of the RRMS patients; see Figure 4).

As mentioned above, shown in Table 6 and described in Example 3 of the Examples section which follows, the prediction power of the selected polynucleotides set forth by SQE ID NOs:1-58 in determining the probability of a subject diagnosed with probable MS to develop definite MS within 2 years was computed using the SVM based on RBF kernel when applied on a set of 40 probable MS subjects, randomly divided to 80% as training set and 20% as test set. The polynucleotide sequence exhibiting the best prediction power as a single gene, which can be used to determine the probability of a subject diagnosed with probable MS to develop definite multiple sclerosis is set forth in SEQ ID NO:4 (average error: 0.216; prediction accuracy of 78.4 %).

As is further shown in Table 6 (Example 3) several groups of genes can predict the probability of a subject diagnosed with probable MS to develop a definite multiple sclerosis within 2 years with about 87 % accuracy (average error of about 0.13).

According to an embodiment of the invention, the polynucleotide sequences which expression level are determined in the cell of the subject diagnosed with probable MS are those depicted in any of the following groups of row numbers of Table 6 in Example 3 of the Examples section which follows: rows 1-33; rows 1-34; rows 1-35; rows 1-40; rows 1-44; and rows 1-45.

As is further shown in Table 6 (Example 3) several groups of genes can predict the probability of a subject diagnosed with probable MS to develop a definite multiple sclerosis within 2 years with about 84-86 % accuracy (average error of about 0.14-0.16).

According to an embodiment of the invention, the polynucleotide sequences which expression level are determined in the cell of the subject diagnosed with probable MS are those depicted in any of the following groups of row numbers of Table 6 in Example 3 of the Examples section which follows: rows 1-6; rows 1-14; rows 1-15; rows 1-16; rows 1-17; rows 1-18; rows 1-19; rows 1-29; rows 1-31; rows 1-32; rows 1-36; rows 1-37; rows 1-38; rows 1-39; rows 1-40; rows 1-41; rows 1-42; rows 1-43; rows 1-46; rows 1-47; rows 1-48; rows 1-49; rows 1-50; and rows 1-52.

As is further shown in Table 6 (Example 3) several groups of genes can predict the probability of a subject diagnosed with probable MS to develop a definite multiple sclerosis diagnosis with about 80-83 % accuracy (average error of about 0.17-0.20).

According to an embodiment of the invention, the polynucleotide sequences which expression level are determined in the cell of the subject diagnosed with probable MS are those depicted in any of the following groups of row numbers of Table 6 in Example 3 of the Examples section which follows: rows 1-7; rows 1-8; rows 1-9; rows 1-10; rows 1-12; rows 1-13; rows 1-20; rows 1-21; rows 1-22; rows 1-23; rows 1-24; rows 1-25; rows 1-26; rows 1-27; rows 1-28; rows 1-30; rows 1-51; rows 1-53; rows 1-54; rows 1-55; rows 1-56; rows 1-57; and rows 1-58.

As is further shown in Table 6 (Example 3) several groups of genes can predict the probability of a subject diagnosed with probable MS to develop a definite multiple sclerosis diagnosis with about 75-79 % accuracy (average error of about 0.21-0.25).

According to an embodiment of the invention, the polynucleotide sequences which expression level are determined in the cell of the subject diagnosed with probable MS are those depicted in any of the following groups of row numbers of Table 6 in Example 3 of the Examples section which follows: rows 1-2; rows 1-3; rows 1-4; rows 1-5; and rows 1-11.

As used herein, the phrase "level of expression" refers to the degree of gene expression and/or gene product activity in a specific cell. For example, up-regulation or down-regulation of various genes can affect the level of the gene product (*i.e*., RNA and/or protein) in a specific cell.

As used herein the phrase "a cell of the subject" refers to any cell content and/or cell secreted content which contains RNA and/or proteins of the subject. Examples include a blood cell, a bone marrow cell, a cell obtained from any tissue biopsy (e.g., CSF, brain biopsy), body fluids such as plasma, serum, saliva, spinal fluid, lymph fluid, the external sections of the skin, respiratory, intestinal, and genitourinary tracts, tears, saliva, sputum and milk. According to an embodiment of the invention, the cell is a blood cell (e.g., white blood cells, macrophages, B- and T-lymphocytes, monocytes, neutrophiles, eosinophiles, and basophiles) which can be obtained using a syringe needle from a vein of the subject. It will be appreciated that PBMC is the most accessible tissue and could be useful as a minimally invasive approach for gene expression differential diagnosis. It should be noted that a "cell of the subject" may also optionally comprise a cell that has not been physically removed from the subject (e.g., in vivo detection).

According to an embodiment of the invention, the white blood cell comprises peripheral blood mononuclear cells (PBMC). The phrase, "peripheral blood mononuclear cells (PBMCs)" as used herein, refers to a mixture of monocytes and lymphocytes. Several methods for isolating white blood cells are known in the art. For example, PBMCs can be isolated from whole blood samples using density gradient centrifugation procedures. Typically, anticoagulated whole blood is layered over the separating medium. At the end of the centrifugation step, the following layers are visually observed from top to bottom: plasma/platelets, PBMCs, separating medium and erythrocytes/granulocytes. The PBMC layer is then removed and washed to remove contaminants (e.g., red blood cells) prior to determining the expression level of the polynucleotide(s) therein.

It will be appreciated that the cell of the subject can be obtained at any time, e.g., immediately after an attack or at any time during remission.

According to preferred embodiments of the present invention, detecting the level of expression of the polynucleotide sequences of the present invention is effected using RNA or protein molecules which are extracted from the cell of the subject.

Methods of extracting RNA or protein molecules from cells of a subject are well known in the art.

Once obtained, the RNA or protein molecules can be characterized for the expression and/or activity level of various RNA and/or protein molecules using methods known in the arts.

Non-limiting examples of methods of detecting RNA molecules in a cell sample include Northern blot analysis, RT-PCR, RNA *in situ* hybridization (using e.g., DNA or RNA probes to hybridize RNA molecules present in the cells or tissue sections), *in situ* RT-PCR (e.g., as described in Nuovo GJ, et al. Am J Surg Pathol. 1993, 17: 683-90; Komminoth P, et al. Pathol Res Pract. 1994, 190: 1017-25), and oligonucleotide microarray (e.g., by hybridization of polynucleotide sequences derived from a sample to oligonucleotides attached to a solid surface [e.g., a glass wafer) with addressable location, such as Affymetrix microarray (Affymetrix®, Santa Clara, CA)].

Non-limiting examples of methods of detecting the level and/or activity of specific protein molecules in a cell sample include Enzyme linked immunosorbent assay (ELISA), Western blot analysis, radio-immunoassay (RIA), Fluorescence activated cell sorting (FACS), immunohistochemical analysis, *in situ* activity assay (using e.g., a chromogenic substrate applied on the cells containing an active enzyme), *in vitro* activity assays (in which the activity of a particular enzyme is measured in a protein mixture extracted from the cells).

For example, in case the detection of the expression level of a secreted protein is desired, ELISA assay may be performed on a sample of fluid obtained from the subject (e.g., serum), which contains cell-secreted content.

As used herein the phrase "reference cell" refers to any cell as described hereinabove of an unaffected subject (*i.e*., a subject devoid of any neurological attack resembling MS or probable MS) such as a healthy subject, which can be an age and/or gender-matched unaffected subject (e.g., a healthy subject from the same age and/or gender as of the subject diagnosed with probable MS). Such a reference cell can be a blood cell, a bone marrow cell, a cell obtained from any tissue biopsy (e.g., CSF), body fluids such as plasma, serum, saliva, spinal fluid, lymph fluid, the external sections of the skin, respiratory, intestinal, and genitourinary tracts, tears, saliva, sputum and milk. It will be appreciated that the level of expression of the above referenced polynucleotides/polypeptides may be obtained from scientific literature.

Since as is shown in Table 5 and is described in Example 2 of the Examples section which follows, 27 polynucleotide sequences displayed elevated expression in the subjects diagnosed with probable MS which further developed the definite diagnosis of MS relative to healthy subjects, in order to determine the probability of a subject diagnosed with probable MS to develop definite MS, the level of expression of at least one polynucleotide sequence selected from the group consisting of SEQ ID NOs:32-58 may be determined and compared to the level of expression of the same polynucleotide sequences in a reference cell derived from an unaffected subject, wherein an upregulation (increase) in the expression level of the at least one polynucleotide sequence above a predetermined threshold relative to the reference cell is indicative of a high probability (e.g., higher than about 75 %, about 80 %, about 85 %, about 87 %) of the subject diagnosed with probable MS to develop definite MS (e.g., to convert to definite MS within a period of about 2 years). On the other hand, downregulation or no significant change in the level of expression of the same at least one polynucleotide sequence relative to the reference cell is indicative of low probability (e.g., less than about 75 %, e.g., less than 50 %, e.g., less than 30 %) of the subject diagnosed with probable MS to develop definite MS (e.g., to convert to definite MS within a period of about 2 years). It will be appreciated that such a subject can eventually develop definite MS following a longer period of time, e.g. more than 2 years, e.g.; 10-20 years.

Additionally or alternatively, since as is further shown in Table 2 and is described in Example 2 of the Examples section which follows, the level of expression of 31 polynucleotide sequences was downregulated in subjects diagnosed with probable MS relative to the healthy control subjects, in order to determine the probability of a subject diagnosed with probable MS to develop definite MS, the level of expression of at least one polynucleotide sequence selected from the group consisting of SEQ ID NOs:1-31 may be determined and compared to the level of expression of the same polynucleotide sequences in a reference cell derived from an unaffected subject, wherein downregulation (decrease) in the expression level of the at least one polynucleotide sequence above a predetermined threshold relative to the reference cell is indicative of high probability (e.g., higher than about 75 %, about 80 %, about 85 %, about 87 %) of the subject diagnosed with probable MS to develop definite MS (e.g., to convert to definite MS within a period of about 2 years). On the other hand, upregulation or no significant change in the level of expression of the same at least one polynucleotide sequence relative to the reference cell is indicative of low probability (e.g., lower than 75 %, e.g., less than 50 %, e.g., less than 30 %) of the subject diagnosed with probable MS to develop definite MS (e.g., to convert to definite MS within a period of about 2 years). It will be appreciated that such a subject can eventually develop definite MS following a longer period of time, e.g., more than 2 years.

As used herein the phrase "an alteration above a predetermined threshold" refers to the increase or decrease (*i.e*., degree of upregulation or downregulation, respectively) which is higher than a predetermined threshold such as at least twice, at least three times, at least four times, at least five times, at least six times, at least seven times, at least eight times, at least nine times, at least 10 times, at least 20 times, at least 50 times, at least 100 times, at least 500 times relative to the reference cell.

For example, as is shown in Table 5, while the level of expression of the polynucleotide sequences set forth by SEQ ID NOs:1-16, is at least twice lower in subjects diagnosed with probable MS which further developed definite MS as compared to unaffected subjects, the level of expression of the polynucleotide sequences set forth by SEQ ID NOs:17-26, the polynucleotide sequences set forth by SEQ ID NOs:27-29, or the polynucleotides set forth by SEQ ID NOs:30-31 is at least 5, 10, or 50 times, respectively, lower in cells of subjects diagnosed with probable MS which further developed definite MS as compared to unaffected, healthy subjects.

In addition, as is further shown in Table 2, while the level of expression of the polynucleotide sequences set forth by SEQ ID NOs:32-46, is at least twice higher in subjects diagnosed with probable MS which further developed definite MS as compared to unaffected, healthy subjects, the level of expression of the polynucleotide sequences set forth by SEQ ID NOs:47-52, the polynucleotides set forth by SEQ ID NOs:53-56, or the polynucleotide set forth by SEQ ID NOs:57-58 is at least 5, 10, or 50 times, respectively, higher in cells of subjects diagnosed with probable MS which further developed definite MS as compared to unaffected, healthy subjects.

It will be appreciated that higher fold change in the expression level of the at least one polynucleotide in the cell of the subject relative the reference cell, and/or alteration in the level of expression of the polynucleotides which exhibit high fold change in Table 5 of Example 2 (e.g., SEQ ID NOs:17-26 and/or 47-52, SEQ ID NOs:27-29 and/or 53-56, 30-31 and/or 57-58), and/or alteration above the predetermined threshold in a significant number of polynucleotides from the polynucleotides set forth by SEQ ID NOs:4, 16, 5, 56, 20, 3, 1, 10, 57, 24, 14, 49, 13, 37, 6, 47, 50, 21, 46, 8, 26, 2, 15, 51, 44, 19, 17, 25, 33, 48, 36, 34, 12, 29, 23, 11, 45, 53, 41, 40, 31, 58, 27, 43, 35, 30, 52, 55, 7, 9, 42, 28, 54, 32, 22, 18, 38, and 39 (e.g., at least 30, at least 40, at least 45, at least 50, or 58) may indicate high probability that the subject diagnosed with probable MS will develop definite MS within a short period of time (during 2 years).

Thus, the method of determining the probability of a subject diagnosed with probable MS to develop definite MS according to the invention enables the classification of probable MS patients to those that will develop definite MS within a predetermined time (e.g., about 2 years, fast convertors) and to those who will sustain the diagnosis of probable MS and will either not convert to definite MS or will convert to definite MS following an extended period of time (e.g., more than 2 years, e.g., at least 10 years).

Thus, the teachings of the present invention can be used to improve the diagnosis of definite MS following the first neurological attack, without needing to rely on the appearance of the second neurological attack.

It will be appreciated that determining the probability of a subject diagnosed with probable MS to develop definite MS can be used to select the treatment regimen of the subject and thereby to treat the subject diagnosed with probable MS.

As used herein the phrase "treating" refers to inhibiting or arresting the development of a pathology [multiple sclerosis, e.g., RRMS or progressive (e.g., primary or secondary) MS] and/or causing the reduction, remission, or regression of a pathology and/or optimally curing the pathology. Those of skill in the art will understand that various methodologies and assays can be used to assess the development of a pathology, and similarly, various methodologies and assays may be used to assess the reduction, remission or regression of the pathology.

Thus, by determining the probability of the subject diagnosed with probable MS to develop definite MS, the subject can be treated early, prior to experiencing the second neurological attack, with suitable therapeutics that can prevent deterioration of clinical symptoms and can increase the chances of achieving cure and remission of symptoms in the affected subjects.

It will be appreciated that classification of subjects diagnosed with probable MS to those that will convert fast to definite MS and to those that will sustain the diagnosis of probable MS can be also used in order to assess the efficacy of a treatment regimen on probable MS patients which are likely to develop definite MS. Thus, by treating subjects with probable MS and high probability to develop definite MS (as determined by the method of the invention) with candidate preventive and/or therapeutic drugs and monitoring the subjects' health in terms of MS progression (e.g., EDSS evaluation and number of relapses), the efficacy of the drugs can be assessed.

The teachings of the invention are of utmost importance and have relevant medical, economical and social aspects. While the MS disease prevalence in USA is at the range of 250.000 to 350.000 cases, the annual cost of MS in USA is anticipated to be 34,000 $ per patient, leading to 2.2 million $ total lifetime cost per patient or 6.8 billion $ yearly, in a conservative estimate of the national annual cost. The possibility to early identify the patients which will develop definite MS among the patients with the diagnosis of probable MS is of utmost importance, as it would be possible to start preventive treatment early and delay accumulation of irreversible neurological disability, inhibition/suppression of disease progression as well as reduce annual cost of disease.

It will be appreciated that the reagents utilized by any of the methods of the present invention which are described hereinabove can form a part of a diagnostic kit/article of manufacture.

As used herein the term "about" refers to ± 10 %.

Additional objects, advantages, and novel features of the present invention will become apparent to one ordinarily skilled in the art upon examination of the following examples, which are not intended to be limiting. Additionally, each of the various embodiments and aspects of the present invention as claimed in the claims section below finds experimental support in the following examples.

### EXAMPLES

Reference is now made to the following examples, which together with the above descriptions; illustrate the invention in a non-limiting fashion.

Generally, the nomenclature used herein and the laboratory procedures utilized in the present invention include molecular, biochemical, microbiological and recombinant DNA techniques. Such techniques are thoroughly explained in the literature. See, for example, "Molecular Cloning: A laboratory Manual" Sambrook et al., (1989); "Current Protocols in Molecular Biology" Volumes I-III Ausubel, R. M., ed. (1994); Ausubel et al., "Current Protocols in Molecular Biology", John Wiley and Sons, Baltimore, Maryland (1989); Perbal, "A Practical Guide to Molecular Cloning", John Wiley & Sons, New York (1988); Watson et al., "Recombinant DNA", Scientific American Books, New York; Birren et al. (eds) "Genome Analysis: A Laboratory Manual Series", Vols. 1-4, Cold Spring Harbor Laboratory Press, New York (1998); methodologies as set forth in U.S. Pat. Nos. 4,666,828; 4,683,202; 4,801,531; 5,192,659 and 5,272,057; "Cell Biology: A Laboratory Handbook", Volumes I-III Cellis, J. E., ed. (1994); "Current Protocols in Immunology" Volumes I-III Coligan J. E., ed. (1994); Stites et al. (eds), "Basic and Clinical Immunology" (8th Edition), Appleton & Lange, Norwalk, CT (1994); Mishell and Shiigi (eds), "Selected Methods in Cellular Immunology", W. H. Freeman and Co., New York (1980); available immunoassays are extensively described in the patent and scientific literature, see, for example, U.S. Pat. Nos. 3,791,932; 3,839,153; 3,850,752; 3,850,578; 3,853,987; 3,867,517; 3,879,262; 3,901,654; 3,935,074; 3,984,533; 3,996,345; 4,034,074; 4,098,876; 4,879,219; 5,011,771 and 5,281,521; "Oligonucleotide Synthesis" Gait, M. J., ed. (1984); "Nucleic Acid Hybridization" Hames, B. D., and Higgins S. J., eds. (1985); "Transcription and Translation" Hames, B. D., and Higgins S. J., Eds. (1984); "Animal Cell Culture" Freshney, R. I., ed. (1986); "Immobilized Cells and Enzymes" IRL Press, (1986); "A Practical Guide to Molecular Cloning" Perbal, B., (1984) and "Methods in Enzymology" Vol. 1-317, Academic Press; "PCR Protocols: A Guide To Methods And Applications", Academic Press, San Diego, CA (1990); Marshak et al., "Strategies for Protein Purification and Characterization - A Laboratory Course Manual" CSHL Press (1996); all of which are incorporated by reference as if fully set forth herein. Other general references are provided throughout this document The procedures therein are believed to be well known in the art and are provided for the convenience of the reader.

### GENERAL MATERIALS AND EXPERIMENTAL METHODS

***Study design and study subjects -*** 40 patients with probable MS of up to 3 months duration according to Poser's criteria (1983, CPMS C3: 1 attack, at least 1 clinical manifestation in addition to positive brain MRI signifying paraclinical evidence) were included in the study. Positive brain MRI was defined according to Fazekas's criteria (1999) by at least 4 focal lesions involving the white matter or 3 lesions if one is periventricular; ≥3 mm in diameter, each. For the evaluation of probable MS related transcription fingerprints the large-scale gene expression profile of patients was compared with the data of 10 sex- and age-matched healthy subjects. Verification of probable PBMC gene expression signature was performed on Signed written informed consent was obtained from all participants.

***Clinical neurological assessment** -* Neurological examination and assessment of disability was performed by the Expanded Disability Status Scale (EDSS) score (17) performed at screening, baseline visit, and at 24, 36 and 48 weeks of follow-up visits The occurrence of a second acute attack, the time to second attack (progression to definite MS) and the change in neurological disability assessed by the EDSS were recorded in each patient. Second attack was defined as the onset of new neurological symptoms or worsening of previous ones occurring at least 30 days after the first attack, lasting for at least 48 hours and involving an objective increase by at least 0.5 point in the EDSS.

***MRI examination* -** Brain MRI was performed using a 3.0 Tesla Imager (GE). Axial dual spin-echo (PD and T2 sequences) and T1-weighted images before and after intravenous administration of Gd-DTPA. Brain lesion load was quantified using the MS Analyze software (18). This automated technique is based on several mathematical algorithms (e.g., Bayesian classification, near-neighbourhood) leading to brain tissue segmentation enabling precise 3-dimentional lesions' identification and volumetric quantification.

***PBMC preparation** -* Blood sample (20 ml) was drawn from all study subjects. No corticosteroid treatment was given for at least 4 weeks prior to blood drawing. PBMC were separated on Ficoll hypaque, washed with PBS and the pellet frozen in liquid nitrogen.

***RNA extraction** -* Frozen PBMC were homogenized in ice cold trizol and total RNA extracted and used as a template for double stranded cDNA synthesis (Affymetrix, Santa Clara, CA). RNA quantity was determined by optical density measurements at 260 nm and its quality by running the RNA on a foramamide-formaldehyde denaturing gel.

***Preparation of labeled cRNA** -* Double stranded cDNA was performed using a cDNA synthesis kit (Life Technologies Superscript cDNA Synthesis System) with an oligo (dT) primer containing a T7 RNA polymerase promoter site added to the 3'. The cDNA was used as a template for *in vitro* transcription with biotin labeled nucleotides (Enzo Diagnostics). Labeled cRNA was used for hybridization.

***Hybridization of microarrays*** - Each Genechip array (U133A) was hybridized with 10 µg/200 µl hybridization mix, stained with streptavidin phycoerythrin (Molecular Probes), hybridized with biotin labeled anti-streptavidin phycoerythrin antibody, re-stained with streptavidin phycoerythrin and scanned (Hewlett Packard, GeneArray-TM scanner G2500A).

***Data Analysis** -* Data were analyzed using ScoreGene software. To correct for multiple testing the False Discovery Rate (FDR) method and the stringent Bonferroni correction were applied. Overabundance analysis was performed to examine the observed results in comparison to expected results. To assess and validate the predictive power of the gene expression signature, the following methods were applied: Leave-One-Out-Cross-Validation (LOOCV) (21; Ben-Dor A et al., 2000) Principal Component Analysis (PCA), and Support vector machine (SVM) [(http://ro.utia.cz/fs/fs_algorithms.html), (19, 20). The study involves various comparisons between subjects of the data set. Due to the reasons of controls compatibility, the number of controls changes from one comparison to another.

***Computation of the average error in determining which probable multiple sclerosis (MS) subjects exhibit high probability (predisposition) to develop the definite diagnosis of MS** -* For each of the 58 differentiating genes (SEQ ID NOs:1-58) the sample of 40 probable MS patients was randomly divided into 80 % as a "training set" and 20 % as a "test set". The SVM used RBF (radial basic function) kernel to build a model based on the "training set", which was further tested on the "test set" while saving the error rate. This procedure was repeated 25 times for each gene and the average error for each gene was calculated. Genes with the lowest average error were selected. Then, for each selected gene, the remaining genes were added one after the other, by selecting the next gene such that the average error after 25 repeats of the group of genes including the new gene has the lowest average error as compared to the addition of another gene. This process was repeated 57 times for each additional genes added to the previous group of genes. The results are shown in Table 6 and described in Example 3 hereinbelow.

### EXAMPLE 1

### IDENTIFICATION OF TRANSCRIPTS DIFFERENTIATING BETWEEN PROBABLE MS AND HEALTHY SUBJECTS

### Experimental and statistical results

***Analysis of large scale gene expression pattern** -* Analysis of large-scale gene expression patterns of PBMC samples obtained from 28 patients with probable MS (mean ± SE, age 36.0 ± 1.9 years, EDSS 1.5 ± 0.2) and 10 healthy matched controls was performed. Gene expression patterns of PBMC in probable MS patients were significantly different from healthy subject. Table 1, hereinbelow, depicts 554 genes that passed the 95 % confidence level in all 3 statistical scores (TNoM, Info, T-test); 352 genes were over-expressed and 202 under-expressed. These genes were defined as the *most informative* (Figure 1a). PCA performed on the 554 *most informative* genes divided all samples into two separated clusters that represented probable MS patients and healthy subjects with only 2 (5 %) classification errors (Figure 1b).

**Table 1**

| ***Differentially expressed markers between probable multiple sclerosis subjects and healthy controls*** | | | | | | | |
|---|---|---|---|---|---|---|---|
| ***Probeset*** | ***GenBank Acc. No.*** | ***Dir*** | ***Gene Symbol*** | ***Probeset*** | ***GenBank Acc. No.*** | ***Dir*** | ***Gene Symbol*** |
| 1494_f_at | M33318 | -1 | CYP2A6 | 203482 at | AL133215 | 1 | C10orf6 |
| 200797_s_at | AI275690 | -1 | MCL1 | 203487_s_at | NM_015396 | 1 | ARMC8 |
| 200798_x_at | NM_021960 | -1 | MCL1 | 203517_at | NM_006554 | 1 | MTX2 |
| 201041_s_at | NM_004417 | -1 | DUSP1 | 203583_at | NM_014044 | 1 | UNC50 |
| 201044_x_at | AA530892 | -1 | DUSP1 | 203683_s_at | NM_003377 | 1 | VEGFB |
| 201109_s_at | AV726673 | -1 | THBS1 | 203710_at | NM_002222 | 1 | ITPR1 |
| 201110_s_at | NM_003246 | -1 | THBS1 | 203721_s_at | NM_016001 | 1 | CGI-48 |
| 201235_s_at | BG339064 | -1 | BTG2 | 203738_at | AI421192 | 1 | FLJ11193 |
| 201236_s_at | BG339064 | -1 | BTG2 | 203775_at | NM_014251 | 1 | SLC25A13 |
| 201464_x_at | BC002646 | -1 | JUN | 203804_s_at | NM_006107 | 1 | CROP |
| 201473_at | NM_002229 | -1 | JUNB | 203865_s_at | NM_015833 | 1 | ADARB1 |
| 201489_at | BC005020 | -1 | PPIF | 203981_s_at | AL574660 | 1 | ABCD4 |
| 201490_s_at | NM_005729 | -1 | PPIF | 204000_at | NM_016194 | 1 | GNB5 |
| 201502_s_at | NM_020529 | -1 | NFKBIA | 204004_at | AI336206 | 1 | PAWR |
| 201531_at | NM_003407 | -1 | ZFP36 | 204060_s_at | NM_005044 | 1 | PRKX, PRKY |
| 201631_s_at | NM_003897 | -1 | IER3 | 204139_x_at | NM_003422 | 1 | ZNF42 |
| 201647_s_at | NM_005506 | -1 | SCARB2 | 204153_s_at | AI738965 | 1 | MFNG |
| 201668_x_at | AW163148 | -1 | MARCKS | 204178_s_at | NM_006328 | 1 | RBM14 |
| 201669_s_at | NM_002356 | -1 | MARCKS | 204185_x_at | NM_005038 | 1 | PPID |
| 201670_s_at | M68956 | -1 | MARCKS | 204202_at | NM_017604 | 1 | IQCE |
| 201693_s_at | NM_001964 | -1 | EGR1 | 204279_at | NM_002800 | 1 | PSMB9 |
| 201694_s_at | NM_001964 | -1 | EGR1 | 204291_at | NM_014803 | 1 | ZNF518 |
| 202672_s_at | NM_001674 | -1 | ATF3 | 204327_s_at | NM_003455 | 1 | ZNF202 |
| 204224_s_at | NM_000161 | -1 | GCHI | 204396_s_at | NM_005308 | 1 | GRK5 |
| 201884_at | NM_004363 | -1 | CEACAM5 | 204410_at | NM_004681 | 1 | EIF1AY |
| 202014_at | NM_014330 | -1 | PPP1R15A | 204510_at | NM_003503 | 1 | CDC7 |
| 202068_s_at | NM_000527 | -1 | LDLR | 204528_s_at | NM_004537 | 1 | NAP1L1 |
| 202081_at | NM_004907 | -1 | IER2 | 204581_at | NM_001771 | 1 | CD22 |
| 202286_s_at | J04152 | -1 | TACSTD2 | 204593_s_at | AA046752 | 1 | FLJ20232 |
| 202340_x_at | NM_002135 | -1 | NR4A1 | 204618_s_at | NM_005254 | 1 | GABPB2 |
| 202570_s_at | NM_014902 | -1 | DLGAP4 | 204640_s_at | NM_003563 | 1 | SPOP |
| 202637_s_at | AI608725 | -1 | ICAM1 | 204772_s_at | NM_007344 | 1 | TTF1 |
| 202638_s_at | NM_000201 | -1 | ICAM1 | 204848_x_at | NM_000559 | 1 | HBG1, HBG2 |
| 202643_s_at | AI738896 | -1 | TNFAIP3 | 204873_at | NM_000466 | 1 | PEX1 |
| 202644_s_at | NM_006290 | -1 | TNFAIP3 | 204950_at | NM_014959 | 1 | CARD8 |
| 202859_x_at | NM_000584 | -1 | IL8 | 205034_at | NM_004702 | 1 | CCNE2 |
| 202895_s_at | D86043 | -1 | PTPNS1 | 205049_s_at | NM_001783 | 1 | CD79A |
| 202924_s_at | AF006005 | -1 | PLAGL2 | 208985_s_at | BC002719 | 1 | EIF3S1 |
| 202925_s_at | NM_002657 | -1 | PLAGL2 | 209685_s_at | M13975 | 1 | PRKCB1 |
| 203317_at | NM_012455 | -1 | PSD4 | 205296_at | AL365505 | 1 | RBL1 |
| 203394_s_at | BE973687 | -1 | HESI | 205297_s_at | NM_000626 | 1 | CD79B |
| 203470_s_at | AI433595 | -1 | PLEK | 205353_s_at | NM_002567 | 1 | PBP |
| 203471_s_at | NM_002664 | -1 | PLEK | 205612_at | NM_007351 | 1 | MMRN1 |
| 203672_x_at | U12387 | -1 | TPMT | 205671_s_at | NM_002120 | 1 | HLA-DOB |
| 203927_at | NM_004556 | -1 | NFKBIE | 205788_s_at | NM_014827 | 1 | NM_014827 |
| 204103_at | NM_002984 | -1 | CCL4 | 205790_at | NM_003726 | 1 | SCAP1 |
| 204393_s_at | NM_001099 | -1 | ACPP | 205933_at | NM_015559 | 1 | SETBP1 |
| 204440_at | NM_004233 | -1 | CD83 | 206272_at | NM_006542 | 1 | SPHAR |
| 204469_at | NM_002851 | -1 | PTPRZ1 | 206493_at | NM_000419 | 1 | ITGA2B |
| 204470_at | NM_001511 | -1 | CXCL1 | 206494_s_at | NM_000419 | 1 | ITGA2B |
| 204533_at | NM_001565 | -1 | CXCL10 | 206652_at | NM_016384 | 1 | DKFZp779H233 |
| 204748_at | NM_000963 | -1 | PTGS2 | 206854_s_at | NM_003188 | 1 | MAP3K7 |
| 204794_at | NM_004418 | -1 | DUSP2 | 207081_s_at | NM_002650 | 1 | PIK4CA |
| 204897_at | AA897516 | -1 | PTGER4 | 207170_s_at | NM_015416 | 1 | LETMD1 |
| 204907_s_at | AI829875 | -1 | BCL3 | 207180_s_at | NM_006410 | 1 | HTATIP2 |
| 204970_s_at | NM_ 002359 | -1 | MAFG | 207314_x_at | NM_006737 | 1 | KIR3DL2 |
| 205045_at | NM_007202 | -1 | AKAP10 | 207405_s_at | NM_002873 | 1 | RAD17 |
| 205067_at | NM_000576 | -1 | IL1B | 207522_s_at | NM_005173 | 1 | ATP2A3 |
| 205114_s_at | NM_002983 | -1 | CCL3, CCL3L1 | 207655_s_at | NM_013314 | 1 | BLNK |
| 205115_s_at | NM_016196 | -1 | RBM19 | 207812_s_at | NM_015530 | 1 | GORASP2 |
| 205207_at | NM_000600 | -1 | IL6 | 207830_s_at | NM_002713 | 1 | PPP1R8 |
| 205220_at | NM_006018 | -1 | GPR109B | 207842_s_at | NM_007359 | 1 | CASC3 |
| 205318_at | NM_004984 | -1 | KIF5A | 208073_x_at | NM_003316 | 1 | TTC3 |
| 205558_at | NM_004620 | -1 | TRAF6 | 208184_s_at | NM_003274 | 1 | TMEM1 |
| 205643_s_at | NM_004576 | -1 | PPP2R2B | 208622_s_at | AA670344 | 1 | VIL2 |
| 205681_at | NM_004049 | -1 | BCL2A1 | 208640_at | BG292367 | 1 | RAC1 |
| 205767_at | NM_001432 | -1 | EREG | 208651_x_at | BG327863 | 1 | CD24 |
| 206025_s_at | AW188198 | -1 | TNFAIP6 | 208722_s_at | BC001081 | 1 | ANAPC5 |
| 206087_x_at | NM_ 000410 | -1 | HFE | 208752_x_at | AI888672 | 1 | NAP1L1 |
| 206157_at | NM_002852 | -1 | PTX3 | 208758_at_ | D89976 | 1 | ATIC |
| 206207_at | NM_001828 | -1 | CLC | 208792_s_at | M25915 | 1 | CLU |
| 206256_at | NM_001308 | -1 | CPN1 | 208809_s_at | AL136632 | 1 | C6orf62 |
| 206295_at | NM_001562 | -1 | IL18 | 208837_at | BC000027 | 1 | C15orf22 |
| 206704_at | NM_000084 | -1 | CLCN5 | 209007_s_at | AF247168 | 1 | NPD014 |
| 206765_at | AF153820 | -1 | KCNJ2 | 209058_at | AB002282 | 1 | EDF1 |
| 206880_at | NM_005446 | -1 | P2RXL1 | 209075_s_at | AY009128 | 1 | ISCU |
| 207075_at | NM_004895 | -1 | CIASI | 209139_s_at | AF083033 | 1 | PRKRA |
| 207113_s_at | NM_000594 | -1 | TNF | 209153_s_at | M31523 | 1 | TCF3 |
| 207287_at | NM_025026 | -1 | FLJ14107 | 209215_at | L11669 | 1 | TETRAN |
| 207490_at | NM_025019 | -1 | TUBA4 | 209234_at | BF939474 | 1 | KIF1B |
| 207535_s_at | NM_002502 | -1 | NFKB2 | 209273_s_at | BG387555 | 1 | HBLD2 |
| 207574_s_at | NM_015675 | -1 | GADD45B | 209284_s_at | AI922509 | 1 | RAP140 |
| 207850_at | NM_002090 | -1 | CXCL3 | 209340_at | S73498 | 1 | UAP1 |
| 207901_at | NM_002187 | -1 | IL12B | 209431_s_at | AF254083 | 1 | ZNF278 |
| 207984_s_at | NM_005374 | -1 | MPP2 | 209447_at | AF043290 | 1 | SYNE1 |
| 208025_s_at | NM_003483 | -1 | HMGA2 | 209449_at | AF196468 | 1 | LSM2 |
| 208039_at | NM_003048 | -1 | NM_003048 | 209452_s_at | AF035824 | 1 | VTI1B |
| 208120_x_at | NM_031221 | -1 | --- | 209482_at | BC001430 | 1 | POP7 |
| 208455_at | NM_002855 | -1 | PVRLI | 209486_at | _BC004546 | 1 | SAS10 |
| 208695_s_at | BC001019 | -1 | RPL39 | 209503_s_at | AF035309 | 1 | PSMC5 |
| 208886_at_ | BC000145 | -1 | H1F0 | 209549_s_at | C001121 | 1 | DGUOK |
| 208961_s_at | AB017493 | -1 | COPEB | 209572_s_at | AF080227 | 1 | EED |
| 209034_at | AF279899 | -1 | PNRCI | 209625_at | BC004100 | 1 | PIGH |
| 209039_x_at | AF001434 | -1 | EHD1 | 209630_s_at | U87460 | 1 | FBXW2 |
| 209124_at | U70451 | -1 | MYD88 | 209659_s_at | AF164598 | 1 | CDC16 |
| 209304_x_at | AF087853 | -1 | GADD45B | 209729_at | BC001782 | 1 | GAS2L1 |
| 209305_s_at | AF078077 | -1 | GADD45B | 209771_x_at | AA761181 | 1 | CD24 |
| 209545_s_at | AF027706 | -1 | RIPK2 | 209778_at | AF007217 | 1 | TRIP11 |
| 209738_x_at | M31125 | -1 | M31125 | 209903_s_at | U49844 | 1 | ATR |
| 209774_x_at | M57731 | -1 | CXCL2 | 209917_s_at | BC002709 | 1 | TP53AP1 |
| 209874_x_at | AK023066 | -1 | CNNM2 | 210046_s_at | U52144 | 1 | IDH2 |
| 209939_x_at | AF005775 | -1 | CFLAR | 210356_x_at | BC002807 | 1 | MS4A1 |
| 210001_s_at | AB005043 | -1 | SOCS1 | 210378_s_at | BC004118 | 1 | SSNA1 |
| 210118_s_at | M15329 | -1 | ILIA | 210627_s_at | BC002804 | 1 | GCS1 |
| 210175_at | BC000853 | -1 | C2orf3 | 210690_at | U96845 | 1 | KLRC4 |
| 210254_at | L35848 | -1 | MS4A3 | 210715_s_at | AF027205 | 1 | SPINT2 |
| 210275_s_at | AF062347 | -1 | ZA20D2 | 210719_s_at | BC002552 | 1 | HMG20B |
| 210414_at | AF169675 | -1 | FLRT1 | 210886_x_at | AB007457 | 1 | TP53AP1 |
| 210592_s_at | M55580 | -1 | SAT | 210927_x_at | BC004239 | 1 | JTB |
| 210651_s_at | L41939 | -1 | EPHB2 | 211036_x_at | BC006301 | 1 | ANAPC5 |
| 211307_s_at | U43677 | -1 | FCAR | 211479_s_at | M81778 | 1 | HTR2C |
| 211332_x_at | AF144241 | -1 | HFE | 211881_x_at | AB014341 | 1 | IGLJ3 |
| 211403_x_at | AF167079 | -1 | VCX-(C,2,3); VCY | 213351_s_at | AB018322 | 1 | KIAA0779 |
| 211434_s_at | AF015524 | -1 | CCRL2 | 213364_s_at | AI052536 | 1 | SNX1 |
| 211506 s at | AF043337 | -1 | IL8 | 211784_s_at | BC006181 | 1 | SFRS1 |
| 211578_s_at | M60725 | -1 | RPS6KB1 | 211945_s_at | BG500301 | I | ITGB1 |
| 211610_at | U51869 | -1 | COPEB | 211954_s_at | BC000947 | 1 | RANBP5 |
| 213575_at | AW978896 | -1 | TRA2A | 211979_at | AB046844 | 1 | GPR107 |
| 215462_at | AI978990 | -1 | LOC149478 | 212040_at | BG249599 | 1 | GOLN2 |
| 211863_x_at | AF079408 | -1 | HFE | 212068_s_at | AB011087 | 1 | KIAA0515 |
| 211924_s_at | AY029180 | -1 | PLAUR | 212114_at | BE967207 | 1 | microtubule protein |
| 211973 at | AW341200 | -1 | NUDT3 | 212121 at | BE962354 | 1 | C10orf61 |
| 212099_at | AI263909 | -1 | RHOB | 212140_at | AB014548 | 1 | SCC-112 |
| 212291_at | AI393355 | -1 | HIPKI | 212201_at | AW274877 | 1 | KIAA0692 |
| 212602_at | AI806395 | -1 | WDFY3 | 212244_at | AL050091 | 1 | GRINLIA |
| 212657_s_at | AW083357 | -1 | IL1RN | 212247_at | AW008531 | 1 | NUP205 |
| 212659_s_at | AW083357 | -1 | IL1RN | 212321_at | BE999972 | 1 | BE999972 |
| 213002_at | AA770596 | -1 | AA770596 | 212326_at | AB007922 | 1 | VPS13D |
| 213038_at | AL031602 | -1 | IBRDC3 | 212361_s_at | AA805753 | 1 | ATP2A2 |
| 213146_at | AA521267 | -1 | JMJD3 | 212548_s_at | BF515124 | 1 | IAA0826 |
| 213281_at | BE327172 | -1 | BE327172 | 212559_at | AU148827 | 1 | AU148827 |
| 213593_s at | AW978896 | -1 | TRA2A | 212566_at | AL523310 | 1 | MAP4 |
| 213632_at | M94065 | -1 | DHODH | 212583_at | AB011132 | 1 | AB011132 |
| 213675_at | W61005 | -1 | FLJ25106 | 212611_at | AV728526 | 1 | DTX4 |
| 213676_at | AL038824 | -1 | AW125688 | 212632_at | N32035 | 1 | STX7 |
| 213988_s_at | BE971383 | -1 | SAT | 212655_at | AB011151 | 1 | ZCCHC14 |
| 214211_at | AA083483 | -1 | FTH1 | 212667_at | AL575922 | 1 | SPARC |
| 214349_at | AV764378 | -1 | ORF2 | 212690_at | AB018268 | 1 | DDHD2 |
| 214421_x_at | AV652420 | -1 | CYP2C9 | 212714_at | AL050205 | 1 | LOC113251 |
| 214637_at | BG437034 | -1 | OSM | 212750_at | AB020630 | 1 | PPP1R16B |
| 214657_s_at | AU134977 | -1 | TncRNA | 212760_at | AB002347 | 1 | C6orf133 |
| 215006_at | AK023816 | -1 | EZH2 | 212813_at | AA149644 | 1 | JAM3 |
| 215078_at | AL050388 | -1 | SOD2 | 212855_at | D87466 | 1 | KIAA0276 |
| 215189_at | X99142 | -1 | KRTHB6 | 212955_s_at | AL037557 | 1 | POLR2I |
| 215223_s_at | W46388 | -1 | SOD2 | 213061_s_at | AA643304 | 1 | NTAN1 |
| 215308_at | AF052148 | -1 | G22P1 | 213065 at | AB011118 | 1 | MGC23401 |
| 215485_s_at | AA284705 | -1 | ICAMI | 213088_s_at | BE551340 | 1 | DNAJC9 |
| 215577_at | AU146791 | -1 | UBE2E1 | 213090_s_at | AI744029 | 1 | TAF4 |
| 215758 x_at | AC007204 | -1 | ZNF505 | 213106_at | AI769688 | 1 | ATP8A1 |
| 215775_at | BF084105 | -1 | BF084105 | 213165_at | AI041204 | 1 | CAP350 |
| 215899_at | AK022331 | -1 | AK022331 | 213213 at | AL035669 | 1 | DATF1 |
| 215987_at | AV654984 | -1 | AV654984 | 213267_at | AL162056 | 1 | KIAA1117 |
| 216015_s_at | AK027194 | -1 | CIAS1 | 213278 at | AW014788 | 1 | MTMR9 |
| 216016_at | AK027194 | -1 | CIAS1 | 213375_s_at | N80918 | 1 | CG018 |
| 216084_at | AL080137 | -1 | LOC389715 | 213408_s_at | AK024034 | 1 | IK4CA |
| 216114_at | AL049430 | -1 | NCKIPSD | 213410_at | AL050102 | 1 | C10orf137 |
| 216153_x_at | AK022897 | -1 | RECK | 213460_x_at | N29665 | 1 | WBSCR20C |
| 216243_s_at | BE563442 | -1 | IL1RN | 213483_at | AK025679 | 1 | KIAA0073 |
| 216336_x_at | AL031602 | -1 | AL031602 | 213515_x_at | AI133353 | 1 | BG2 |
| 216366_x_at | AF047245 | -1 | AF047245 | 213528_at | AL035369 | 1 | MGC9084 |
| 216438_s_at | AL133228 | -1 | TMSB4X, TMSL3 | 213626_at | AL049442 | 1 | CBR4 |
| 216678_at | AK000773 | -1 | AK000773 | 213634_s_at | AL031588 | 1 | TRMT1 |
| 216774 at | AK025325 | -1 | AK025325 | 213653 at | AW069290 | 1 | METTL3 |
| 216973_s_at | S49765 | -1 | HOXB7 | 213659 at | AA209420 | 1 | ZNF75 |
| 216997 x at | AL358975 | -1 | TLE4 | 213672 at | AA621558 | 1 | MARS |
| 217362_x_at | AF005487 | -1 | HLA-DRB6 | 213838_at | AA191426 | 1 | AA191426 |
| 217415_at | M21610 | -1 | M21610 | 214130_s_at | AI821791 | 1 | PDE4DIP |
| 217489_s_at | S72848 | -1 | IL6R | 214669_x_at | BG485135 | 1 | BG485135 |
| 217741_s_at | NM_006007 | -1 | ZA20D2 | 214749_s_at | AK000818 | 1 | ARMCX6 |
| 217996_at | NM_007350 | -1 | PHLDA1 | 214948_s_at | AL050136 | 1 | TMF1 |
| 218177_at | NM_020412 | -1 | CHMP1.5 | 215158_s_at | AK022531 | | DEDD |
| 218198_at | NM_018180 | -1 | DHX32 | 215227_x_at | BG035989 | 1 | ACP1 |
| 218611_at | NM_016545 | -1 | IER5 | 215318_at | AL049782 | 1 | CG012 |
| 218810_at | NM_025079 | -1 | FLJ23231 | 215343_at | AF070587 | 1 | 0610010D24Rik |
| 219312_s_at | NM_023929 | -1 | ZBTB10 | 215648_at | AU144324 | 1 | KIAA1068 |
| 219358_s_at | NM_018404 | -1 | CENTA2 | 215925_s_at | AF283777 | 1 | CD72 |
| 219397_at | NM_025147 | -1 | FLJ13448 | 216237_s_at | AA807529 | 1 | MCM5 |
| 219450_at | NM_018302 | -1 | FLJ11017 | 216261_at | AI151479 | 1 | ITGB3 |
| 219617_at | NM_024766 | -1 | FLJ23451 | 216338_s_at | AK021433 | 1 | C6orf109 |
| 219901_at | NM_018351 | -1 | FGD6 | 218100_s_at | NM_018010 | 1 | ESRRBL1 |
| 219935_at | NM_007038 | -1 | ADAMTS5 | 219802_at | NM_024854 | 1 | FLJ22028 |
| 220054_at | NM_016584 | -1 | IL23A | 216379_x_at | AK000168 | 1 | CD24 |
| 220091_at | NM_017585 | -1 | SLC2A6 | 216907_x_at | X93596 | 1 | KIR3DL2 |
| 220215_at | NM_024804 | -1 | FLJ12606 | 216956_s_at | AF098114 | 1 | ITGA2B |
| 220712_at | NM_024984 | -1 | NM 024984 | 217043_s_at | U95822 | 1 | MFN1 |
| 220737_at | AF184965 | -1 | AF184965 | 217418_x_at | X12530 | 1 | MS4A1 |
| 220740_s_at | NM_005135 | -1 | SLC12A6 | 217645_at | AW088547 | 1 | AW088547 |
| 220776_at | NM_013348 | -1 | KCNJ14 | 217730_at | NM_022152 | 1 | PP1201 |
| 220924_5_art | NM_018976 | -1 | SLC38A2 | 217772_s_at | NM_014342 | 1 | MTCH2 |
| 221236_s_at | NM 030795 | -1 | STMN4 | 217792_at | NM_014426 | 1 | SNX5 |
| 221323_at | NM_025218 | -1 | ULBP1 | 217815_at | NM_007192 | 1 | SUPT16H |
| 221345_at | NM_005306 | -1 | GPR43 | 217896_s_at | NM_024946 | 1 | NIP30 |
| 221477_s_at | BC001980 | -1 | gasdermin | 217906_at | NM_014315 | 1 | KLHDC2 |
| 222136_x_at | AK022905 | -1 | ZNF43 | 217908_s_at | NM_018442 | 1 | IQWD1 |
| 222303 at | AV700891 | -1 | AV700891 | 217938_s_at | AI743396 | 1 | KCMF1 |
| 222326_at | AW973834 | -1 | AW973834 | 217978_s_at | NM_017582 | 1 | UBE2Q |
| 222329_x_at | AW974816 | -1 | AW974816 | 217979_at | NM_014399 | 1 | TM4SF13 |
| 36564_at | W27419 | -1 | IBRDC3 | 217986_s_at | AA102574 | 1 | BAZ1A |
| 37028_at | U83981 | -1 | PPP1R15A | 218004_at | NM_018045 | 1 | FLJ10276 |
| 39402_at | M15330 | -1 | IL1B | 218082_s_at | NM_014517 | 1 | UBP1 |
| 41386_i_at | AB002344 | -1 | JMJD3 | 218124_at | NM_017750 | 1 | FLJ20296 |
| 41387_r_at | AB002344 | -1 | JMJD3 | 218132_s_at | NM_024075 | 1 | LENG5 |
| 200023_s_at | AI001896 | 1 | EIF3S5 | 218179_s_at | NM_021942 | 1 | FLJ12716 |
| 200028_s_at | NM_020151 | 1 | STARD7 | 218212_s_at | NM_004531 | 1 | MOCS2 |
| 200030_s_art | NM_002635 | I | SLC25A3 | 218220_at | NM_021640 | 1 | C12orf10 |
| 200083_at | AA621731 | 1 | USP22 | 218237_s_at | NM_030674 | 1 | SLC38A1 |
| 200098_s_at | T33068 | 1 | ANAPC5 | 218250_s_at | NM_013354 | 1 | CNOT7 |
| 200609_s_at | NM 017491 | 1 | WDR1 | 218301 at | NM_018226 | 1 | RNPEPL1 |
| 200625_s_at | NM_006367 | 1 | CAP1 | 218328_at | NM_016035 | 1 | COQ4 |
| 200688_at | D13642 | 1 | SF3B3 | 218339 at | NM_014180 | 1 | MRPL22 |
| 200816_s_at | NM 000430 | 1 | PAFAH1B1 | 218361_at | NM_018178 | 1 | GPP34R |
| 200830_at | NM_002808 | 1 | PSMD2 | 218371_s_at | AA969958 | 1 | PSPCI |
| 200840_at | NM_005548 | 1 | KARS | 218432_at | NM_012175 | 1 | FBXO3 |
| 200870_at | NM_007178 | 1 | STRAP | 218440_at | NM_020166 | 1 | MCCC1 |
| 200899_s_at | AK002091 | 1 | MGEAS | 218456_at | NM_023925 | 1 | C1QDC1 |
| 200910_at | NM_005998 | 1 | CCT3 | 218462_at | NM_025065 | 1 | RPF1 |
| 200932_s_art | NM_006400 | 1 | DCTN2 | 218504_at | NM_016044 | 1 | FAHD2A |
| 200961_at | NM_012248 | 1 | SEPHS2 | 218533_s_at | NM_017859 | 1 | UCKL1 |
| 201010_s_at | NM_006472 | 1 | TXNIP | 218575_at | NM_22662 | 1 | ANAPC1 |
| 201027_s_at | NM_015904 | 1 | EIF5B | 218607_s_at | NM_018115 | 1 | SDAD1 |
| 201036_s_at | NM_005327 | 1 | HADHSC | 218608_at | NM_022089 | 1 | HSA9947 |
| 201106_at | NM_002085 | 1 | GPX4 | 218626_at | NM_019843 | 1 | EIF4ENIF1 |
| 201174_s_at | NM_018975 | 1 | TERF21P | 218639_s_at | NM_025112 | 1 | MGC11349 |
| 201229_s_at | BC000422 | 1 | ARIH2 | 218654_s_at | NM_016071 | 1 | MRPS33 |
| 201405_s_at | NM_006833 | 1 | COPS6 | 218667_at | NM_022368 | 1 | PJA1 |
| 201540_at | NM_001449 | 1 | FHL1 | 218679_s_at | NM_016208 | 1 | VPS28 |
| 201541_s_at | NM_006349 | 1 | ZNHIT1 | 218771_at | NM_018216 | 1 | PANK4 |
| 201569_s_at | NM_015380 | 1 | CGI-51 | 219067_s_at | NM_017615 | 1 | C10orf86 |
| 201612_at | NM_000696 | 1 | ALDH9A1 | 219126_at | NM_018288 | 1 | PHF10 |
| 201672_s_at | NM_005151 | 1 | USP14 | 219151_s_at | NM_007081 | 1 | RABL2A, RABL2B |
| 201688_s_at | BG389015 | 1 | TPD52 | 219180_s_at | AI817074 | 1 | PEX26 |
| 201689_s_at | NM_005079 | 1 | TPD52 | 219185_at | NM_012241 | 1 | SIRT5 |
| 202042_at | NM_002109 | 1 | HARS | 219283_at | NM_014158 | 1 | CIGALT2 |
| 202658_at | NM_003846 | 1 | PEX11B | 219292_at | NM_018105 | 1 | THAP1 |
| 202836_s_at | BC001046 | 1 | TXNL4A | 219317_at | NM_007195 | 1 | POLI |
| 204220_at | NM_004877 | 1 | GMFG | 219470_x_at | NM_019084 | 1 | CCNJ |
| 201805_at | NM_002733 | 1 | PRKAG1 | 219581_at | NM_025265 | 1 | SEN2L |
| 201863_at | NM_014077 | 1 | FAM32A | 219627_at | NM_024910 | 1 | FLJ12700 |
| 201959_s_at | AA488899 | 1 | MYCBP2 | 219822_at | NM_004294 | 1 | MTRF1 |
| 201964_at | N64643 | 1 | N64643 | 219880 at | NM 022907 | 1 | FLJ23053 |
| 202009_at | NM_007284 | 1 | PTK9L | 219979_s_at | NM_016401 | 1 | HSPC138 |
| 202020_s_at | NM_006055 | 1 | LANCL1 | 220044_x at | NM 016424 | 1 | CROP |
| 202057_at | AW051311 | 1 | KPNA1 | 220059 at | NM_012108 | 1 | BRDG1 |
| 202116_at | NM_06268 | 1 | DPF2 | 220169 at | NM_024943 | 1 | FLJ23235 |
| 202127_at | AB011108 | 1 | PRPF4B | 220216_at | NM_019607 | 1 | FLJ11267 |
| 202167_s_at | NM_022362 | 1 | MMS19L | 220261_s_at | NM_018106 | 1 | ZDHHC4 |
| 202220_at | NM_014949 | 1 | KIAA0907 | 220387_s_at | NM_007071 | 1 | HHLA3 |
| 202244_at | NM_002796 | 1 | PSMB4 | 220418_at | NM_018961 | 1 | UBASH3A |
| 202259_s_at | NM_014887 | 1 | PFAAP5 | 220936_s_at | NM_018267 | 1 | H2AFJ - |
| 202265_at | NM_005180 | 1 | BMI1 | 221208_s_at | NM_024631 | 1 | FLJ23342 |
| 202271_at | AB007952 | 1 | AB007952 | 221253_s_at | NM_030810 | 1 | TXNDC5 |
| 202296_s_at | NM_007033 | 1 | RER1 | 221476_s_at | AF279903 | 1 | RPL15 |
| 202306_at | NM_002696 | 1 | POLR2G | 221515_s_at | BC001214 | 1 | LCMT1 |
| 202342_s_at | NM_015271 | 1 | TRIM2 | 221516_s_at | BC002587 | 1 | FLJ20232 |
| 202353_s_at | NM_002816 | 1 | PSMD12 | 221548_s_at | AY024365 | 1 | ILKAP |
| 202365_at | BC004815 | 1 | MGC5139 | 221586_s_at | U15642 | 1 | E2F5 |
| 202439_s_at | NM_000202 | 1 | IDS | 221736 at | BG236163 | 1 | KIAA1219 |
| 202475_at | NM_006326 | 1 | NIFIE14 | 221791_s_at | BG167522 | 1 | HSPC016 |
| 202529_at | NM_002766 | 1 | PRPSAP1 | 221811 at | BF033007 | 1 | PERLD1 |
| 202560_s_at | NM_015607 | 1 | DKFZP547E1010 | 221932_s_at | AA133341 | 1 | C14orf87 |
| 202564_x_at | NM 001667 | 1 | ARL2 | 222111_at | AU145293 | 1 | KlAA1164 |
| 202568_s_at | AI745639 | 1 | MARK3 | 222130_s_at | AK024635 | 1 | FTSJ2 |
| 202759_s_at | BE879367 | 1 | PALM2-AKAP2 | 222155_s_at | AK021918 | 1 | GPR172A |
| 202761_s_at | NM_015180 | 1 | SYNE2 | 222233_s_at | AK022922 | 1 | DCLREIC |
| 202792_s_at | NM_014678 | 1 | KIAA0685 | 222273_at | AI419423 | 1 | AI419423 |
| 202811_at | NM_006463 | 1 | STAMBP | 266_s_at | L33930 | 1 | CD24 |
| 202892_at | NM_004661 | 1 | CDC23 | 32259_at | AB002386 | 1 | EZH1 |
| 202960_s_at | NM_000255 | 1 | MUT | 33307_at | AL022316 | 1 | CGI-96 |
| 202983_at | A1760760 | 1 | SMARCA3 | 37965_at | AA181053 | 1 | PARVB |
| 203117_s_at | NM_014871 | 1 | USP52 | 37966_at | AA187563 | 1 | PARVB |
| 203194_s_at | NM_005387 | 1 | NUP98 | 38043_at | X55448 | 1 | FAM3A |
| 203221_at | NM_005077 | 1 | TLE1 | 45828_at | AI768100 | 1 | FLJ10241 |
| 203264_s_at | NM_015185 | 1 | ARHGEF9 | 48825 at | AA887083 | 1 | ING4 |
| 203266_s_at | NM_003010 | 1 | MAP2K4 | 50374_at | AA150503 | I | LOC339229 |
| 203301_s_at | NM_021145 | 1 | DMTF1 | 52164_at | AA065185 | 1 | C11orf24 |
| 203378_at | AB020631 | 1 | PCF11 | 64432_at | W05463 | 1 | FLJ39616 |
| 203445_s_at | NM_005730 | 1 | CTDSP2 | 65472_at | AI161338 | 1 | AI161338 |

| | | | | | | | |
|---|---|---|---|---|---|---|---|
| Table 1: Provided are genetic markers which are differentially expressed between subjects diagnosed with probable multiple sclerosis and healthy controls (the Probeset ID of the Affymetrix Gene Chip), along with the corresponding GenBank accession number (GenBank Acc. No.), the gene symbol and the direction (Dir) of change in gene expression ("1"- upregulation; "-1" - downregulation). Note that the p values of the TNOM, Info and t-Test statistical tests all passed the 95 % confidence level. | | | | | | | |

***Independent verification by support vector machine (SVM)** -* Verification of the probable PBMC gene expression signature (554 genes) was performed on an independent group of 15 subjects (12 patients, 3 controls) by SVM analysis and resulted in high classification rate of 80 %. These findings suggest that the identified gene expression signature in probable MS is reliable and not related to spurious difference due to multiple testing.

### EXAMPLE 2

### GENE EXPRESSION ANALYSIS OF SUBJECTS WHOSE DIAGNOSIS CONVERTED TO DEFINITE MS OR SUSTAINED PROBABLE MS

### Experimental results

***Conversion to definite MS - 2-year results** -* During the follow up period of 2 year, 30 % of patients (12/40) experienced a second attack and progressed to definite MS disease (defined as early convectors to definite MS). Comparison of the gene expression pattern of only these probable patients who further experienced a second attack and therefore defined as definite MS (12 patients) (using the blood cell samples obtained when the subjects were defined as probable multiple sclerosis, i.e., after the first neurological attack) to matched control group (11 subjects) identified 1517 most informative genes (Table 2, hereinbelow and Figure 2a), all passed FDR at p < 0.03 and 8 genes - Bonfferoni correction at p < 3.2 x 10⁻⁵. PCA performed on these 1517 most informative genes demonstrated correct classification rate with no errors (Figure 2b).

**Table 2**

| ***Differentially expressed markers between probable multiple sclerosis subjects which further developed definite multiple sclerosis and healthy controls.*** | | | | | | | |
|---|---|---|---|---|---|---|---|
| ***Probeset*** | ***GenBank Acc No.*** | ***Dir*** | ***Gene Symbol*** | ***Probeset*** | ***GenBank Acc. No.*** | ***Dir*** | ***Gene Symbol*** |
| 214376_at | AI263044 | -1 | AI263044 | 221596_s_at | AL136619 | 1 | DKFZP564O052 3 |
| 217446_x_a t | AL080160 | -1 | AL080160 | 202537_s_at | NM_01404 3 | 1 | DKFZP564O123 |
| 215185_at | AK024177 | -1 | AK024177 | 210006_at | BC002571 | 1 | DKFZP564O243 |
| 217449_at | AL137284 | -1 | DKFZp434D151 6 | 213149_at | AW299740 | 1 | DLAT |
| 221877_at | BF508835 | -1 | FKSG27 | 205677_s_at | NM_00588 | 1 | DLEU1 |
| 222135_at | AK022663 | -1 | KIAA1956 | 208810_at | AF080569 | 1 | DNAJB6 |
| 217381_s_at | X69383 | -1 | T-cell receptor gamma | 212467_at | AB014578 | 1 | DNAJC13 |
| 221477_s_at | BC001980 | -1 | NP_079012.2 | 213088_s_at | BE551340 | 1 | DNAJC9 |
| 215995_x_a | AUI47598 | -1 | LOC283585 | 213092_x_a t | AW241779 | 1 | DNAJC9 |
| 214826_at | U79276 | -1 | 2'-PDE | 217917_s_at | NM_01418 3 | 1 | DNCL2A |
| 44702_at | R77097 | -1 | 7h3 | 212538_at | AL576253 | 1 | DOCK9 |
| 201269_s_at | AB028991 | -1 | AB028991 | 221677_s_at | AF232674 | 1 | DONSON |
| 214979_at | AK000791 | -1 | ABCC3 | 219452_at | NM_02235 5 | 1 | DPEP2 |
| 205566_at | NM_00701 | -1 | ABED2 | 219373_at | NM_01897 3 | 1 | DPM3 |
| 207268_x_at | NM_00575 9 | -1 | ABI2 | 208370_s_at | NM_00441 4 | 1 | DSCR1 |
| 2170M_x_at | AC004832 | -1 | AC004832 | 218576_s_at | NM_00724 0 | 1 | DUSP12 |
| 206776_x_at | NM_00161 2 | -1 | ACRV1 | 208956_x_a t | AB049113 | 1 | DUT |
| 207973_x_at | NM_02011 0 | -1 | ACRV1 | 209033_s_at | D86550 | 1 | DYRK1A |
| 207990_x_at | NM_02011 4 | -1 | ACRV1 | 207232_s_at | NM_01464 8 | 1 | DZIP3 |
| 207991_x_at | NM_02011 3 | -1 | ACRV1 | 221586_s_at | U15642 | 1 | E2F5 |
| 211489_at | D32201 | -1 | ADRA1A | 220942_x_at | NM_01436 7 | 1 | E2IG5 |
| 38447_at | U08438 | -1 | ADRBK1 | 219787_s_at | NM_01809 8 | 1 | ECT2 |
| 201205_at | AF006751 | -1 | AF006751 | 208883_at | BF515424 | 1 | EDD |
| 216366_x_at | AF047245 | -1 | AF047245 | 209058_at | AB002282 | 1 | EDF1 |
| 211189_x_at | AF054816 | -1 | AF054816 | 209059_s_at | AB002282 | 1 | EDF1 |
| 215837_x_at | AF113018 | -1 | AF113018 | 212410_at | AI346431 | 1 | EFHA1 |
| 208849_at | AF118091 | -1 | AF118091 | 218438_s_at | NM_02520 5 | 1 | EG1 |
| 219558_at | NM_02452 4 | -1 | AFURS1 | 218696_at | NM_00483 6 | 1 | EIF2AK3 |
| 205357_s_at | NM_00068 5 | -1 | AGTR1 | 218287_s_at | NM_01219 9 | 1 | EIF2C1 |
| 207464_at | NM_01412 1 | -1 | AHCYL1 | 212716_s_at | AW083133 | 1 | eIF3k |
| 214139_at | AI051476 | -1 | AI051476 | 208756_at | U36764 | 1 | EIF3S2 |
| 221960_s_at | AI189609 | -1 | AI189609 | 201437_s_at | NM_00196 8 | 1 | EIF4E |
| 214379_at | AI954458 | -1 | AI954458 | 214919_s_at | R39094 | 1 | EIF4EBP3, MASK-BP3 |
| 216678_at | AK000773 | -1 | AK000773 | 210839_s_at | D45421 | 1 | ENPP2 |
| 216682_s_at | AK021457 | -1 | AK021457 | 202596_at | BC000436 | 1 | ENSA |
| 216051_x_at | AK022045 | -1 | AK022045 | 212681_at | AI770004 | 1 | EPB41L3 |
| 217335_at | AK023539 | -1 | AK023539 | 202909_at | NM_01480 5 | 1 | EPM2AIP1 |
| 216739_at | AK024527 | -1 | AK024527 | 202776_at | NM_01459 7 | 1 | ERBP |
| 216746_at | AK024606 | -1 | AK024606 | 200043_at | NM_00445 0 | 1 | ERH |
| 216437_at | AK024949 | -1 | AK024949 | 218100_s_at | NM_01801 0 | 1 | ESRRBL1 |
| 215405_at | AK025072 | -1 | AK025072 | 202942_at | NM_00198 5 | 1 | ETFB |
| 216774_at | AK025325 | -1 | AK025325 | 205530_at | NM_00445 3 | 1 | ETFDH |
| 217218_at | AK027005 | -1 | AK027005 | 217838_s_at | NM_01633 7 | 1 | EVL |
| 210517_s_at | AB003476 | -1 | AKAP12 | 215136_s_at | AL050353 | 1 | EXOSC8 |
| 205359_at | NM_00427 | -1 | AKAP6 | 32259_at | AB002386 | 1 | EZH1 |
| 211172_x_a | AF161075 | -1 | AKAP7 | 202862_at | NM_00013 7 | 1 | FAH |
| 204151_x_a | NM_00135 | -1 | AKR1C1 | 218504_at | NM_01604 4 | 1 | FAHD2A |
| 21636_x_a | AL031602 | -1 | AL031602 | 220643_s_at | NM_01814 7 | 1 | FAIM |
| 41856_at | AL049370 | -1 | AL049370 | 209405_s_at | BC002934 | 1 | FAM3A |
| 216499_at | AL137590 | -1 | AL137590 | 38043_at | X55448 | 1 | FAM3A |
| 216828_at | AL441988 | -1 | AL441988 | 201889_at | NM_01488 8 | 1 | FAM3C |
| 210544_s_at | BC002430 | -1 | ALDH3A2 | 204283_at | NM_00656 7 | 1 | FARS1 |
| 214423_x_a | AV652403 | - 1 | ALDOB | 211623_s_at | M30448 | 1 | FBL |
| 207755_at | NM_02501 7 | - 1 | AMOTL2 | 222119_s_at | AL117620 | 1 | FBXO11 |
| 213001_at | AF007150 | -1 | ANGPTL2 | 212231_at | AB020682 | 1 | FBXO21 |
| 218418_s_at | NM_01549 3 | -1 | ANKRD25 | 218432_at | NM_01217 5 | 1 | FBXO3 |
| 203074_at | NM_00163 0 | -1 | ANXA8 | 206759_at | NM_00200 2 | 1 | FCER2 |
| 21155_x_a | AF149794 | -1 | APAF1 | 210889_s_at | M31933 | 1 | FCGR2B |
| 206350_at | NM_00163 9 | -1 | APCS | 203620_s_at | NM_01482 4 | 1 | FCHSD2 |
| 202914_s_at | NM_01478 4 | -1 | ARHGEF11 | 203115_at | AU152635 | 1 | FECH |
| 207919_at | NM_00431 4 | -1 | ART1 | 201540_at | NM_00144 9 | 1 | FHL1 |
| 202672_s_at | NM_00167 4 | -1 | ATF3 | 214505_s_at | AF220153 | 1 | FHL1 |
| 216070_at | AL049331 | -1 | ATP1B1 | 221007_s_at | NM_03091 7 | 1 | FIP1L1 |
| 214132_at | BG232034 | -1 | ATP5C1 | 219117_s_at | NM_01659 4 | 1 | FKBP11 |
| 204624_at | NM_00005 3 | -1 | ATP7B | 219118_at | NM_01659 4 | 1 | FKBP11 |
| 222303_at | AV700891 | -1 | AV700891 | 218003_s_at | M90820 | 1 | FKBP3 |
| 206251_s_at | NM_00070 6 | -1 | AVPR1A | 218349_s_at | AA824298 | 1 | FLJ10036 |
| 212999_x_a t | AW276186 | -1 | AW276186 | 205510_s_at | NM_01797 6 | 1 | FLJ0038 |
| 222363_at | AW979018 | -1 | AW979018 | 219648_at | NM_01800 0 | 1 | FLJ10116 |
| 219326_s_at | AF288208 | -1 | B3GNT1 | 218067_s_at | NM_01801 1 | 1 | FLJ10154 |
| 209413_at | BC002431 | -1 | B4GALT2 | 218974_at | NM_01801 3 | 1 | FLJ10159 |
| 91920_at | AI205180 | -1 | BCAN | 218004_at | NM_01804 5 | 1 | FLJ10276 |
| 219444_at | NM_021946 | -1 | BCORL1 | 217900_at | NM_01806 0 | 1 | FLJ10326 |
| 213281_at | BE327172 | -1 | BE327172 | 221934_s_at | BF941492 | 1 | FLJ10496 |
| 221917_s_at | BF058465 | -1 | BF058465 | 218314_s_at | AA024582 | 1 | FLJ10726 |
| 213561_at | BF062335 | -1 | BF062335 | 217884_at | NM_02466 2 | 1 | FLJ10774 |
| 215775_at | BF084105 | -1 | BF084105 | 218125_s_at | NM_01824 6 | 1 | FLJ10853 |
| 212002_at | BG171020 | -1 | BG171020 | 203941_at | NM_01825 0 | 1 | FLJ10871 |
| 220580_at | NM_02504 4 | -1 | BICC1 | 53720_at | AI862559 | 1 | FLJ11286 |
| 218955_at | NM_01831 0 | -1 | BRF2 | 213064_at | N64802 | 1 | FLJ11806 |
| 201235_s_at | BG339064 | -1 | BTG2 | 218341_at | NM_02466 4 | 1 | FLJ11838 |
| 215072_x_a | AK025270 | -1 | C10orf137 | 219022_at | NM_02289 5 | 1 | FLJ12448 |
| 43934_at | AA479495 | -1 | C11ORF4 | 217866_at | NM_02481 1 | 1 | FLJ12529 |
| 219720_s_at | NM_01797 2 | -1 | C14orf118 | 46142_at | AI003763 | 1 | FLJ12681 |
| 215219_at | AK025095 | -1 | C21orf5 | 218179_s_at | NM_02194 2 | 1 | FLJ12716 |
| 48030_i_at | H93077 | -1 | C5orf4 | 220199_s_at | NM_02283 1 | 1 | FLJ12806 |
| 220231_at | NM_00665 8 | -1 | C7orfl6 | 44065_at | AI937468 | 1 | FLJ14827 |
| 61874_at | AL042496 | -1 | C9orf7 | 219798_s_at | NM_01960 6 | 1 | FLJ20257 |
| 205949_at | M33987 | -1 | CA1 | 209672_s_at | AL136892 | 1 | FLJ20323 |
| 205199_at | NM_00121 6 | -1 | CA9 | 206583_at | NM_01777 6 | 1 | FLJ20344 |
| 214845_s_at | AF257659 | -1 | CALU | 221222_s_at | NM_01786 0 | 1 | FLJ20519 |
| 34846_at | AF112472 | -1 | CAMK2B | 219751_at | NM_02486 0 | 1 | FLJ11H48 |
| 210787_s_at | AF140507 | -1 | CAMKK2 | 218531_at | NM_02512 4 | 1 | FLJ21749 |
| 205114_s_at | NM_00298 3 | -1 | CCL3, CCL3L1, MGC12815 | 218262_at | NM_02276 2 | 1 | FLJ22318 |
| 202705_at | NM_00470 1 | -1 | CCNB2 | 219176_at | NM_02452 0 | 1 | FLJ22555 |
| 205098_at | AI421071 | -1 | CCR1 | 219880_at | NM_02290 7 | 1 | FLJ23053 |
| 208304_at | NM_00183 7 | -1 | CCR3 | 220169_at | NM_02494 3 | 1 | FLJ23235 |
| 207445_s_at | AF145439 | -1 | CCR9 | 64432_at | W05463 | 1 | FLJ39616 |
| 210325_at | M28825 | -1 | CD1A | 218053_at | NM_01789 2 | 1 | FNBP3 |
| 207176_s_at | NM_00519 1 | -1 | CD80 | 202304_at | NM_01492 3 | 1 | FNDC3 |
| 211190_x_a t | AF054817 | -1 | CD84 | 203064_s_at | NM_00451 4 | 1 | FOXK2 |
| 209287_s_at | AI754416 | -1 | CDC42EP3 | 209702_at | U79260 | 1 | FTO |
| 209057_x_a | AB007892 | -1 | CDC5L | 205324_s_at | NM_01228 0 | 1 | FTSJ1 |
| 220115_s_at | NM_00672 7 | -1 | CDH10 | 218356_at | NM_01339 3 | 1 | FTSJ2 |
| 211804_s_at | AB012305 | -1 | CDK2 | 212847_at | AL036840 | 1 | FUBP1 |
| 211883_x_a | M76742 | -1 | CEACAM1 | 214093_s_at | AA156865 | 1 | FUBP1 |
| 40020_at | AB011536 | -1 | CELSR3 | 202231_at | NM_00636 0 | 1 | GA17 |
| 220885_s_at | NM_01845 1 | -1 | CENPJ | 204618_s_at | NM_00525 4 | 1 | GABPB2 |
| 202938_x_a t | NM 01570 3 | -1 | CGI-96, dJ222E13.2 | 219815_at | NM_02463 7 | 1 | GAL3ST4 |
| 207486_x_a t | NM_00406 7 | -1 | CHN2 | 213049_at | BG436400 | 1 | GARNL1 |
| 206635_at | NM_00074 8 | -1 | CHRNB2 | 214855_s_at | AL050050 | 1 | GARNL1 |
| 215916_at | AL157418 | -1 | CHRNE | 209729_at | BC001782 | 1 | GAS2L1 |
| 207075_at | NM_00489 5 | -1 | CIAS1 | 201738_at | NM_00587 5 | 1 | GC20 |
| 216016_at | AK027194 | -1 | CIAS1 | 212139_at | D86973 | 1 | GCN1L1 |
| 206818_s_at | NM_01764 9 | -1 | CNNM2 | 208913_at | AA868560 | 1 | GGA2 |
| 209874_x_a t | AK023066 | -1 | CNNM2 | 200681_at | NM_00670 8 | 1 | GLO1 |
| 206586_at | NM_00184 1 | -1 | CNR2 | 209883_at | AF288389 | 1 | GLT25D2 |
| 211980_at | AI922605 | -1 | COL4A1 | 218913_s_at | NM_01657 3 | 1 | GMIP |
| 216898_s_at | U02520 | -1 | COL4A3 | 214157_at | AA401492 | 1 | GNAS |
| 52651_at | AI806793 | -1 | COL8A2 | 202382_s_at | NM_00547 1 | 1 | GNPDA1 |
| 205624_at | NM_00187 0 | -1 | CPA3 | 202106_at | 5 | 1 | GOLGA3 |
| 222301_at | BF530257 | -1 | CROC4 | 201056_at | N53479 | 1 | GOLGB1 |
| 202468_s_at | NM_00379 8 | -1 | CTNNAL1 | 208843_s_at | BC001408 | 1 | GORASP2 |
| 219080_s_at | NM_01985 7 | -1 | CTPS2 | 209470_s_at | D49958 | 1 | GPM6A |
| 206297_at | NM_00727 2 | -1 | CTRC | 204793_at | NM_0147 0 1 | | GPRASP1 |
| 205927_s_at | NM_000191 0 | -1 | CTSE | 201106_at | NM_00208 5 | 1 | GPX4 |
| 206775_at | NM_00108 1 | -1 | CUBN | 204396_s_at | NM_00530 8 | 1 | GRK5 |
| 204470_at | NM_00151 1 | -1 | CXCL1 | 201520_s_at | BF034561 | 1 | GRSF1 |
| 209774_x_a t | M57731 | -1 | CXCL2 | 215438_x_a t | BE906054 | 1 | GSPT1 |
| 207850_at | NM_00209 0 | -1 | CXCL3 | 201912_s_at | NM_00209 4 | 1 | GSPT1 |
| 206336_at | NM_00299 3 | -1 | CXCL6 | 202680_at | NM_00209 5 | 1 | GTF2E2 |
| 205088_at | NM_00549 1 | -1 | CXorf6 | 213357_at | AV701318 | 1 | GTF2H5 |
| 214610_at | AV702430 | -1 | CYP11B1 | 201338_x_a t 7 | NM_00209 | 1 | GTF3A |
| 217558_at | BE971373 | -1 | CYP2C9 | 215091_s_at | BE542815 | 1 | GTF3A |
| 213873_at | D29810 | -1 | D29810 | 218343_s_at | NM_01208 6 | 1 | GTF3C3 |
| 217025_s_at | AL110225 | -1 | DBN1 | 200075_s_at | BC006249 | 1 | GUK1 |
| 19490_s_at | NM_02283 6 | -1 | DCLRE1B | 220936_s_at | NM_01826 7 | 1 | H2AFJ |
| 205338_s_at | NM_00192 2 | -1 | DCT | 200853_at | NM_00210 6 | 1 | H2AFZ |
| 213632_at | M94065 | -1 | DHODH | 200080_s_at | AI955655 | 1 | H3F3A |
| 219799_s_at | NM_00577 1 | -1 | DHRS9 | 208630_at | AI972144 | 1 | HADHA |
| 204494_s_at | AW516789 | -1 | DKFZP434H132 | 201036_s_at | NM_00532 7 | 1 | HADHSC |
| 214699_x_a | AK024279 | -1 | DKFZP434J154 | 211569_s_at | AF001903 | 1 | HADHSC |
| 219872_at | NM_01661 3 1 | -1 | DKFZp434L142 | 202042_at | NM_00210 | 1 | HARS |
| 78495_at | R61320 | -1 | DKFZp762P211 1 | 203138_at 2 | NM_00364 | 1 | HAT1 |
| 208216_at | NM_00193 4 | -1 | DLX4 | 204018_x_a t 8 | NM_00055 | 1 | HBA1 HBA2 |
| 33768_at | L19267 | -1 | DMWD | 214414_x_a t | T50399 | 1 | HBA2 |
| 202866_at | BG283782 | -1 | DNAJB12 | 209116_x_a t | M25079 | 1 | HBB |
| 219746_at | NM_01207 4 | -1 | DPF3 | 211696_x_a t | AF349114 | 1 | HBB |
| 206590_x_a | NM_01657 4 | -1 | DRD2 | 209274_s_at | BC002675 | 1 | HBLD2 |
| 216938_x_a t | S69899 | -1 | DRD2 | 202299_s_at | NN_00640 2 | 1 | HBXIP |
| 211541_s_at | U52373 | -1 | DYRK1A | 202300_at | NM_00640 2 | 1 | HBXIP |
| 218660_at | NM_00349 4 | -1 | DYSF | 215985_at | X92110 | 1 | HCG8 |
| 203692_s_at | AI640363 | -1 | E2F3 | 202957_at | NM_00533 5 | 1 | HCLS1 |
| 208112_x_a | NM_00679 | -1 | EHD1 | 218620_s_at | NM_01617 3 | 1 | HEMK1 |
| 209038_s_at | AL579035 | -1 | EHD1 | 200093_s_at | N32864 | 1 | HINT1 |
| 209039_x_a | AF001434 | -1 | EHD1 | 218946_at | NM_01570 0 | 1 | HIRIP5 |
| 210376_x_a | M25269 | -1 | ELK1 | 205671_s_at | NM_00212 0 | 1 | HLA-DOB |
| 205994_at | NM_00197 3 | -1 | ELK4 | 203290_at | NM_00212 2 | 1 | HLA-DQA1 |
| 396_f_at | X97671 | -1 | EPOR | 200679_x_a t | NM_00212 8 | 1 | HMGB1 |
| 206794_at | NM_00523 5 | -1 | ERBB4 | 208808_s_at | BC000903 | 1 | HMGB2 |
| 205225_at | NM_00012 5 | -1 | ESR1 | 200943_at | NM_00496 5 | 1 | HMGN1 |
| 206501_x_a | NM_00495 6 | -1 | ETV1 | 200944_s_at | NM_00496 5 | 1 | HMGN1 |
| 215006_at | AK023816 | -1 | EZH2 | 209787_s_at | BC001282 | 1 | HMGN4 |
| 205189_s_at | NM_00013 6 | -1 | FANCC | 209068_at | D89678 | 1 | HNRPDL |
| 204819_at | NM_00446 3 | -1 | FGD1 | 208990_s_at | AI912352 | 1 | HNRPH3 |
| 219901_at | NM_01835 1 | -1 | FGD6 | 214918_at | AK024911 | 1 | HNRPM |
| 208417_at | NM_02099 6 | -1 | FGF6 | 208766_s_at | BC001449 | 1 | HNRPR |
| 219389_at | NM_01798 2 | -1 | FLJ10052 | 208713_at | BF724216 | 1 | HNRPUL1 |
| 219501_at | NM_01799 3 | -1 | FLJ10094 | 203202_at | AI950314 | 1 | HRB2 |
| 218815_s_at | NM_01802 2 | -1 | FLJ10199 | 203203_s_at | NM_00704 3 | 1 | HRB2 |
| 218814_s_at | NM_01825 2 | -1 | FLJ10874 | 202098_s_at | NM_00153 5 | 1 | HRMT1L1 |
| 219450_at | NM_01830 2 | -1 | FLJ11017 | 221564_at | AL570294 | 1 | HRMT1L1 |
| 220465_at | NM_02498 8 | -1 | FLJ12355 | 206445_s_at | NM_00153 6 | 1 | HRMTIL2 |
| 220578_at | NM_02500 8 | -1 | FLJ13544 | 209657_s_at | M65217 | 1 | HSF2 |
| 220149_at | NM_02486 1 | -1 | FLJ22671 | 220839_at | NM_01416 8 | 1 | HSPC133 |
| 221224_s_at | NM_02481 9 | -1 | FLJ22955 | 221570_s_at | AF201938 | 1 | HSPC133 |
| 215062_at | AL390143 | -1 | FMNL2 | 217774_s_at | NM_01640 4 | 1 | HSPCI52 |
| 206263_at | NM_00202 2 | -1 | FMO4 | 218728_s_at | AK024569 | 1 | HSPC163 |
| 202768_at | NM_00673 2 | -1 | FOSB | 210211_s_at | AF028832 | 1 | HSPCA |
| 219889_at | NM_00547 9 | -1 | FRAT1 | 205133_s_at | NM_00215 7 | 1 | HSPE1 |
| 211628_x_a | J04755 | -1 | FTHP1 | 202602_s_at | NM_01450 0 | 1 | HTATSF1 |
| 215744_at | AW514140 | -1 | FUS | 212493_s_at | AI761110 | 1 | HYPB |
| 209893_s_at | M58596 | -1 | FUT4 | 204744_s_at | NM_01341 7 | 1 | IARS |
| 202488_s_at | NM_00597 1 | -1 | FXYD3 | 210970_s_at | AF235049 | 1 | IBTK |
| 204452_s_at | AF072872 | -1 | FZD1 | 204868_at | NM SO154 5 | 1 | ICT1 |
| 213524_s_at | NM_01571 4 | -1 | GOS2 | 202070_s_at | NM-00553 0 | 1 | IDH3A |
| 214772_at | H08993 | -1 | G2 | 222285_at | AW134608 | 1 | IGHD |
| 208217_at | NM_00204 3 | -1 | GABRR2 | 209374_s_at | BC001872 | 1 | IGHM |
| 207574_s_at | NM_01567 5 | -1 | GADD45B | 212827_at | X17115 | 1 | IGHM |
| 209305_s_at | AF078077 | -1 | GADD45B | 209341_s_at | AU153366 | 1 | IKBKB |
| 207357_s_at | NM_01754 0 | -1 | GALNT10 | 204116_at | NM_00020 6 | 1 | IL2RG |
| 220929_at | NM_01741 7 | -1 | GALNT8 | 221548_s_at | AY024365 | 1 | ILKAP |
| 207954_at | NM_00205 0 | -1 | GATA2 | 212411_at | BE747342 | 1 | IMP4 |
| 209710_at | AL563460 | -1 | GATA2 | 205981_s_at | NM_00156 4 | 1 | INGIL |
| 210358_x_a t | BC002557 | -1 | GATA2 | 48825_at | AA887083 | 1 | ING4 |
| 221314_at | NM_00526 0 | -1 | GDF9 | 204552_at | AA355179 | 1 | INPP4A |
| 205100_at | NM_0511 0 | -1 | GFPT2 | 204202_at | NM_01760 4 | 1 | IQCE |
| 211815_s_at | AF219138 | -1 | GGA3 | 217908_s_at | NM01844 2 | 1 | IQWD1 |
| 207131_x_a | NM_01343 0 | -1 | GGT1 | 209075_s_at | AY009128 | 1 | ISCU |
| 208284_x_a t | NM_01342 1 | -1 | GGT1 | 205055_at | NM_00220 8 | 1 | ITGAE |
| 209919_x_a t | L20490 | -1 | GGT1 | 216178_x_a t | AA215854 | 1 | ITGB1 |
| 211417_x_a t | L20493 | -1 | GGT1 | 202660_at | AA834576 | 1 | ITPR2 |
| 215977_x_a t | X68285 | -1 | GK | 215791_at | AF003738 | 1 | ITSN1 |
| 27034_s_at | NM-03037 9 | -1 | GL12 | 212287_at | BF382924 | 1 | JJAZI |
| 204762_s_at | NM_02098 8 | -1 | GNAO1 | 210878_s_at | BC001202 | 1 | JMJD1B |
| 209220_at | L47125 | -1 | GPC3 | 214861_at | AI341811 | 1 | JMJD2C |
| 206264_at | L11702 | -1 | GPLD1 | 200048_s_at | NM_00669 4 | 1 | JTB |
| 209168_at | AW148844 | -1 | GPM6B | 218570_at | NM-01809 5 | 1 | KBTBD4 |
| 211977_at | AK024651 | -1 | GPR107 | 45653_at | AW026481 | 1 | KCTD13 |
| 205220_at | NM_00601 8 | -1 | GPR109B | 218823_s_at | NM_01763 4 | 1 | KCTD9 |
| 221394_at | NM_01462 6 | -1 | GPR58 | 212733_at | AI798908 | 1 | KIAA0226 |
| 214655_at | U18549 | -1 | GPR6 | 212735_at | AI798908 | 1 | KIAA0226 |
| 220481_at | NM_00679 4 | -1 | GPR75 | 212474_at | D87682 | 1 | KIAA024 1 |
| 205276_s_at | U87964 | -1 | GTPBP1 | 212450_at | D87445 | 1 | KIAA0256 |
| 44783_s_at | R61374 | -1 | HEY1 | 209256_s_at | AF277177 | 1 | KIAA0265 |
| 210864_x_a t | AF144240 | -1 | HFE | 212855_at | D87466 | 1 | KIAA0276 |
| 211328_x_a t | AF144244 | -1 | HFE | 212621_at | AB006624 | 1 | KIAA0286 |
| 211329_x_a t | AF115264 | -1 | HFE | 214356_s_at | AI272899 | 1 | KIAA0368 |
| 211332_x_a t | AF144241 | -1 | HFE | 212480_at | AB002374 | 1 | KIAA0376 |
| 211863_x_a t | AF079408 | -1 | HFE | 202713_s_at | AA129755 | 1 | KIAA0391 |
| 205425_at | NM_00533 8 | -1 | HIP1 | 212068_s_at | AB011087 | 1 | KIAA0515 |
| 205426_s_at | NM_0533 8 | -1 | HIP1 | 212485_at | AU146596 | 1 | KIAA0553 |
| 209398_at | BC002649 | -1 | HISTIH1C | 212675_s_at | AB011154 | 1 | KIAA0582 |
| 214290_s_at | AI313324 | -1 | HIST2H2AA | 34406_at | AB011174 | 1 | KIAA0602 |
| 218280_x_a t | BC001629 | -1 | HIST2H2AA | 201965_s_at 6 | NM_015046 | 1 | KIAA0625 |
| 208812_x_a t | BC004489 | -1 | HLA-C, HLA-B | 212200_at | AW274877 | 1 | KIAA0692 |
| 211911_x_at | L07950 | -1 | HLA-C, HLA-B | 212311_at | AA522514 | 1 | KIAA0746 |
| 211654_x_a | M17565 | -1 | HLA-DQB1 | 209553_at | BC001001 | 1 | KIAA0804 |
| 211528_x_at | M90685 | -1 | HLA-G | 212546_s_at | AI126634 | 1 | KIAA0826 |
| 211529_x_at | M90684 | -1 | HLA-G | 204568_at | NM-01492 4 | 1 | KIAA0831 |
| 213844_at | NM_01910 2 | -1 | HOXA5 | 205594_at | NM_01489 7 | 1 | KIAA0924 |
| 205601_s_at | NM_00214 7 | -1 | HOXB5 | 209654_at | BC004902 | 1 | KIAA0947 |
| 216973_s_at | S49765 | -1 | HOXB7 | 203831_at | NM_01492 5 | 1 | KIAA1002 |
| 204221_x_at | U16307 | -1 | HRB2 | 200861_at | NM_01628 5 | 1 | KIAA1007 |
| 214085_x_at | AI912583 | -1 | HRB2 | 55872_at | AI493119 | 1 | KIAA1196 |
| 209192_x_at | BC000166 | -1 | HTATIP | 221736_at | BG236163 | 1 | KIAA1219 |
| 207404_s_at | NM_00086 5 | -1 | HTR1E | 219520_s_at | NM_01845 8 | 1 | KIAA1280 |
| 36564_at | W27419 | -1 | IBRDC3 | 207314_x_a t | NM_00673 7 | 1 | KIR3DL2 |
| 202637_s_at | AI608725 | -1 | ICAM1 | 217906_at | NM_01431 5 | 1 | KLHDC2 |
| 202638_s_at | NM_00020 1 | -1 | ICAM1 | 208975_s_at | BC003572 | 1 | KPNB1 |
| 215485_s_at | AA284705 | -1 | ICAM1 | 204009_s_at | NM_00498 5 | 1 | KRAS2 |
| 203328_x_a t | NM_00496 9 | -1 | IDE | 200914_x_a t | NM_00498 6 | 1 | K1N1 |
| 202081_at | NM 00490 7 | -1 | IER2 | 200915_x_at | NM 00498 t 6 | 1 | KTN1, TXNDC7 |
| 201631_s_at | NM_00389 7 | -1 | IER3 | 214709_s_at | Z22551 | 1 | KTN1, TXNDC7 |
| 208261_x_at | NM_00217 1 | -1 | IFNA10 | 200771_at | NM_00229 3 | 1 | LAMC1 |
| 214569_at | NM_00216 9 | -1 | IFNA5 | 212137_at | AV746402 | 1 | LARP |
| 208548_at | BM_02100 2 | -1 | IFNA6 | 34764_at | D21851 | 1 | LARS2 |
| 208441_at | NM_015883 | -1 | IGF1R | 212446_s_at | AI658534 | 1 | LASS6 |
| 202421_at | AB007935 | -1 | IGSF3 | 221515_s_at | BC001214 | 1 | LCMT1 |
| 209032_s_at | AF132811 | -1 | IGSF4 | 202726_at | NM_00023 4 | 1 | LIG1 |
| 207901_at | NM_00218 7 | -1 | IL12B | 204357_s_at | NM_00231 4 | 1 | LIMK1 |
| 64440_at | AI560217 | -1 | IL17RC | 202386_s_at | NM_01908 1 | 1 | LKAP |
| 206295_at | NM-20156 2 | -1 | IL18 | 213527_s_at | AC002310 | 1 | LOC146542 |
| 205067_at | NM_00057 6 | -1 | IL1B | 213703_at | W95043 | 1 | LOC150759 |
| 39402_at | M15330 | -1 | IL1B | 221973_at | AI983904 | 1 | LOCI50759 |
| 212659_s_at | AW083357 | -1 | IL1RN | 214801_at | W88821 | 1 | LOC163590 |
| 216243_s_at | BE563442 | -1 | IL1RN | 211325_x_a t | U72518 | 1 | LOC171220 |
| 220056_at | NM-02125 8 | -1 | IL22RAI | 212866_a | AI081543 | 1 | LOC203069 |
| 202859_x_at | NM_00058 4 | -1 | IL8 | 213725_x_at | AI693140 | 1 | LOC283824 |
| 211506_s_at | AF043337 | -1 | IL8 | 219043_s_at | NM_02406 5 | 1 | LOC285359, PDCL3 |
| 202531_at | NM_00219 8 | -1 | IRF1 | 221797_at | AY007126 | 1 | LOC339229 |
| 206766_at | AF112345 | -1 | ITGA10 | 50374_at | AA150503 | 1 | LOC339229 |
| 37201_at | D38535 | -1 | 1TIH4 | 222000_at | AI915947 | 1 | LOC339448 |
| 203682_s_a at | M_00222 5 | -1 | IVD | 218303_x_at | NM_01661 8 | 1 | LOC51315 |
| 209098_s_at | U73936 | -1 | JAG1 | 218616_at | NM_02039 5 | 1 | LOC57117 |
| 201465_s_at | BC002646 | -1 | JUN | 203897_at | BE963444 | 1 | LOC57149 |
| 211806_s_at | D87291 | -1 | KCNJ15 | 213346_at | BE748563 | 1 | LOC93081 |
| 58916_at | AI672101 | -1 | KCTD14 | 218096_at | NM_01836 1 | 1 | LPAAT-e |
| 211028_s_at | BC006233 | -1 | KHK | 216250_s_at | X77598 | 1 | LPXN |
| 216251_s_at | BF965437 | -1 | KIAAQ153 | 221640_s_at | AF274972 | 1 | LRDD |
| 210954_s_at | AF201292 | -1 | KIAA0669 | 211615_s_at | M92439 | 1 | LRPPRC |
| 31826_at | AB014574 | -1 | KIAA0674 | 209449_at | AF196468 | 1 | LSM2 |
| 212054_x_at | AK026096 | -1 | KIAA0676 | 202737_s_at | AA112507 | 1 | LSM4 |
| 213358_at | AB018345 | -1 | KIAA0802 | 211747_s_at | BC005938 | 1 | LSM5 |
| 207705_s_at | NM_02517 6 | -1 | KIAA0980 | 204559_s_at | NM_01619 9 | 1 | LSM7 |
| 213478_at | AB028949 | -1 | KIAA1026 | 212248_at | AI886796 | 1 | LYRIC |
| 216294_s_at | AL137254 | -1 | KIAA1109 | 217536_x_at | M78162 | 1 | M78162 |
| 221078_s_at | NM_01808 4 | -1 | KIAA1212 | 209014_at | AF217963 | 1 | MAGED1 |
| 222367_at | AI921841 | -1 | KIAA1971, LOC339005 | 220925_at | NM_02192 9 | 1 | MAK10 |
| 205318_at | NM_4_0498 4 | -1 | KIF5A | 214703_s_at | AW954107 | 1 | MAN2B2 |
| 207908_at | NM_00042 3 | -1 | KRT2A | 202670_at | AI571419 | 1 | MAP2K1 |
| 215189_at | X99142 | -1 | KRTHB6 | 206854_s_at | NM_00318 8 | 1 | MAP3K7 |
| 221717_at | L25664 | -1 | L25664 | 202569_s_at | NM_00237 6 | 1 | MARK3 |
| 219813_at | NM_00469 0 | -1 | LATS1 | 213671_s_at | AA621558 | 1 | MARS |
| 204012_s_at | AL529189 | -1 | LCMT2 | 212064_x_at | AI471665 | 1 | MAZ |
| 215462_at | AI978990 | -1 | LOC149478 | 209580_s_at | AL556619 | 1 | MBD4 |
| 51774_s_at | AW014299 | -1 | LOC222070 | 201620_at | NM_00379 1 | 1 | MBTPS1 |
| 58900_at | AW025284 | -1 | LOC222070 | 218440_at | NM_62016 6 | 1 | MCCC1 |
| 217520_x_at | BG396614 -1 | | L0C283683 | 200978_at | NM_00591 7 | 1 | MDH1 |
| 213751_at | AW873594 | -1 | LOC284352 | 209036_s_at | BC001917 | 1 | MDH2 |
| 214838_at | AL035297 | -1 | LOC375035 | 212693_at | BE670928 | 1 | MDN1 |
| 219071_x_at | NM_01645 | -1 | LOC51236 | 218288_s_at | NM_021825 | 1 | MDS025 |
| 221629_x_at | AF151022 | -1 | LOC51236 | 22t706_s_at | BC006005 | 1 | MDS032 |
| 220244_at | NM_013343 | -1 | LOH3CR2A | 218061_at | NM_014623 | 1 | MEA |
| 34697_at | AF074264 | -1 | LRP6 | 202645_s_at | NM_000244 | 1 | MEN1 |
| 219886_at | NM_024548 | -1 | LRRIQ2 | 207098_s_at | NM_01792 | 1 | MFN1 |
| 204682_at | NM_000428 | -1 | LTBP2 | 204153_s_at | AI738965 | 1 | MFNG |
| 202018_s_at | NM_002343 | -1 | LTF | 220189_s_at | NM_014275 | 1 | MGAT4B |
| 209480_at | M16276 | -1 | M16276 | 219001_s_at | NM-024345 | 1 | MGC10765 |
| 209738_x_at | M31125 | -1 | M31125 | 213104_at | AI799802 | 1 | MGC24381 |
| 211241_at | M62895 | -1 | M62895 | 218903_s_at | NM_024068 | 1 | MGC2731 |
| 217920_at | BE543064 | -1 | MAN1A2 | 200076_s_at | BC006479 | 1 | MGC2749 |
| 209951_s_at | AF006689 | -1 | MAP2K7 | 218714_at | NM_024031 | 1 | MGC3121 |
| 214969_at | AF251442 | -1 | MAP3K9 | 209191_at | BC002654 | 1 | MGC4083 |
| 204813_at | U34819 | -1 | MAPK10 | 202365_at | BC004815 | 1 | MGC5139 |
| 201668_x_at | AW163148 | -1 | MARCKS | 221580_s_at | BC001972 | 1 | MGC5306 |
| 202485_s_at | NM_003927 | -1 | MBD2 | 200847_s_at | NM_016127 | 1 | MGC8721 |
| 200797_s_at | AI275690 | -1 | MCL1 | 200899_s_at | AK002091 | 1 | MGEA5 |
| 202618_s_at | L37298 | -1 | MECP2 | 217871_s_at | BC000447 | 1 | MIF |
| 206028_s_at | NM_00634 | -1 | MERTK | 214246_x_at | AI859060 | 1 | MINK |
| 211913_s_at | L08961 | -1 | MERTK | 221824_s_at | AA770170 | 1 | MIR |
| 211599_x_at | U19348 | -1 | MET | 209845_at | AF117233 | 1 | MKRN1 |
| 209758_s_at | U37283 | -1 | MFAP5 | 204173_at | NM_002475 | 1 | MLC1SA |
| 211717_at | BC005853 | -1 | MGC15396 | 202520_s_at | NM_00249 | 1 | MLH1 |
| 212340_at | BE673723 | -1 | MGC21416 | 204206_at | NM_020310 | 1 | MNT |
| 217548_at | AA491625 | -1 | MGC61550 | 212508_at | AK024029 | 1 | MOAP1 |
| 205905_s_at | NM_000247 | -1 | MICA, MICB | 201994_at | NM_012286 | 1 | MORF4L2 |
| 208384_s_at | NM_012216 | -1 | MID2 | 215731_s_at | X98258 | 1 | MPHOSPH9 |
| 212021_s_at | AU132185 | -1 | MKI67 | 219162_s_at | NM_016050 | 1 | MRPL11 |
| 218211_s_at | NM_024101 | -1 | MLPH | 220526_s_at | NM_017971 | 1 | MRPL20 |
| 160020_at | Z48481 | -1 | MMP14 | 218339_at | NM_014180 | 1 | MRPL22 |
| 203936_s_at | NM_004994 | -1 | MMP9 | 213897_s_at | AI832239 | 1 | MRPL23 |
| 203949_at | NM_000250 | -1 | MPO | 209609_s_at | BC004517 | 1 | MRPL9 |
| 206186_at | NM_001932 | -1 | MPP3 | 203800_s_at | BG254653 | 1 | MRPS14 |
| 210594_x_at | AF239756 | -1 | MPZL1 | 221437_s_at | NM_031280 | 1 | MRPS15 |
| 210223_s_at | AF031469 | -1 | MR1 | 212604_at | AI937794 | 1 | MRPS31 |
| 210224_at | AF031469 | -1 | MR1 | 218654_s_at | NM_016071 | 1 | MRPS33 |
| 210242_x_at | AF249277 | -1 | MTHFS | 217942_at | NM_021821 | 1 | MRPS35 |
| 212093_s_at | AI695017 | -1 | MTUS1 | 211783_s_at | BC006177 | 1 | MTA1 |
| 207847_s_at | NM_002456 | -1 | MUC1 | 210212_x_at | BC002600 | 1 | MTCP1 |
| 212365_at | BF215996 | -1 | MYO1B | 216862_s_at | Z24459 | 1 | MTCP1 |
| 33197_at | U39226 | -1 | MYO7A | 202309_at | NM_005956 | 1 | MTHFD1 |
| 210048_at | BC001889 | -1 | NAPG | 216095_x_at | AF057354 | 1 | MTMR1 |
| 218742_at | NM_022493 | -1 | NARFL | 219822_at | NM_004294 | 1 | MTRF1 |
| 217045_x_at | AL136967 | -1 | NCR2 | 203199_s_at | N29717 | 1 | MTRR |
| 202150_s_at | U64317 | -1 | NEDD9 | 205145_s_at | NM_002477 | 1 | MYL5 |
| 203927_at | NM_004556 | -1 | NFKBIE | 222018_at | AI992187 | 1 | NACA |
| 207986_x_at | NM_001915 | -1 | NM_001915 | 204528_s_at | NM_00453 | 1 | NAP1L1 |
| 208039_at | NM_003048 | -1 | NM_003048 | 208753_s_at | BC002387 | 1 | NAP1L1 |
| 208180_s_at | NM_003543 | -1 | NM_003543 | 204749_at | NM_004538 | 1 | NAPIL3 |
| 220814_at | NM_017964 | -1 | NM_017964 | 218713_at | NM_024611 | 1 | NARG2 |
| 221105_at | NM_018395 | -1 | NM_018395 | 201517_at | BC001255 | 1 | NCBP2 |
| 220872_at | NM_018547 | -1 | NM_018547 | 218697_at | NM_016453 | 1 | NCKIPSD |
| 208540_x_at | NM_021039 | -1 | NM_021039 | 200610_s_at | NM_005381 | 1 | NCL |
| 219680_at | NM_024618 | -1 | NOD9 | 217286_s_at | BC001805 | 1 | NDRG3 |
| 39548_at | U77970 | -1 | NPAS2 | 202298_at | NM_004541 | 1 | NDUFA1 |
| 210730_s_at | U36269 | -1 | NPY2R | 209224_s_at | BC003674 | 1 | NDUFA2 |
| 202340_x_at | NM_002135 | -1 | NR4A1 | 217773_s_at | NM_002489 | 1 | NDUFA4 |
| 204105_s_at | NM_005010 | -1 | NRCAM | 202785_at | NM_00500 | 1 | NDUFA7 |
| 216959_x_at | U55258 | -1 | NRCAM | 208969_at | AF050641 | 1 | NDUFA9 |
| 210022_at | BC004952 | -1 | NSPC1 | 202077_at | NM_00500 | 1 | NDUFAB1 |
| 217377_x_at | AF041811 | -1 | NTRK3 | 218320_s_at | NM_019056 | 1 | NDUFB11 |
| 206553_at | NM_002535 | -1 | OAS2 | 202839_s_at | NM_004146 | 1 | NDUFB7 |
| 205660_at | NM_003733 | -1 | OASL | 203478_at | NM_002494 | 1 | NDUFC1 |
| 214485_at | NM_024410 | -1 | ODF1 | 218101_s_at | NM_004549 | 1 | NDUFC2 |
| 219105_x_at | NM_014321 | -1 | ORC6L | 201740_at | NM_004551 | 1 | NDUFS3 |
| 205729_at | NM_00399 | -1 | OSMR | 203189_s_at | NM_002496 | 1 | NDUFS8 |
| 205815_at | NM_002580 | -1 | PAP | 203190_at | NM_002496 | 1 | NDUFS8 |
| 213332_at | AL031290 | -1 | PAPPA2 | 213331_s_at | AV700007 | 1 | NEK1 |
| 204752_x_at | NM_005484 | -1 | PARP2 | 203413_at | NM_006159 | 1 | NELL2 |
| 215773_x_at | AJ236912 | -1 | PARP2 | 218129_s_at | BC005316 | 1 | NFYB |
| 215418_at | AK022316 | -1 | PARVA | 202475_at | NM_006326 | 1 | NIFIE14 |
| 121_at | X69699 | -1 | PAX8 | 212483_at | AB019494 | 1 | NIPBL |
| 205656_at | NM_014459 | -1 | PCDH17 | 203830_at | NM_022344 | 1 | NJMU-R1 |
| 211066_x_at | BC006439 | -1 | PDGHGA1-12 PDGHGB1-7 | 202294_at | NM_005862 | 1 | NM_005862 |
| 206388_at | U36798 | -1 | PDE3A | 217946_s_at | NM_016402 | 1 | NM_016402 |
| 216061_x_at | AK022920 | -1 | PDGFB | 219817_at | NM_016534 | 1 | NM_016534 |
| 220236_at | NM_017990 | -1 | PDPR | 218506_x_at | NM_018459 | 1 | NM_018459 |
| 220944 at | NM_020393 | -1 | PGLYRP4 | 218517_at | NM_024900 | 1 | NM_024900 |
| 215622_x_at | AL137671 | -1 | PHF7 | 212739_s_at | AL523860 | 1 | NME4 |
| 209346_s_at | AL561930 | -1 | PI4KII | 200875_s_at | NM_006392 | 1 | NOL5A |
| 215832_x_at | AV722190 | -1 | PICALM | 202882_x_at | NM_01616 | 1 | NOL7 |
| 210256_s_at | U78576 | -1 | PIP5K1A | 204791_at | NM_003297 | 1 | NR2C1 |
| 204267_x_at | NM_004203 | -1 | PKMYT1 | 204651_at | AW003022 | 1 | NRF1 |
| 202924_s_at | AF006005 | -1 | PLAGL2 | 213061_s_at | AA643304 | 1 | NTAN1 |
| 202925_s_at | NM_00265 | -1 | PLAGL2 | 213062_at | AA643304 | 1 | NTAN1 |
| 203471_s_at | NM_002664 | -1 | PLEK | 212605_s_at | AK025759 | 1 | NUDT3 |
| 39854_r_at | AF055000 | -1 | PNPLA2 | 212182_at | AB007956 | 1 | NUDT4 |
| 206654_s_at | NM_006467 | -1 | POLR3G | 202900_s_at | NM_002532 | 1 | NUP88 |
| 210809_s_at | D13665 | -1 | POSTN | 213018_at | AI337901 | 1 | ODAG |
| 207725_at | NM_004575 | -1 | POU4F2 | 203569_s_at | NM_003611 | 1 | OFD1 |
| 216330_s_at | L14482 | -1 | POU6F1 | 219073_s_at | NM_017784 | 1 | OSBPL10 |
| 212226_s_at | AA628586 | -1 | PPAP2B | 208717_at | 8C001669 | 1 | OXA1L |
| 210235_s_at | U22815 | -1 | PPFIA1 | 206637_at | NM_014879 | 1 | P2RY14 |
| 201489_at | BC005020 | -1 | PPIF | 218131_s_at | AK024670 | 1 | p66alpha |
| 201490_s_at | NM_005729 | -1 | PPIF | 201545_s_at | NM_004643 | 1 | PABPN1 |
| 202014_at | NM_014330 | -1 | PPP1R15A | 200816_s_at | NM_000430 | 1 | PAFAH1B1 |
| 37028_at | U83981 | -1 | PPP1R15A | 208051_s_at | NM_006451 | 1 | PAIPI |
| 221088_s_at | NM_017650 | -1 | PPP1R9A | 209064_x_at | BF248165 | 1 | PAIP1 |
| 204506_at | AL544951 | -1 | PPP3R1 | 210466_s_at | BC002488 | 1 | PAI-RBP1 |
| 210499_s_at | AB041834 | -1 | PQBP1 | 202759_s_at | BE879367 | 1 | PALM2-AKAP2 |
| 209766_at | AF118073 | -1 | PRDX3 | 204715_at | NM_015368 | 1 | PANX1 |
| 219732_at | NM_017753 | -1 | PRG-3 | 212718_at | BF797555 | 1 | PAPOLA |
| 217269_s_at | AP001672 | -1 | PRSS7 | 200006_at | NM_007262 | 1 | PARK7 |
| 210195_s_at | M34715 | -1 | PSG1 | 203905_at | NM_002582 | 1 | PARN |
| 204897_at | AA897516 | -1 | PTGER4 | 219033_at | NM_02461 | 1 | PARP8 |
| 204748_at | NM_000963 | -1 | PTGS2 | 213534_s_at | D50925 | 1 | PASK |
| 202895_s_at | D86043 | -1 | PTPNS1 | 204004_at | AI336206 | 1 | PAWR |
| 206157_at | NM_00285 | -1 | PTX3 | 205353_s_at | NM_00256 | 1 | PBP |
| 202148_s_at | NM_006907 | -1 | PYCR1 | 210825_s_at | AF130103 | 1 | PBP |
| 211471_s_at | AF133588 | -1 | RAB36 | 211941_s_at | 8E96967 1 | 1 | PBP |
| 37793_r_at | AF034956 | -1 | RAD51L3 | 214177_s_at | AI935162 | 1 | PBXIP1 |
| 204916_at | NM_005855 | -1 | RAMP1 | 212694_s_at | NM_000532 | 1 | PCCB |
| 217020_at | X04014 | -1 | RARB | 219737_s_at | NM_020403 | 1 | PCDH9 |
| 215688_at | AL359931 | -1 | RASGRF1 | 203378_at | AB020631 | 1 | PCF11 |
| 205115_s_at | NM_016196 | -1 | RBM19 | 218260_at | NM_024050 | 1 | PCIA1 |
| 215761_at | AK000156 | -1 | RC3 | 202174_s_at | NM_006197 | 1 | PCM1 |
| 216153_x_at | AK022897 | -1 | RECK | 218014_at | NM_024844 | 1 | PCNT1 |
| 205879_x_at | BC004257 | -1 | RET | 205559_s_at | NM_00620 | 1 | PC8K5 |
| 207936_x_at | NM_006604 | -1 | RFPL3 | 212422_at | AL547263 | 1 | PDCD11 |
| 209637_s_at | AF030111 | -1 | RGS12 | 204025_s_at | NM_002598 | 1 | PDCD2 |
| 209545_s_at | AF027706 | -1 | RIPK2 | 213581_at | BF446180 | 1 | PDCD2 |
| 221287_at | NM_021133 | -1 | RNASEL | 203415_at | NM_013232 | 1 | PDCD6 |
| 208270_s_at | NM_020216 | -1 | RNPEP | 204491_at | R40917 | 1 | PDE4D |
| 218441_s_at | NM_015540 | -1 | RPAP1 | 214129_at | AI821791 | 1 | PDE4DIP |
| 213223_at | AK025866 | -1 | RPL28 | 219575_s_at | NM_022341 | 1 | PDF, COG8 |
| 211180_x_at | D89788 | -1 | RUNX1 | 221811_at | BF033007 | 1 | PERLD1 |
| 205485_at | NM_000540 | -1 | RYR1 | 219180_s_at | AI817074 | 1 | PEX26 |
| 217033_x_at | 576475 | -1 | S76475 | 205361_s_at | A1718295 | 1 | PFDN4 |
| 210593_at | M55580 | -1 | SAT | 210908_s_at | AB055804 | 1 | PFDN5 |
| 217331_at | U63542 | -1 | SCC-112 | 204604_at | NM_012395 | 1 | PFTK1 |
| 59705_at | AA911739 | -1 | SCLY | 222125_s_at | BC000580 | 1 | PH-4 |
| 207413_s_at | NM_000335 | -1 | SCN5A | 212542_s_at | BF224151 | 1 | PHIP |
| 203788_s_at | AI962897 | -1 | SEMA3C | 202738_s_at | BG149218 | 1 | PHKB |
| 35666_at | U38276 | -1 | SEMA3F | 202739_s_at | NM_00029 3 | 1 | PHKB |
| 218122_s_at | NM_02162 7 | -1 | SENP2 | 203335_at | NM_00621 4 | 1 | PHYH |
| 209722_s_at | BC002538 | -1 | SERPINB9 | 209625_at | BC004100 | 1 | PIGH |
| 214197_s_at | AI762193 | -1 | SETDB1 | 209998_at | BC001030 | 1 | PIGO |
| 33323_r_at | X57348 | -1 | SFN | 202927_at | NM_00622 1 | 1 | PIN1 |
| 204051_s_at | NM_00301 4 | -1 | SFRP4 | 205632_s_at | NM_00355 8 | 1 | PIP5K1B |
| 201742_x_at | M69040 | -1 | SFRS1 | 213111_at | AB023198 | 1 | PIP5K3 |
| 37004_at | J02761 | -1 | SFTPB | 218667_at | NM_02236 8 | 1 | PJA1 |
| 201739_at | NM_00562 7 | -1 | SGK | 54051_at | H59033 | 1 | PKNOX1 |
| 211211_x_at | AF100542 | -1 | SH2D1A | 205372_at | NM_00265 5 | 1 | PLAG1 |
| 210796_x_at | D86359 | -1 | SIGLEC6 | 213309_at | AL117515 | 1 | PLCL2 |
| 207250_at | NM_00737 4 | -1 | SIX6 | 219024_at | NM_02162 2 | 1 | PLEKHA1 |
| 207095_at | NM_00045 2 | -1 | SLC10A2 | 201682_at | NM_00427 9 | 1 | PMPCB |
| 217473_x_at | AF229163 | -1 | SLC11A1 | 213677_s_at | BG434893 | 1 | PMS1 |
| 203125_x_at | AF046997 | -1 | SLC11A2 | 218961_s_at | NM_00725 4 | 1 | PNKP |
| 202236_s_at | NM_00305 1 | -1 | SLC16A1 | 205901_at | NM_00622 8 | 1 | PNOC |
| 204230_s_at | NM_02030 9 | -1 | SLCI7A7 | 209740_s_at | U03886 | 1 | PNPLA4 |
| 208389_s_at | NM_00417 1 | -1 | SLC1A2 | 201115_at | NM_00623 0 | 1 | POLD2 |
| 207408_at | NM_00480 3 | -1 | SLC22A14 | 217806_s_at | NM_01558 4 | 1 | POLDIP2 |
| 219344_at | NM_01834 4 | -1 | SLC29A3 | 203366_at | NM_00269 3 | 1 | POLG |
| 216236_s_at | AL110298 | -1 | SLC2A3 | 212955_s_at | AL037557 | 1 | POLR2I |
| 219991_at | NM_02004 1 | -1 | SLC2A9 | 218016_s_at | NM_01811 9 | 1 | POLR3E |
| 210739_x_a t | AF069510 | -1 | SLC4A4 | 203782_s_at | NM_00503 5 | 1 | POLRMT |
| 211123_at | D87920 | -1 | SLC5A5 | 213360_s_at | AA514622 | 1 | POM121, LOC340318 |
| 205920_at | NM_00304 3 | -1 | SLC6A6 | 204839_at | NM_01591 8 | 1 | POP5 |
| 216092_s_at | AL365347 | -1 | SLC7A8 | 209482_at | BC001430 | 1 | POP7 |
| 207499_x_a t | NM_01797 9 | -1 | SMAP-1 | 205267_at | NM_00623 5 | 1 | POU2AF1 |
| 214708_at | BG484314 | -1 | SNTB1 | 205661_s_at | NM_02520 7 | 1 | PP591 |
| 215366_at | AL353943 | -1 | SNX13 | 202494_at | NM_00611 2 | 1 | PPIE |
| 205482_x_at | NM_01330 6 | -1 | SNX15 | 212750_at | AB020630 | 1 | PPP1R16B |
| 206663_at | NM_00311 2 | -1 | SP4 | 41577_at | AB020630 | 1 | PPP1R16B |
| 205406_s_at | NM_01742 5 | -1 | SPA17 | 202165_at | BF966540 | 1 | PPP1R2 |
| 206239_s_at | NM_00312 2 | -1 | SPINK1 | 207830_s_at | NM_00271 3 | 1 | PPP1R8 |
| 216981_x_at | X60502 | -1 | SPN | 221772_s_at | AI459157 | 1 | PPP2R2D |
| 219677_at | NM_02510 6 | -1 | SSB1 | 201877_s_at | NM_00271 9 | 1 | PPP2R5C |
| 203019_x_at | NM_01402 1 | -1 | SSX2IP | 202432_at | NM_02113 2 | 1 | PPP3CB |
| 214441_at | NM_00581 9 | -1 | STX6 | 202741_at | AA130247 | 1 | PRKACB |
| 204287_at | NM_00471 1 | -1 | SYNGRI | 207957_s_at | NM_00273 8 | 1 | PRKCB1 |
| 203999_at | AV731490 | -1 | SYT1 | 209685_s_at | M13975 | 1 | PRKCB1 |
| 212679_at | AK026529 | -1 | TBL2 | 208694_at | U47077 | 1 | PRKDC |
| 206838_at | NM_00514 9 | -1 | TBX19 | 220553_s_at | NM_01833 3 | 1 | PRPF39 |
| 211590_x_at | U11271 | -1 | TBXA2R | 209161_at | AI184802 | 1 | PRPF4 |
| 205513_at | NM_00106 2 | -1 | TCN1 | 202127_at | AB011108 | 1 | PRPF4B |
| 221473_x_at | U49188 | -1 | TDE1 | 209440_at | BC001605 | 1 | PRPS1 |
| 215902_at | AF009267 | -1 | TEB4 | 203401_at | NM_00276 5 | 1 | PRPS2 |
| 204653_at | BF343007 | -1 | TFAP2A | 202529_at | NM_00276 6 | 1 | PRPSAP1 |
| 205016_at | NM_00323 6 | -1 | TGFA | 203089_s_at | NM_01324 7 | 1 | PRSS25 |
| 203834_s_at | NM_00646 4 | -1 | TGOLN2 | 209337_at | AF063020 | 1 | PSIP1 |
| 204064_at | NM_00513 1 | -1 | THOC1 | 202244_at | NM_00279 6 | 1 | PSMB4 |
| 203167_at | NM_00325 5 | -1 | TIMP2 | 209503_s_at | AF035309 | 1 | PSMC5 |
| 210176_at | AL050262 | -1 | TLR1 | 212296_at | NM_00580 5 | 1 | PSMDI4 |
| 206271_at | NM_00326 5 | -1 | TLR3 | 200830_at | NM_00280 8 | 1 | PSMD2 |
| 214657_s_at | AU134977 | -1 | TncRNA | 212219_at | D38521 | 1 | PSME4 |
| 207113_s_at | NM_00059 4 | -1 | TNF | 218371_s_at | AA969958 | 1 | PSPC1 |
| 206025_s_at | AW188198 | -1 | TNFAIP6 | 219293_s_at | NM_01334 1 | 1 | PTD004 |
| 209294_x_at | BC001281 | -1 | TNFRSF10B | 201433_s_at | NM_01475 4 | 1 | PTDSS1 |
| 210405_x_at | AF153687 | -1 | TNFRSF10B | 213795_s_at | AL121905 | 1 | PTPRA |
| 206729_at | NM_00124 3 | -1 | TNFRSF8 | 201166_s_at | NM_01467 6 | 1 | PUM1 |
| 211333_s_at | AF288573 | -1 | TNFSF6 | 216221_s_at | D87078 | 1 | PUM2 |
| 206990_at | NM_00328 5 | -1 | TNR | 201608_s_at | NM_00706 2 | 1 | PWP1 |
| 202807_s_at | NM_00548 8 | -1 | TOM1 | 201568_at | NM_1440 2 | 1 | QP-C |
| 204946_s_at | NM_00461 8 | -1 | TOP3A | 202754_at | NM_01536 1 | 1 | R3HDM |
| 215781_s_at | D87012 | -1 | TOP3B | 217763_s_at | AF183421 | 1 | RAB31 |
| 211943_x_a | AL565449 | -1 | TPT1 t | 217764_s_at | AF183421 | 1 | RAB31 |
| 36742_at | U34249 | -1 | TRIM15 | 202373_s_at | AF255648 | 1 | RAB3-GAP150 |
| 206911_at | NM_00508 2 | -1 | TRIM25 | 218699_at | NM_00392 9 | 1 | RAB7L1 |
| 208178_x_a t | NM_00711 8 | -1 | TRIO | 213313_at | AI922519 | 1 | RABGAP1 |
| 208349_at | NM_00733 2 | -1 | TRPA1 | 209181_s_at | U49245 | 1 | RABGGTB |
| 214205_x_a t | AK022131 | -1 | TXNL2 | 219151_s_at | NM_00708 1 | 1 | RABL2A, RABL2B |
| 210803_at | AF201385 | -1 | TXNRD2 | 207405_s_at | NM_00287 3 | 1 | RAD17 |
| 215511_at | U19345 | -1 | U19345 | 204199_at | NM_01463 6 | 1 | RALGPS1 |
| 209947_at | BC003170 | -1 | UBAP2L | 220338_at | NM_01803 7 | 1 | RALGPS2 |
| 220422_at | NM_01748 1 | -1 | UBQLN3 | 221809_at | AB040897 | 1 | RANBP10 |
| 221323_at | NM_02521 8 | -1 | ULBP1 | 211954_s_at | BC000947 | 1 | RANBP5 |
| 205536_at | NM_00337 1 | -1 | VAV2 | 202582_s_at | AF306510 | 1 | RANBP9 |
| 210513_s_at | AF091352 | -1 | VEGF | 218526_s_at | NM_01418 5 | 1 | RANGNRF |
| 209822_s_at | L22431 | -1 | VLDLR | 209444_at | BC001851 | 1 | RAP1GDS1 |
| 204787_at | NM_00726 8 | -1 | VSIG4 | 205169_at | NM_00505 7 | 1 | RBBP5 |
| 38964_r_at | U12707 | -1 | WAS | 212781_at | AK026954 | 1 | RBBP6 |
| 221927_s_at | AI923458 | -1 | WBSCR21 | 201092_at | NM-00289 3 | 1 | RBBP7 |
| 71933_at | AI218134 | -1 | WNT6 | 57540_at | AI823980 | 1 | RBKS |
| 210200_at | BC000108 | -1 | WWP2 | 204178_s_at | NM_00632 8 | 1 | RBM14 |
| 206365_at | NM_00299 5 | -1 | XCL1 | 218117_at | NM_01424 8 | 1 | RBX1 |
| 216809_at | Z22780 | -1 | Z22780 | 201486_at | NM_00290 2 | 1 | RCN2 |
| 201531_at | NM-00340 7 | -1 | ZFP36 | 203898_at | AU154853 | 1 | RCP9 |
| 212892_at | AW130128 | -1 | ZNF282 | 222203_s_at | AK023625 | 1 | RDH14 |
| 216692_at | AL137428 | -1 | ZNF337 | 221532_s_at | AF309553 | 1 | REC14 |
| 219089_s_at | NM_02432 7 | -1 | ZNF576 | 210568_s_at | BC001052 | 1 | RECQL |
| 215376_at | AU147830 | 1 | MAMMA10018 18 | 219041_s_at | NM_01437 4 | 1 | REPIN1 |
| 212829_at | BE878277 | 1 | OVARC1000964 | 202296_s_at | NM_00703 3 | 1 | RER1 |
| 213686_at | AI186145 | 1 | AI186145 | 218428_s_at | NM_01631 6 | 1 | REV1L |
| 215287_at | AA975427 | 1 | ELISC-1 | 208070_s_at | NM_00291 2 | 1 | REV3L |
| 213750_at | AA928506 | 1 | YH77E09 | 203659_s_at | NM_0579 8 | 1 | RFP2 |
| 217529 at | BE547674 | 1 | LOC402578 | 202976_s_at | NM_01489 9 | 1 | RHOBTB3 |
| 215343_at | AF070587 | 1 | 0610010D24Rik | 202130_at | NM_00383 1 | 1 | RIOK3 |
| 212114_at | BE967207 | 1 | microtubule proteins | 214663_at | AB007941 | 1 | RIPK5 |
| 212413_at | D50918 | 1 | 6-Sep | 213397_x_a t | AI761728 | 1 | RNASE4 |
| 212414_s_at | D50918 | 1 | 6-Sep | 218496_at | BG534527 | 1 | RNASEH1 |
| 213970_at | AA744682 | 1 | AA744682 | 202683_s_at | NM_00379 9 | 1 | RNMT |
| 209307_at | AB014540 | 1 | AB014540 | 200087_s_at | AK024976 | 1 | RNP24 |
| 207819_s_at | NM_00044 3 | 1 | ABCB4 | 212430_at | AL109955 | 1 | RNPC1 |
| 209620_s_at | AB005289 | 1 | ABCB7 | 218462_at | NM_02506 5 | 1 | RPF1 |
| 214274_s_at | AI860341 | 1 | ACAA1 | 222229_x_a t | AL121871 | 1 | RPL26 |
| 205355_at | NM_00160 9 | 1 | ACADSB | 218830_at | NM_01609 3 | 1 | RPL26L1 |
| 219962_at | NM_02180 4 | 1 | ACE2 | 221593_s_at | BC001663 | 1 | RPL31 |
| 201715_s_at | NM_01497 7 | 1 | ACIN1 | 213687_s_at | BE968801 | 1 | RPL35A |
| 215227_x_at | BG035989 | 1 | ACP1 | 210034_s_at | AA582460 | 1 | RPL5 |
| 202135_s_at | NM_00573 | 1 | ACTR1B | 211542_x_at | BC004334 | 1 | RPS10 |
| 205260_s_at | NM_00110 7 | 1 | ACYP1 | 200095_x_a t | AA320764 | 1 | RPS10 |
| 206833_s_at | NM_00110 | 1 | ACYP2 | 213890_x_at | AI200589 | 1 | RPS16 |
| 208268_at | NM_02177 7 | 1 | ADAM28 | 200949_x_at | NM_00102 3 | 1 | RPS20 |
| 209321_s_at | AF033861 | 1 | ADCY3 | 200091_s_at | AA888388 | 1 | RPS25 |
| 208030_s_at | NM_00111 9 | 1 | ADD1 | 200741_s_at | NM_00103 0 | 1 | RPS27 |
| 201773_at | NM_01533 9 | 1 | ADNP | 200017_at | NM_00295 4 | 1 | RPS27A |
| 201281_at | NM_00700 2 | 1 | ADRM1 | 218007_s_at | BC003667 | 1 | RPS27L |
| 202144_s_at | NM_00002 6 | 1 | ADSL | 208904_s_at | BF431363 | 1 | RPS28 |
| 217729_s_at | NM_00113 0 | 1 | AES | 204635_at | NM_00475 5 | 1 | RPS6KA5 |
| 216609_at | AF065241 | 1 | AF065241 | 205540_s_at | NM_01665 6 | 1 | RRAGB |
| 211071_s_at | BC006471 | 1 | AF1Q | 218088_s_at | AK023373 | 1 | RRAGC |
| 210111_s_at | AF277175 | 1 | AF277175 | 208456_s_at | NM_01225 0 | 1 | RRAS2 |
| 204333_s_at | NM_00002 7 | 1 | AGA | 203704_s_at | AW118862 | 1 | RREB1 |
| 203566_s_at | NM_00064 5 | 1 | AGL | 201975_at | NM_00295 6 | 1 | RSN |
| 200850_s_at | NM_00662 1 | 1 | AHCYL1 | 212319_at | AB007857 | 1 | RUTBC1 |
| 202820 at | NM_00162 1 | 1 | AHR | 201459_at | NM_00666 6 | 1 | RUVBL2 |
| 212980_at | AL050376 | 1 | AHSA2 | 219598_s_at | NM_01610 4 | 1 | RWDD1 |
| 204057_at | AI073984 | 1 | AI073984 | 205087_at | NM_01548 5 | 1 | RWDD3 |
| 212543_at | U83115 | 1 | AIM1 | 212438_at | BG252325 | 1 | RY1 |
| 214102_at | AK023737 | 1 | AK023737 | 213262_at | AI932370 | 1 | SACS |
| 201675_at | NM_00348 8 | 1 | AKAP1 | 203280_at | NM_01464 9 | 1 | SAFB2 |
| 218580_x_a t | NM_01790 0 | 1 | AKIP | 209486_at | BC004546 | 1 | SAS10 |
| 202139_at | NM_00368 9 | 1 | AKR7A2 | 218276_s_at | AI679398 | 1 | SAV1 |
| 215307_at | AL109722 | 1 | AL109722 | 213244_at | AI207792 | 1 | SCAMP4 |
| 216421_at | AL121886 | 1 | AL121886 | 205790_at | NM_00372 6 | 1 | SCAP1 |
| 216490_x_at | AL133267 | 1 | AL133267 | 212140_at | AB014548 | 1 | SCC-112 |
| 201612_at | NM_00069 6 | 1 | ALDH9A1 | 202541_at | BF589679 | 1 | SCYE1 |
| 218203_at | NM_0133 8 | 1 | ALG5 | 218607_s_at | NM_01811 5 | 1 | SDAD1 |
| 203545_at | NM_02407 9 | 1 | ALG8 | 218649_x_at | NM_00471 3 | 1 | SDCCAG1 |
| 214220_s_at | AW003635 | 1 | ALMS1 | 218427_at | NM_00664 3 | 1 | SDCCAG3 |
| 204976_s_at | AK023637 | 1 | AMMECR1 | 200945_s_at | NM_01493 3 | 1 | SEC31L1 |
| 208722_s_at | BC001081 | 1 | ANAPC5 | 219349_s_at | NM_01830 3 | 1 | SEC5L1 |
| 218769_s_at | NM_02303 9 | 1 | ANKRA2 | 201916_s_at | NM_00721 4 | 1 | SEC63 |
| 218093_s_at | NM_01766 4 | 1 | ANKRD10 | 212630_at | AF055006 | 1 | SEC6L1 |
| 212211_at | AI986295 | 1 | ANKRD17 | 218265__at | NM_02407 7 | 1 | SECISBP2 |
| 202442_at | NM_00128 4 | 1 | AP3S1 | 219351_at | NM_01456 | 1 | SEDL |
| 210278_s_at | AF155159 | 1 | AP4S1 1 | 221931_s_at | AV701173 | 1 | SEH1L |
| 213115_at | AL031177 | 1 | APG4A | 204563_at | NM-00065 5 | 1 | SELL |
| 202512_s_at | AK001899 | 1 | APG5L | 201194_at | NM_00300 9 | 1 | SEPW1 |
| 202630_at | AA046411 | 1 | APPBP2 | 209723_at | BC002538 | 1 | SERPINB9 |
| 213892_s_at | AA927724 | 1 | APRT | 205352_at | NM_00502 5 | 1 | SERPINI1 |
| 201526_at | NM_00166 2 | 1 | ARF5 | 205933_at | NM_01555 9 | 1 | SETBP1 |
| 218870_at | NM_01846 0 | 1 | ARHGAP15 | 216457_s_at | AK026080 | 1 | SF3A1 |
| 213039_at | AB011093 | 1 | ARHGEF18 | 203818_s_at | NM_00680 2 | 1 | SF3A3 |
| 218964_at | NM_00646 5 | 1 | ARID3B | 200685_at | AU146237 | 1 | SFRS11 |
| 221230_s_at | NM_01637 4 | 1 | ARlD4B | 200686_s_at | NM_00476 8 | 1 | SFRS11 |
| 201229_s_at | BC000422 | 1 | ARIH2 | 214853_s_at | AI091079 | 1 | SHC1 |
| 201230_s_at | NM_00632 1 | 1 | ARIH2 | 205063_at | NM_00361 6 | 1 | SIP1 |
| 218868_at | NM_2044 5 | 1 | ARP3BETA | 213600_at | AA425633 | 1 | SIPA1L3 |
| 215457_at | AF070647 | 1 | ARPC1A | 219185_at | NM_01224 1 | 1 | SIRT5 |
| 207988_s_at | NM_00573 1 | 1 | ARPC2 | 203489_at | NM_00642 7 | 1 | SIVA |
| 213513_x_a | BG034239 | 1 | ARPC2 | 200718_s_at | AA927664 | 1 | SKP1A |
| 215684_s_at | AL096741 | 1 | ASCC2 | 207974_s_at | NM_00693 0 | 1 | SKP1A |
| 212672_at | U82828 | 1 | ATM | 205234_at | NM_00469 0 | 1 | SLC16A4 |
| 211755_s_at | BC005960 | 1 | ATP5F1 | 203775_at | NM_01425 1 | 1 | SLC25A13 |
| 207508 at | NM_00168 9 | 1 | ATP5G3 | 218989_x_a | NM_02290 2 | 1 | SLC30A5 |
| 209492_x_at | BC003679 | 1 | ATP5I | 213082_s_at | AJ005866 | 1 | SLC35D2 |
| 202325_s_at | NM_00168 5 | 1 | ATP5J | 218928_s_at | NM_01896 4 | 1 | SLC37A1 |
| 200818_at | NM_00169 7 | 1 | ATP5O | 202111_at | NM_00304 0 | 1 | SLC4A2 |
| 214934_at | AW411030 | 1 | ATP9B | 209884_s_at | AF047033 | 1 | SLC4A7 |
| 209903_s_at | U49844 | 1 | ATR | 215043_s_at | X83301 | 1 | SMA3, SMA5 |
| 208833_s_at | AF119662 | 1 | ATXN10 | 201784_s_at | NM_01426 7 | 1 | SMAP |
| 214691_x_a t | AU121431 | 1 | AU121431 | 206544_x_at | NM_00307 0 | 1 | SMARCA2 |
| 222297_x_a t | AV738806 | 1 | AV738806 | 218452_at | NM_01414 0 | 1 | SMARCAL1 |
| 221589_s_at | AW612403 | 1 | AW612403 | 201827_at | AF113019 | 1 | SMARCD2 |
| 202387_at | NM_00432 3 | 1 | BAG1 | 218781_at | NM_02462 4 | 1 | SMC6L1 |
| 219667_s_at | NM_01793 5 | 1 | BANK1 | 205596_s_at | AY014180 | 1 | SMURF2 |
| 202326_at | NM_00670 9 | 1 | BAT8 | 202690_s_at | BC001721 | 1 | SNRPD1 |
| 211833_s_at | U19599 | 1 | BAX | 203832_at | NM_00309 | 1 | SNRPF |
| 200837_at | NM_00574 5 | 1 | BCAP31 | 212777_at | L13857 | 1 | SOS1 |
| 210347_s_at | AF080216 | 1 | BCL11A | 221239_s_at | NM_03076 4 | 1 | SPAP1 |
| 219497_s_at | NM_01801 | 1 | BCL11A | 217927_at | NM_01404 1 | 1 | SPC12 |
| 202315_s_at | NM_00432 7 | 1 | BCR | 212526_at | AK002207 | 1 | SPG20 |
| 213282_at | BE501952 | 1 | BE501952 | 205861_at | NM_00312 1 | 1 | SPIB |
| 213637_at | BE503392 | 1 | BE503392 | 211704_s_at | AF356353 | 1 | SPIN2 |
| 215440_s_at | AL523320 | 1 | BEXL1 | 212071_s_at | BE968833 | 1 | SPTBN1 |
| 222026_at | BF437591 | 1 | BF437591 | 218407_x_at | NM_01334 9 | 1 | SPUF |
| 221847_at | BF665706 | 1 | BF665706 | 209218_at | AF098865 | 1 | SQLE |
| 206255_at | NM_00171 5 | 1 | BLK | 210959_s_at | AF113128 | 1 | SRD5A1 |
| 207655_s_at | NM_01331 4 | 1 | BLNK | 201247_at | BE513151 | 1 | SREBF2 |
| 201849_at | NM_00405 2 | 1 | BNIP3 | 205335_s_at | NM_00313 5 | 1 | SRP19 |
| 212563_at | BG491842 | 1 | BOP1 | 201273_s_at | NM_00313 3 | 1 | SRP9 |
| 203502_at | NM_00172 4 | 1 | BPGM | 202200_s_at | NM_00313 7 | 1 | SRPK1 |
| 213473_at | AL042733 | 1 | BRAP | 218140_x_ at | NM_02120 3 | 1 | SRPRB |
| 204531_s_at | NM_00729 5 | 1 | BRCA1 | 201138_s_at | NM_00314 2 | 1 | SSB |
| 220059_at | NM_01210 8 | 1 | BRDG1 | 201139_s_at | NM_00314 2 | 1 | SSB |
| 219177_at | NM_01832 1 | 1 | BRIX | 202591_s_at | NM_00314 3 | 1 | SSBP1 |
| 201458_s_at | NM_00472 5 | 1 | BUB3 | 208666_s_at | BE866412 | 1 | ST13 |
| 209974_s_at | AF047473 | 1 | BUB3 | 208667_s_at | U17714 | 1 | ST13 |
| 202808_at | AK000161 | 1 | C10orf26 | 209023_s_at | BC001765 | 1 | STAG2 |
| 55662_at | H27225 | 1 | C10orf76 | 212549_at | BE645861 | 1 | STAT5B |
| 219067_s_at | NM_01761 5 | 1 | C10orf86 | 211505_s_at | AL136601 | 1 | STAU |
| 218213_s_at | NM_01420 6 | 1 | C11orf10 | 208855_s_at | AF083420 | 1 | STK24 |
| 52164_at | AA065185 | 1 | C11orf24 | 217934_x_at | NM_00586 1 | 1 | STUB1 |
| 218220_at | NM_02164 0 | 1 | C12orf10 | 203310_at | NM_00726 9 | 1 | STXBP3 |
| 44790_s_at | AI129310 | 1 | C13orf18 | 221213_s_at | NM_01766 1 | 1 | SUHW4 |
| 218940_at | NM_02455 8 | 1 | C14orf138 | 202829_s_at | NM_00563 8 | 1 | SYBL1 |
| 217768_at | NM_01603 9 | 1 | C14orf166 | 219156_at | NM_01837 3 | 1 | SYNJ2BP |
| 221932_s_at | AA133341 | 1 | C14orf87 | 200055_at | NM_00628 4 | 1 | TAF10 |
| 201685_s_at | NM_01482 8 | 1 | C14orf92 | 206613_s_at | NM_00568 1 | 1 | TAF1A |
| 208837_at | BC000027 | 1 | C15orf22 | 221508_at | BC002756 | 1 | TAOK3 |
| 219439_at | NM_02015 6 | 1 | C1GALT1 | 201263_at | NM_00319 1 | 1 | TARS |
| 212164_at | AL522296 | 1 | Clorf37 | 209154_at | AF234997 | 1 | TAX1BP3 |
| 203960_s_at | NM_061612 6 | 1 | C1orf41 | 218466_at | NM_02468 2 | 1 | TBC1D17 |
| 214214_s_at | AU151801 | 1 | C1QBP | 203667_at | NM_00460 7 | 1 | TBCA |
| 209422_at | AY027523 | 1 | C20orf104 | 203715_at | NM_00319 3 | 1 | TBCE |
| 218448_at | NM_01789 6 | 1 | C20orf11 | 213400_s_at | AV753028 | 1 | TBL1X |
| 219443_at | NM_01771 4 | 1 | C20orf13 | 209820_s_at | BC002361 | 1 | TBL3 |
| 217737_x_a t | NM_01640 7 | 1 | C20orf43 | 216241_s_at | X57198 | 1 | TCEA1 |
| 217851_s_at | NM_01604 5 | 1 | C20orf45 | 202371_at | NM_02486 3 | 1 | TCEAL4 |
| 218859_s_at | NM_01664 9 | 1 | C20orf6 | 209153_s_at | M31523 | 1 | TCF3 |
| 202217_at | NM_00464 9 | 1 | C21orf33 | 222146_s_at | AK026674 | 1 | TCF4 |
| 218123_at | NM_01783 5 | 1 | C21orf59 | 222010_at | BF224073 | 1 | TCP1 |
| 221211_s_at | NM_02015 2 | 1 | C21orf7 | 222011_s_at | BF224073 | 1 | TCP1 |
| 218037_at | NM_02429 3 | 1 | C2orf17 | 203054_s_at | NM_02217 1 | 1 | TCTA |
| 221984_s_at | AL040896 | 1 | C2orf17 | 203448_s_at | AI347136 | 1 | TERF1 |
| 219137_s_at | NM_02019 4 | 1 | C2orf33 | 212330_at | R60866 | 1 | TFDP1 |
| 218518_at | NM_01660 3 | 1 | C5orf5 | 218996_at | NM_01334 2 | 1 | TFPT |
| 212176_at | AA902326 | 1 | C6orf111 | 214977_at | AK023852 | 1 | TG |
| 208809_s_at | AL136632 | 1 | C6orf62 | 212910_at | W19873 | 1 | THAP11 |
| 203259_s_at | BC001671 | 1 | C6orf74 | 213043_s_at | AI023317 | 1 | THRAP4 |
| 41047_at | AI885170 | 1 | C9orf16 | 43544_at | AA314406 | 1 | THRAP5 |
| 218992_at | NM_01846 5 | 1 | C9orf46 | 218188_s_at | NM_01245 8 | 1 | TIMM13 |
| 211984_at | AI653730 | 1 | CALM1 | 203222_s_at | NM_00507 7 | 1 | TLE1 |
| 200622_x_at | AV685208 | 1 | CALM3 | 211077_s_at | Z25421 | 1 | TLK1 |
| 203538_at | NM_00174 5 | 1 | CAMLG | 212997_s_at | AU151689 | 1 | TLK2 |
| 218929_at | NM_01763 2 | 1 | CARF | 208942_s_at | U93239 | 1 | TLOC1 |
| 202402_s_at | NM_00175 1 | 1 | CARS | 217979_at | NM_01439 9 | 1 | TM4SF13 |
| 213373_s_at | BF439983 | 1 | CASP8 | 208184_s_at | NM_00327 4 | 1 | TMEM1 |
| 200037_s_at | NM_01658 7 | 1 | CBX3 | 209412_at | U61500 | 1 | TMEM1 |
| 219174_at | NM_02510 3 | 1 | CCDC2 | 218477_at | NM_01405 1 | 1 | TMEM14A |
| 219470_x_a t | NM_01908 4 | 1 | CCNJ | 221452_s_at | NM_03096 9 | 1 | TMEM14B |
| 204645_at | NM_00124 1 | 1 | CCNT2 | 202857_at | NM_01425 5 | 1 | TMEM4 |
| 200910_at | NM_00599 8 | 1 | CCT3 | 209796_s_at | BC001027 | 1 | TMEM4 |
| 200812_at | NM_00642 9 | 1 | CCT7 | 215346_at | BF664114 | 1 | TNFRSF5 |
| 206398_s_at | NM_00177 0 | 1 | CD19 | 206150_at | NM_00124 2 | 1 | TNFRSF7 |
| 209583_s_at | AF063591 | 1 | CD200 | 48531_at | AA522816 | 1 | TNIP2 |
| 204581_at | NM_00177 1 | 1 | CD22 | 202561_at | AF070613 | 1 | TNKS |
| 38521_at | X59350 | 1 | CD22 | 210886_x_a t | AB007457 | 1 | TP53AP1 |
| 208650_s_at | BG327863 | 1 | CD24 | 220865_s_at | NM_01431 7 | 1 | TPRT |
| 208651_x_at | BG327863 | 1 | CD24 | 213334_x_a t | BE676218 | 1 | TREX2 |
| 209771_x_at | AA761181 | 1 | CD24 | 210846_x_at | AF220130 | 1 | TRIM 14 |
| 216379_x_at | AK000168 | 1 | CD24 | 212544_at | AI131008 | 1 | STRIP3 |
| 203593_at | NM_01212 0 | 1 | CD2AP | 202642_s_at | NM_00349 6 | 1 | TRRAP |
| 213856_at | BG230614 | 1 | CD47 | 215735_s_at | AC005600 | 1 | TSC2 |
| 200984_s_at | X16447 | 1 | CD59 | 214606_at | BF129969 | 1 | TSPAN-2 |
| 200985_s_at | NM_00061 1 | 1 | CD59 | 212928_at | AL050331 | 1 | TSPYL4 |
| 215925_s_at | AF283777 | 1 | CD72 | 217968_at | NM_00331 0 | 1 | TSSC1 |
| 205049_s_at | NM_00178 | 1 | CD79A | 36936_at | U58766 | 1 | TSTA3 |
| 205297_s_at | NM_00062 | 1 | CD79B | 201434_at | NM_003314 | 1 | TTC1 |
| 206761_at | NM_00581 | 1 | CD96 | 217964_at | NM_017775 | 1 | TTC19 |
| 205288_at | NM_00367 | 1 | CDC14A | 210645_s_at | D83077 | 1 | TTC3 |
| 203468_at | NM_00367 | 1 | CDK10 | 213174_at | BE675549 | 1 | TTC9 |
| 212899_at | AB028951 | 1 | CDK11 | 202266_at | NM_016614 | 1 | TTRAP |
| 204252_at | M68520 | 1 | CDK2 | 209077_at | AL022313 | 1 | TXN2 |
| 34210_at | N90866 | 1 | CDW52 | 201010_s_at | NM_006472 | 1 | TXNIP |
| 218592_s_at | NM_017829 | 1 | CECR5 | 37577_at | U79256 | 1 | U79256 |
| 205642_at | NM_00701 | 1 | CEP1 | 202151-s-at | NM_016172 | 1 | UBADC1 |
| 209194_at | BC005334 | 1 | CETN2 | 209115_at | AL117566 | 1 | UBE1C |
| 215318_at | AL049782 | 1 | CG012 | 201343_at | BE621259 | 1 | UBE2D2 |
| 218628_at | NM_016053 | 1 | CGI-116 | 201344_at | BF196642 | 1 | UBE2D2 |
| 219030_at | NM_016058 | 1 | CGI-121 | 210024_s_at | AB017644 | 1 | UBE2E3 |
| 218102_at | NM_015954 | 1 | CGI-26 | 201002_s_at | NM_003349 | 1 | UBE2V1, Kua-UEV |
| 219590_x_at | NM_015958 | 1 | CGI-30 | 218082_s_at | NM_014517 | 1 | UBP1 |
| 203721_s_at | NM_01600 | 1 | CGI-48 | 205687 at | NM_019116 | 1 | UBPH |
| 201569_s_at | NM_01538 | 1 | CGI-51 | 212008_at | N29889 | 1 | UBXD2 |
| 218642_s_at | NM_0024300 | 1 | CHCHD7 | 218533_s_at | NM_017859 | 1 | UCKL1 |
| 208968_s_at | BC002568 | 1 | CIAPIN1 | 209103_s_at | BC001049 | 1 | UFD1L |
| 200999_s_at | NM_006825 | 1 | CKAP4 | 212074_at | BE972774 | 1 | UNC84A |
| 201897_s_at | BC001425 | 1 | CKS1B | 206958_s_at | AF318575 | 1 | UPF3A |
| 212752_at | AA176798 | 1 | CLASP1 | 206959_s_at | AF318575 | 1 | UPF3A |
| 219859_at | NM_01435 | 1 | CLECSF9 | 214323_s_at | N36842 | 1 | UPF3A |
| 204085_s_at | NM_00649 | 1 | CLN5 | 202090_s_at | NM_00683 | 1 | UQCR |
| 204577_s_at | NM_024793 | 1 | CLUAP1 | 208909_at | BC000649 | 1 | UQCRFS1 |
| 218111_s_at | NM_018686 | 1 | CMAS | 208970_s_at | M14016 6 | 1 | UROD |
| 208912_s_at | BC001362 | 1 | CNP | 203031_s_at | NM_000375 | 1 | UROS |
| 203642_s_at | NM_01490 | 1 | COBLL1 | 200083_at | AA621731 | 1 | USP22 |
| 203073_at | NM_007357 | 1 | COG2 | 212388_at | AB028980 | 1 | USP24 |
| 213243_at | AI052003 | 1 | COH1 | 220079_s_at | NM_01839 | 1 | USP48 |
| 201652_at | NM_006837 | 1 | COPS5 | 203117_s_at | NM_014871 | 1 | USP52 |
| 218328_at | NM_01603 | 1 | COQ4 | 202745_at | NM_005154 | 1 | USP8 |
| 200086_s_at | AA854966 | 1 | COX4I1 | 221514_at | BC001149 | 1 | UTP14A |
| 203663_s_at | NM_00425 | 1 | COX5A | 218495_at | NM_004182 | 1 | UXT |
| 211025_x_at | BC006229 | 1 | COX5B | 207100_s_at | NM_016830 | 1 | VAMP1 |
| 201754_at | NM_004374 | 1 | COX6C | 214792_x_at | AI955119 | 1 | VAMP2 |
| 213846_at | AA382702 | 1 | COX7C | 202550_s_at | AF160212 | 1 | VAPB |
| 218142_s_at | NM_016302 | 1 | CRBN | 212038_s_at | AL515918 | 1 | VDAC1 |
| 208670_s_at | AF109873 | 1 | CRI1 | 203683_s_at | NM_003377 | 1 | VEGFB |
| 205081_at | NM_001311 | 1 | CRIP1 | 208623_s_at | J05021 | 1 | VIL2 |
| 204349_at | BC005250 | 1 | CRSP9 | 209950_s_at | BC004300 | 1 | VILL |
| 220753_s_at | NM_015974 | 1 | CRYL1 | 205844_at | NM_004666 | 1 | VNN1 |
| 221139_s_at | NM_01598 | 1 | CSAD | 204376_at | NM_014703 | 1 | VprBP |
| 201160_s_at | AL556190 | 1 | CSDA | 220068_at | NM_013378 | 1 | VPREB3 |
| 212073_at | AI631874 | 1 | CSNK2A1 | 212326_at | AB007922 | 1 | VPS13D |
| 203575_at | NM_001896 | 1 | CSNK2A2 | 218022_at | NM_016440 | 1 | VRK3 |
| 213980_s_at | AA053830 | 1 | CTBP1 | 221998_s_at | BF062886 | 1 | VRK3 |
| 221742_at | AI472139 | 1 | CUGBP1 | 213048_s_at | W26593 | 1 | W26593 |
| 201372_s_at | AU145232 | 1 | CUL3 | 213598_at | W87688 | 1 | W87688 |
| 218970_s_at | NM_015960 | 1 | CUTC | 221725_at | AI962978 | 1 | WASF2 |
| 214974_x_at | AK026546 | 1 | CXCL5 | 200609_s_at | NM_017491 | 1 | WDR1 |
| 215726_s_at | M22976 | 1 | CYB5 | 209216_at | BC000464 | 1 | WDR45 |
| 220999_s_at | NM_030778 | 1 | CYFIP2 | 212533_at | X62048 | 1 | WEE1 |
| 221905_at | BF516433 | 1 | CYLD | 203112_s_at | NM_005663 | 1 | WHSC2 |
| 207786_at | NM_024514 | 1 | CYP2R1 | 34225_at | AF101434 | 1 | WHSC2 |
| 216060_s_at | AK021890 | 1 | DAAM1 | 20S672_at | NM_000380 | 1 | XPA |
| 201624_at | NM_001349 | 1 | DARS | 212160_at | A1984005 | 1 | XPOT |
| 218443_s_at | NM_018959 | 1 | DAZAP1 1 | 213077_at | AL049305 | 1 | YTHDC2 |
| 202428_x_at | NM_020548 | 1 | DBI | 219186_at | NM_020224 | 1 | ZBTB7 |
| 211070_x_at | BC006466 | 1 | DBI | 217594_at | R25849 | 1 | ZCCHC11 |
| 201572_x_at | NM_00192 | 1 | DCTD | 212655_at | AB011151 | 1 | ZCCHC14 |
| 208619_at | L40326 | 1 | DDB1 | 212860_at | BGI68720 | 1 | ZDHHC18 |
| 212690_at | AB018268 | 1 | DDHD2 | 220261_s_at | NM_01810 | 1 | ZDHHC4 |
| 208674_x_at | D29643 | 1 | DDOST | 220206_at | NM_024772 | 1 | ZMYM1 |
| 203785_s_at | NM_018380 | 1 | DDX28 | 207605_x_at | NM_024498 | 1 | ZNF117 |
| 221699_s_at | AF334103 | 1 | DDX50 | 37254_at | U09366 | 1 | ZNF133 |
| 220482_s_at | NM_01213 9 | 1 | DELGEF | 209565_at | BC000832 | 1 | ZNF183 |
| 221509_at | AB014731 | 1 | DENR | 202778_s_at | NM_00345 3 | 1 | ZNF198 |
| 206752_s_at | NM_00440 2 | 1 | DFFB | 214823_at | AF033199 | 1 | ZNF204 |
| 203816_at | NM_00192 9 | 1 | DGUOK | 219925_at | NM_00716 | 1 | ZNF258 |
| 209549_s_at | BC001121 | 1 | DGUOK | 211009_s_at | AF15956 7 | 1 | ZNF271 |
| 202481_at | NM_00475 3 | 1 | DHRS3 | 216399_s_at | AK025663 | 1 | ZNF291 |
| 202031_s_at | NM_01561 0 | 1 | DKFZP434J154 | 211678_s_at | AF090934 | 1 | ZNF313 |
| 213701_at | AW299245 | 1 | DKFZp43N2030 | 209538_at | U69645 | 1 | ZNF32 |
| 222149_x_at | AL137398 | 1 | DKFZp434P162 | 218079_s_at | NM_024835 | 1 | ZNF403 |
| 212936_at | AI927701 | 1 | DKFZP564D172 | 221626_at | AL136548 | 1 | ZNF506 |
| 208091_s_at | NM_03079 6 | 1 | DKFZP564K0822 | 204291_at | NM_01480 3 | 1 | ZNF518 |
| | | | | 221645_s_at | M27877 | 1 | ZNF83 |

| | | | | | | | |
|---|---|---|---|---|---|---|---|
| Table 2: Genetic markers which are differentially expressed between probable multiple sclerosis which further converted to the diagnosis of definite MS during a two-years follow up and healthy controls are provided (the Probeset ID of the Affymetrix Gene Chip), along with the corresponding GenBank accession number (GenBank Acc. No.), the gene symbol and the direction of change in gene expression ("1"- upregulation; "-1" - downregulation). Note that the p values of the TNOM, Info and t-Test statistical tests all passed the 95 % confidence level. | | | | | | | |

***Sustained probable MS: Analysis of the signature of non-convertors -*** Analyzing of probable MS patients that did not convert to definite MS during the 2-year follow-up period as compared to healthy controls identified a specific gene expression signature of 503 *most informative* genes that is characteristic to these patients (Table 3, hereinbelow).

**Table 3**

| ***Differentially expressed markers between probable multiple sclerosis subjects which did not convert to the diagnosis of definite multiple sclerosis (non-convertors) and healthy controls*** | | | | | | | |
|---|---|---|---|---|---|---|---|
| ***Probeset*** | ***GenBank Acc. No.*** | ***Dir*** | ***Gene Symbol*** | ***Probeset*** | ***GenBank Acc. No.*** | ***Dir*** | ***Gene Symbol*** |
| 212126_at | | -1 | BG391282 | 220059_at | NM_012108 | 1 | BRDG1 |
| 2t3002_at | AA770596 | -1 | AA770596 | 207369_at | Z97632 | 1 | BRS3 |
| 207593_at | NM_022169 | -1 | ABCG4 | 213410_at | AL050102 | 1 | C10orf137 |
| 219935_at | NM_007038 | -1 | ADAMTS5 | 219012_s_at | AK023651 1 | 1 | C11or130 |
| 216678_at | AK000773 | -1 | AK000773 | 220240_s_at | NM_017905 | 1 | C13orf11 |
| 222252_x_at | AK023354 | -1 | AK023354 | 216263_s_at | AK022215 | 1 | C14orf120 |
| 216746_at | AK024606 | -1 | AK024606 | 213508_at | AA142942 | 1 | C14orf147 |
| 216774_at | AK025325 | -1 | AK025325 | 221932_s_at | AA133341 | 1 | C14orf87 |
| 216336_x_at | AL031602 | -1 | AL031602 | 52285_f_at | AW002970 | 1 | C18orf9 |
| 216822_x_at | AL359763 | -1 | AL359763 | 219283-at | NM_014158 | 1 | C1GALT2 |
| 216813_at | AL512728 | -1 | AL512728 | 2t7737_x_at | NM_016407 | 1 | C20orf43 |
| 202920_at | NM_001148 | -1 | ANK2 | 221196_x_at | NM_024332 | 1 | C6.1A |
| 215764_x_at | AA877641 | -1 | AP2A2 | 220329_s_at | NM_017909 | 1 | C6orf96 |
| 201168_x_at | NM_004309 | -1 | ARHGDIA | 204480_s_at | NM_024112 | 1 | C9orf16 |
| 213138_at | M62324 | -1 | ARID5A | 200622_x_at | AV685208 | 1 | CALM3 |
| 202208_s_at | BC001051 | -1 | ARL7 | 205034_at | NM_004702 | 1 | CCNE2 |
| 219996_at | NM_024708 | -1 | ASB7 | 2t6379_x_at | AK000168 | 1 | CD24 |
| 215987_at | AV654984 | -1 | AV654984 | 200983_x_at | BF983379 | 1 | CD59 |
| 222303_at | AV700891 | -1 | AV700891 | 215925_s_at | AF283777 | 1 | CD72 |
| 222348_at | AW971134 | -1 | AW971134 | 202892_at | NM_004661 | 1 | CDC23 |
| 222329_x_at | AW974816 | -1 | AW974816 | 211804_s_at | AB012305 | 1 | CDK2 |
| 205681_at | NM_004049 | -1 | BCL2A1 | 204661_at | NM_001803 | 1 | CDW52 |
| 213281_at | BE327172 | -1 | BE327172 | 219375 at | NM_006090 | 1 | CEPT1 |
| 217921_at | BE543064 | -1 | BE543064 | 213375_s_at | N80918 | 1 | CG018 |
| 215775_at | BF084105 | -1 | BF084105 | 218102_at | NM_015954 | 1 | CGI-26 |
| 217591_at | BF725121 | -1 | BF725121 | 33307_at | AL022316 | 1 | CGI-96 |
| 201235_s_at | BG339064 | -1 | BTG2 | 214426_x_at | BF062223 | 1 | CHAF1A |
| 220509_at | NM_018605 | -1 | C13orf10 | 220496_at | NM_016509 | 1 | CLEC2 |
| 201309_x_at | NM_004772 | -1 | C5orf13 | 202799_at | NM_006012 | 1 | CLPP |
| 220614_s_at | NM_024694 | -1 | C6orf103 | 222043_at | A1982754 | 1 | CLU |
| 205476_at | NM_004591 | -1 | CCL20 | 219301_s_at | NM_014141 | 1 | CNTNAP2 |
| 205114_s_at | NM_002983 | -1 | CCL3, CCL3L1, MGC12815 | 212189_s_at | AK022874 | 1 | COG4 |
| 201884_at | NM_004363 | -1 | CEACAM5 | 218328_at | NM_016035 | 1 | COQ4 |
| 218177_at | NM_020412 | -1 | CHMP1.5 | 213735_s_at | AI557312 | 1 | COX5B |
| 216016_at | AK027194 | -1 | CIAS1 | 201597_at | NM_001865 | 1 | COX7A2 |
| 216015_s_at | AK027194 | -1 | CIAS1 | 204920_at | AF154830 | 1 | CPS1 |
| 206207_at | NM_001828 | -1 | CLC | 203633_at | BF001714 | 1 | CPT1A |
| 213622_at | A1733465 | -1 | COL9A2 | 203804_s_at | NM_006107 | 1 | CROP |
| 213504_at | W63732 | -1 | COPS6 | 203445_s_at | NM_005730 | 1 | CTDSP2 |
| 204533_at | NM_001565 | -1 | CXCL10 | 200932_s_at | NM_006400 | 1 | DCTN2 |
| 203666_at | NM_000609 | -1 | CXCL12 | 203785_s_at | NM_018380 | 1 | DDX28 |
| 209774_x_at | M57731 | -1 | CXCL2 | 209549_s_at | BC001121 | 1 | DGUOK |
| 207850_at | NM_002090 | -1 | CXCL3 | 202532_s_at | NM_000791 | 1 | DHFR |
| 214421_x_at | AV652420 | -1 | CYP2C9 | 220985_s_at | NM_030954 | 1 | DKFZP564A 022 |
| 218013_x_at | NM_016221 | -1 | DCTN4 | 213647_at | D42046 | 1 | DNA2L |
| 22t780_s_at | AF336851 | -1 | DDX27 | 221689_s_at | AB035745 | 1 | DSCRS |
| 220004_at | NM_018665 | -1 | DDX43 | 204841_s_at | NM_003566 | 1 | EEA1 |
| 221293_s_at | NM_022047 | -1 | DEF6 | 206254_at | NM_001963 | 1 | EGF |
| 213632_at | M94065 | -1 | DHODH | 204410_at | NM_004681 | 1 | EIFIAY |
| 33768_at | L19267 | -1 | DMWD | 202461_at | NM_014239 | 1 | EIF2B2 |
| 215151_at | AB014594 | -1 | DOCK10 | 208985_s_at | BC002719 | 1 | EIF3S1 |
| 201044_x_at | AA530892 | -1 | DUSP1 | 208688_x_at | U78525 | 1 | EIF3S9 |
| 201041_s_at | NM_004417 | -1 | DUSP1 | 204505_s_at | NM_001978 | 1 | EPB49 |
| 204794_at | NM_004418 | -1 | DUSP2 | 203249_at | AB002386 | 1 | EZH1 |
| 213477_x_at | AL515273 | -1 | EEF1A1 | 218504_at | NM_016044 | 1 | FAHD2A |
| 201694_s_at | NM_001964 | -1 | EGR1 | 222056_s_at | AA723370 | 1 | FAHD2A |
| 201693_s_at | NM_001964 | -1 | EGR1 | 201863_at | NM_014077 | 1 | FAM32A |
| 200596_s_at | BE614908 | -1 | EIF3S10 | 38043_at | X55448 | 1 | FAM3A |
| 204513_s_at | NM_014800 | -1 | ELMO1 | 220408_x_at | NM_017569 | 1 | FAM48A |
| 210651_s_at | L41939 | -1 | EPHB2 | 209630_s_at | U87460 | 1 | FBXW2 |
| 205767_at | NM_001432 | -1 | EREG | 210889_s_at | M31933 | 1 | FCGR2B |
| 212106_at | AB020694 | -1 | ETEA | 217518_at | BF056029 | 1 | FERIL3 |
| 201329_s_at | NM_005239 | -1 | ETS2 | 210298_x_at | AF098518 | 1 | FHL1 |
| 211307_s_at | U43677 | -1 | FCAR | 201540_at | NM_001449 | 1 | FHL1 |
| 218814_s_at | NM_018252 | -1 | FLJ10874 | 208255_s_at | NM_012181 | 1 | FKBP8 |
| 220215_at | NM_024804 | -1 | FLJ12606 | 219130_at | NM_019083 | 1 | FLJ10287 |
| 219397_at | NM_025147 | -1 | FLJ13448 | 218179_s_at | NM_021942 | 1 | FLJ12716 |
| 218810_at | NM_025079 | -1 | FLJ23231 | 221777_at | BE966197 | 1 | FLJ14827 |
| 58367_s_at | AA429615 | -1 | FLJ23233 | 219802_at | NM_024854 | 1 | FLJ22028 |
| 219617_at | NM_024766 | -1 | FLJ23451 | 220169_at | NM_024943 | 1 | FLJ23235 |
| 206548_at | NM_024880 | -1 | FLJ23556 | 205140_at | NM_003838 | 1 | FPGT |
| 210414_at | AF169675 | -1 | FLRT1 | 204145_at | NM_004477 | 1 | FRG1 |
| 210933_s_at | BC004908 | -1 | FSCN1 | 209729_at | BC001782 | 1 | GAS2L1 |
| 217370_x_at | S75762 | -1 | FUS | 204220_at | NM_004877 | 1 | GMFG |
| 209416_s_at | AF083810 | -1 | FZRI | 204000_at | NM_016194 | 1 | GNB5 |
| 215308_at | AF052148 | -1 | G22P1 | 218361_at | NM_018178 | 1 | GPP34R |
| 209305_s_at | AF078077 | -1 | GADD45B | 222155_s_at | AK021918 | 1 | GPR172A |
| 219954_s_at | NM_020973 | -1 | GBA3 | 201106_at | NM_002085 | 1 | GPX4 |
| 207034_s_at | NM_030379 | -1 | GLI2 | 201501_s_at | NM_002092 | 1 | GRSF1 |
| 205220_at | NM_006018 | -1 | GPR109B | 203577_at | NM_001517 | 1 | GTF2H4 |
| 208524_at | NM_005290 | -1 | GPR15 | 218343_s_at | NM_012086 | 1 | GTF3C3 |
| 20994S_s_at | BC000251 | -1 | GSK3B | 221942_s_at | AI719730 | 1 | GUCY1A3 |
| 212291_at | AI393355 | -1 | HIPK1 | 203817_at | W93728 | 1 | GUCY1B3 |
| 208026_at | NM_003540 | -1 | HIST1H4F | 2135t5_x_at | AI133353 | 1 | HBG2 |
| 208729_x_at | D83043 | -1 | HLA-B | 209273_s_at | BG387555 | 1 | HBLD2 |
| 208812_x_at | BC004489 | -1 | HLA-C, HLA-B | 204689_at | NM_001529 | 1 | HHEX |
| 222126_at | A1247494 | -1 | 215933_s_at | IRS3L | Z21533 | 1 | HHEX |
| 206708_at | NM_002158 | -1 | HTLF | 220387_s_at | NM_007071 | 1 | HHLA3 |
| 36564_at | W27419 | -1 | IBRDC3 | 21852S_s_at | NM_017902 | 1 | HIF1AN |
| 202637_s_at | AI608725 | -1 | ICAM1 | 203932_at | NM_002H8 | 1 | HLA-DMB |
| 215485_s_at | AA284705 | -1 | ICAM1 | 205671_s_at | NM_002120 | 1 | HLA-DOB |
| 202638_s_at | NM_000201 | -1 | ICAM1 | 200904_at | X56841 | 1 | HLA-E |
| 202438_x_at | BF346014 | -1 | IDS | 213793_s_at | BE550452 | 1 | HOMER1 |
| 202081_at | NM_004907 | -1 | IER2 | 203914_x_at | NM_000860 | 1 | HPGD |
| 201631_s_at | NM_003897 | -1 | IER3 | 210112_at | U96721 | 1 | HPS1 |
| 207901_at | NM_002187 | -1 | IL12B | 217869_at | NM_016142 | 1 | HSD17B12 |
| 210118_s_at | M15329 | -1 | IL1A | 221791_s_at | BG167522 | 1 | HSPC016 |
| 205067_at | NM_000576 | -1 | IL1B | 221711_s_at | BC006244 | 1 | HSPC142 |
| 39402_at | M15330 | -1 | IL1B | 221622_s_at | AF246240 | 1 | HT007 |
| 212657_s_at | AW083357 | -1 | IL1RN | 207180_s_at | NM_006410 | 1 | HTATIP2 |
| 211506_s_at | AF043337 | -1 | IL8 | 209586_s_at | AF123539 | 1 | HTCD37 |
| 202859_x_at | NM_000584 | -1 | IL8 | 219209_at | NM_022168 | 1 | IFIH1 |
| 208364_at | NM_001566 | -1 | INPP4A | 203595_s_at | N47725 | 1 | IFIT5 |
| 213146_at | AA521267 | -1 | JMJD3 | 211868_x_at | AJ225092 | 1 | IGHG1 |
| 41386_i_at | AB002344 | -1 | JMJD3 | 212592_at | AV733266 | 1 | IGJ |
| 41387_r_at | AB002344 | -1 | JMJD3 | 221651_x_at | BC005332 | 1 | IGKC |
| 201465_s_at | BC002646 | -1 | JUN | 216207_x_at | AW408194 | 1 | IGKV1D-13 |
| 203751_x_at | NM_005354 | -1 | JUND | 215121_x_at | AA680302 | 1 | IGLC2 |
| 207141_s_at | U39196 | -1 | KCNJ3 | 48825_at | AA887083 | 1 | ING4 |
| 212037_at | AA206161 | -1 | KIAA0182 | 204202_at | NM_017604 | 1 | IQCE |
| 215137_at | H92070 | -1 | KIAA0508 | 206493_at | NM_000419 | 1 | ITGA2B |
| 204403_x_at | NM_014719 | -1 | KIAA0738 | 206494_s_at | NM_000419 | 1 | TTGA2B |
| 206966_s_at | NM_016285 | -1 | KLF12 | 211945_s_at | BG500301 | 1 | ITGB1 |
| 208467_at | NM_007249 | -1 | KLF12 | 216261_at | AI151479 | 1 | ITGB3 |
| 204012_s_at | AL529189 | -1 | LCMT2 | 213483_at | AK025679 | 1 | KIAA0073 |
| 202068_s_at | NM_000527 | -1 | LDLR | 202503_s_at | NM_014736 | 1 | KIAA0101 |
| 215462_at | AI978990 | -1 | LOC149478 | 212149_at | AW470003 | 1 | KIAA0143 |
| 216084_at | AL080137 | -1 | LOC389715 | 212523_s_at | D63480 | 1 | KIAA0146 |
| 64899_at | AA209463 | -1 | LPPR2 | 212733_at | AI798908 | 1 | KIAA0226 |
| 205193_at | NM_012323 | -1 | MAFF | 204876_at | NM_014699 | 1 | KIAA0296 |
| 219442_at | NM_024048 | -1 | MGC3020 | 76897_s_at | AA628140 | 1 | KIAA0674 |
| 209231_s_at | BC004191 | -1 | MGC3248 | 212548_s_at | BF515124 | 1 | KIAA0826 |
| 207984_s_at | NM_005374 | -1 | MPP2 | 207314_x_at | NM_006737 | 1 | KIR3DL2 |
| 210254_at | L35848 | -1 | MS4A3 | 201553_s_at | NM_005561 | 1 | LAMP1 |
| 202247_s_at | NM_004689 | -1 | MTA1 | 203042_at | NM_002294 | 1 | LAMP2 |
| 212452_x_at | AF113514 | -1 | MYST4 | 221515_s_at | BC0012t4 | 1 | LCMT1 |
| 205669_at | NM_004540 | -1 | NCAM2 | 221274_s_at | NM_030805 | 1 | LMAN2L |
| 200855_at | AW771910 | -1 | NCOR1 | 213408_s_at | AK024034 | 1 | LOC220686, PIK4CA |
| 204888_s_at | AF029729 | -1 | NEURL | 216250_s_at | X77598 | 1 | LPXN |
| 207535_s_at | NM_002502 | -1 | NFKB2 | 35974_at | U10485 | 1 | LRMP |
| 201502_s_at | NM_020529 | -1 | NFKBIA | 210044_s_at | BC002796 | 1 | LYL1 |
| 203927_at | NM_004556 | -1 | NFKBIE | 201384_s_at | NM_005899 | 1 | M17S2 |
| 215720_s_at | AL031778 | -1 | NFYA | 218573_at | NM_014061 | 1 | MAGEH1 |
| 220856_x_at | NM_014128 | -1 | NM-01412 8 | 206854_s_at | NM_003188 | 1 | MAP3K7 |
| 207783_x_at | NM_017627 | -1 | NM 01762 | 222036 s at | AI859865 | 1 | MCM4 |
| 208120_x_at | NM_031221 | -1 | NM_03122 | 209199_s_at 1 | L08895 | 1 | MEF2C |
| 202340_x_at | NM_002135 | -1 | NR4A1 | 202645_s_at | NM_000244 | 1 | MEN1 |
| 210226_at | D85245 | -1 | NR4A1 | 217043_s_at | U95822 | 1 | MFN1 |
| 211973_at | AW341200 | -1 | NUDT3 | 217022_s_at | S55735 | 1 | MGC27165 |
| 204435_at | NM_014778 | -1 | NUPL1 | 201764_at | NM_024056 | 1 | MGC5576 |
| 208274_at | NM_022375 | -1 | OCLM | 214364_at | W84525 | 1 | MGC61716 |
| 213131_at | R38389 | -1 | OLFM1 | 213528_at | AL035369 | 1 | MGC9084 |
| 221344_at | NM_013936 | -1 | OR12D2 | 205612_at | NM_007351 | 1 | MMRN1 |
| 214637_at | BG437034 | -1 | OSM | 218853_s_at | NM_019556 | 1 | MOSPD1 |
| 205432_at | NM_002557 | -1 | OVGP1 | 218339_at | NM_014180 | 1 | MRPL22 |
| 206880_at | NM_005446 | -1 | P2RXL1 | 203095_at | NM_002453 | 1 | MTIF2 |
| 215823_x_at | U64661 | -1 | PABPC1, PABPC3 | 210386_s_at | BC001906 | 1 | MTX1 |
| 204267_x_at | NM_004203 | -1 | PKMYT1 | 203517_at | NM_006554 | 1 | MTX2 |
| 207717_s_at | NM_004572 | -1 | PKP2 | 202960_s_at | NM_000255 | 1 | MUT |
| 204691_x_at | NM_003560 | -1 | PLA2G6 | 203359_s_at | NM_012333 | 1 | MYCBP |
| 202924_s_at | AF006005 | -1 | PLAGL2 | 200027_at | NM_004539 | 1 | NARS |
| 205934_at | NM_006226 | -1 | PLCL1 | 202607_at | AL526632 | 1 | NDST1 |
| 203471_s_at | NM_002664 | -1 | PLEK | 209224_s_at | BC003674 | 1 | NDUFA2 |
| 201489_at | BC005020 | -1 | PPIF | 202839_s_at | NM_004146 | 1 | NDUFB7 |
| 37028_at | U83981 | -1 | PPP1R15A | 218101_s_at | NM_004549 | 1 | NDUFC2 |
| 202014_at | NM_014330 | -1 | PPP1R15A | 217896_s_at | NM_024946 | 1 | NIP30 |
| 205643_s_at | NM_004576 | -1 | PPP2R2B | 213682_at | AL036344 | 1 | NUP50 |
| 203317_at | NM_012455 | -1 | PSD4 | 213018_at | AI337901 | 1 | ODAG |
| 204748_at | NM_000963 | -1 | PTGS2 | 205301_s_at | NM_016820 | 1 | OGGI |
| 206157_at | NM_002852 | -1 | PTX3 | 203351_s_at | NM_002552 | 1 | ORC4L |
| 34478_at | X79780 | -1 | RAB11B | 37966_at | AA187563 | 1 | PARVB |
| 205461_at | NM_006861 | -1 | RAB35 | 37965_at | AA181053 | 1 | PARVB |
| 204543_at | NM_005312 | -1 | RAPGEF1 | 216253_s_at | N73272 | 1 | PARVB |
| 203750_s_at | NM_000964 | -1 | RARA | 213652_at | AU152579 | 1 | PCSK5 |
| 209936_at | AF107493 | -1 | RBM5 | 207414_s_at | NM_002570 | 1 | PCSK6 |
| 216153_x_at | AK022897 | -1 | RECK | 219180_s_at | A1817074 | 1 | PEX26 |
| 209637_s_at | AF030111 | -1 | RGS12 | 206390_x_at | NM_002619 | 1 | PF4 |
| 221989_at | AW057781 | -1 | RPL10 | 202739_s_at | NM_000293 | 1 | PHKB |
| 215620_at | AU147182 | -1 | RREB1 | 217097_s_at | AC004990 | 1 | PHTF2 |
| 205485_at | NM_000540 | -1 | RYR1 | 207081_s_at | NM_002650 | 1 | PIK4CA |
| 215670_s_at | AK022844 | -1 | SCAND2 | 202732_at | NM_007066 | 1 | PKIG |
| 217331_at | U63542 | -1 | SCC-112 | 201410_at | AI983043 | 1 | PLEKHB2 |
| 208124_s_at | NM_004263 | -1 | SEMA4F | 212719_at | AB011178 | 1 | PLEKHE1 |
| 213742_at | AW241752 | -1 | SFRS11 | 201682_at | NM_004279 | 1 | PMPCB |
| 222169_x_at | N71739 | -1 | SH2D3A | 219317_at | NM_007t95 | 1 | POLI |
| 214623_at | AA845710 | -1 | SHFM3P1 | 202306_at | NM_002696 | 1 | POLR2G |
| 220000_at | NM_003830 | -1 | SIGLEC5 | 212955_s_at | AL037557 | 1 | POLR2I |
| 210796_x_at | D86359 | -1 | SIGLEC6 | 209382_at | U93867 | 1 | POLR3C |
| 203125_x_at | AF046997 | -1 | SLC11A2 | 209482_at | BC001430 | 1 | POP7 |
| 208389_s_at | NM_004171 | -1 | SLC1A2 | 202884_s_at | T79584 | 1 | PPP2R1B |
| 220091_at | NM_017585 | -1 | SLC2A6 | 200844_s_at | BE869583 | 1 | PRDX6 |
| 210001_s_at | AB005043 | -1 | SOCS1 | 201805_at | NM_002733 | 1 | PRKAG1 |
| 203372_s_at | NM_003877 | -1 | SOCS2 | 207808_s_at | NM_000313 | 1 | PROS1 |
| 215223_s_at | W46388 | -1 | SOD2 | 202529_at | NM_002766 | I | PRPSAP 1 |
| 215078_at | AL050388 | -1 | SOD2 | 201316_at | AL523904 | 1 | PSMA2 |
| 217576_x_at | BF692958 | -1 | SOS2 | 204279_at | NM_002800 | 1 | PSMB9 |
| 210693_at | BC001788 | -1 | SPPL2B | 201068_s_at | NM_002803 | 1 | PSMC2 |
| 202021_x_at | AF083441 | -1 | SUI1 | 200830_at | NM_002808 | 1 | PSMD2 |
| 207684_at | NM_004608 | -1 | TBX6 | 202009_at | NM_007284 | 1 | PTK9L |
| 221473_x_at | U49188 | -1 | TDE1 | 219178_at | NM_024638 | 1 | QTRTD1 |
| 211769_x_at | BC006088 | -1 | TDE1 | 220964_s_at | NM_030981 | 1 | RABIB |
| 201109_s_at | AV726673 | -1 | THBS1 | 217763_s_at | AF183421 | 1 | RAB31 |
| 201110_s_at | NM_003246 | -1 | THBS1 | 217764_s_at | AF183421 | 1 | RAB31 |
| 214657_s_at | AU134977 | -1 | TncRNA | 2145S2_s_at | AF098638 | 1 | RABEP1 |
| 207113_s_at | NM_000594 | -1 | TNF | 203020_at | NM_014857 | 1 | RABGAP1L |
| 202643_s_at | AI738896 | -1 | TNFAIP3 | 222077_s_at | AU153848 | 1 | RACGAP1 |
| 202644_s_at | NM_006290 | -1 | TNFAIP3 | 221809_at | AB040897 | 1 | RANBP10 |
| 206025_s_at | AW188198 | -1 | TNFAIP6 | 202297_s_at | AF157324 | 1 | RER1 |
| 216042_at | AI275938 | -1 | TNFRSF25 | 202296_s_at | NM_007033 | 1 | ER1 |
| 212260_at | AL045800 | -1 | TNRC15 | 209882_at | AF084462 | 1 | RIT1 |
| 204080_at | NM_025077 | -1 | TOE1 | 218301_at | NM_018226 | 1 | RNPEPL1 |
| 212869_x_at | AI721229 | -1 | TPT1 | 218583_s_at | NM_020640 | 1 | RP42 |
| 211943_x_at | AL565449 | -1 | TPT1 | 209773_s_at | BC001886 | 1 | RRM2 |
| 214327_x_at | AI888178 | -1 | TPT1 | 209486_at | BC004546 | 1 | SAS10 |
| 212284_x_at | BG498776 | -1 | TPT1 | 202084_s_at | NM_003003 | 1 | SEC14L1 |
| 220205_at | NM_013315 | -1 | TPTE | 200961_at | NM_012248 | 1 | SEPHS2 |
| 213593_s_at | AW978896 | -1 | TRA2A | 217756_x_at | NM_005770 | 1 | SERF2 |
| 205558_at | NM_004620 | -1 | TRAF6 | 205933_at | NM_015559 | 1 | SETBP1 |
| 213191_at | AF070530 | -1 | TRIP | 204688_at | NM_003919 | 1 | SGCE |
| 209013_x_at | AF091395 | -1 | TRIO | 213355_at | AI989567 | 1 | SIAT10 |
| 207490_at | NM_025019 | -1 | TUBA4 | 56256_at | AA150165 | 1 | SIDT2 |
| 201378_s_at | NM_014847 | -1 | UBAP2L | 219185_at | NM_012241 | 1 | SIRT5 |
| 215577_at | AU146791 | -1 | UBE2E1 | 201575_at | NM_012245 | 1 | SKIIP |
| 211403_x_at | AF167079 | -1 | VCX-C, VCX2, VCX3, VCY, VCX | 203775_at | NM_014251 | 1 | SLC25A13 |
| 214758_at | AL080157 | -1 | WDR21 | 203658_at | BC001689 | 1 | SLC25A20 |
| 218851_s_at | NM_018383 | -1 | WDR33 | 215043_s_at | X83301 | 1 | SMA3, SMA5 |
| 216036_x_at | AK001734 | -1 | WDTC1 | 207827_x_at | NM_007308 | 1 | SNCA |
| 222180_at | AU147889 | -1 | YES1 | 206272_at | NM_006542 | 1 | SPHAR |
| 219312_s_at | NM_023929 | -1 | ZBTB10 | 201273_s_at | NM_003133 | 1 | SRP9 |
| 203602_s_at | NM_003443 | -1 | ZBTB17 | 202811_at | NM_006463 | 1 | STAMBP |
| 216350_s_at | X52332 | -1 | ZNF10 | 200028_s_at | NM_020151 | 1 | STARD7 |
| 219228_at | NM_018555 | -1 | ZNF331 | 208855_s_at | AF083420 | 1 | STK24 |
| 220836_at | NM_017757 | -1 | ZNF407 | 212990_at | AB020717 | 1 | SYNJ1 |
| 220086_at | NM_022466 | -1 | ZNFN1A5 | 201259_s_at | AI768845 | 1 | SYPL |
| 43511 s at | AI201594 | 1 | DKFZp762 M127 | 221397 at | NM_023921 | 1 | TAS2R10 |
| 217480_x_at | M20812 | 1 | Ig kappa chain | 209152_s_at chain | M31523 | 1 | TCF3 |
| 212993_at | AA114166 | 1 | XP 316923. 1 | 219292_at | NM_018105 | 1 | THAP1 |
| 212583_at | AB011132 | 1 | AB011132 | 201447_at | AL567227 | 1 | TIA1 |
| 209307_at | AB014540 | 1 | AB014540 | 217979_at | NM_014399 | 1 | TM4SF13 |
| 217239_x_at | AF044592 | 1 | AF044592 | 221246_x_at | NM_018274 | 1 | TNS |
| 210183_x_at | AF112222 | 1 | AF112222 | 209917_s_at | BC002709 | 1 | TP53AP1 |
| 217939_s_at | NM_017657 | 1 | AFTIPHILI N | 218095_s_at | BC003545 | 1 | TPARL |
| 204057_at | AI073984 | 1 | AI073984 | 220865_s_at | NM_014317 | 1 | TPRT |
| 222273_at | AI419423 | 1 | AI419423 | 203512_at | NM_014408 | 1 | TRAPPC3 |
| 206513_at | NM_004833 | 1 | AIM2 | 217958_at | NM_016146 | 1 | TRAPPC4 |
| 214259_s_at | AI144075 | 1 | AKR7A2 | 204341_at | NM_006470 | 1 | TRIM16 |
| 209200_at | AL536517 | 1 | AL536517 | 210705_s_at | AF220028 | 1 | TRIM5 |
| 218203_at | NM_013338 | 1 | ALG5 | 209778_at | AF007217 | 1 | TRIP11 |
| 209425_at | AA888589 | 1 | AMACR | 215735_s_at | AC005600 | 1 | TSC2 |
| 202204_s_at | NM_001144 | 1 | AMFR | 221253_s_at | NM_030810 | 1 | TXNDC5 |
| 218230_at | AL044651 | 1 | ARFIP1 | 201588_at | NM_004786 | 1 | TXNL1 |
| 201229_s_at | BC000422 | 1 | ARIH2 | 218289_s_at | NM_024818 | 1 | UBE1DC1 |
| 202564_x_at | NM_001667 | 1 | ARL2 | 203281_s_at | NM_003335 | 1 | UBEIL |
| 203487_s_at | NM_015396 | 1 | ARMC8 | 218533_s_at | NM_017859 | 1 | UCKL1 |
| 214749_s_at | AK000818 | 1 | ARMCX6 | 203583_at | NM_014044 | 1 | UNC50 |
| 213238_at | AI478147 | 1 | ATP10D | 209268_at | AF165513 | 1 | VPS45A |
| 208898_at | AF077614 | 1 | ATP6V1D | 209452_s_at | AF035824 | 1 | VTI1B |
| 212559_at | AU148827 | 1 | AU148827 | 220917_s_at | NM_025132 | 1 | WDR19 |
| 219667_s_at | NM_017935 | 1 | BANK1 | 217784_at | NM_006555 | 1 | YKT6 |
| 214836_x_at | BG536224 | 1 | BG536224 | 220261_s_at | NM_018106 | 1 | ZDHHC4 |
| 207655_s_at | NM_013314 | 1 | BLNK | 202939_at | NM_005857 | 1 | ZMPSTE24 |
| | | | | 201541_s_at | NM_006349 | 1 | ZNHIT1 |

| | | | | | | | |
|---|---|---|---|---|---|---|---|
| Table 3: Provided are genetic markers which are differentially expressed between subjects diagnosed with probable multiple sclerosis which did not convert to the diagnosis of definite MS (non-convertors) during a two-years follow up and healthy controls (the Probeset ID of the Affymetrix Gene Chip), along with the corresponding GenBank accession number (GenBank Acc. No.), the gene symbol and the direction (Dir) of change in gene expression ("1"- upregulation; "-1" - downregulation). Note that the p values of the TNOM, Info and t-Test statistical tests all passed the 95 % confidence level. | | | | | | | |

***Gene expression pattern of subjects with definite diagnosis of multiple** sclerosis -* Table 4, hereinbelow, depict 722 genetic markers which are differentially expressed between subjects with a definite diagnosis of multiple sclerosis (both from relapse and remitting phases; blood samples were taken after the diagnosis of definite MS was confirmed, i.e., at least after the second neurological attack) and healthy controls.

**Table 4**

| ***Differentially expressed markers between subjects with the diagnosis of definite multiple sclerosis and healthy controls*** | | | | | |
|---|---|---|---|---|---|
| ***GenBank Accession No,*** | ***TNOM PValue*** | ***Info PValue*** | ***t-Test PValue*** | ***Dir*** | ***Gene Symbol*** |
| AI252582 | 5.31E-06 | 5.31E-06 | 2.46E-05 | -1 | ATP6V0E |
| NM_017686 | 5.31E-06 | 5.31E-06 | 1.44E-05 | -1 | GDAP2 |
| NM_006404 | 2.90E-05 | 4.28E-05 | 1.23E-05 | -1 | PROCR |
| NM_003916 | 1.49E-04 | 1.49E-04 | 8.21E-04 | -1 | AP1S2 |
| NM_015396 | 1.49E-04 | 1.49E-04 | 2.46E-03 | -1 | ARMC8 |
| NM_007212 | 1.49E-04 | 3.72E-05 | 5.28E-05 | -1 | RNF2 |
| AL138761 | 1.49E-04 | 1.49E-04 | 3.67E-03 | -1 | --- |
| NM_020474 | 2.70E-04 | 1.90E-04 | 2.85E-02 | -1 | GALNT1 |
| N21138 | 2.70E-04 | 1.90E-04 | 2.98E-04 | -1 | RHOBTB3 |
| NM_015904 | 1.82E-03 | 1.47E-03 | 7.88E-03 | -1 | EIF5B |
| NM_003201 | 1.82E-03 | 1.47E-03 | 4.89E-03 | -1 | TFAM |
| NM_005263 | 1.82E-03 | 1.79E-03 | 1.67E-02 | -1 | GFI1 |
| NM_022335 | 1.82E-03 | 2.96E-03 | 1.64E-04 | -1 | --- |
| NM_001222 | 1.82E-03 | 1.79E-03 | 3.87E-03 | -1 | SRP72 |
| BC002456 | 1.82E-03 | 2.96E-03 | 1.85E-04 | -1 | --- |
| AW268817 | 1.82E-03 | 1.47E-03 | 3.21E-02 | -1 | CDC5L |
| AB044661 | 1.82E-03 | 3.63E-04 | 7.50E-05 | -1 | XAB1 |
| AK024044 | 1.82E-03 | 1.47E-03 | 8.77E-04 | -1 | SSA2 |
| D42055 | 1.82E-03 | 1.79E-03 | 2.32E-03 | -1 | NEDD4 |
| BF680255 | 1.82E-03 | 9.10E-04 | 4.33E-03 | -1 | --- |
| AW238654 | 1.82E-03 | 1.47E-03 | 1.83E-03 | -1 | --- |
| AK024584 | 1.82E-03 | 1.47E-03 | 2.95E-02 | -1 | --- |
| L40992 | 1.82E-03 | 1.79E-03 | 2.40E-02 | -1 | RUNX2 |
| NM_014159 | 1.82E-03 | 1.47E-03 | 2.75E-03 | -1 | HYPB |
| AL137750 | 1.82E-03 | 1.47E-03 | 1.87E-02 | -1 | ETNKI |
| AL567227 | 2.01E-03 | 2.88E-03 | 3.52E-02 | -1 | TIA1 |
| NM_014774 | 2.01E-03 | 2.88E-03 | 4.00E-02 | -1 | KIAA0494 |
| AU146275 | 2.01E-03 | 1.69E-03 | 1.55E-02 | -1 | ZNF161 |
| NM_002717 | 2.01E-03 | 1.69E-03 | 6.85E-03 | -1 | PPP2R2A |
| NM_004301 | 2.01E-03 | 2.88E-03 | 1.52E-03 | -1 | ACTL6A |
| BE622627 | 2.01E-03 | 1.69E-03 | 1.83E-02 | -1 | PIK3R3 |
| NM_005977 | 2.01E-03 | 2.55E-04 | 7.91E-04 | -1 | RNF6 |
| NM_003616 | 2.01E-03 | 2.88E-03 | 9.81E-03 | -1 | SIP1 |
| NM_005531 | 2.01E-03 | 1.69E-03 | 7.12E-04 | -1 | IFI16 |
| NM_005849 | 2.01E-03 | 2.88E-03 | 1.29E-03 | -1 | IGSF6 |
| NM_006065 | 2.01E-03 | 5.47E-04 | 1.08E-03 | -1 | SIRPB1 |
| NM_003452 | 2.01E-03 | 2.88E-03 | 1.16E-02 | -1 | ZNF189 |
| NM_003539 | 2.01E-03 | 2.88E-03 | 2.71E-03 | -1 | HIST1H4D |
| NM_006016 | 2.01E-03 | 1.69E-03 | 1.81E-03 | -1 | CD164 |
| AI332962 | 2.01E-03 | 1.69E-03 | 2.36E-03 | -1 | ---- |
| BC004421 | 2.01E-03 | 1.21E-03 | 1.18E-03 | -1 | ZNF330 |
| AB037701 | 2.01E-03 | 2.88E-03 | 5.57E-03 | -1 | SIP1 |
| AB000815 | 2.01E-03 | 2.88E-03 | 8.15E-04 | -1 | ARNTL |
| BC006403 | 2.01E-03 | 1.21E-03 | 1.38E-04 | -1 | NCK1 |
| AB037703 | 2.01E-03 | 2.88E-03 | 1.25E-02 | -1 | SIP1 |
| U90142 | 2.01E-03 | 1.21E-03 | 3.63E-04 | -1 | BTN2A1 |
| AF234262 | 2.01E-03 | 2.88E-03 | 1.75E-03 | -1 | MAP3K2 |
| M60725 | 2.01E-03 | 2.55E-04 | 1.06E-04 | -1 | RPS6KB1 |
| AI057093 | 2.01E-03 | 1.69E-03 | 1.21E-03 | -1 | RDX |
| AL079292 | 2.01E-03 | 2.88E-03 | 1.92E-02 | -1 | --- |
| AB002347 | 2.01E-03 | 2.55E-04 | 2.15E-03 | -1 | C6orf133 |
| AL033377 | 2.01E-03 | 2.88E-03 | 4.63E-02 | -1 | GPR126 |
| AI811577 | 2.01E-03 | 1.69E-03 | 2.03E-03 | -1 | ZNF184 |
| AI860341 | 2.01E-03 | 1.69E-03 | 1.06E-03 | -1 | ACAA1 |
| AF052146 | 2.01E-03 | 2.88E-03 | 1.12E-03 | -1 | --- |
| AK025174 | 2.01E-03 | 2.88E-03 | 4.88E-03 | -1 | GSDML |
| AL096729 | 2.01E-03 | 1.69E-03 | 2.80E-02 | -1 | GSTA1 |
| AK026712 | 2.01E-03 | 2.88E-03 | 4.93E-03 | -1 | --- |
| AL049325 | 2.01E-03 | 2.88E-03 | 2.30E-02 | -1 | CCM1 |
| BC005316 | 2.01E-03 | 2.88E-03 | 2.52E-02 | -1 | NFYB |
| NM_016618 | 2.01E-03 | 5.47E-04 | 6.92E-03 | -1 | LOC51315 |
| NM_017423 | 2.01E-03 | 2.55E-04 | 6.86E-04 | -1 | GALNT7 |
| NM_018046 | 2.01E-03 | 2.88E-03 | 7.75E-03 | -1 | VG5Q |
| NM_018489 | 2.01E-03 | 1.69E-03 | 3.98E-04 | -1 | ASH1L |
| NM_018293 | 2.01E-03 | 1.69E-03 | 1.80E-03 | -1 | FLJ10997 |
| NM_018398 | 2.01E-03 | 1.69E-03 | 8.61E-03 | -1 | CACNA2D3 |
| NM_018042 | 2.01E-03 | 2.88E-03 | 1.97E-03 | -1 | FLJ10260 |
| NM_022488 | 2.01E-03 | 2.88E-03 | 5.03E-03 | -1 | APG3L |
| NM_030934 | 2.01E-03 | 1.69E-03 | 9.89E-03 | -1 | Clorf25 |
| AU157915 | 2.01E-03 | 2.88E-03 | 8.04E-03 | -1 | YTHDF3 |
| BG167522 | 2.01E-03 | 2.88E-03 | 2.61E-02 | -1 | HSPC016 |
| W72694 | 2.01E-03 | 2.55E-04 | 1.72E-03 | -1 | FAM26B |
| NM_002136 | 9.49E-03 | 8.40E-03 | 1.84E-02 | -1 | HNRPA1 |
| NM_005520 | 9.49E-03 | 8.40E-03 | 4.11E-03 | -1 | HNRPH1 |
| NM_020474 | 9.49E-03 | 1.37E-02 | 1.53E-02 | -1 | GALNT1 |
| NM_004048 | 9.49E-03 | 3.33E-03 | 1.95E-02 | -1 | B2M |
| NM_005415 | 9.49E-03 | 7.52E-03 | 2.69E-03 | -1 | SLC20A1 |
| AW051311 | 9.49E-03 | 1.56E-02 | 1.20E-02 | -1 | KPNA1 |
| AB020335 | 9.49E-03 | 7.52E-03 | 1.85E-02 | -1 | SEL1L |
| NM_002023 | 9.49E-03 | 1.37E-02 | 6.51E-03 | -1 | FMOD |
| NM_005103 | 9.49E-03 | 1.37E-02 | 1.21E-03 | -1 | FEZ1 |
| NM_000609 | 9.49E-03 | 7.52E-03 | 4.73E-02 | -1 | CXCL12 |
| NM_003759 | 9.49E-03 | 1.37E-02 | 2.07E-02 | -1 | SLC4A4 |
| NM_005589 | 9.49E-03 | 1.56E-02 | 1.03E-02 | -1 | ALDH6A1 |
| NM_005630 | 9.49E-03 | 1.56E-02 | 2.48E-02 | -1 | SLCO2A1 |
| NM_001046 | 9.49E-03 | 1.37E-02 | 2.06E-03 | -1 | SLC12A2 |
| NM_005388 | 9.49E-03 | 7.52E-03 | 2.40E-02 | -1 | PDCL |
| NM_005261 | 9.49E-03 | 8.40E-03 | 7.52E-03 | -1 | GEM |
| NM_014254 | 9.49E-03 | 1.56E-02 | 1.21E-02 | -1 | TMEM5 |
| NM_016656 | 9.49E-03 | 8.40E-03 | 7.06E-04 | -1 | RRAGB |
| NM_002147 | 9.49E-03 | 7.52E-03 | 1.86E-02 | -1 | HOXB5 |
| NM_004758 | 9.49E-03 | 1.37E-02 | 8.74E-03 | -1 | BZRAP1 |
| NM_002547 | 9.49E-03 | 1.56E-02 | 5.79E-03 | -1 | OPHN1 |
| NM_007366 | 9.49E-03 | 1.37E-02 | 8.98E-03 | -1 | PLA2R1 |
| NM_001340 | 9.49E-03 | 8.40E-03 | 3.60E-03 | -1 | CYLC2 |
| NM_015623 | 9.49E-03 | 7.52E-03 | 1.05E-02 | -1 | DKFZP564D166 |
| AF116710 | 9.49E-03 | 7.52E-03 | 1.10E-02 | -1 | RPS14 |
| BC000914 | 9.49E-03 | 7.52E-03 | 2.46E-03 | -1 | SFRS3 |
| U40763 | 9.49E-03 | 1.37E-02 | 7.34E-03 | -1 | PPIG |
| AB023200 | 9.49E-03 | 1.56E-02 | 4.79E-03 | -1 | C22orf19 |
| L07515 | 9.49E-03 | 3.33E-03 | 3.32E-04 | -1 | CBX5 |
| AB056663 | 9.49E-03 | 7.52E-03 | 1.00E-02 | -1 | ITCH |
| AW003989 | 9.49E-03 | 3.63E-04 | 4.13E-03 | -1 | C19orf2 |
| AF130104 | 9.49E-03 | 7.52E-03 | 1.37E-02 | -1 | --- |
| BC002713 | 9.49E-03 | 7.52E-03 | 1.04E-02 | -1 | MXD4 |
| AF229180 | 9.49E-03 | 3.33E-03 | 4.12E-04 | -1 | AASS |
| BE737027 | 9.49E-03 | 7.52E-03 | 1.79E-03 | -1 | --- |
| AI972268 | 9.49E-03 | 7.52E-03 | 1.65E-02 | -1 | PSME4 |
| AA148507 | 9.49E-03 | 2.52E-03 | 6.95E-04 | -1 | SLC7A1 |
| BG111635 | 9.49E-03 | 1.56E-02 | 2.16E-02 | -1 | CAST |
| AI022882 | 9.49E-03 | 1.37E-02 | 2.36E-03 | -1 | PAM |
| L43577 | 9.49E-03 | 7.52E-03 | 1.52E-02 | -1 | CXorf40 |
| BE312027 | 9.49E-03 | 8.40E-03 | 1.81E-03 | -1 | --- |
| AK022846 | 9.49E-03 | 8.40E-03 | 1.60E-02 | -1 | INPP5B |
| BG260658 | 9.49E-03 | 7.52E-03 | 2.00E-02 | -1 | --- |
| AW007137 | 9.49E-03 | 3.63E-04 | 2.91E-03 | -1 | --- |
| AI970731 | 9.49E-03 | 1.37E-02 | 2.38E-02 | -1 | RPS7 |
| H71805 | 9.49E-03 | 1.56E-02 | 2.40E-03 | -1 | --- |
| AV717561 | 9.49E-03 | 1.23E-02 | 1.55E-03 | -1 | --- |
| AI613383 | 9.49E-03 | 1.56E-02 | 4.68E-03 | -1 | EEF1D |
| W87901 | 9.49E-03 | 1.37E-02 | 4.57E-03 | -1 | --- |
| AK022065 | 9.49E-03 | 8.40E-03 | 1.10E-03 | -1 | RAB5A |
| AJ010395 | 9.49E-03 | 3.33E-03 | 1.27E-02 | -1 | DKC1 |
| AF113008 | 9.49E-03 | 2.52E-03 | 1.04E-03 | -1 | --- |
| AL359578 | 9.49E-03 | 3.33E-03 | 1.21E-03 | -1 | --- |
| AL049997 | 9.49E-03 | 8.40E-03 | 4.08E-02 | -1 | --- |
| U80771 | 9.49E-03 | 1.56E-02 | 9.34E-03 | -1 | --- |
| AF041811 | 9.49E-03 | 1.56E-02 | 1.10E-02 | -1 | NTRK3 |
| AW088547 | 9.49E-03 | 7.52E-03 | 3.59E-02 | -1 | --- |
| NM_006717 | 9.49E-03 | 8.40E-03 | 2.15E-03 | -1 | SPIN |
| NM_018145 | 9.49E-03 | 1.56E-02 | 3.83E-03 | -1 | FLJ10579 |
| AA824298 | 9.49E-03 | 7.52E-03 | 3.28E-03 | -1 | FLJ10036 |
| NM_016649 | 9.49E-03 | 1.37E-02 | 2.29E-03 | -1 | C20orf6 |
| NM_018320 | 9.49E-03 | 1.56E-02 | 1.44E-02 | -1 | RNF121 |
| NM_003973 | 9.49E-03 | 8.40E-03 | 4.82E-03 | -1 | --- |
| NM_01757 | 9.49E-03 | 8.40E-03 | 3.67E-03 | -1 | KIAA1212 |
| NM_022101 | 9.49E-03 | 1.37E-02 | 1.07E-02 | -1 | FLJ22965 |
| NM_014155 | 9.49E-03 | 7.52E-03 | 2.76E-02 | -1 | HSPC063 |
| NM_024312 | 9.49E-03 | 8.40E-03 | 1.51E-02 | -1 | MGC4170 |
| NM_024966 | 9.49E-03 | 3.33E-03 | 1.18E-03 | -1 | SEMA6D |
| NM_018655 | 9.49E-03 | 7.52E-03 | 1.39E-02 | -1 | LENEP |
| AF077053 | 9.49E-03 | 2.52E-03 | 2.54E-03 | -1 | TAF9L |
| BE972394 | 9.49E-03 | 1.56E-02 | 1.79E-03 | -1 | ZNF131 |
| BF195165 | 9.49E-03 | 1.56E-02 | 8.62E-03 | -1 | --- |
| BF224259 | 1.74E-02 | 2.19E-02 | 2.51E-02 | -1 | SMNDC1 |
| BG168896 | 1.74E-02 | 2.72E-02 | 1.16E-02 | -1 | FNTA |
| NM_012248 | 1.74E-02 | 7.75E-03 | 2.21E-03 | -1 | SEPHS2 |
| BF739979 | 1.74E-02 | 2.72E-02 | 1.64E-02 | -1 | FLJ16518 |
| AF053641 | 1.74E-02 | 5.43E-03 | 2.99E-02 | -1 | CSE1L |
| BC002513 | 1.74E-02 | 2.72E-02 | 1.15E-02 | -1 | EIF2S1 |
| NM_001020 | 1.74E-02 | 5.43E-03 | 3.65E-02 | -1 | RPS16 |
| NM_003589 | 1.74E-02 | 2.19E-02 | 2.94E-02 | -1 | CUL4A |
| AL567227 | 174E-02 | 2.19E-02 | 1.49E-02 | -1 | TIA1 |
| NM_022037 | 1.74E-02 | 5.43E-03 | 1.66E-03 | -1 | TIA1 |
| NM_002806 | 1.74E-02 | 5.43E-03 | 4.98E-03 | -1 | PSMC6 |
| D42063 | 1.74E-02 | 2.72E-02 | 1.35E-02 | -1 | RANBP2 |
| AF185696 | 1.74E-02 | 2.19E-02 | 2.04E-02 | -1 | OSBP |
| NM_001560 | 1.74E-02 | 2.19E-02 | 4.92E-02 | -1 | IL13RA1 |
| AI984051 | 1.74E-02 | 5.47E-04 | 4.24E-05 | -1 | THRAP1 |
| AA156948 | 1.74E-02 | 2.19E-02 | 7.17E-03 | -1 | PRPF4B |
| NM_016553 | 1.74E-02 | 7.75E-03 | 2.86E-03 | -1 | NUP62 |
| AF302110 | 1.74E-02 | 5.43E-03 | 7.85E-03 | -1 | AASDHPPT |
| NM_003601 | 1.74E-02 | 2.19E-02 | 1.40E-02 | -1 | SMARCA5 |
| NM_006178 | 1.74E-02 | 5.43E-03 | 4.00E-03 | -1 | NSF |
| BC000365 | 1.74E-02 | 2.19E-02 | 8.40E-03 | -1 | GTF2H1 |
| AA834576 | 1.74E-02 | 5.43E-03 | 1.05E-02 | -1 | ITPR2 |
| NM_003903 | 1.74E-02 | 2.19E-02 | 9.52E-03 | -1 | CDC16 |
| NM_006460 | 1.74E-02 | 5.43E-03 | 7.32E-03 | -1 | HIS1 |
| NM_004661 | 1.74E-02 | 2.19E-02 | 4.09E-02 | -1 | CDC23 |
| NM_003850 | 1.74E-02 | 5.43E-03 | 1.17E-02 | -1 | SUCLA2 |
| NM_006421 | 1.74E-02 | 2.72E-02 | 4.86E-02 | -1 | ARFGEF1 |
| NM_000255 | 1.74E-02 | 2.19E-02 | 1.71E-02 | -1 | MUT |
| NM_005783 | 1.74E-02 | 5.43E-03 | 3.12E-02 | -1 | TXNDC9 |
| NM_013450 | 1.74E-02 | 5.43E-03 | 1.03E-02 | -1 | BAZ2B |
| NM_003194 | 1.74E-02 | 2.19E-02 | 3.67E-02 | -1 | TBP |
| NM_014663 | 1.74E-02 | 2.72E-02 | 1.21E-02 | -1 | JMJD2A |
| BG111661 | 1.74E-02 | 2.72E-02 | 4.74E-02 | -1 | GOLGA1 |
| AU157008 | 1.74E-02 | 2.72E-02 | 5.92E-03 | -1 | PSMD5 |
| NM_003674 | 1.74E-02 | 5.43E-03 | 3.21E-02 | -1 | CDK10 |
| NM_025137 | 1.74E-02 | 5.43E-03 | 1.19E-02 | -1 | FLJ21439 |
| NM_021645 | 1.74E-02 | 5.43E-03 | 3.23E-02 | -1 | UTP14C |
| NM_001114 | 1.74E-02 | 5.43E-03 | 3.34E-03 | -1 | ADCY7 |
| NM_005923 | 1.74E-02 | 1.18E-02 | 1.58E-03 | -1 | MAP3K5 |
| NM_002118 | 1.74E-02 | 2.19E-02 | 3.48E-02 | -1 | HLA-DMB |
| NM_007049 | 1.74E-02 | 2.72E-02 | 8.46E-03 | -1 | BTN2A1 |
| NM_005999 | 1.74E-02 | 2.19E-02 | 4.44E-02 | -1 | TSNAX |
| No_006493 | 1.74E-02 | 2.19E-02 | 2.88E-02 | -1 | CLN5 |
| U16307 | 1.74E-02 | 5.43E-03 | 2.50E-03 | -1 | HRB2 |
| NM_004379 | 1.74E-02 | 2.19E-02 | 1.85E-03 | -1 | CREB 1 |
| NM_002048 | 1.74E-02 | 2.19E-02 | 2.16E-02 | -1 | GAS1 |
| AB011092 | 1.74E-02 | 1.18E-02 | 1.68E-03 | -1 | ADCY9 |
| NM_004898 | 1.74E-02 | 2.19E-02 | 9.45E-03 | -1 | CLOCK |
| NM_003631 | 1.74E-02 | 2.43E-02 | 6.33E-03 | -1 | PARG |
| NM_002643 | 1.74E-02 | 2.72E-02 | 4.22E-02 | -1 | PIGF |
| NM_014950 | 1.74E-02 | 2.19E-02 | 9.23E-03 | -1 | ZBTB1 |
| NM_003838 | 1.74E-02 | 5.43E-03 | 1.77E-03 | -1 | FPGT |
| NM_006299 | 1.74E-02 | 5.43E-03 | 4.66E-02 | -1 | ZNF193 |
| AF082283 | 1.74E-02 | 2.19E-02 | 4.89E-02 | -1 | BCL10 |
| NM_007309 | 1.74E-02 | 2.72E-02 | 1.60E-02 | -1 | DIAPH2 |
| NM_020423 | 1.74E-02 | 2.72E-02 | 1.01E-02 | -1 | PACE-1 |
| NM_012135 | 1.74E-02 | 2.19E-02 | 4.11E-03 | -1 | FAM50B |
| NM_006300 | 1.74E-02 | 2.72E-02 | 3.98E-02 | -1 | ZNF230 |
| NM_006588 | 1.74E-02 | 2.19E-02 | 5.41E-03 | -1 | HSGP25L2G |
| NM_003265 | 1.74E-02 | 2.72E-02 | 4.50E-02 | -1 | TLR3 |
| NM_002158 | 1.74E-02 | 2.19E-02 | 1.63E-03 | -1 | -HTLF |
| NM_001499 | 1.74E-02 | 2.72E-02 | 1.35E-02 | -1 | GLE1L |
| NM_000647 | 1.74E-02 | 5.47E-04 | 8.05E-04 | -1 | CCR2 |
| NM_004858 | 1.74E-02 | 2.19E-02 | 1.38E-03 | -1 | SLC4A8 |
| NM_015384 | 1.74E-02 | 5.47E-04 | 3.22E-03 | -1 | NIPBL |
| NM_003438 | 1.74E-02 | 5.47E-04 | 4.76E-04 | -1 | ZNF137 |
| NM_024986 | 1.74E-02 | 2.19E-02 | 9.65E-03 | -1 | FLJ12331 |
| NM_014812 | 1.74E-02 | 5.43E-03 | 3.21E-03 | -1 | KAB |
| NM_017522 | 1.74E-02 | 2.19E-02 | 4.19E-02 | -1 | LRP8 |
| NM_004324 | 1.74E-02 | 2.72E-02 | 3.10E-02 | -1 | BAX |
| AK024823 | 1.74E-02 | 2.19E-02 | 4.04E-03 | -1 | SUMO2 |
| AL570661 | 1.74E-02 | 2.72E-02 | 8.23E-03 | -1 | MCP |
| U72937 | 1.74E-02 | 2.19E-02 | 3.01E-02 | -1 | ATRX |
| AW073672 | 1.74E-02 | 5.43E-03 | 3.21E-03 | -1 | CTNND1 |
| BG534245 | 1.74E-02 | 4.17E-03 | 2.31E-03 | -1 | CSNK1A1 |
| AI659005 | 1.74E-02 | 7.75E-03 | 2.60E-03 | -1 | LGALS8 |
| BC005374 | 1.74E-02 | 2.19E-02 | 4.87E-02 | -1 | TXNDC4 |
| AF208043 | 1.74E-02 | 1.18E-02 | 2.83E-04 | -1 | IFI16 |
| BC002719 | 1.74E-02 | 2.72E-02 | 1.91E-02 | -1 | EIF3S1 |
| AF247168 | 1.74E-02 | 2.43E-02 | 3.04E-02 | -1 | NPD014 |
| AF006516 | 1.74E-02 | 2.19E-02 | 5.99E-03 | -1 | ABI1 |
| NM_001253 | 1.74E-02 | 1.18E-02 | 3.54E-03 | -1 | CDC5L |
| BC003600 | 1.74E-02 | 2.19E-02 | 8.59E-03 | -1 | LMO4 |
| AV701283 | 1.74E-02 | 2.19E-02 | 3.30E-03 | -1 | SEC22L1 |
| AI753638 | 1.74E-02 | 2.19E-02 | 2.93E-02 | -1 | OSBPL2 |
| AF165513 | 1.74E-02 | 2.19E-02 | 4.41E-03 | -1 | VPS45A |
| AF008442 | 1.74E-02 | 2.19E-02 | 8.61E-03 | -1 | POLRIC |
| AF112207 | 1.74E-02 | 2.19E-02 | 1.73E-03 | -1 | --- |
| BE963245 | 1.74E-02 | 2.19E-02 | 4.82E-03 | -1 | FBXW11 |
| N25915 | 1.74E-02 | 2.72E-02 | 1.41E-02 | -1 | CUGBP1 |
| AK001280 | 1.74E-02 | 2.19E-02 | 2.35E-02 | -1 | HDGFRP3 |
| AL133600 | 1.74E-02 | 5.47E-04 | 1.41E-03 | -1 | STAM2 |
| AF182198 | 1.74E-02 | 2.72E-02 | 8.03E-03 | -1 | ITSN2 |
| AF176699 | 1.74E-02 | 2.72E-02 | 5.37E-03 | -1 | FBXL4 |
| U22815 | 1.74E-02 | 2.19E-02 | 1.20E-02 | -1 | PPFIA1 |
| BC001265 | 1.74E-02 | 2.19E-02 | 3.12E-03 | -1 | DJ462023.2 |
| U89358 | 1.74E-02 | 2.19E-02 | 3.57E-02 | -1 | L3MBTL |
| AB034951 | 1.74E-02 | 2.72E-02 | 4.79E-02 | -1 | HSPA8 |
| BC002635 | 1.74E-02 | 2.19E-02 | 4.32E-02 | -1 | CSF2RA |
| AF274935 | 1.74E-02 | 2.19E-02 | 9.75E-03 | -1 | LOC54499 |
| BC005259 | 1.74E-02 | 2.19E-02 | 8.41E-03 | -1 | XRCC4 |
| AB002382 | 1.74E-02 | 2.19E-02 | 4.24E-03 | -1 | CTNND1 |
| BC006181 | 1.74E-02 | 2.19E-02 | 1.59E-02 | -1 | SFRS1 |
| AI359472 | 1.74E-02 | 2.19E-02 | 9.72E-03 | -1 | XTP2 |
| M27487 | 1.74E-02 | 2.72E-02 | 3.95E-02 | -1 | HLA-DPA1 |
| AU143855 | 1.74E-02 | 2.72E-02 | 2.08E-02 | -1 | PSME4 |
| BG111260 | 1.74E-02 | 2.19E-02 | 3.20E-03 | -1 | HIPK1 |
| AK022910 | 1.74E-02 | 5.43E-03 | 4.83E-04 | -1 | TNPO3 |
| AA195936 | 1.74E-02 | 5.43E-03 | 2.79E-02 | -1 | MGC21416 |
| AV715767 | 1.74E-02 | 2.19E-02 | 1.14E-02 | -1 | LIM |
| AV745949 | 1.74E-02 | 2.72E-02 | 2.35E-02 | -1 | SCAMP1 |
| D26069 | 1.74E-02 | 2.72E-02 | 1.34E-02 | -1 | CENTB2 |
| AL080111 | 1.74E-02 | 2.19E-02 | 1.71E-02 | -1 | NEK7 |
| AL562282 | 1.74E-02 | 2.19E-02 | 4.97E-02 | -1 | PP591 |
| BF970829 | 1.74E-02 | 2.19E-02 | 5.55E-03 | -1 | OSBPL8 |
| AI991252 | 1.74E-02 | 2.19E-02 | 2.36E-02 | -1 | BTN3A2 |
| AI742305 | 1.74E-02 | 4.17E-03 | 9.52E-05 | -1 | CHD9 |
| AA524345 | 1.74E-02 | 2.19E-02 | 1.02E-02 | -1 | SNX4 |
| AI752257 | 1.74E-02 | 2.72E-02 | 5.98E-03 | -1 | ZNF3 |
| BG548738 | 1.74E-02 | 2.72E-02 | 1.08E-02 | -1 | KIAA1040 |
| AB020684 | 1.74E-02 | 2.19E-02 | 1.15E-02 | -1 | KIAA0877 |
| AI040324 | 1.74E-02 | 2.19E-02 | 2.72E-03 | -1 | NCOA2 |
| AA284075 | 1.74E-02 | 2.72E-02 | 3.24E-03 | -1 | KNS2 |
| BE786164 | 1.74E-02 | 7.75E-03 | 2.14E-03 | -1 | --- |
| AP000693 | 1.74E-02 | 1.18E-02 | 2.00E-03 | -1 | ZCWCC3 |
| N22548 | 1.74E-02 | 2.19E-02 | 1.10E-03 | -1 | --- |
| BF970253 | 1.74E-02 | 2.19E-02 | 2.64E-02 | -1 | FLJ11806 |
| AV682436 | 1.74E-02 | 5.43E-03 | 1.97E-03 | -1 | PIK3C2A |
| Z78330 | 1.74E-02 | 5.43E-03 | 5.22E-03 | -1 | ACTR3 |
| AI041204 | 1.74E-02 | 5.47E-04 | 3.79E-03 | -1 | CAP350 |
| AL162056 | 1.74E-02 | 2.19E-02 | 4.90E-02 | -1 | KIAA1117 |
| AI823592 | 1.74E-02 | 1.18E-02 | 3.18E-03 | -1 | KIAA0423 |
| AI989567 | 1.74E-02 | 2.19E-02 | 3.94E-02 | -1 | SIAT10 |
| BF223370 | 1.74E-02 | 2.72E-02 | 2.52E-02 | -1 | MGC11332 |
| AW190088 | 1.74E-02 | 2.72E-02 | 3.63E-02 | -1 | ZNF307 |
| AI655015 | 1.74E-02 | 2.72E-02 | 1.05E-02 | -1 | DUSP7 |
| AA053830 | 1.74E-02 | 2.72E-02 | 3.29E-02 | -1 | CTBP1 |
| BE501352 | 1.74E-02 | 2.72E-02 | 9.70E-03 | -1 | DKFZp667G2110 |
| AB011097 | 1.74E-02 | 2.19E-02 | 9.50E-03 | -1 | ARTS-1 |
| M19720 | 1.74E-02 | 4.17E-03 | 1.92E-03 | -1 | --- |
| AI912583 | 1.74E-02 | 4.17E-03 | 2.31E-03 | -1 | HRB2 |
| AA890010 | 1.74E-02 | 2.19E-02 | 1.46E-02 | -1 | --- |
| AI376724 | 1.74E-02 | 5.43E-03 | 5.94E-04 | -1 | COX11 |
| AA209332 | 1.74E-02 | 2.19E-02 | 2.04E-02 | -1 | OPA1 |
| N58120 | 1.74E-02 | 2.19E-02 | 6.36E-03 | -1 | --- |
| M34356 | 1.74E-02 | 2.19E-02 | 4.08E-02 | -1 | CREB1 |
| AK026142 | 1.74E-02 | 5.43E-03 | 4.71E-04 | -1 | ODAG |
| AI357539 | 1.74E-02 | 4.17E-03 | 1.80E-03 | -1 | LRCH3 |
| AI571996 | 1.74E-02 | 2.19E-02 | 3.71E-02 | -1 | STAM2 |
| AA830884 | 1.74E-02 | 2.19E-02 | 9.07E-03 | -1 | FMR1 |
| AA573862 | 1.74E-02 | 2.72E-02 | 2.11E-02 | -1 | HLA-A |
| AL121936 | 1.74E-02 | 7.75E-03 | 2.66E-03 | -1 | BTN2A1 |
| AC005614 | 1.74E-02 | 2.43E-02 | 2.52E-03 | -1 | LOC163131 /// LOC284323 |
| AL163248 | 1.74E-02 | 2.19E-02 | 1.33E-02 | -1 | ZNF294 |
| AK024456 | 1.74E-02 | 5.47E-04 | 5.10E-03 | -1 | FGD2 |
| AK001861 | 1.74E-02 | 4.17E-03 | 5.47E-04 | -1 1 | SNX13 |
| AK025663 | 1.74E-02 | 5.43E-03 | 1.00E-02 | -1 | ZNF291 |
| AK024606 | 1.74E-02 | 5.43E-03 | 1.73E-03 | -1 | --- |
| AK025097 | 1.74E-02 | 2.19E-02 | 4.60E-02 | -1 | --- |
| AF035299 | 1.74E-02 | 5.47E-04 | 5.00E-03 | -1 | DOK1 |
| X69383 | 1.74E-02 | 2.43E-02 | 1.30E-02 | -1 | --- |
| S67289 | 1.74E-02 | 5.43E-03 | 3.46E-03 | -1 | CYBB |
| AI339732 | 1.74E-02 | 1.21E-03 | 1.55E-03 | -1 | CIAO1 |
| AA719797 | 1.74E-02 | 2.19E-02 | 4.46E-02 | -1 | OR7E18P |
| NM_014052 | 1.74E-02 | 2.19E-02 | 1.90E-03 | -1 | YWHAB |
| NM_014426 | 1.74E-02 | 2.72E-02 | 6.25E-03 | -1 | SNX5 |
| NM_018184 | 1.74E-02 | 4.17E-03 | 7.32E-03 | -1 | ARL10C |
| NM_016166 | 1.74E-02 | 2.19E-02 | 1.59E-02 | -1 | PIAS1 |
| NM_018442 | 1.74E-02 | 2.19E-02 | 2.87E-02 | -1 | IQWD1 |
| NM_018244 | 1.74E-02 | 2.19E-02 | 4.15E-02 | -1 | C20orf44 |
| NM_015153 | 1.74E-02 | 2.19E-02 | 1.15E-02 | -1 | PHF3 |
| NM_015961 | 1.74E-02 | 2.72E-02 | 3.91E-03 | -1 | SNF7DC2 |
| NM_018361 | 1.74E-02 | 2.19E-02 | 1.76E-02 | -1 | LPAAT-e |
| BC005316 | 1.74E-02 | 2.72E-02 | 4.18E-02 | -1 | NFYB |
| NM_018229 | 1.74E-02 | 2.19E-02 | 1.59E-02 | -1 | C14orf108 |
| AF195514 | 1.74E-02 | 5.47E-04 | 9.81E-04 | -1 | VPS4B |
| NM_024573 | 1.74E-02 | 5.43E-03 | 3.35E-03 | -1 | C6orf211 |
| NM_014028 | 1.74E-02 | 2.19E-02 | 4.90E-02 | -1 | OSTM1 |
| NM_001668 | 1.74E-02 | 2.19E-02 | 3.53E-02 | -1 | ARNT |
| NM_022494 | 1.74E-02 | 2.19E-02 | 9.09E-03 | -1 | ZDHHC6 |
| NM_022776 | 1.74E-02 | 5.47E-04 | 8.77E-04 | -1 | OSBPL11 |
| NM_022067 | 1.74E-02 | 2.19E-02 | 1.91E-02 | -1 | C14orf133 |
| NM_020640 | 1.74E-02 | 2.19E-02 | 1.07E-02 | -1 | RP42 |
| NM_018115 | 1.74E-02 | 1.18E-02 | 8.56E-04 | -1 | SDAD1 |
| NM_017646 | 1.74E-02 | 2.19E-02 | 9.56E-04 | -1 | TRIT1 |
| NM_003929 | 1.74E-02 | 2.72E-02 | 2.23E-02 | -1 | RAB7L1 |
| NM_017850 | 1.74E-02 | 7.75E-03 | 1.01E-03 | -1 | FLJ20508 |
| NM_012123 | 1.74E-02 | 2.72E-02 | 2.21E-02 | -1 | MTO1 |
| AK024569 | 1.74E-02 | 5.43E-03 | 1.05E-02 | -1 | HSPC163 |
| NM_023010 | 1.74E-02 | 2.19E-02 | 2.12E-03 | -1 | UPF3B |
| NM_025080 | 1.74E-02 | 5.43E-03 | 5.58E-03 | -1 | ASRGL1 |
| NM_017810 | 1.74E-02 | 2.72E-02 | 1.59E-02 | -1 | ZNF434 |
| NM_018569 | 1.74E-02 | 2.19E-02 | 8.22E-03 | -1 | C4orf16 |
| NM_019083 | 1.74E-02 | 2.72E-02 | 2.38E-02 | -1 | FLJ10287 |
| AF119868 | 1.74E-02 | 5.43E-03 | 1.89E-02 | -1 | KIAA1922 |
| NM_016020 | 1.74E-02 | 2.19E-02 | 1.58E-02 | -1 | TFB1M |
| NM_024638 | 1.74E-02 | 1.21E-03 | 1.92E-03 | -1 | QTRTD1 |
| NM_022168 | 1.74E-02 | 2.72E-02 | 9.45E-03 | -1 | IFIH1 |
| NM_024828 | 1.74E-02 | 2.19E-02 | 1.58E-03 | -1 | C9orf82 |
| NM_024546 | 1.74E-02 | 2.19E-02 | 1.30E-02 | -1 | C13orf7 |
| NM_017912 | 1.74E-02 | 2.72E-02 | 4.68E-02 | -1 | HERC6 |
| NM_017687 | 1.74E-02 | 2.19E-02 | 2.54E-03 | -1 | NHLRC2 |
| NM_024862 | 1.74E-02 | 7.75E-03 | 4.33E-03 | -1 | FBXO38 |
| NM_025027 | 1.74E-02 | 2.72E-02 | 3.08E-02 | -1 | ZNF606 |
| NM_025231 | 1.74E-02 | 4.17E-03 | 1.49E-03 | -1 | ZNF435 |
| NM_022147 | 1.74E-02 | 2.19E-02 | 3.26E-02 | -1 | IFRG28 |
| NM_004923 | 1.74E-02 | 2.72E-02 | 1.87E-02 | -1 | MTL5 |
| NM_024838 | 1.74E-02 | 5.43E-03 | 1.90E-02 | -1 | --- |
| NM_024833 | 1.74E-02 | 2.19E-02 | 3.46E-02 | -1 | FLJ23506 |
| NM_007210 | 1.74E-02 | 1.21E-03 | 2.38E-03 | -1 | GALNT6 |
| NM_005774 | 1.74E-02 | 2.43E-02 | 2.15E-02 | -1 | ZNF224 |
| NM_016424 | 1.74E-02 | 2.19E-02 | 4.40E-03 | -1 | CROP |
| NM_018372 | 1.74E-02 | 2.72E-02 | 3.91E-02 | -1 | RIF1 |
| NM_013240 | 1.74E-02 | 2.19E-02 | 2.64E-03 | -1 | C21orf127 |
| NM_018327 | 1.74E-02 | 5.43E-03 | 2.08E-02 | -1 | C20orf38 |
| NM_015510 | 1.74E-02 | 2.72E-02 | 1.83E-02 | -1 | DKFZp566O084 |
| No_018976 | 1.74E-02 | 5.43E-03 | 8.66E-03 | -1 | SLC38A2 |
| NM_030917 | 1.74E-02 | 2.19E-02 | 1.22E-02 | -1 | FIP1L1 |
| NM_030911 | 1.74E-02 | 1.21E-03 | 4.68E-04 | -1 | CDADC1 |
| NM_030963 | 1.74E-02 | 5.43E-03 | 1.52E-02 | -1 | RNF146 |
| AL120704 | 1.74E-02 | 2.19E-02 | 3.01E-02 | -1 | KPNA3 |
| BC000039 | 1.74E-02 | 1.21E-03 | 6.70E-04 | -1 | FAM26B |
| AF257135 | 1.74E-02 | 2.19E-02 | 3.46E-02 | -1 | WBSCR5 |
| AF274950 | 1.74E-02 | 5.43E-03 | 4.34E-02 | -1 | FLJ10637 |
| BC003073 | 1.74E-02 | 5.43E-03 | 1.21E-03 | -1 | FLJ10521 |
| AL565516 | 1.74E-02 | 2.19E-02 | 2.11E-02 | -1 | PANK3 |
| AI433464 | 1.74E-02 | 2.19E-02 | 3.87E-02 | -1 | PGM3 |
| NM_004251 | 1.74E-02 | 2.19E-02 | 3.14E-02 | -1 | RAB9A |
| AW162015 | 1.74E-02 | 2.72E-02 | 6.39E-03 | -1 | ZNF143 |
| AW002578 | 1.74E-02 | 2.19E-02 | 3.05E-02 | -1 | MGC3731 |
| AI983115 | 1.74E-02 | 2.72E-02 | 9.07E-03 | -1 | IL27RA |
| AW008921 | 1.74E-02 | 2.19E-02 | 3.59E-02 | -1 | SENP5 |
| AL137398 | 1.74E-02 | 2.72E-02 | 2.16E-03 | -1 | DKFZp434P162 |
| R43279 | 1.74E-02 | 4.17E-03 | 1.74E-03 | -1 | SNAPC3 |
| AW975638 | 1.74E-02 | 2.19E-02 | 4.47E-02 | -1 | --- |
| AF070618 | 1.74E-02 | 2.19E-02 | 1.09E-02 | -1 | MRPS22 |
| U07139 | 1.74E-02 | 2.19E-02 | 4.38E-02 | -1 | CACNB3 |
| D29642 | 1.74E-02 | 5.47E-04 | 1.84E-02 | -1 | ARHGAP25 |
| AI458463 | 1.74E-02 | 5.43E-03 | 9.08E-03 | -1 | PACE-1 |
| Z48481 | 3.95E-02 | 2.49E-02 | 2.75E-02 | -1 | MMP14 |
| AV702810 | 3.95E-02 | 2.49E-02 | 6.09E-03 | -1 | SET |
| NM_016451 | 3.95E-02 | 4.88E-03 | 4.12E-03 | -1 | COPB |
| AF205218 | 3.95E-02 | 2.52E-03 | 1.02E-03 | -1 | IVNSIABP |
| AW968555 | 3.95E-02 | 3.54E-02 | 4.32E-02 | -1 | TBL1X |
| NM_007214 | 3.95E-02 | 3.54E-02 | 3.59E-03 | -1 | SEC63 |
| NM_006838 | 3.95E-02 | 4.91E-02 | 1.87E-02 | -1 | --- |
| BC000603 | 3.95E-02 | 3.54E-02 | 3.10E-03 | -1 | --- |
| BE250417 | 3.95E-02 | 4.91E-02 | 9.76E-03 | -1 | ZMYND11 |
| AW183478 | 3.95E-02 | 9.26E-03 | 1.65E-02 | -1 | STK17A |
| NM_015641 | 3.95E-02 | 2.49E-02 | 1.28E-02 | -1 | TES |
| NM_014872 | 3.95E-02 | 4.91E-02 | 2.61E-02 | -1 | ZBTB5 |
| NM_014753 | 3.95E-02 | 4.91E-02 | 2.64E-02 | -1 | BMS1L |
| NM_001482 | 3.95E-02 | 9.26E-03 | 7.96E-03 | -1 | GATM |
| NM_000702 | 3.95E-02 | 2.49E-02 | 5.02E-03 | -1 | ATP1A2 |
| NM_006214 | 3.95E-02 | 3.54E-02 | 3.50E-02 | -1 | PHYH |
| BG034328 | 3.95E-02 | 4.88E-03 | 5.06E-03 | -1 | TFDP2 |
| BG252490 | 3.95E-02 | 4.91E-02 | 2.36E-02 | -1 | DNAJB4 |
| NM_016024 | 3.95E-02 | 4.91E-02 | 3.39E-02 | -1 | RBMX2 |
| NM_001353 | 3.95E-02 | 2.32E-02 | 6.14E-03 | -1 | AKR1C1 |
| NM_000428 | 3.95E-02 | 2.49E-02 | 1.45E-02 | -1 | LTBP2 |
| BF224146 | 3.95E-02 | 4.91E-02 | 3.64E-03 | -1 | TMEM5 |
| NM_002830 | 3.95E-02 | 3.54E-02 | 2.79E-02 | -1 | PTPN4 |
| NM_021077 | 3.95E-02 | 4.91E-02 | 4.77E-02 | -1 | NMB |
| NM_004944 | 3.95E-02 | 1.23E-02 | 3.94E-03 | -1 | DNASE1L3 |
| NM_016352 | 3.95E-02 | 9.26E-03 | 1.01E-02 | -1 | CPA4 |
| NM_003151 | 3.95E-02 | 3.54E-02 | 1.24E-02 | -1 | STAT4 |
| NM_002649 | 3.95E-02 | 3.54E-02 | 2.51E-02 | -1 | PIK3CG |
| NM_024506 | 3.95E-02 | 4.91E-02 | 1.05E-02 | -1 | GLB1L |
| NM_016436 | 3.95E-02 | 1.23E-02 | 2.13E-03 | -1 | C20orf104 |
| NM_001240 | 3.95E-02 | 2.32E-02 | 5.79E-03 | -1 | CCNT1 |
| NM_006610 | 3.95E-02 | 2.32E-02 | 3.17E-02 | -1 | MASP2 |
| NM_002385 | 3.95E-02 | 3.54E-02 | 4.24E-02 | -1 | MBP |
| BG494940 | 3.95E-02 | 4.91E-02 | 1.96E-02 | -1 | SSA2 |
| NM_014257 | 3.95E-02 | 1.75E-02 | 626E-03 | -1 | CD209L |
| NM_007068 | 3.95E-02 | 3.54E-02 | 3.77E-02 | -1 | DMC1 |
| AF208850 | 3.95E-02 | 4.91E-02 | 1.60E-02 | -1 | PTP4A2 |
| AF116710 | 3.95E-02 | 1.75E-02 | 9.09E-03 | -1 | RPS14 |
| BC001019 | 3.95E-02 | 1.23E-02 | 1.21E-02 | -1 | RPL39 |
| BC000734 | 3.95E-02 | 4.58E-02 | 1.57E-02 | -1 | EIF3S6 |
| AF226044 | 3.95E-02 | 4.91E-02 | 4.07E-02 | -1 | SNRK |
| AY008372 | 3.95E-02 | 3.54E-02 | 2.90E-02 | -1 | OSBPL3 |
| AF271775 | 3.95E-02 | 3.54E-02 | 5.26E-03 | -1 | LAT1-3TM |
| U79526 | 3.95E-02 | 3.54E-02 | 221E-02 | -1 | CMKLR1 |
| AF031137 | 3.95E-02 | 3.54E-02 | 9.11E-03 | -1 | NCR3 |
| AB006589 | 3.95E-02 | 2.49E-02 | 3.80E-02 | -1 | ESR2 |
| AF000381 | 3.95E-02 | 4.58E-02 | 9.92E-03 | -1 | --- |
| BE466128 | 3.95E-02 | 3.54E-02 | 2.57E-02 | -1 | RBM25 |
| AI631874 | 3.95E-02 | 3.54E-02 | 7.58E-03 | -1 | CSNK2A1 |
| BG403834 | 3.95E-02 | 2.49E-02 | 2.54E-02 | -1 | KPNA6 |
| AW593213 | 3.95E-02 | 1.23E-02 | 1.83E-03 | -1 | KIAA1078 |
| BF214492 | 3.95E-02 | 7.66E-04 | 3.51E-03 | -1 | RPL5 |
| AA521269 | 3.95E-02 | 4.58E-02 | 1.11E-02 | -1 | --- |
| BF739959 | 3.95E-02 | 1.23E-02 | 1.31E-02 | -1 | MFHAS1 |
| AW302047 | 3.95E-02 | 4.91E-02 | 6.80E-03 | -1 | --- |
| AI252582 | 3.95E-02 | 2.49E-02 | 1.10E-03 | -1 | --- |
| AI539361 | 3.95E-02 | 1.23E-02 | 1.39E-02 | -1 | --- |
| NM_012081 | 3.95E-02 | 4.58E-02 | 1.28E-02 | -1 | ELL2 |
| AA400421 | 3.95E-02 | 4.91E-02 | 2.82E-02 | -1 | TWISTNB |
| AK023851 | 3.95E-02 | 4.88E-03 | 1.60E-03 | -1 | CAPN2 |
| BE000837 | 3.95E-02 | 1.23E-02 | 1.96E-03 | -1 | KIAA0220 |
| AL121891 | 3.95E-02 | 3.54E-02 | 3.37E-03 | -1 | UBCE7IP5 |
| AK023621 | 3.95E-02 | 9.26E-03 | 1.74E-02 | -1 | RHOBTB3 |
| S69182 | 3.95E-02 | 4.88E-03 | 3.02E-03 | -1 | --- |
| AK026521 | 3.95E-02 | 3.54E-02 | 6.64E-03 | -1 | TAF1B |
| AL354872 | 3.95E-02 | 3.54E-02 | 4.77E-02 | -1 | CTH |
| NM_022731 | 3.95E-02 | 4.88E-03 | 4.52E-03 | -1 | NUCKS |
| NM_020239 | 3.95E-02 | 3.54E-02 | 1.25E-02 | -1 | CDC42SE1 |
| NM_022365 | 3.95E-02 | 3.54E-02 | 2.20E-02 | -1 | DNAJC1 |
| AV682567 | 3.95E-02 | 3.54E-02 | 4.39E-02 | -1 | MDS010 |
| NM_024779 | 3.95E-02 | 2.49E-02 | 2.19E-02 | -1 | PIP5K2C |
| NM_016052 | 3.95E-02 | 4.88E-03 | 2.26E-03 | -1 | CGI-115 |
| NM_022073 | 3.95E-02 | 4.91E-02 | 4.41E-02 | -1 | EGLN3 |
| NM_013281 | 3.95E-02 | 2.49E-02 | 3.12E-02 | -1 | FLRT3 |
| NM_016122 | 3.95E-02 | 3.54E-02 | 9.15E-03 | -1 | NY-REN-SS |
| NM_024084 | 3.95E-02 | 2.49E-02 | 8.27E-03 | -1 | MGC3196 |
| NM_017860 | 3.95E-02 | 3.54E-02 | 3.82E-03 | -1 | FLJ20519 |
| NM_023034 | 3.95E-02 | 2.32E-02 | 1.26E-02 | -1 | WHSC1L1 |
| NM_002548 | 3.95E-02 | 2.49E-02 | 2.42E-02 | -1 | OR1D2 |
| AL136733 | 3.95E-02 | 4.91E-02 | 1.29E-02 | -1 | UBAP1 |
| BC005934 | 3.95E-02 | 2.32E-02 | 1.83E-03 | -1 | FLJ21168 |
| AW303136 | 3.95E-02 | 4.88E-03 | 2.28E-03 | -1 | --- |
| W87634 | 3.95E-02 | 2.49E-02 | 6.65E-03 | -1 | CXorf33 |
| AI669379 | 3.95E-02 | 4.91E-02 | 1.28E-02 | -1 | --- |
| BG025063 | 3.95E-02 | 3.54E-02 | 3.63E-02 | -1 | --- |
| NM_014622 | 1.49E-04 | 1.49E-04 | 2.75E-03 | 1 | LOH11CR2A |
| M87268 | 1.49E-04 | 1.49E-04 | 1.71 E-03 | 1 | IGHG1 |
| NM_024588 | 1.49E-04 | 1.49E-04 | 4.18E-03 | 1 | FLJ23584 |
| NM_030926 | 1.49E-04 | 3.72E-05 | 2.14E-04 | 1 | ITM2C |
| NM_021173 | 2.70E-04 | 2.34E-04 | 1.25E-04 | 1 | POLD4 |
| NM_014403 | 2.70E-04 | 4.40E-04 | 1.20E-04 | 1 | SIAT7D |
| NM_004930 | 1.82E-03 | 9.10E-04 | 8.75E-04 | 1 | CAPZB |
| AF129756 | 1.82E-03 | 2.96E-03 | 1.39E-02 | 1 | BAT2 |
| AL096733 | 1.82E-03 | 2.96E-03 | 2.18E-03 | 1 | ATP6V0A1 |
| W74620 | 1.82E-03 | 1.47E-03 | 8.07E-04 | 1 | HNRPD |
| AC002544 | 1.82E-03 | 1.79E-03 | 2.14E-02 | 1 | LOC283970 |
| AF008937 | 1.82E-03 | 8.95E-05 | 4.76E-06 | 1 | STX16 |
| NM_006284 | 2.01E-03 | 2.88E-03 | 3.75E-02 | 1 | TAF10 |
| BF969352 | 2.01E-03 | 2.88E-03 | 6.33E-04 | 1 | ECE1 |
| AA675892 | 2.01E-03 | 1.69E-03 | 9.55E-04 | 1 | TOB1 |
| NM_004395 | 2.01E-03 | 2.88E-03 | 1.56E-02 | 1 | DBN1 |
| NM_000558 | 2.01E-03 | 2.88E-03 | 1.18E-03 | 1 | HBA1 /// HBA2 |
| NM_002412 | 2.01E-03 | 2.88E-03 | 2.62E-03 | 1 | MGMT |
| NM_003955 | 2.01E-03 | 1.69E-03 | 3.20E-02 | 1 | SOCS3 |
| NM_004089 | 2.01E-03 | 1.69E-03 | 4.98E-04 | 1 | DSIPI |
| AF022231 | 2.01E-03 | 2.88E-03 | 2.04E-03 | 1 | CTDSP2 |
| AL110191 | 2.01E-03 | 1.69E-03 | 4.57E-04 | 1 | DSIPI |
| M25079 | 2.01E-03 | 2.88E-03 | 1.68E-03 | 1 | HBB |
| L01639 | 2.01E-03 | 2.88E-03 | 5.25E-03 | 1 | CXCR4 |
| AF105974 | 2.01E-03 | 2.88E-03 | 1.36E-03 | 1 | HBA1 /// HBA2 |
| L07555 | 2.01E-03 | 2.88E-03 | 9.46E-03 | 1 | CD69 |
| AF167343 | 2.01E-03 | 2.88E-03 | 1.04E-02 | 1 | IL1RAP |
| AF116676 | 2.01E-03 | 2.88E-03 | 1.99E-02 | 1 | MYL4 |
| AF349114 | 2.01E-03 | 2.88E-03 | 1.56E-03 | 1 | HBB |
| AF349571 | 2.01E-03 | 2.88E-03 | 1.59E-03 | 1 | HBA1 /// HBA2 |
| BC005931 | 2.01E-03 | 2.88E-03 | 1.33E-03 | 1 | HBA1 |
| AJ225092 | 2.01E-03 | 2.88E-03 | 5.25E-03 | 1 | IGHG1 |
| AF348491 | 2.01E-03 | 2.88E-03 | 7.22E-03 | 1 | CXCR4 |
| T50399 | 2.01E-03 | 2.88E-03 | 1.29E-03 | 1 | HBA2 |
| AJ275355 | 2.01E-03 | 1.21E-03 | 5.42E-04 | 1 | MGC27165 |
| AJ249377 | 2.01E-03 | 2.88E-03 | 5.89E-03 | 1 | 1GLJ3 |
| AF059180 | 2.01E-03 | 2.88E-03 | 1.99E-03 | 1 | HBB |
| AJ275374 | 2.01E-03 | 1.69E-03 | 3.02E-03 | 1 | --- |
| V00489 | 2.01E-03 | 2.88E-03 | 1.63E-03 | 1 | HBA2 |
| NM_024299 | 2.01E-03 | 1.69E-03 | 1.98E-02 | 1 | C20orf149 |
| NM_020360 | 2.01E-03 | 1.69E-03 | 3.92E-03 | 1 | PLSCR3 |
| NM_017679 | 2.01E-03 | 1.21E-03 | 1.93E-04 | 1 | BCAS3 |
| AF230924 | 2.01E-03 | 2.88E-03 | 6.68E-03 | 1 | C6orf82 |
| AA523441 | 2.01E-03 | 2.88E-03 | 6.97E-04 | 1 | PEX16 |
| NM_005186 | 9.49E-03 | 1.56E-02 | 1.12E-02 | 1 | CAPN1 |
| BF304759 | 9.49E-03 | 7.52E-03 | 3.75E-02 | 1 | LRP1 |
| NM_001662 | 9.49E-03 | 8.40E-03 | 4.40E-03 | 1 | ARF5 |
| AF113019 | 9.49E-03 | 7.52E-03 | 3.36E-03 | 1 | SMARCD2 |
| NM_003025 | 9.49E-03 | 7.52E-03 | 2.68E-02 | 1 | SH3GL1 |
| NM_014846 | 9.49E-03 | 1.37E-02 | 4.68E-02 | 1 | KIAA0196 |
| NM_017458 | 9.49E-03 | 123E-02 | 2.56E-03 | 1 | MVP |
| NM_006243 | 9.49E-03 | 1.56E-02 | 4.40E-02 | 1 | PPP2R5A |
| NM_001985 | 9.49E-03 | 1.56E-02 | 3.40E-02 | 1 | ETFB |
| NM_016621 | 9.49E-03 | 1.37E-02 | 3.12E-02 | 1 | BHC80 |
| NM_004565 | 9.49E-03 | 1.23E-02 | 1.37E-03 | 1 | PEX14 |
| NM_005509 | 9.49E-03 | 1.56E-02 | 1.43E-02 | 1 | DMXL1 |
| NM_005224 | 9.49E-03 | 9.26E-03 | 8.52E-03 | 1 | ARID3A |
| NM_003070 | 9.49E-03 | 1.56E-02 | 1.21E-02 | 1 | SMARCA2 |
| AI810484 | 9.49E-03 | 3.63E-04 | 4.25E-03 | 1 | CBFA2T2 |
| AF226990 | 9.49E-03 | 1.37E-02 | 2.39E-02 | 1 | HLA-G |
| U78774 | 9.49E-03 | 7.52E-03 | 1.03E-02 | 1 | NFYC |
| U71088 | 9.49E-03 | 3.63E-04 | 1.69E-03 | 1 | MAP2K5 |
| AA085748 | 9.49E-03 | 1.56E-02 | 1.69E-02 | 1 | LOC149603 |
| AB011126 | 9.49E-03 | 8.40E-03 | 2.72E-03 | 1 | FNBP1 |
| BF570210 | 9.49E-03 | 1.37E-02 | 4.19E-03 | 1 | PNPLA2 |
| AI343248 | 9.49E-03 | 1.37E-02 | 7.03E-03 | 1 | SRP46 |
| AA126728 | 9.49E-03 | 7.52E-03 | 2.86E-02 | 1 | ICAM2 |
| N36926 | 9.49E-03 | 8.40E-03 | 1.13E-02 | 1 | GNA11 |
| AA602532 | 9.49E-03 | 8.40E-03 | 1.41E-02 | 1 | CLN2 |
| BE898639 | 9.49E-03 | 8.40E-03 | 1.18E-02 | 1 | ADD1 |
| AV724215 | 9.49E-03 | 1.56E-02 | 2.07E-02 | 1 | --- |
| AK001574 | 9.49E-03 | 1.37E-02 | 3.28E-03 | 1 | GORASP1 |
| BF034906 | 9.49E-03 | 8.40E-03 | 2.75E-03 | 1 | PL6 |
| AL121981 | 9.49E-03 | 3.33E-03 | 1.69E-03 | 1 | DLG1 |
| NM_024531 | 9.49E-03 | 7.52E-03 | 1.12E-02 | 1 | GPR172A |
| NM_016274 | 9.49E-03 | 8.40E-03 | 1.75E-03 | 1 | CKIP-1 |
| NM_004542 | 9.49E-03 | 7.52E-03 | 1.25E-02 | 1 | NDUFA3 |
| NM_007254 | 9.49E-03 | 1.37E-02 | 4.31E-03 | 1 | PNKP |
| NM_015711 | 9.49E-03 | 1.37E-02 | 1.96E-03 | 1 | GLTSCR1 |
| NM_022350 | 9.49E-03 | 1.56E-02 | 4.57E-02 | 1 | LRAP |
| NM_017915 | 9.49E-03 | 1.56E-02 | 3.46E-02 | 1 | FLJ20641 |
| AK026970 | 9.49E-03 | 7.52E-03 | 1.53E-03 | 1 | STX16 |
| AF078847 | 9.49E-03 | 1.37E-02 | 3.27E-02 | 1 | GTF2H2 |
| AI828531 | 9.49E-03 | 1.56E-02 | 1.01E-02 | 1 | WIZ |
| NM_001344 | 1.74E-02 | 2.19E-02 | 2.80E-02 | 1 | DAD1 |
| NM_006184 | 1.74E-02 | 2.19E-02 | 3.03E-03 | 1 | NUCB1 |
| NM_002743 | 1.74E-02 | 2.19E-02 | 8.32E-03 | 1 | PRKCSH |
| NM_007245 | 1.74E-02 | 2.43E-02 | 3.73E-03 | 1 | ATXN2L |
| NM_015853 | 1.74E-02 | 2.19E-02 | 7.03E-03 | 1 | LOC51035 |
| NM_003367 | 1.74E-02 | 2.72E-02 | 3.92E-02 | 1 | USF2 |
| NM_025195 | 1.74E-02 | 2.19E-02 | 4.92E-03 | 1 | TRIB1 |
| BE675849 | 1.74E-02 | 2.19E-02 | 4.29E-02 | 1 | C9orf60 |
| BC000436 | 1.74E-02 | 2.19E-02 | 1.14E-02 | 1 | ENSA |
| NM_006732 | 1.74E-02 | 2.19E-02 | 1.84E-02 | 1 | FOSB |
| NM_019058 | 1.74E-02 | 2.72E-02 | 1.70E-02 | 1 | DDIT4 |
| NM_020248 | 1.74E-02 | 2.19E-02 | 2.01E-02 | 1 | CTNNBIP1 |
| NM_003364 | 1.74E-02 | 2.72E-02 | 3.03E-02 | 1 | UPP1 |
| NM_016294 | 1.74E-02 | 2.19E-02 | 9.73E-03 | 1 | PPP6C |
| NM_014569 | 1.74E-02 | 2.19E-02 | 1.31E-02 | 1 | ZFP95 |
| NM_005354 | 1.74E-02 | 2.72E-02 | 3.91E-02 | 1 | JUND |
| NM_004994 | 1.74E-02 | 2.72E-02 | 3.80E-03 | 1 | MMP9 |
| NM_021127 | 1.74E-02 | 2.19E-02 | 4.47E-02 | 1 | PMAIP1 |
| NM_002460 | 1.74E-02 | 2.19E-02 | 4.63E-02 | 1 | IRF4 |
| NM_002201 | 1.74E-02 | 2.72E-02 | 3.88E-03 | 1 | ISG20 |
| NM_004073 | 1.74E-02 | 2.19E-02 | 1.47E-02 | 1 | PLK3 |
| NM_005738 | 1.74E-02 | 2.19E-02 | 4.15E-02 | 1 | ARL4A |
| NM_014716 | 1.74E-02 | 2.19E-02 | 1.32E-02 | 1 | CENTB1 |
| BG035761 | 1.74E-02 | 5.43E-03 | 1.26E-02 | 1 | SOCS3 |
| NM_018134 | 1.74E-02 | 2.19E-02 | 3.27E-02 | 1 | IQCC |
| NM_000519 | 1.74E-02 | 2.19E-02 | 2.23E-02 | 1 | HBD |
| NM_003811 | 1.74E-02 | 1.18E-02 | 1.57E-02 | 1 | TNFSF9 |
| NM_005191 | 1.74E-02 | 2.19E-02 | 8.99E-03 | 1 | CD80 |
| NM_017528 | 1.74E-02 | 4.17E-03 | 9.17E-04 | 1 | WBSCR22 |
| NM_002620 | 1.74E-02 | 2.72E-02 | 4.54E-02 | 1 | PF4V1 |
| NM_000423 | 1.74E-02 | 2.19E-02 | 7.31E-04 | 1 | KRT2A |
| AL578551 | 1.74E-02 | 2.19E-02 | 4.25E-03 | 1 | RNA10 |
| U91543 | 1.74E-02 | 2.72E-02 | 1.55E-02 | 1 | CHD3 |
| BC004242 | 1.74E-02 | 2.19E-02 | 6.76E-03 | 1 | HNRPUL1 |
| U03886 | 1.74E-02 | 2.72E-02 | 2.05E-02 | 1 | PNPLA4 |
| M36172 | 1.74E-02 | 2.19E-02 | 3.03E-02 | 1 | MYL4 |
| U20498 | 1.74E-02 | 2.19E-02 | 6.66E-03 | 1 | CDKN2D |
| BC000383 | 1.74E-02 | 4.17E-03 | 3.83E-02 | 1 | WTAP |
| BC006383 | 1.74E-02 | 5.43E-03 | 4.42E-04 | 1 | GPAA1 |
| AY026505 | 1.74E-02 | 5.43E-03 | 8.99E-04 | 1 | KIF2C |
| U17074 | 1.74E-02 | 2.19E-02 | 1.23E-02 | 1 | CDKN2C |
| D87858 | 1.74E-02 | 2.19E-02 | 3.94E-02 | 1 | FCAR |
| AI827941 | 1.74E-02 | 5.43E-03 | 1.32E-03 | 1 | MYH9 |
| BE968833 | 1.74E-02 | 2.72E-02 | 2.71E-02 | 1 | SPTBN1 |
| AK021419 | 1.74E-02 | 2.19E-02 | 2.12E-02 | 1 | SMARCB1 |
| D50918 | 1.74E-02 | 5.43E-03 | 3.23E-03 | 1 | |
| H65865 | 1.74E-02 | 2.72E-02 | 1.69E-02 | 1 | FLJ13910 |
| M62324 | 1.74E-02 | 5.43E-03 | 7.50E-03 | 1 | ARID5A |
| AW168132 | 1.74E-02 | 2.19E-02 | 3.40E-03 | 1 | KIAA0404 |
| AA514622 | 1.74E-02 | 2.19E-02 | 3.47E-02 | 1 | POM121 /// LOC340318 |
| AL578583 | 1.74E-02 | 5.47E-04 | 1.21E-03 | 1 | CORT |
| AA022949 | 1.74E-02 | 2.19E-02 | 1.18E-02 | 1 | --- |
| BG485135 | 1.74E-02 | 2.72E-02 | 1.23E-02 | 1 | --- |
| BG540628 | 1.74E-02 | 2.19E-02 | 1.73E-02 | 1 | --- |
| BG482805 | 1.74E-02 | 2.19E-02 | 1.53E-02 | 1 | --- |
| AC007842 | 1.74E-02 | 2.19E-02 | 4.29E-02 | 1 | LOC163131 |
| BG325734 | 1.74E-02 | 2.72E-02 | 1.53E-02 | 1 | MAPKAPK2 |
| AW404894 | 1.74E-02 | 2.72E-02 | 1.37E-02 | 1 | --- |
| W46388 | 1.74E-02 | 2.72E-02 | 1.58E-02 | 1 | SOD2 |
| AL008730 | 1.74E-02 | 4.17E-03 | 3.33E-03 | 1 | C6orf4 |
| X58851 | 1.74E-02 | 2.19E-02 | 4.04E-02 | 1 | MY4 |
| L14482 | 1.74E-02 | 2.19E-02 | 2.57E-02 | 1 | POU6F1 |
| AL050332 | 1.74E-02 | 2.19E-02 | 1.57E-03 | 1 | LYPLA2P1 |
| D84143 | 1.74E-02 | 2.19E-02 | 5.78E-03 | 1 | --- |
| AJ224869 | 1.74E-02 | 2.19E-02 | 7.85E-03 | 1 | CXCR4 |
| AL022067 | 1.74E-02 | 2.72E-02 | 2.51E-02 | 1 | PRDM1 |
| AL512687 | 1.74E-02 | 5.43E-03 | 1.56E-03 | 1 | NOMO2 /// NOMO1 /// NOMO3 |
| D84140 | 1.74E-02 | 2.19E-02 | 4.15E-03 | I | --- |
| AF043583 | 1.74E-02 | 2.19E-02 | 1.59E-02 | 1 | --- |
| AI042030 | 1.74E-02 | 5.43E-03 | 3.57E-03 | 1 | SMCIL1 |
| NM_005746 | 1.74E-02 | 2.72E-02 | 9.92E-03 | I | PBEF1 |
| NM_005746 | 1.74E-02 | 2.72E-02 | 1.28E-02 | 1 | PBEF1 |
| AL523965 | 1.74E-02 | 2.19E-02 | 1.86E-02 | 1 | C6orf106 |
| NM_017874 | 1.74E-02 | 2.19E-02 | 2.42E-03 | 1 | C20ort27 |
| NM_022452 | 1.74E-02 | 5.43E-03 | 1.14E-02 | 1 | FBS1 |
| NM_024324 | 1.74E-02 | 2.19E-02 | 2.83E-03 | 1 | --- |
| NM_024535 | 1.74E-02 | 2.19E-02 | 1.05E-03 | 1 | CORO7 |
| NM_023948 | 1.74E-02 | 2.19E-02 | 1.92E-02 | 1 | MOSPD3 |
| NM_022341 | 1.74E-02 | 5.43E-03 | 1.97E-03 | I | PDF /// COG8 |
| NM_016633 | 1.74E-02 | 2.19E-02 | 2.74E-02 | 1 | ERAF |
| NM_012447 | 1.74E-02 | 2.72E-02 | 1.41E-02 | 1 | STAG3 |
| NM_020533 | 1.74E-02 | 2.19E-02 | 4.81E-02 | 1 | MCOLN1 |
| NM_019891 | 1.74E-02 | 2.19E-02 | 2.86E-02 | 1 | ERO1LB |
| NM_018113 | 1.74E-02 | 2.19E-02 | 4.51E-02 | 1 | LIMR |
| NM_017774 | 1.74E-02 | 5.43E-03 | 7.44E-03 | 1 | CDKAL1 |
| NM_024669 | 1.74E-02 | 2.72E-02 | 3.93E-03 | 1 | FLJ11795 |
| NM_014076 | 1.74E-02 | 7.75E-03 | 7.57E-04 | 1 | GPR97 |
| N39536 | 1.74E-02 | 5.43E-03 | 1.95E-03 | 1 | NOMO2 /// NOMO1 /// NOMO3 |
| L14754 | 1.74E-02 | 2.19E-02 | 2.49E-02 | 1 | IGHMBP2 |
| Y14330 | 1.74E-02 | 2.43E-02 | 4.44E-03 | 1 | JAG2 |
| U88964 | 1.74E-02 | 2.19E-02 | 1.28E-02 | 1 | ISG20 |
| AL096779 | 1.74E-02 | 2.72E-02 | 2.66E-02 | 1 | C22orf4 |
| AC005954 | 1.74E-02 | 4.17E-03 | 4.12E-03 | 1 | TJP3 |
| AF022991 | 1.74E-02 | 2.72E-02 | 1.02E-02 | 1 | PERI |
| AB002328 | 1.74E-02 | 2.72E-02 | 1.88E-02 | 1 | CABIN1 |
| AI150117 | 1.74E-02 | 2.72E-02 | 1.52E-02 | 1 | TOPORS |
| AI743331 | 1.74E-02 | 2.19E-02 | 7.64E-03 | 1 | C20orf67 |
| L38487 | 3.95E-02 | 3.54E-02 | 1.21E-02 | 1 | ESRRA |
| BC002356 | 3.95E-02 | 4.58E-02 | 8.37E-03 | 1 | NUCB1 |
| NM_000177 | 3.95E-02 | 7.66E-04 | 3.31E-03 | 1 | GSN |
| NM_000018 | 3.95E-02 | 4.91E-02 | 1.50E-02 | 1 | ACADVL |
| NM_002332 | 3.95E-02 | 3.54E-02 | 2.62E-02 | 1 | LRP1 |
| AI920976 | 3.95E-02 | 3.54E-02 | 1.14E-02 | 1 | RERE |
| AL523776 | 3.95E-02 | 9.26E-03 | 1.65E-02 | 1 | OTUB1 |
| NM_003097 | 3.95E-02 | 4.91E-02 | 4.68E-02 | 1 | SNURF /// SNRPN |
| NM_006295 | 3.95E-02 | 7.66E-04 | 4.08E-03 | 1 | VARS2 |
| NM_001540 | 3.95E-02 | 2.49E-02 | 2.73E-02 | 1 | HSPB1 |
| NM_003040 | 3.95E-02 | 2.52E-03 | 5.86E-03 | 1 | SLC4A2 |
| NM_015049 | 3.95E-02 | 3.54E-02 | 5.05E-03 | 1 | ALS2CR3 |
| BC005003 | 3.95E-02 | 4.91E-02 | 2.77E-02 | 1 | NFYC |
| BC000120 | 3.95E-02 | 2.49E-02 | 1.09E-02 | 1 | GTF2F1 |
| NM_005334 | 3.95E-02 | 3.54E-02 | 1.79E-02 | 1 | HCFC1 |
| NM_004216 | 3.95E-02 | 3.54E-02 | 1.04E-02 | 1 | DEDD |
| NM_006700 | 3.95E-02 | 1.23E-02 | 8.01E-04 | 1 | FLN29 |
| NM_004514 | 3.95E-02 | 2.49E-02 | 2.54E-02 | 1 | FOXK2 |
| NM_005077 | 3.95E-02 | 3.54E-02 | 5.69E-03 | 1 | TLE1 |
| NM_005641 | 3.95E-02 | 4.91E-02 | 9.70E-03 | 1 | TAF6 |
| NM 003704 | 3.95E-02 | 7.66E-04 | 3.50E-03 | 1 | C4orf8 |
| NM_006547 | 3.95E-02 | 3.54E-02 | 2.21E-02 | 1 | IMP-3 |
| AL136771 | 3.95E-02 | 2.32E-02 | 2.43E-03 | 1 | ELL |
| NM_000265 | 3.95E-02 | 4.88E-03 | 2.45E-03 | 1 | NCF1 |
| NM_002555 | 3.95E-02 | 9.26E-03 | 2.18E-03 | 1 | SLC22A18 |
| NM_003043 | 3.95E-02 | 3.54E-02 | 2.64E-02 | 1 | SLC6A6 |
| NM_014813 | 3.95E-02 | 1.23E-02 | 3.47E-03 | 1 | LRIG2 |
| NM_001157 | 3.95E-02 | 4.88E-03 | 5.60E-03 | 1 | ANXA11 |
| NM_006865 | 3.95E-02 | 4.58E-02 | 1.11E-02 | 1 | LILRA3 |
| NM_004258 | 3.95E-02 | 7.66E-04 | 3.58E-02 | 1 | IGSF2 |
| AF052179 | 3.95E-02 | 3.54E-02 | 3.45E-02 | 1 | ARF1 |
| BE674658 | 3.95E-02 | 2.32E-02 | 2.24E-02 | 1 | FLJ13052 |
| BC003381 | 3.95E-02 | 3.54E-02 | 1.54E-03 | 1 | KIAA0217 |
| K02920 | 3.95E-02 | 9.26E-03 | 7.34E-03 | 1 | GBA |
| D87454 | 3.95E-02 | 4.91E-02 | 4.30E-02 | 1 | KIAA0265 |
| U66879 | 3.95E-02 | 1.75E-02 | 3.88E-03 | 1 | BAD |
| AB011179 | 3.95E-02 | 2.32E-02 | 3.44E-03 | 1 | NCDN |
| D37781 | 3.95E-02 | 4.91E-02 | 1.33E-02 | 1 | PTPRJ |
| U22815 | 3.95E-02 | 2.49E-02 | 2.64E-03 | 1 | PPFIA1 |
| U81802 | 3.95E-02 | 2.49E-02 | 7.85E-03 | 1 | PIK4CB |
| BC004516 | 3.95E-02 | 9.26E-03 | 4.07E-02 | 1 | MAX |
| AF056322 | 3.95E-02 | 2.49E-02 | 2.77E-02 | 1 | SP100 |
| AF234161 | 3.95E-02 | 4.91E-02 | 5.17E-03 | 1 | CIZ1 |
| U90940 | 3.95E-02 | 4.91E-02 | 4.23E-02 | 1 | FCGR2C |
| AL046054 | 3.95E-02 | 2.32E-02 | 1.78E-02 | 1 | PTOV1 |
| AI762552 | 3.95E-02 | 4.91E-02 | 9.59E-03 | 1 | HNRPDL |
| BF974389 | 3.95E-02 | 3.54E-02 | 7.94E-03 | 1 | MTVR1 |
| AW237172 | 3.95E-02 | 2.32E-02 | 2.21E-02 | 1 | JMJD2B |
| AA877910 | 3.95E-02 | 1.23E-02 | 3.15E-03 | 1 | ATP2A3 |
| AW157619 | 3.95E-02 | 9.26E-03 | 3.57E-03 | 1 | CES2 |
| AW451236 | 3.95E-02 | 2.49E-02 | 2.11E-02 | 1 | TCEB3 |
| BE856549 | 3.95E-02 | 3.54E-02 | 4.99E-02 | 1 | KIAA0974 |
| AW072388 | 3.95E-02 | 1.75E-02 | 4.60E-03 | 1 | NCF1 |
| AK025604 | 3.95E-02 | 3.54E-02 | 9.44E-03 | 1 | M-RIP |
| AL096741 | 3.95E-02 | 4.58E-02 | 5.49E-03 | 1 | ASCC2 |
| AK025271 | 3.95E-02 | 3.54E-02 | 4.30E-02 | 1 | CHCHD3 |
| AB035175 | 3.95E-02 | 2.32E-02 | 2.57E-03 | 1 | IGHG1 |
| NM_016145 | 3.95E-02 | 4.91E-02 | 1.95E-02 | 1 | PTD008 |
| BE891920 | 3.95E-02 | 2.49E-02 | 4.78E-02 | 1 | ARPC4 |
| NM_024293 | 3.95E-02 | 9.26E-03 | 3.73E-03 | 1 | C2orf17 |
| NM_014164 | 3.95E-02 | 3.54E-02 | 1.73E-03 | 1 | FXYD5 |
| AL561281 | 3.95E-02 | 3.54E-02 | 4.57E-02 | 1 | MAP4K4 |
| NM_006342 | 3.95E-02 | 3.54E-02 | 3.08E-02 | 1 | TACC3 |
| NM_017684 | 3.95E-02 | 3.54E-02 | 1.76E-02 | 1 | VPS13C |
| NM_022089 | 3.95E-02 | 2.32E-02 | 4.84E-03 | 1 | HSA9947 |
| NM_018053 | 3.95E-02 | 2.49E-02 | 2.04E-02 | 1 | FLJ10307 |
| NM_024604 | 3.95E-02 | 4.91E-02 | 3.07E-02 | 1 | FLJ21908 |
| NM_018694 | 3.95E-02 | 3.54E-02 | 4.96E-02 | 1 | ARL6IP4 |
| NM_019096 | 3.95E-02 | 4.58E-02 | 6.10E-03 | 1 | GTPBP2 |
| AB016531 | 3.95E-02 | 2.52E-03 | 1.67E-02 | 1 | PEX16 |
| AI660075 | 3.95E-02 | 3.54E-02 | 3.63E-02 | 1 | LRCH4 |
| AF052151 | 3.95E-02 | 4.88E-03 | 7.99E-03 | 1 | MTVR1 |
| AI744083 | 3.95E-02 | 3.54E-02 | 6.98E-03 | 1 | MOSPD2 |

| | | | | | |
|---|---|---|---|---|---|
| Table 4: Provided are genetic markers which are differentially expressed between subjects diagnosed with definite multiple sclerosis and healthy controls (the Probeset ID of the Affymetrix Gene Chip), along with the corresponding GenBank accession number (GenBank Acc. No.), the gene symbol and the direction (Dir) of change in gene expression ("1"- upregulation; "-1" - downregulation). Note that the p values of the TNOM, Info and t-Test statistical tests all passed the 95 % confidence level. | | | | | |

### EXAMPLE 3

### IDENTIFICATION OF GENES WHICH EXPRESSION PATTERN IS CHARACTERISTICS TO PROBABLE MULTIPLE SCLEROSIS SUBJECTS WHICH FURTHER CONCERT TO DEFINITE MULTIPLE SCLEROSIS

***Probable vs. definite gene expression patterns** -* To identify genes which expression pattern, i.e., upregulation or downregulation is characteristics to probable multiple sclerosis subjects who further convert to definite multiple sclerosis (within a 2 years period), the PBMC expression pattern of genes differentially expressed between definite RRMS/ healthy controls (722 genetic markers shown in Table 4, hereinabove) was compared to the expression pattern of probable MS who converted to definite MS (12 patients, converted within 2-years)/ healthy controls (1517 genetic markers shown in Table 2, hereinabove).

This intersection disclosed 58 universal genes that characterize probable (who are predisposed to develop definite MS) and definite MS disease (Figure 3 and Table 5, hereinbelow). This signature included MMP genes: MMP9, MMP14; antigen presenting genes like: B7-1 (CD80, CD28); T-cells receptor genes and neuron survival genes: SIP1, TCRγV.

**Table 5**

| ***Universal expression pattern of markers common to subjects with probable MS diagnosis which later converted to definite diagnosis of MS and subjects with definite diagnosis of MS*** | | | | | |
|---|---|---|---|---|---|
| ***Probeset*** | ***SEQ ID NO:*** | ***GenBank Accession No.*** | ***Dir*** | ***Fold Change*** | ***Gene Symbol*** |
| 203335_at | 1 | NM_006214 | -1 | 2.0 | PHYH |
| 216746_at | 2 | AK024606 | -1 | 2.1 | AK024606 |
| 217377_x_at | 3 | AF041811 | -1 | 2.3 | NTRK3 |
| 214274_s_at | 4 | AI860341 | -1 | 2.4 | ACAA1 |
| 204151_x_at | 5 | NM_001353 | -1 | 2.6 | AKR1C1 |
| 203468_at | 6 | NM_003674 | -1 | 2.9 | CDK10 |
| 218303_x_at | 7 | NM_016618 | -1 | 2.9 | LOC51315 |
| 204221_x_at | 8 | U16307 | -1 | 2.9 | HRB2 |
| 218129_s_at | 9 | BC005316 | -1 | 3.1 | NFYB |
| 212073_at | 10 | AI631874 | -1 | 3.2 | CSNK2A1 |
| 218607_s_at | 11 | NM_018115 | -1 | 3.2 | SDAD1 |
| 206271_at | 12 | NM_003265 | -1 | 3.4 | TLR3 |
| 217908_s_at | 13 | NM_018442 | -1 | 3.5 | IQWD1 |
| 160020_at | 14 | Z48481 | -1 | 3.7 | MMP14 |
| 217381_s_at | 15 | X69383 | -1 | 4.1 | TCR |
| 218349_s_at | 16 | AA824298 | -1 | 4.8 | FLJ10036 |
| 221007_s_at | 17 | NM_030917 | -1 | 5.0 | FIP1L1 |
| 218728_s_at | 18 | AK024569 | -1 | 5.7 | HSPC163 |
| 218096_at | 19 | NM_018361 | -1 | 6.2 | LPAAT-e |
| 213980_s_at | 20 | AA053830 | -1 | 6.4 | CTBP1 |
| 222149_x_at | 21 | AL137398 | -1 | 6.6 | DKFZp434P162 |
| 221222_s_at | 22 | NM_017860 | -1 | 6.8 | FLJ20519 |
| 202660_at | 23 | AA834576 | -1 | 8.1 | ITPR2 |
| 210235_s_at | 24 | U22815 | -1 | 8.3 | PPFIA1 |
| 205540_s_at | 25 | NM_016656 | -1 | 8.8 | RRAGB |
| 204682_at | 26 | NM_000428 | -1 | 9.0 | LTBP2 |
| 218859_s_at | 27 | NM_016649 | -1 | 12.2 | C20orf6 |
| 218699_at | 28 | NM_003929 | -1 | 26.5 | RAB7L1 |
| 205063_at | 29 | NM_003616 | -1 | 31.3 | SIP1 |
| 214085_x_at | 30 | AI912583 | -1 | 66.8 | HRB2 |
| 216399_s_at | 31 | AK025663 | -1 | 82.9 | ZNF291 |
| 219575_s_at | 32 | NM_022341 | 1 | 2.0 | PDF, COG8 |
| 216330_s_at | 33 | L14482 | 1 | 2.1 | POU6F1 |
| 203222_s_at | 34 | NM_005077 | 1 | 2.1 | TLE1 |
| 203064_s_at | 35 | NM_004514 | 1 | 2.2 | FOXK2 |
| 207176_s_at | 36 | NM_005191 | 1 | 2.2 | CD80 |
| 213360_s_at | 37 | AA514622 | 1 | 2.2 | POM121 |
| 205920_at | 38 | NM_003043 | 1 | 2.4 | SLC6A6 |
| 206544_x_at | 39 | NM_003070 | 1 | 2.4 | SMARCA2 |
| 218961_s_at | 40 | NM_007254 | 1 | 2.7 | PNKP |
| 201526_at | 41 | NM_001662 | 1 | 2.9 | ARF5 |
| 203936_s_at | 42 | NM_004994 | 1 | 3.0 | MMP9 |
| 207908_at | 43 | NM_000423 | 1 | 3.1 | KRT2A |
| 202942_at | 44 | NM_001985 | 1 | 3.3 | ETFB |
| 211696_x_at | 45 | AF349114 | 1 | 4.3 | HBB |
| 218037_at | 46 | NM_024293 | 1 | 4.9 | C2orf17 |
| 209116_x_at | 47 | M25079 | 1 | 5.3 | EBB |
| 204018_x_at | 48 | NM_000558 | 1 | 6.4 | HBA1, HBA2 |
| 200055_at | 49 | NM_006284 | 1 | 6.5 | TAF10 |
| 202596_at | 50 | BC000436 | 1 | 6.6 | ENSA |
| 201827_at | 51 | AF113019 | 1 | 7.2 | SMARCD2 |
| 214414_x_at | 52 | T50399 | 1 | 7.6 | HBA2 |
| 202768_at | 53 | NM_006732 | 1 | 14.0 | FOSB |
| 215684_s_at | 54 | AL096741 | 1 | 14.9 | ASCC2 |
| 212071_s_at | 55 | BE968833 | 1 | 21.8 | SPTBN1 |
| 202111_at | 56 | NM_003040 | 1 | 28.8 | SLC4A2 |
| 212413_at | 57 | D50918 | 1 | 105.1 | D50918 |
| 209740_s_at | 58 | U03886 | 1 | 106.7 | PNPLA4 |

| | | | | | |
|---|---|---|---|---|---|
| Table 5: Genetic markers which are differentially expressed between probable multiple sclerosis which further developed definite diagnosis of MS and healthy controls are provided (the Probeset ID of the Affymetrix Gene Chip), along with the corresponding GenBank accession number, the gene symbol, the SEQ ID NO., the direction of change in gene expression ("1"- upregulation; "-1" - downregulation) and the fold change in subjects diagnosed with probable MS (who are predisposed to develop definite MS) as compared to healthy control (probable MS/healthy control). Note that the p values of the TNOM, Info and t-Test statistical tests all passed the 95 % confidence level. | | | | | |

***Determination of the prediction power of selected genes which differentiate between probable MS subjects who are predisposed to develop definite MS and healthy controls** -* To evaluate the power of each of the 58 differentiating genes (SEQ ID NOs:1-58) identified in this study to predict the predisposition of a probable MS subject to develop a definite MS diagnosis, the study sample of 40 probable patients was randomly divided into 80 % of the subjects as a "training set" and 20 % (to confirm) of the subjects as a "test set" and a model was build using the SVM based on RBF kernel. For each of the differentiating genes the predictability of the training set on the test set was computed and the average error following 25 permutations was calculated. Genes with the lowest average error were selected, then, for each selected gene, the remaining genes were added one after the other, by selecting the next gene such that the average error after 25 repeats of the group of genes including the new gene has the lowest average error as compared to the addition of another gene. This process was repeated 57 times for each additional gene added to the previous group of genes. The resulting average error for each gene combination is depicted in Table 6, hereinbelow, wherein the first gene in row number 1 (SEQ ID NO:4) exhibits the best predictive power (error average of "0.21 ") as a single gene.

**Table 6**

| ***Row Number*** | ***SEQ ID NO:*** | ***Probeset ID*** | ***Gene Bank ID*** | ***Error Average*** | ***Gene Symbol*** |
|---|---|---|---|---|---|
| 1 | 4 | 214274_s_at | AI860341 | 0.216363636 | ACAA1 |
| 2 | 16 | 218349_s_at | NM_017975 | 0.216363636 | FLJ10036 |
| 3 | 5 | 204151_x_at | NM_001353 | 0.247272727 | AKR1C1 |
| 4 | 56 | 202111_at | NM_003040 | 0.230909091 | SLC4A2 |
| 5 | 20 | 213980_s_at | AA053830 | 0.223636364 | CTBP1 |
| 6 | 3 | 217377_x_at | AF041811 | 0.158181818 | NTRK3 |
| 7 | 1 | 203335_at | NM_006214 | 0.169090909 | PHYH |
| 8 | 10 | 212073_at | AI631874 | 0.176363636 | CSNK2A1 |
| 9 | 57 | 212413_at | D50918 | 0.169090909 | 6-Sep |
| 10 | 24 | 210235_s_at | U22815 | 0.194545455 | PPFIA1 |
| 11 | 14 | 160020_at | Z48481 | 0.214545455 | MMP14 |
| 12 | 49 | 200055_art | NM_006284 | 0.201818182 | TAF10 |
| 13 | 13 | 217908_s_at | NM_018442 | 0.181818182 | PC326 |
| 14 | 37 | 213360_s_at | AA514622 | 0.163636364 | na |
| 15 | 6 | 203468_at | NM_003674 | 0.165454545 | CDK10 |
| 16 | 47 | 209116_x_at | M25079 | 0.158181818 | HBB |
| 17 | 50 | 202596_at | BC000436 | 0.163636364 | ENSA |
| 18 | 21 | 222149_x_at | AL137398 | 0.154545455 | --- |
| 19 | 46 | 218037_at | NM_024293 | 0.143636364 | MGC3035 |
| 20 | 8 | 204221_x_at | U16307 | 0.165454545 | GLIPR1 |
| 21 | 26 | 204682_at | NM_000428 | 0.174545455 | LTBP2 |
| 22 | 2 | 216746_at | AK024606 | 0.169090909 | --- |
| 23 | 15 | 217381_s_at | X69383 | 0.172727273 | --- |
| 24 | 51 | 201827_at | AF113019 | 0.167272727 | SMARCD2 |
| 25 | 44 | 202942_at | NM_001985 | 0.170909091 | ETFB |
| 26 | 19 | 218096_at | NM_018361 | 0.167272727 | LPAAT-e |
| 27 | 17 | 221007_s_at | NM_030917 | 0.176363636 | FIP1L1 |
| 28 | 25 | 205540_s_at | NM_016656 | 0.176363636 | RRAGB |
| 29 | 33 | 216330_s_at | L14482 | 0.161818182 | POU6F1 |
| 30 | 48 | 204018_x_at | NM_000558 | 0.174545455 | HBA1 |
| 31 | 36 | 207176_s_at | NM_005191 | 0.161818182 | CD80 |
| 32 | 34 | 203222_s_at | NM_005077 | 0.158181818 | TLE1 |
| 33 | 12 | 206271 at | NM_003265 | 0.132727273 | TLR3 |
| 34 | 29 | 205063_at | NM_003616 | 0.136363636 | SIP1 |
| 35 | 23 | 202660 at | AA834576 | 0.132727273 | --- |
| 36 | 11 | 218607_s_at | NM_018115 | 0.150909091 | FLJ10498 |
| 37 | 45 | 211696_x_at | AF349114 | 0.149090909 | HBB |
| 38 | 53 | 202768_at | NM_006732 | 0.16 | FOSB |
| 39 | 41 | 201526_at | NM_001662 | 0.156363636 | ARF5 |
| 40 | 40 | 218961_s_at | NM_007254 | 0.130909091 | PNKP |
| 41 | 31 | 216399_s_at | AK025663 | 0.154545455 | ZNF291 |
| 42 | 58 | 209740_s_at | U03886 | 0.143636364 | DXS1283E |
| 43 | 27 | 218859_s_at | NM_016649 | 0.147272727 | C20orf6 |
| 44 | 43 | 207908_at | NM_000423 | 0.132727273 | KRT2A |
| 45 | 35 | 203064_s_at | NM 004514 | 0.132727273 | ILF1 |
| 46 | 30 | 214085_x_at | AI912583 | 0.16 | HRB2 |
| 47 | 52 | 214414_x_at | T50399 | 0.156363636 | HBA1 |
| 48 | 55 | 212071_s_at | BE968833 | 0.147272727 | SPTBN1 |
| 49 | 7 | 218303_x_at | NM 016618 | 0.16 | LOC51315 |
| 50 | 9 | 218129_s_at | NM_006166 | 0.161818182 | NFYB |
| 51 | 42 | 203936_s_at | NM_004994 | 0.167272727 | MMP9 |
| 52 | 28 | 218699_at | BG338251 | 0.145454545 | RAB7L1 |
| 53 | 54 | 215684_s_at | AL096741 | 0.170909091 | FLJ21588 |
| 54 | 32 | 219575_s_at | NM_022341 | 0.167272727 | COG8 |
| 55 | 22 | 221222_s_at | NM_017860 | 0.178181818 | Fly20519 |
| 56 | 18 | 218728_s_at | NM_014184 | 0.181818182 | HSPC163 |
| 57 | 38 | 205920_at_ | NM_003043 | 0.190909091 | SLC6A6 |
| 58 | 39 | 206544_x_at | NM_003070 | 0.194545455 | SMARCA2 |

| | | | | | |
|---|---|---|---|---|---|
| Table 6: Shown are the average errors of predicting a probability of a probable MS subject to develop the diagnosis of definite MS within a 2-years period based on a model computed for each gene or a group of genes in the MS training set group. The ascending order of genes reflects combinations of genes, where each row includes the gene specified in that row and in all preceding rows. For example, the average error presented in row number 4 reflects the average error in predicting a probability of a probable MS subject to develop the diagnosis of definite MS within a 2-years period using the group of genes described in rows 1, 2, 3 and 4 (i.e., SEQ ID NOs:4, 16. 5 and 56). Probe set ID = Affymetrix ID. | | | | | |

As shown in Table 6 hereinabove, the predictive power of each set of genes was evaluated using the MS training and test sets of samples. The polynucleotide exhibiting the best predictive power in determining the probability of a probable MS subject to convert to the diagnosis of definite MS was the polynucleotide set forth by SEQ ID NO:4 (GenBank Accession No. AI860341; row No. 1 in Table 6), in which the average error between the test and training groups was 0.216. Similarly, the combination genes set forth by SEQ ID NOs:4 and 16 (GenBank Accession No. NM_017975; row No. 2 in Table 6) displayed a predictive power with 0.216 average error. Another exemplary combination, which provides an even higher prediction power (with a smaller average error) is shown in row number 6 in Table 6, in which the combination of the polynucleotide sequences set forth in SEQ ID NOs:4, 16, 5, 56, 20 and 3 displayed a high predictive power with 0.158 average error. Yet another exemplary combination, which provides an even higher prediction power (with a smaller average error) is shown in row number 35 in Table 6, in which the combination of the polynucleotide sequences set forth in SEQ ID NOs:4, 16, 5, 56, 20, 3, 1, 10, 57, 24, 14, 49, 13, 37, 6, 47, 50, 21, 46, 8, 26, 2, 15, 51, 44, 19, 17, 25, 33, 48, 36, 34, 12, 29 and 23 displayed a high predictive power with 0.132 average error. Thus, this analysis enables one skilled in the art to select a group of polynucleotides which can give the best predictive power for prediction of the probability of a subject diagnosed with probable MS (after the first neurological attack) to develop the diagnosis of definite MS within 2 years.

### Conclusions

1. PBMC gene expression signature distinguished probable MS patients from healthy subjects.
2. Patients that experience a second relapse and converted to definite MS during 2 years of follow-up period have a specific gene expression signature.
3. Patients with probable and definite MS demonstrate a universal gene expression signature.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination.

### REFERENCES

### (Additional references are cited in text)

1. Anderson DW, Ellenberg JH, Leventhal CM, et al. Revised estimate of the prevalence of multiple sclerosis in the United States. Ann Neurol. 1992 Mar; 31(3):333-6.
2. Weinshenker BC. Natural history of multiple sclerosis. Ann Neurol. 1994;36 Suppl:S6-11.
3. Confavreux C, Vukusic S, Moreau T, et al. Relapses and progression of disability in multiple sclerosis. N Engl J Med. 2000 Nov 16;343(20):1430-8.
4. Poser CM, Paty DW, Scheinberg L, et al. New diagnostic criteria for multiple sclerosis: guidelines for research protocols. Ann Neurol. 1983 Mar;13 (3):227-31.
5. Oksenberg JR, Baranzini SE, Barcellos LF, et al. Multiple sclerosis: genomic rewards. J Neuroimmunol. 2041 Feb 15;113(2):171-84.
6. Compston A, Coles A. Genetic analysis of multiple sclerosis. Curr Neurol Neurosci Rep. 2002 May;2(3):259-66.
7. Achiron A, Barak Y. Multiple sclerosis-from probable to definite diagnosis: a 7-year prospective study. Arch Neurol. 2000 Jul;57(7):974-9.
8. O'Riordan JI, Tompson AJ, Kingsley DP, et al. The prognostic value of brain MRI in clinically isolated syndromes of the CNS. A 10-year follow-up. Brain. 1998 Mar;121 (Pt 3):495-503.
9. Morrissey SP, Miller DH, Kendall BE, et al. The significance of brain magnetic resonance imaging abnormalities at presentation with clinically isolated syndromes suggestive of multiple sclerosis: a 5-year follow-up study. Brain. 1993;116:135-146.
10. Graves DJ. Powerful tools for genetic analysis come of age. Trends Biotechnol. 1999 ; 17 : :127-134.
11. Duggan DJ, Bittner M, Chen Y, et al. Expression profiling using cDNA microarrays. Nat Genet 1999;21 (1 Suppl):10-14.
12. Greenberg SA. DNA microarray gene expression analysis technology and its application to neurological disorders. Neurology 2001;57:755-61.
13. Whitney LW, Becker KG, Tresser NJ, Caballero-Ramos CI, Munson PJ, Prabhu VV, Trent JM, McFarland HF, Biddison WE. Analysis of gene expression in multiple sclerosis lesions using cDNA microarrays. Ann Neurol. 1999; 46(3): 425-8.
14. Lock CB, Heller RA. Gene microarray analysis of multiple sclerosis lesions. Trends Mol Med 2003; 9: 535-41.
15. Ramanathan M, Weinstock G, Nguyen LT, et a1. In vivo gene expression revealed by cDNA arrays: the pattern in relapsing remitting multiple sclerosis patients compared with normal subjects. J Neuroimmunol 2001;116:213 219.
16. Achiron, A., Gurevich, M., Friedman, N., Kaminski, N. and Mandel, M. Blood transcriptional signatures of multiple sclerosis: unique gene expression of disease activity. Ann Neurol 2004; 55: 410-7.
17. Kurtzke JF. Rating neurologic impairment in multiple sclerosis an expanded disability status scale (EDSS). Neurology 1983; 33:1444-1452.
18. Achiron A, Gicquel S, Miron S, Faibel M. Brain MRI lesion load quantification in multiple sclerosis: a comparison between automated multispectral and semi-automated thresholding computer-assisted techniques. Magn Reson Imaging 2002;20:713-20.
19. Furey TS, Cristianini N, Duffy N, Bednarski DW, Schummer M, Haussler D. Support vector machine classification and validation of cancer tissue samples using microarray expression data. Bioinformatics 2000; 16:906-14.
20. Statnikov A, Aliferis CF, Tsamardinos I, Hardin D, Levy S. A comprehensive evaluation of multicategory classification methods for microarray gene expression cancer diagnosis. Bioinformatics 2005;21:631-43.
21. Ben-Dor A, Bruhn L, Friedman N, Nachman I, Schummer M, Yakhini Z. Tissue classification with gene expression profiles. J Comput Biol 2000; 7: 559-83.

## Claims

1. A method of determining a probability of a subject diagnosed with probable multiple sclerosis by the experience of one neurological attack affecting the central nervous system (CNS) and accompanied by demyelinating lesions on brain magnetic resonance imaging (MRI) to develop definite multiple sclerosis by the experience of at least a second neurological attack affecting the CNS and accompanied by demyelinating lesions on brain MRI, the method comprising determining in a cell of the subject a level of expression of a polynucleotide set forth by SEQ ID NO:4, wherein an alteration above a predetermined threshold in said level of expression of said polynucleotide sequence in said cell of the subject relative to a level of expression of said polynucleotide sequence in a reference cell is indicative of the probability of the subject diagnosed with probable multiple sclerosis to develop definite multiple sclerosis.

2. The method of claim 1, wherein said reference cell is of an unaffected subject.

3. The method of claim 2, wherein said alteration is downregulation.

4. The method of claim 3, wherein said probability of the subject diagnosed with probable multiple sclerosis to develop definite multiple sclerosis is higher than about 75 %.

5. The method of claim 1, wherein said detecting said level of expression is effected using an RNA detection method.

6. The method of claim 1, wherein said cell of the subject is a blood cell.

7. The method of claim 1, wherein said detecting said level of expression is effected at the protein level.

## Patentansprüche

1. Verfahren zum Bestimmen einer Wahrscheinlichkeit, dass ein Proband, dem eine wahrscheinliche multiple Sklerose durch das Erleiden eines neurologischen Anfalls diagnostiziert wird, die das zentrale Nervensystem (CNS) angreift und bei der Magnetresonanztomografie (MRI) des Gehirns von Demyelinationsläsionen begleitet ist, durch das Erleiden mindestens eines zweiten neurologischen Anfalls eine definitive multiple Sklerose entwickelt, die das CNS angreift und bei der Gehirn-MRI von Demyelinationsläsionen begleitet ist, wobei das Verfahren ein Bestimmen in einer Zelle des Probanden eines Expressionsspiegels eines Polynucleotids umfasst, das durch SEQ ID NO:4 dargelegt ist, wobei eine Änderung des Expressionsspiegels der Polynualeotidsequenz in der Zelle des Probanden über eine vorgegebene Schwelle in Bezug auf einen Expressionsspiegel der Polynucleotidsequenz in einer Vergleichszelle die Wahrscheinlichkeit anzeigt, dass der Proband, dem eine wahrscheinliche multiple Sklerose diagnostiziert wird, eine definitive multiple Sklerose entwickelt.

2. Verfahren nach Anspruch 1, wobei die Vergleichszelle von einem nicht angegriffenen Probanden ist.

3. Verwendung nach Anspruch 2, wobei es sich bei der Änderung um Herunterregulation handelt.

4. Verfahren nach Anspruch 3, wobei die Wahrscheinlichkeit, dass der Proband, dem eine wahrscheinliche multiple Sklerose diagnostiziert wird, eine definitive Sklerose entwickelt, über etwa 75 % liegt.

5. Verfahren nach Anspruch 1, wobei das Nachweisen des Expressionsspiegels unter Verwendung eines RNA-Nachweisverfahrens erfolgt.

6. Verfahren nach Anspruch 1, wobei es sich bei der Zelle des Probanden um eine Blutzelle handelt.

7. Verfahren nach Anspruch 1, wobei das Nachweisen des Expressionsspiegels am Proteinspiegel erfolgt.

## Revendications

1. Procédé pour déterminer la probabilité, chez un sujet ayant reçu un diagnostic de sclérose en plaques probable suite à une atteinte neurologique affectant le système nerveux central (SNC), et s'accompagnant de lésions démyélinisantes identifiées par une imagerie par résonance magnétique (IRM) du cerveau, de développer une sclérose en plaques définitive suite à au moins une deuxième atteinte neurologique affectant le SNC, et s'accompagnant de lésions démyélinisantes identifiées par une IRM du cerveau ; le procédé consistant à déterminer dans une cellule du sujet un taux d'expression d'un polynucléotide décrit par SEQ ID NO: 4, dans lequel une altération excédant un seuil prédéterminé dudit taux d'expression de ladite séquence polynucléotidique dans ladite cellule du sujet, par rapport à un taux d'expression de ladite séquence polynucléotidique dans une cellule de référence, indique la probabilité du sujet ayant reçu un diagnostic de sclérose en plaques probable de développer une sclérose en plaques définitive.

2. Procédé selon la revendication 1, dans lequel ladite cellule de référence provient d'un sujet non affecté.

3. Procédé selon la revendication 2, dans lequel ladite altération est une régulation négative.

4. Procédé selon la revendication 3, dans lequel ladite probabilité du sujet ayant reçu un diagnostic de sclérose en plaques probable de développer une sclérose en plaques définitive est supérieure à environ 75 %.

5. Procédé selon la revendication 1, dans lequel ladite détection dudit taux d'expression est réalisée en utilisant un procédé de détection d'ARN.

6. Procédé selon la revendication 1, dans lequel ladite cellule du sujet est une cellule sanguine.

7. Procédé selon la revendication 1, dans lequel ladite détection dudit taux d'expression est réalisée au niveau protéique.
